(19) Europäisches Patentamt / European Patent Office / Office européen des brevets

(11) EP 4 036 087 A1

(12) EUROPEAN PATENT APPLICATION
published in accordance with Art. 153(4) EPC

(43) Date of publication:
**03.08.2022 Bulletin 2022/31**

(21) Application number: **20870084.9**

(22) Date of filing: **24.09.2020**

(51) International Patent Classification (IPC):
***C07D 401/14*** (2006.01) ***C07D 401/12*** (2006.01)
***C07D 401/10*** (2006.01) ***A61K 31/501*** (2006.01)
***A61K 31/495*** (2006.01) ***A61K 31/395*** (2006.01)
***A61P 7/02*** (2006.01) ***A61P 9/10*** (2006.01)

(52) Cooperative Patent Classification (CPC):
**A61K 31/395; A61K 31/495; A61K 31/501; A61P 7/02; A61P 9/10; C07D 401/10; C07D 401/12; C07D 401/14**

(86) International application number:
**PCT/CN2020/117257**

(87) International publication number:
**WO 2021/057818 (01.04.2021 Gazette 2021/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.09.2019 CN 201910923960**
**19.12.2019 CN 201911318870**
**01.09.2020 CN 202010902000**

(71) Applicant: **Shenzhen Salubris Pharmaceuticals Co. Ltd**
**Shenzhen, Guangdong 518040 (CN)**

(72) Inventors:
- **WU, Junjun**
  **Shenzhen, Guangdong 518040 (CN)**
- **LU, Yinsuo**
  **Shenzhen, Guangdong 518040 (CN)**
- **XIAO, Ying**
  **Shenzhen, Guangdong 518040 (CN)**
- **HONG, Zexin**
  **Shenzhen, Guangdong 518040 (CN)**
- **WU, Jianli**
  **Shenzhen, Guangdong 518040 (CN)**
- **XING, Wei**
  **Shenzhen, Guangdong 518040 (CN)**

(74) Representative: **Altmann Stößel Dick Patentanwälte PartG mbB**
**Theodor-Heuss-Anlage 2**
**68165 Mannheim (DE)**

# (54) FXIA INHIBITORS AND PREPARATION METHOD THEREFOR AND PHARMACEUTICAL USE THEREOF

(57) Provided in the present invention is a series of selective Factor XIa (FXIa) inhibitors, relating to the technical field of chemical drugs. The present invention also relates to pharmaceutical compositions containing said compounds and a use of said compounds in drugs for the treatment of diseases such as thromboembolism.

EP 4 036 087 A1

## Description

## FIELD OF THE INVENTION

The invention belongs to the technical field of chemical drugs, and provides a series of inhibitors of selective Factor XIa (FXIa for short). The present invention also relates to pharmaceutical compositions containing these compounds and their use in medicines for treating diseases such as thromboembolism.

## BACKGROUND OF THE INVENTION

Cardiovascular and cerebrovascular diseases such as cerebrovascular disease, cerebral infarction, myocardial infarction, coronary heart disease and arteriosclerosis kill nearly 12 million people in the world every year, which is close to 1/4 of the total number of deaths in the world, and become number one enemy for human health. More than 2.6 million people die of cardiovascular disease in China every year, and 75% of the surviving patients are disabled, of which more than 40% are severely disabled. The thrombosis caused by cardiovascular and cerebrovascular diseases, diabetes and their complications has become an urgent problem to be solved today.

The human blood coagulation process is composed of intrinsic pathway, extrinsic pathway and common pathway (Annu.Rev.Med.2011.62:41-57), which is a chain reaction in which the process is continuously strengthened and amplified through the sequential activation of various zymogens. The coagulation cascade is initiated by the endogenous pathway (also known as the contact activation pathway) and the exogenous pathway (also known as the tissue factor pathway) to generate FXa, and then generates thrombin (FIIa) through the common pathway, and finally forms fibrin.

The intrinsic pathway refers to the process in which factor XII is activated to form XIa-VIIIa-$Ca^{2+}$-PL complex and activate factor X, while the extrinsic coagulation pathway refers to the process in which tissue factor (TF) is released, TF-VIIa-$Ca^{2+}$ complex forms and then activates factor X. The common pathway refers to the process of combining the two pathways into one after the formation of factor Xa, activating prothrombin and finally generating fibrin, in which FXI is necessary to maintain the endogenous pathway, and it plays a key role in the amplification of the coagulation cascade. In the coagulation cascade reaction, thrombin can activate FXI feedback, and the activated FXI (FXIa) in turn promotes the production of thrombin, thereby amplifying the coagulation cascade reaction. Therefore, antagonists of FXI have been widely developed for the treatment of various thrombi.

Traditional anticoagulant drugs, such as warfarin, heparin, low molecular weight heparin (LMWH), and new drugs launched in recent years, such as FXa inhibitors (Rivaroxaban, Apixaban, etc.) and thrombin inhibitors (Dabigatran etexilate, Hirudin, etc.), all have good effects on reducing thrombosis, and occupy the vast cardiovascular and cerebrovascular market with their significant effectiveness. However, their side effects are becoming more and more significant. Among them, the "bleeding risk" is one of the most serious problems (N Engl J Med 1991; 325: 153-8, Blood. 2003; 101: 4783-4788).

Studies have found that in the thrombosis model, inhibition of FXIa factor can effectively inhibit the formation of thrombus, but in more severe thrombosis, the effect of FXIa is minimal (Blood. 2010; 116(19): 3981-3989). Clinical statistics show that increasing the amount of FXIa increases the prevalence of VTE (Blood 2009;114:2878-2883), while those with severe FXIa deficiency have a reduced risk of DVT (Thromb Haemost 2011;105:269-273).

FXIa is currently an emerging target for inhibiting thrombosis, and patent for compounds with FXIa inhibitory activity are disclosed as WO9630396, WO9941276, WO2013093484, WO2004002405, WO2013056060, WO2017005725, WO2017/023992, WO2018041122, etc. Among them, only Bayer's antisense oligonucleotide BAY-2306001 has entered the Phase II clinical study.

The compounds of the present application have higher activity. In particular, the compound of the present application exhibits excellent anticoagulant effect on human blood, has good pharmacokinetic activity, and can be used for effective treatment and/or prevention of cardiovascular and cerebrovascular diseases and thrombosis symptoms.

## SUMMARY OF THE INVENTION

The present application provides a series of oxopyridazinamide derivatives, preparation methods therefor and pharmaceutical use thereof.

In particular, the present application provides a compound of formula (I), or a stereoisomer, a tautomer, a pharmaceutically acceptable salt thereof, wherein all variables are as defined herein.

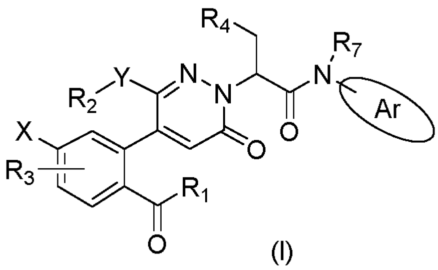

(I)

These compounds are selective factor XIa (FXIa) inhibitors. The present invention also relates to pharmaceutical compositions containing these compounds and use of these compounds in medicines for treating diseases such as thromboembolism.

Specifically, the present invention provides following technical solutions:
A compound of formula (I), or a stereoisomer, a tautomer, a pharmaceutically acceptable salt thereof,

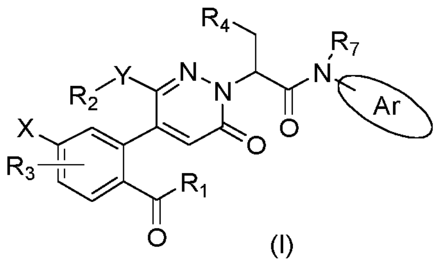

(I)

wherein:

- $R_1$ is selected from the group consisting of alkyl, haloalkyl, alkoxy, alkoxyalkyl, hydroxyalkyl;
- X is selected from the group consisting of halogen, alkoxy, and haloalkyl;
- $R_3$ is hydrogen or halogen;
- Y is selected from the group consisting of oxygen, nitrogen, and a bond;
- $R_2$ is selected from the group consisting of hydrogen, benzene ring, alkyl, alkoxy, alkoxyalkyl, hydroxyalkyl, haloalkyl, heterocycloalkyl, and cycloalkylmethylene;
- $R_4$ is selected from the group consisting of alkyl, benzyl, and aryl or heteroaryl substituted by one $R_6$, wherein $R_6$ is selected from the group consisting of alkyl, halogen, cyano, substituted or unsubstituted amido, substituted or unsubstituted oxopiperazinyl, and substituted or unsubstituted 2-piperidinonyl, wherein substituted amido, substituted oxopiperazinyl, and substituted 2-piperidinonyl is substituted by a substituent selected from the group consisting of alkyl, cycloalkyl, and alkoxy alkyl;
- Ar is selected from the group consisting of benzene ring and indole substituted with one or two $R_5$, indazole, quinoxaline, benzimidazole, indolin-2-one, isoquinolin-1(2*H*)-one, and 3,4-dihydroquinolin-2(1H)-one, wherein $R_5$ is selected from the group consisting of hydrogen, halogen, alkoxy, hydroxyl, carboxyl, sulfonic acid group, sulfonamido, and amide group; and
- $R_7$ is hydrogen or alkyl.

As a preferred embodiment of the present invention, the alkyl is $C_{1-4}$ alkyl, wherein the $C_{1-4}$ alkyl is selected from the group consisting of methyl, ethyl, propyl, isopropyl, n-butyl, isopropyl butyl, sec-butyl, and tert-butyl.

As a preferred embodiment of the present application, the alkoxy group is $C_{1-4}$ alkoxy, wherein the $C_{1-4}$ alkoxy is selected from the group consisting of methoxy, ethoxy, propoxy, isopropoxy , n-butoxy, isobutoxy, sec-butoxy, and tert-butoxy.

As a preferred embodiment of the present invention, the alkoxyalkyl is $C_{1-4}$ alkoxy $C_{1-4}$ alkyl, wherein the $C_{1-4}$ alkoxy $C_{1-4}$ alkyl is selected from the group consisting of methoxymethyl, methoxyethyl, methoxypropyl, methoxy butyl, ethoxymethyl, ethoxyethyl, ethoxypropyl, ethoxybutyl, propoxymethyl, propoxyethyl, propoxypropyl, propoxybutyl, butoxymethyl, butoxy ethyl, butoxypropyl, and butoxybutyl and the like.

As a preferred embodiment of the present invention, the halogen is selected from the group consisting of fluorine, chlorine, bromine and iodine. The haloalkyl means that one or more hydrogen atoms of the alkyl are substituted by halogen, and the hydroxyalkyl means that one or more hydrogen atoms of the alkyl are substituted by hydroxyl. The heterocycloalkyl means that one or more hydrogen atoms of the alkyl are substituted by heterocyclic ring. The cycloalkylmethylene means that one or more hydrogen atoms of the methyl are substituted by cycloalkyl.

As a preferred embodiment of the present invention, the heterocycloalkyl is 4- to 10-membered heterocycloalkyl, wherein the 4- to 10-membered heterocycloalkyl is selected from the group consisting of

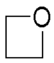, 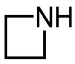, 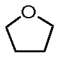, 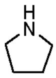, 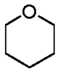, 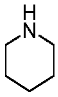, 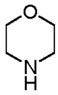, 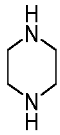, 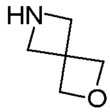,

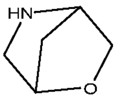

; the aryl is phenyl; the heteroaryl is 5- to 12-membered heteroaryl, wherein the 5- to 12-membered heteroaryl is selected from the group consisting of

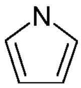, 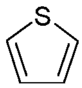, 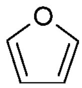,

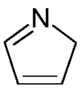, 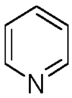, 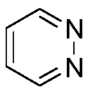, 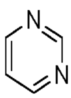, 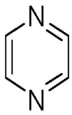, 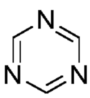, 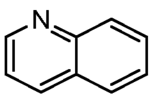, 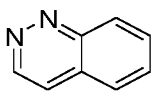, 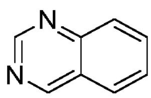,

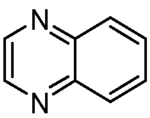, 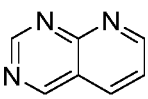, 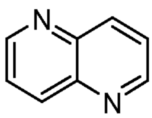 and 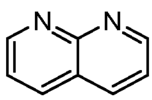.

As a preferred embodiment of the present invention, the cycloalkyl is $C_{3-6}$ cycloalkyl, wherein the $C_{3-6}$ cycloalkyl is selected from the group consisting of cyclopropyl, cyclobutyl, cyclopentyl, and cyclohexyl.

As a preferred version of the present invention, $R_1$ is selected from the group consisting of methyl, ethyl, hydroxymethyl, difluoromethyl, fluoromethyl, and methoxymethyl;
X is selected from the group consisting of chlorine, fluorine, and trifluoromethyl;
$R_3$ is hydrogen;
Y is a bond and $R_2$ is hydrogen or

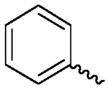,

or Y is oxygen and $R_2$ is selected from the group consisting of hydrogen, methyl, ethyl, phenyl, hydroxyethyl, cyclopropylmethyl, methoxyethyl, isopropyl, difluoromethyl,

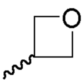

and $CF_3CH_{2-}$;
$R_4$ is selected from the group consisting of phenyl, 4-fluorophenyl, 4-bromophenyl, 3-methylphenyl, 4-methylphenyl,

benzyl, isopropyl,

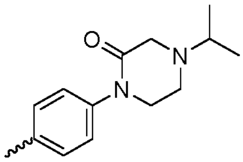

,

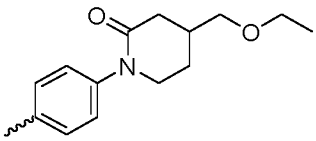

,

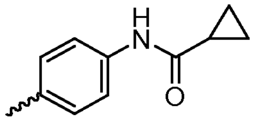

,

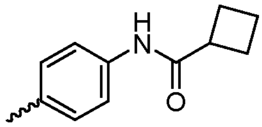

, and

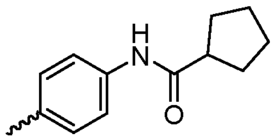

;

Ar is selected from the group consisting of

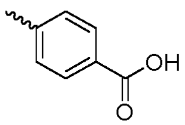

,

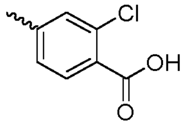

,

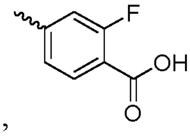

,

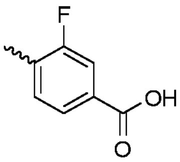

,

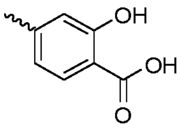

,

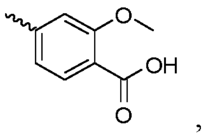

,

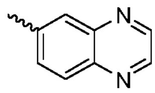

,

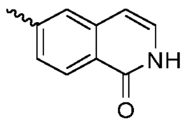

,

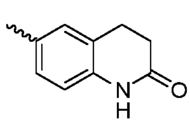

,

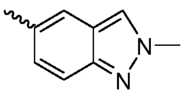

,

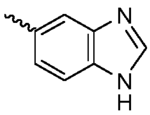

,

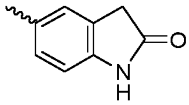

,

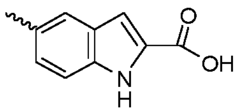

,

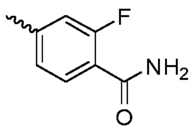

,

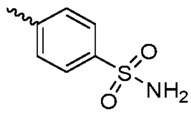

,

and

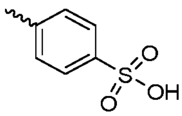

;

$R_7$ is hydrogen or methyl.

As a preferred embodiment of the present invention, the compound or the pharmaceutically acceptable salt thereof is selected from following compounds:

| example | structural formula | example | structural formula |
|---|---|---|---|
| 1 | 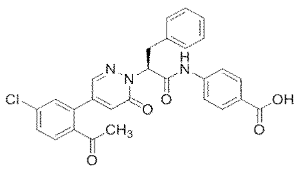 | 24 | 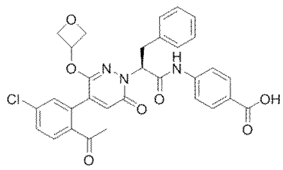 |

(continued)

| example | structural formula | example | structural formula |
|---|---|---|---|
| 2 | 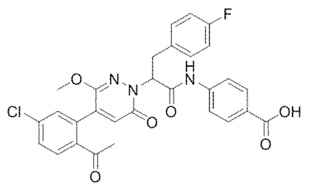 | 25 | 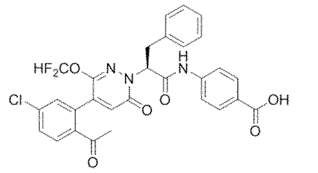 |
| 3 | 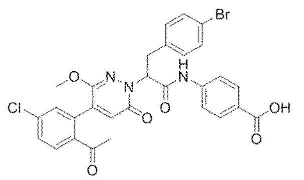 | 26 | 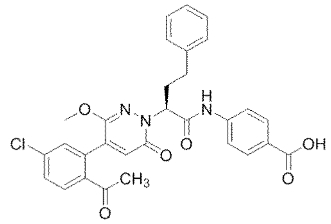 |
| 4 | 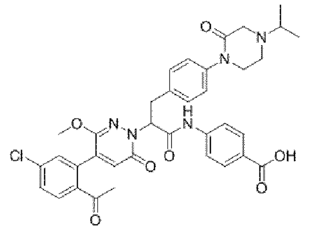 | 27 | 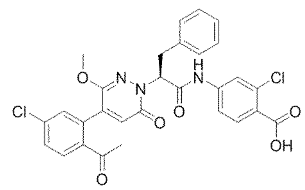 |
| 5 | 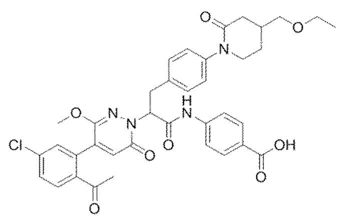 | 28 | 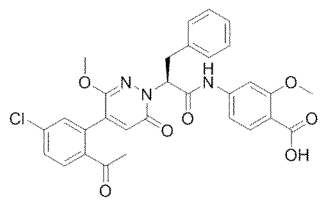 |
| 6 | 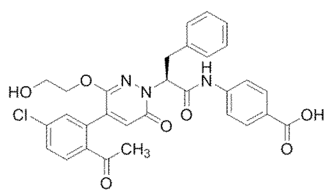 | 29 | 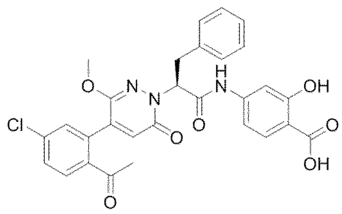 |
| 7 | 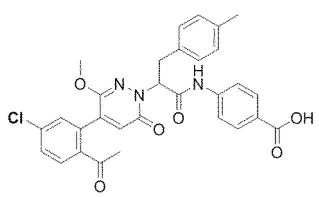 | 30 | 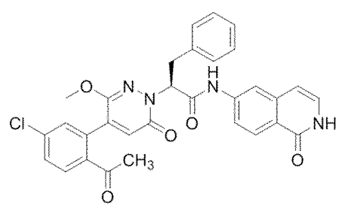 |
| 8 | 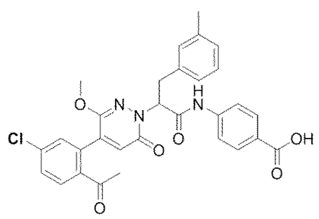 | 31 | 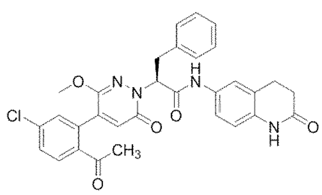 |

(continued)

| example | structural formula | example | structural formula |
|---|---|---|---|
| 9 | 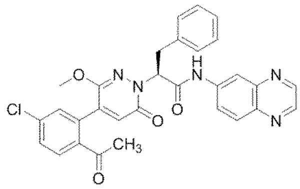 | 32 | 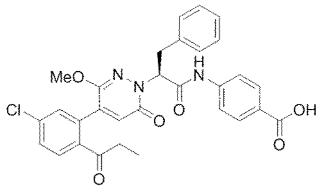 |
| 10 | 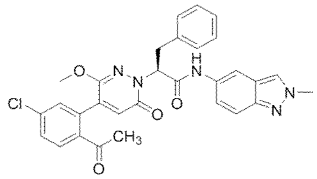 | 33 | 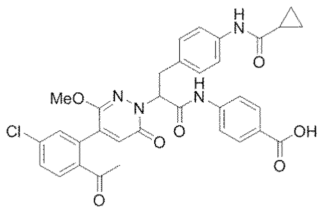 |
| 11 | 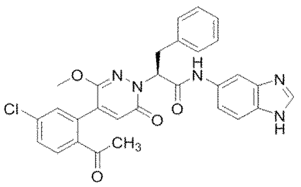 | 34 | 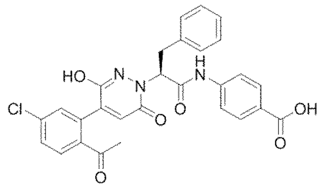 |
| 12 | 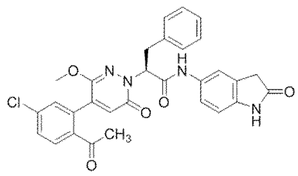 | 35 | 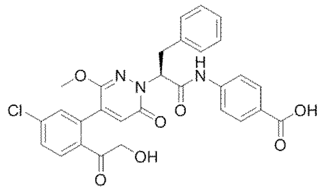 |
| 13 | 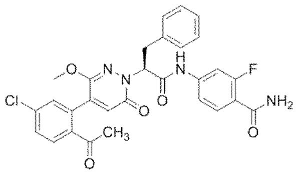 | 36 | 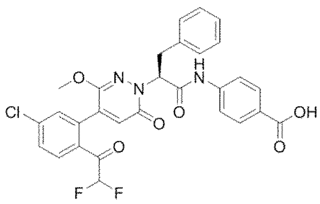 |
| 14 | 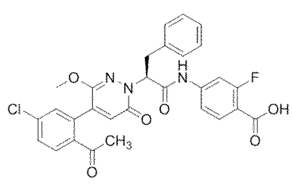 | 37 | 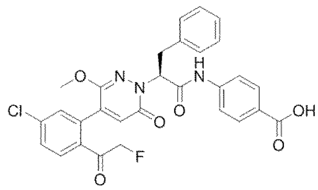 |
| 15 | 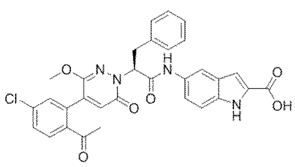 | 38 | 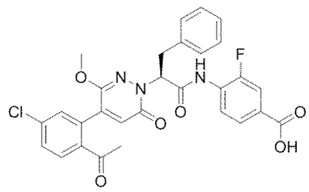 |

(continued)

| example | structural formula | example | structural formula |
|---|---|---|---|
| 16 | 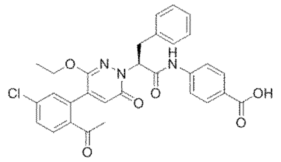 | 39 | 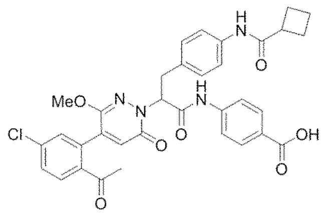 |
| 17 | 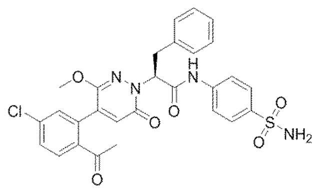 | 40 | 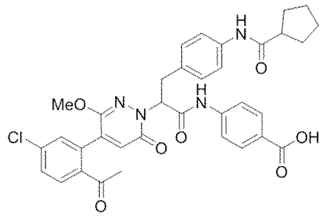 |
| 18 | 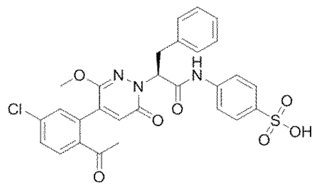 | 41 | 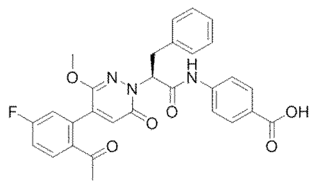 |
| 19 | 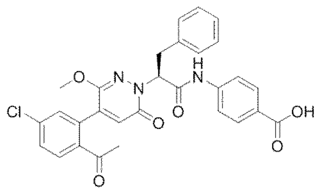 | 43 | 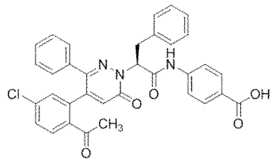 |
| 20 | 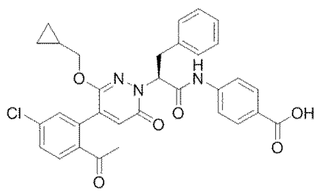 | 44 | 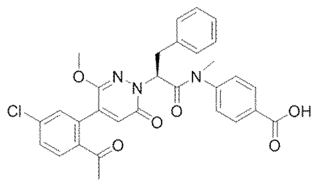 |
| 21 | 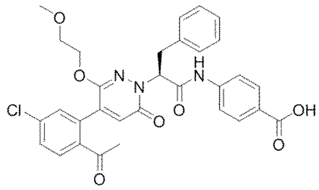 | 45 | 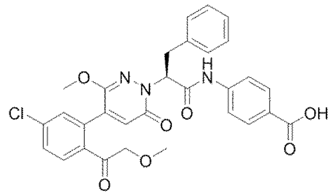 |
| 22 | 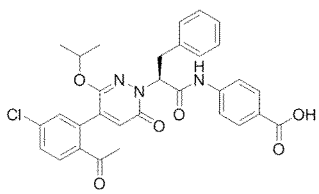 | 46 | 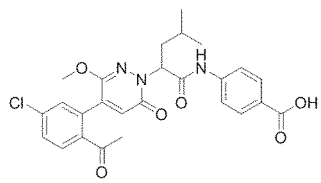 |

(continued)

| example | structural formula | example | structural formula |
|---|---|---|---|
| 23 | 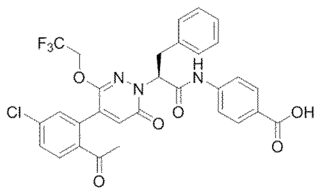 | 47 | 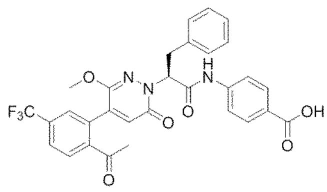 |

As a preferred embodiment of the present invention, the pharmaceutically acceptable salt refers to a salt prepared by the compound and a pharmaceutically acceptable acid or base.

As a preferred embodiment of the present invention, more than one hydrogen atoms of the compound are substituted by the isotope deuterium.

Another object of the present invention is to provide a pharmaceutical composition comprising the aforementioned compound of formula (I), or the stereoisomer, the tautomer, the pharmaceutically acceptable salt thereof, and one or more pharmaceutically acceptable carriers.

Another object of the present invention is to provide use of the aforementioned compound of formula (I), or the stereoisomer, the tautomer, the pharmaceutically acceptable salt thereof in manufacture of a medicament for treating FXIa-related diseases, specifically, it relates to the use of a medicament for treating thrombosis-related diseases.

Unless otherwise specified, the following terms and phrases used herein are intended to have the following meanings. A specific term or phrase should not be considered indeterminate or unclear without specific definitions, but should be understood in its ordinary meaning. When a trade name appears herein, it is intended to refer to its corresponding commercial product or its active ingredient. As used herein, the term "pharmaceutically acceptable" refers to those compounds, materials, compositions and/or dosage forms, within the scope of sound medical judgment, are suitable for use in contacting with human and animal tissue, without excessive toxicity, irritation, allergic reactions or other problems or complications, and are commensurate with a reasonable benefit/risk ratio.

The term "pharmaceutically acceptable salt" refers to a salt of a compound of the present invention prepared by the compound of the present invention with a specified substituent and a pharmaceutically acceptable acid or base.

In addition to salt forms, the compounds provided herein also exist in prodrug forms. Prodrugs of the compounds described herein are readily chemically altered under physiological conditions to convert to the compounds of the present invention. Furthermore, prodrugs can be converted to the compounds of the present invention by chemical or biochemical methods in an *in vivo* environment.

Certain compounds of the present application may exist in unsolvated and solvated forms, comprising hydrated forms. In general, solvated and unsolvated forms are equivalent and are intended to be included within the scope of the present invention.

The compounds of the present invention may exist in specific geometric or stereoisomeric forms. The present invention contemplates all of such compounds, comprising cis and trans isomers, (-)- and (+)-enantiomers, (R)- and (S)-enantiomers, diastereomers isomers, (D)-isomers, (L)-isomers, and racemic and other mixtures thereof, such as enantiomer- or diastereoisomer-enriched mixtures, all of these mixtures fall within the scope of the present invention. Additional asymmetric carbon atoms may be present in substituents such as alkyl. All of such isomers, as well as mixtures thereof, are included within the scope of the present invention.

Optically active (R)- and (S)-isomers, as well as D and L isomers, can be prepared by chiral synthesis or chiral reagents or other conventional techniques. If one enantiomer of the compound of the present invention is desired, it can be prepared by asymmetric synthesis or derivatization with a chiral auxiliary, wherein the resulting mixture of diastereomers is separated and the auxiliary group is cleaved to provide pure desired enantiomer. Alternatively, when the molecule contains a basic functional group (such as an amino group) or an acidic functional group (such as a carboxyl group), a diastereomeric salt is formed with an appropriate optically active acid or base, then the diastereoisomers are resolved by conventional methods known in the art and the pure enantiomers are recovered. In addition, separation of enantiomers and diastereomers is usually accomplished by the use of chromatography employing a chiral stationary phase, optionally in combination with chemical derivatization (e.g., from amine to carbamate).

The atoms of the molecules of the compounds of the present invention are isotopes, and the isotope derivatization can usually prolong the half-life, reduce the clearance rate, stabilize the metabolism and improve the activity in vivo. Also, an embodiment is included in which at least one atom is replaced by an atom having the same atomic number (number of protons) and a different mass number (sum of protons and neutrons). Examples of isotopes included in the compounds of the present invention comprise hydrogen atom, carbon atom, nitrogen atom, oxygen atom, phosphorus

atom, sulfur atom, fluorine atom, chlorine atom, which respectively comprise $^{2}H$, $^{3}H$, $^{13}C$, $^{14}C$, $^{15}N$, $^{17}O$, $^{18}O$, $^{31}P$, $^{32}P$, $^{35}S$, $^{18}F$, $^{36}Cl$. In particular, radioisotopes that emit radiation as they decay, such as $^{3}H$ or $^{14}C$, are useful in the topological examination of pharmaceutical formulations or compounds *in vivo.* Stable isotopes will not decay or change with their amount and they are not radioactive, thus are safe to use. When the atoms constituting the molecules of the compounds of the present invention are isotopes, the isotopes can be converted according to general methods by substituting the reagents used in the synthesis with reagents containing the corresponding isotopes.

The compounds of the present invention may contain unnatural proportions of atomic isotopes at one or more atoms that constitute the compound. For example, compounds can be labeled with radioactive isotopes, such as deuterium ($^{2}H$), iodine-125 ($^{125}I$) or C-14 ($^{14}C$). All transformations of the isotopic composition of the compounds of the present invention, regardless of whether radioactive or not, are included within the scope of the present invention.

Further, one or more hydrogen atoms of the compounds of the present invention are substituted by the isotope deuterium ($^{2}H$). After deuteration, the compounds of the present invention have the effects of prolonging the half-life, reducing the clearance rate, stabilizing the metabolism and improving the *in vivo* activity.

The preparation method of the isotopic derivatives generally comprises a phase transfer catalysis method. For example, the preferred deuteration method employs a phase transfer catalyst (e.g., tetraalkylammonium salts, $NBu_4HSO_4$). The methylene protons of diphenylmethane compounds are exchanged using the phase transfer catalyst, resulting in that higher levels of deuterium are introduced than reduction with deuterated silanes (e.g. triethyldeuterosilane) in the presence of an acid (e.g., methanesulfonic acid) or with a Lewis acid such as aluminum trichloride with sodium deuteroborate.

The term "pharmaceutically acceptable carrier" refers to any formulation carrier or medium capable of delivering an effective amount of the active substance of the present invention, without interfering with the biological activity of the active substance, and without toxic side effects to the host or patient. A representative carrier comprises water, oil, vegetables, minerals, cream base, lotion matrix, ointment matrix, and the like. Such matrixes comprise suspending agents, tackifiers, penetration enhancers, and the like. Their formulations are well known to those skilled in the cosmetic or topical pharmaceutical field. For additional information about carriers, please refer to Remington: The Science and Practice of Pharmacy, 21 st Ed., Lippincott, Williams & Wilkins (2005), the contents of which are incorporated herein by reference.

The term "excipient" generally refers to the carrier, diluent and/or medium required to formulate an effective pharmaceutical composition.

The term "effective amount" or "therapeutically effective amount" with respect to a drug or pharmacologically active agent refers to a nontoxic but sufficient amount of the drug or agent to achieve the desired effect. For oral dosage forms of the present invention, an "effective amount" of one active substance in a composition refers to the amount required to achieve the desired effect when used in combination with another active substance in the composition. The determination of the effective amount varies from person to person, depends on the age and general condition of the recipient, and also depends on the specific active substance. The appropriate effective amount in individual cases can be determined by those skilled in the art based on routine experiments.

The terms "active ingredient", "therapeutic agent", "active substance" or "active agent" refer to a chemical entity that is effective in treating the target disorder, disease or condition.

The terms "optional" or "optionally" means that the subsequently described event or circumstance may occur or not occur, and this description includes instances in which said event or circumstance occurs and instances in which said event or circumstance does not occur. "〰" indicates a bond.

The compounds of the present invention can be prepared by a variety of synthetic methods well known to those skilled in the art, including the specific embodiments enumerated below, embodiments formed in combination with other chemical synthesis methods, and equivalent alternatives well known to those skilled in the art, preferred embodiments include, but are not limited to, the example of the present invention.

## DETAILED DESCRIPTION OF THE INVENTION

The present invention will be further described in detail below with reference to the examples, but the embodiments of the present invention are not limited to these examples.

The structures of compounds were determined by nuclear magnetic resonance (NMR) or mass spectrometry (MS). NMR shifts ($\delta$) were given in units of $10^{-6}$ (ppm). NMR was measured by Bruker AVANCE-III nuclear magnetic instrument, and the solvent was deuterated dimethyl sulfoxide (DMSO-d6), deuterated chloroform ($CDCl_3$), and the internal standard was tetramethylsilane (TMS).

The MS was measured using an ISQ EC mass spectrometer (manufacturer: Thermo, model: ISQ EC).

High performance liquid chromatography (HPLC) analysis was performed using a Thermo U3000 HPLC DAD high performance liquid chromatograph.

The CombiFlash Rapid Preparation System was the CombiFlash Rf+ LUMEN (TELEDYNE ISCO).

The thin layer chromatography silica gel plate was Yantai Yinlong HSGF254 or GF254 silica gel plate, the specification of the silica gel plate used for thin layer chromatography (TLC) was 0.17mm - 0.23mm, and the specification of the TLC separation and purification products was 0.4mm - 0.5mm.

Silica gel column chromatography used Rushan Shangbang silica gel 100 - 200 mesh silica gel as the carrier.

**Example 1**

**Synthesis of (S)-4-(2-(4-(2-acetyl-5-chlorophenyl)-6-oxopyrrolidin-1(6*H*)-yl)-3-phenylpropanamido)benz oic acid**

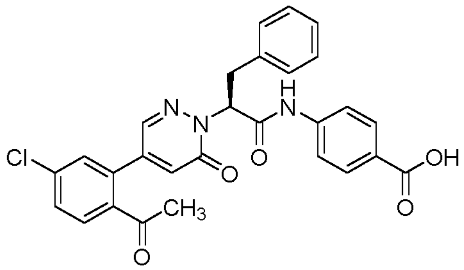

The specific synthetic route was as follows:

Step A: Synthesis of 1-bromo-4-chloro-2-vinylbenzene

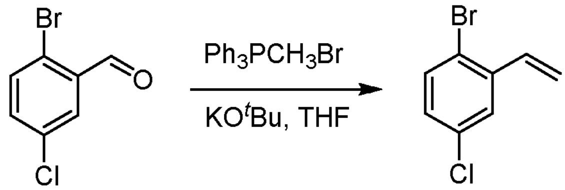


2-bromo-5-chlorobenzaldehyde (3.00 g, 13.6 mmol) was dissolved in tetrahydrofuran (40.0 ml). Subsequently, bromomethyl triphenylphosphine (5.86 g, 16.0 mmol) and potassium tert-butoxide (3.00 g, 27.0 mmol) were added to the above solution and nitrogen replacement was performed for three times. It was stirred at 60 °C for 4 hours.

The reaction solution was diluted by slowly adding 50 ml of water dropwise. The mixed solution was extracted with ethyl acetate (100 ml × 3). The organic phases were combined. The combined organic phase was washed with saturated brine water (100 ml), then dried with anhydrous sodium sulfate, and concentrated under reduced pressure. The resulting residue was purified by silica gel column chromatography (eluent: ethyl acetate/petroleum ether = 1/15). 2.00 g of oily product 1-bromo-4-chloro-2-vinyl benzene was obtained (yield: 66.0%). LCMS: RT = 4.56 min, $[M+H]^+$ = 217.14.

Step B: Synthesis of 2-(2-bromo-5-chlorophenyl)acetaldehyde

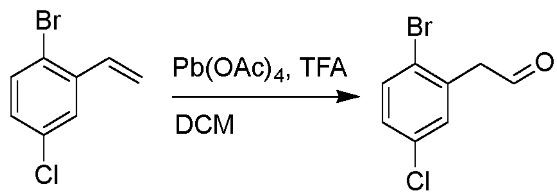


1-bromo-4-chloro-2-vinylbenzene (1.20 g, 5.5 mmol) and lead acetate (9.70 g, 22.0 mmol) were dissolved in

dichloromethane (30.0 mL). Subsequently, trifluoroacetic acid (10 mL) was added to the above solution. It was stirred at room temperature for 4 hours.

The small samples (500 mg of raw materials) were combined, and saturated sodium bicarbonate solution (100 ml) was added to the reaction solution to quench the reaction. The mixed solution was extracted with ethyl acetate (50 mL × 3 times). The organic phases were combined. The combined organic phase was washed with saturated brine (50 ml × 3 times), then dried with anhydrous sodium sulfate, and concentrated under reduced pressure. The resulting residue was purified by silica gel column chromatography (eluent: ethyl acetate/petroleum ether=1/10). 1.50 g of solid 2-(2-bromo-5-chlorophenyl)acetaldehyde was obtained (yield: 82.0%). LCMS: RT = 4.33 min, $[M+H]^+$ = 233.26.

**Step C: Synthesis of 4-(2-bromo-5-chlorophenyl)-5-hydroxyfuran-2(5*H*)-one**

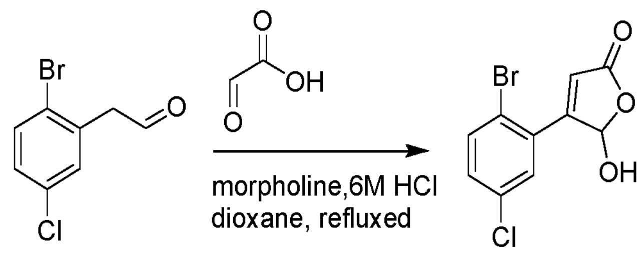


2-(2-bromo-5-chlorophenyl)acetaldehyde (1.50 g, 6.4 mmol) and 2-oxoacetic acid (713 mg, 9.60 mmol) were dissolved in 1,4-dioxane (20.0 mL). Subsequently, morpholine (547 mg, 6.4 mmol) and hydrochloric acid (6 mol/L, 4.0 mL) were added to the above solution. It was stirred at 110 °C for 14 hours.

Saturated sodium bicarbonate solution (50 mL) was added to the reaction solution saturated to quench the reaction. The mixed solution was extracted with ethyl acetate (50 mL × 3 times). The organic phases were combined. The combined organic phase was washed with saturated brine (50 ml × 3 times), then dried with anhydrous sodium sulfate, and concentrated under reduced pressure. The residue was purified by silica gel column chromatography (eluent: ethyl acetate/petroleum ether=1/5). 900 mg of solid 4-(2-bromo-5-chlorophenyl)-5-hydroxyfuran-2(5*H*)-one was obtained (yield: 51.0%). LCMS: RT = 3.88 min, $[M+H]^+$ = 289.16.

**Step D: Synthesis of 5-(2-bromo-5-chlorophenyl)pyridazin-3(2*H*)-one**

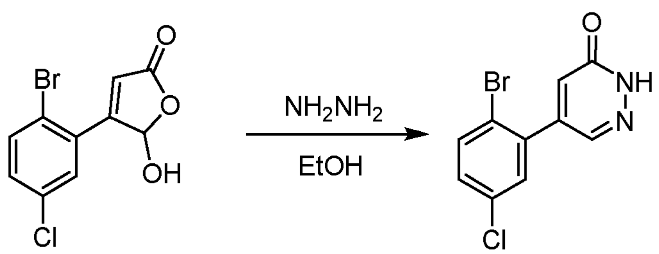


4-(2-bromo-5-chlorophenyl)-5-hydroxyfuran-2(5*H*)-one (600 mg, 2.0 mmol) and hydrazine (132 mg, 4.1 mmol) were dissolved in ethanol (10.0 mL). It was stirred at room temperature for 16 hours.

Water (50 mL) was added to the reaction to dilute the reaction solution. The mixed solution was extracted with ethyl acetate (40 ml × 3 times). The organic phases were combined. The combined organic phase was washed with saturated brine (30 ml × 3 times), then dried with anhydrous sodium sulfate, and concentrated under reduced pressure. The residue was purified by silica gel column chromatography (eluent: ethyl acetate/petroleum ether=1/5). 380 mg of white solid 5-(2-bromo-5-chlorophenyl)pyridazin-3(2*H*)-one was obtained (yield: 64.2%). LCMS: RT = 2.87 min, $[M+H]^+$ = 285.20.

**Step E: Synthesis of tert-butyl (R)- 4-(2-hydroxy-3-phenylpropanamido)benzoate**

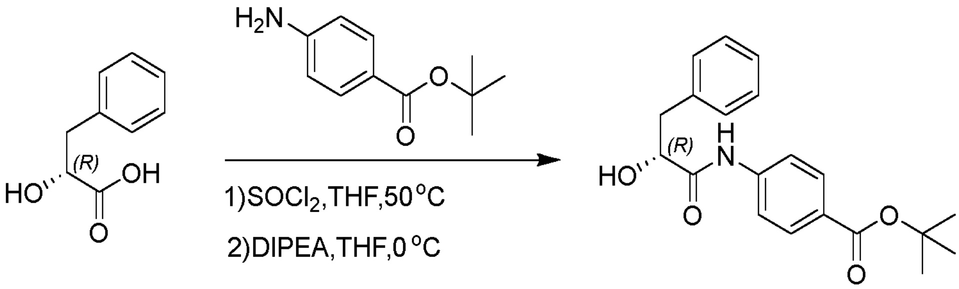


D-phenyllactic acid (23.0 g, 138 mmol) was dissolved in dry tetrahydrofuran (400 mL), placed in a dry three-necked flask, stirred in an ice bath for 15 minutes under nitrogen protection. Thionyl chloride ( 20 ml, 207 mmol) was slowly added dropwise to the reaction solution, the dropwise addition was completed after 30 minutes. The reaction solution was heated to 50°C and stirred at a constant temperature for 3 hours. The reaction solution was cooled to room temperature, spin-dried, and vacuumed by an oil pump for 15 minutes, then dissolved with THF to prepare solution A. Tert-butyl 4-aminobenzoate (20 g, 110 mmol) and diisopropylethylamine (68 mL, 414 mmol) were dissolved in dry tetrahydrofuran (200 mL), placed in a dry three-necked flask. The mixed solution was stirred in an ice bath for 15 minutes under nitrogen protection. The solution A was slowly added dropwise to the mixed solution under an ice bath for 1 hour.
Water was added to the reaction solution to quench the reaction, the mixed solution was extracted with ethyl acetate (200 mL × 3 times), the organic phases were combined, The combined organic phase was washed with saturated brine (100 mL × 3 times), and then dried with anhydrous sodium sulfate, and concentrated under reduced pressure. The resulting residue was purified by silica gel column chromatography (eluent: ethyl acetate/petroleum ether=1/4). 19 g of yellow solid tert-butyl (R)-4-(2-hydroxy-3-phenylpropanamido)benzoate was obtained (yield: 53%). LCMS: RT = 4.16 min, $[M-H]^-$ = 340.09.

Step F: Synthesis of tert-butyl (R)-4-(2-(((4-nitrophenyl)sulfonyl)oxy)-3-phenylpropanamido)benzoate

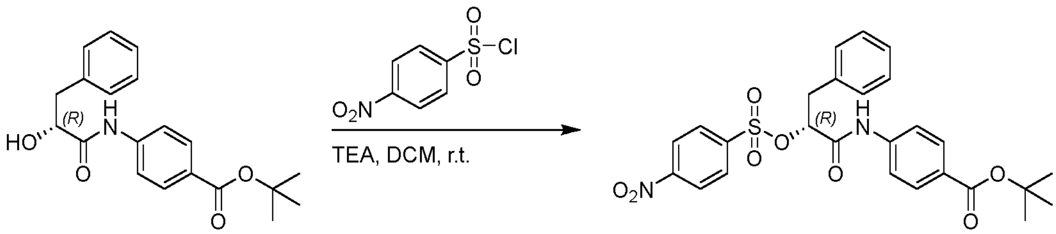


tert-butyl (R)-4-(2-hydroxy-3-phenylpropanamido)benzoate (19 g, 55.7 mmol) and triethylamine (21.6 mL, 167.1 mmol) were dissolved in dichloromethane (100.0 ml). 4-nitrobenzenesulfonyl chloride (18.5 g, 165.6 mmol) was added to the reaction solution under an ice bath. It was stirred at room temperature for 2 hours.
Saturated sodium bicarbonate solution (100 mL) was added to the reaction solution to quench the reaction. The mixed solution was extracted with ethyl acetate (200 mL × 3 times). The organic phases were combined. The combined organic phase was washed with saturated brine (100 ml × 3 times), then dried with anhydrous sodium sulfate, and concentrated under reduced pressure. The resulting residue was dissolved in dichloromethane (40 mL), and added dropwise to n-hexane (400 mL) with stirring. A large amount of white solid was precipitated, filtered, and the filter cake was collected to obtain 11.2 g of white solid tert-butyl (R)-4-(2-(((4-nitrophenyl)sulfonyl)oxy)-3-phenylpropanamido)benzoate (yield: 38%). LCMS: RT = 4.39 min.

**Step G: Synthesis of tert-butyl (S)-4-(2-(4-(2-bromo-5-chlorophenyl)-6-oxopyridazin-1(6*H*)-yl)-3-phenylpropanamido)benz oate**

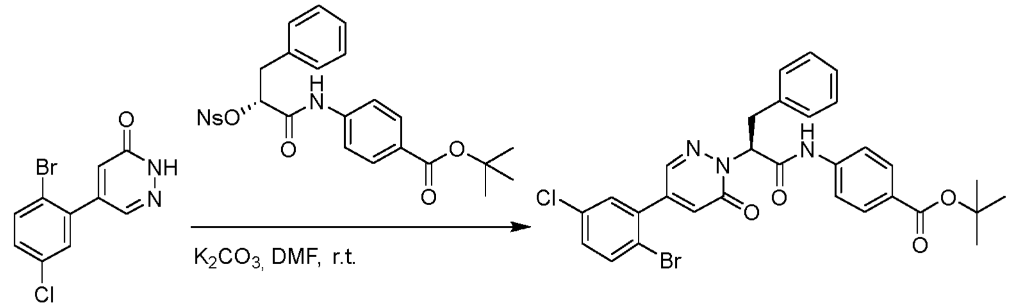


5-(2-bromo-5-chlorophenyl)pyridazin-3(2*H*)-one (380 mg, 1.33 mmol) and tert-butyl (R)-4-(2-((((4-nitrophenyl)sulfonyl)oxy)-3-phenylpropanamido)benzoate (840 mg, 1.60 mmol) were dissolved in ethanol (10.0 mL). Subsequently, potassium carbonate (367 mg, 2.66 mmol) was added to the above solution. It was stirred at room temperature for 18 hours.

Water (50 mL) was added to the reaction solution to dilute the reaction solution. The mixed solution was extracted with ethyl acetate (40 ml × 3 times). The organic phases were combined. The combined organic phase was washed with saturated brine (30 ml × 3 times), then dried with anhydrous sodium sulfate, and concentrated under reduced pressure. The residue was purified by silica gel column chromatography (eluent: ethyl acetate/petroleum ether=1/5). 460 mg of white solid tert-butyl (S)-4-(2-(4-(2-bromo-5-chlorophenyl)-6-oxopyridazin-1(6*H*)-yl)-3-phenylpropanamido)benzoate was obtained (yield: 56.0%). LCMS: RT = 3.95 min, $[M+H]^+$ = 608.06.

**Step H: Synthesis of tert-butyl (S)-4-(2-(4-(2-acetyl-5-chlorophenyl)-6-oxopyridazin-1(6*H*)-yl)-3-phenylpropanamido)benz oate**

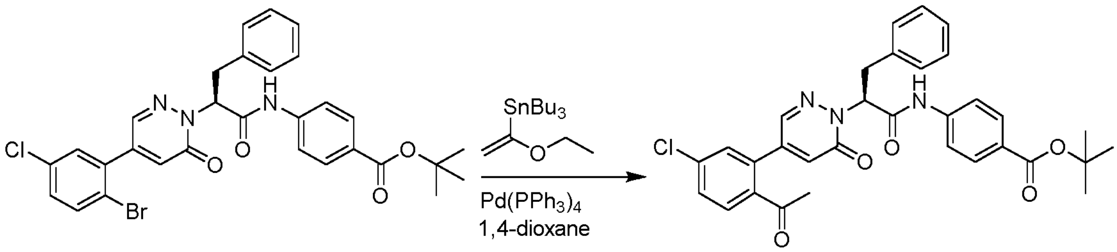


tert-butyl (S)-4-(2-(4-(2-bromo-5-chlorophenyl)-6-oxopyridazin-1(6*H*)-yl)-3-phenylpropanamido)benzoate (300 mg, 0.49 mmol) and tributyl(1-ethoxyvinyl)stannane (213 mg, 0.59 mmol) were dissolved in 1,4-dioxane (15.0 mL). Subsequently, tetrakis(triphenylphosphine) palladium (56 mg, 0.049 mmol) was added to the above solution. It was stirred at 100 °C for 18 hours.

The reaction solution was added with hydrochloric acid (1 mol/L, 10 mL), followed by stirring for 1 hour. The mixed solution was extracted with ethyl acetate (40 mL × 3 times). The organic phases were combined. The combined organic phase was washed with saturated brine (30 ml × 3 times), then dried with anhydrous sodium sulfate, and concentrated under reduced pressure. The residue was purified by silica gel column chromatography (eluent: ethyl acetate/petroleum ether=1/5). 250 mg of white solid tert-butyl (S)-4-(2-(4-(2-acetyl-5-chlorophenyl)-6-oxopyridazin-1(6*H*)-yl)-3-phenylpropanamido)benzoate was obtained (yield: 70.0%). LCMS: RT = 4.17 min, $[M+H]^+$ = 572.03.

**Step I: Synthesis of (S)-4-(2-(4-(2-acetyl-5-chlorophenyl)-6-oxopyrrolidin-1(6*H*)-yl)-3-phenylpropanamido)benz oic acid**

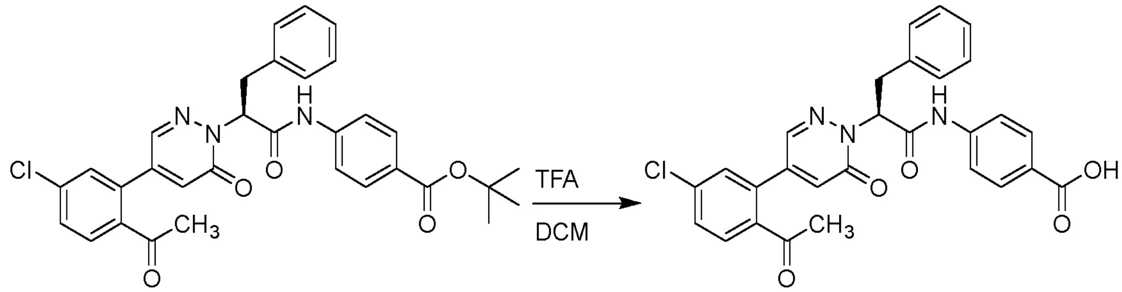


tert-butyl (S)-4-(2-(4-(2-acetyl-5-chlorophenyl)-6-oxopyridazin-1(6*H*)-yl)-3-phenylpropanamido)benzoate (240 mg, 350 mmol) was dissolved in dichloromethane (4 mL). Subsequently, trifluoroacetic acid (1 ml) was added to the above solution. It was stirred at room temperature for 2 hours.

The reaction solution was concentrated under reduced pressure and purified by preparative high performance liquid phase. 207 mg of white solid (S)-4-(2-(4-(2-acetyl-5-chlorophenyl)-6-oxopyrrolidine-1(6*H*)-yl)-3-phenylpropanamido)benzoic acid was obtained (yield: 95.0%). LCMS: RT = 3.94 min, $[M+H]^+$ = 516.10. $^1$H NMR (400 MHz, DMSO) δ 12.72 (s, 1H), 10.59 (s, 1H), 8.03 (d, J = 8.4 Hz, 1H), 7.93 - 7.86 (m, 3H), 7.74 - 7.66 (m, 3H), 7.55 (d, J = 2.1 Hz, 1H), 7.31 - 7.22 (m, 4H), 7.20 - 7.14 (m, 1H), 6.87 (d, J = 2.2 Hz, 1H), 5.82 (dd, J = 9.6, 5.5 Hz, 1H), 3.46 (ddd, J = 19.7, 14.1, 7.6 Hz, 2H), 2.55 (s, 3H).

**Example 2**

**Synthesis of (S)-4-(2-(4-(2-acetyl-5-chlorophenyl)-6-oxo-3-phenylpyridazin-1(6*H*)-yl)-3-phenylpropana mido)benzoic acid**

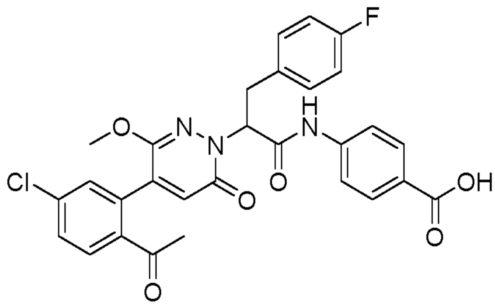

The specific synthetic route was as follows:

**Step A: Synthesis of tert-butyl 2-(4-(2-acetyl-5-chlorophenyl)-3-methoxy-6-oxopyridazin-1(6*H*)-yl)acetate**

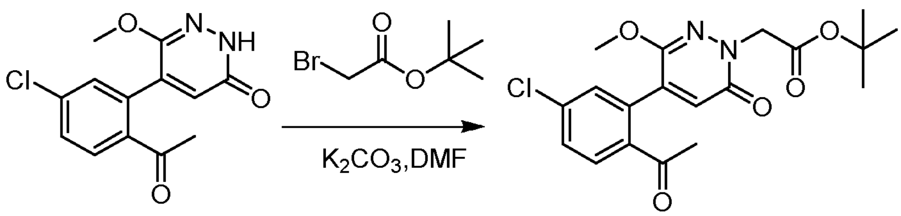


5-(2-acetyl-5-chlorophenyl)-6-methylpyridin-3(2*H*)-one (800 mg, 2.8 mmol) and tert-butyl 2-bromoacetate (671 mg, 3.4 mmol) were dissolved in N,N-dimethylformamide (20.0 mL). Subsequently, potassium carbonate (793 mg, 5.7 mmol) was added to the above solution. It was stirred at room temperature for 4 hours.

Water (50 mL) was added to the reaction solution to dilute the reaction solution. The mixed solution was extracted with ethyl acetate (10 mL × 3 times). The organic phases were combined. The combined organic phase was

washed with saturated brine (30 ml × 3 times), then dried with anhydrous sodium sulfate, and concentrated under reduced pressure. The residue was slurried with n-hexane/ethyl acetate. 780 mg of yellow solid tert-butyl 2-(4-(2-acetyl-5-chlorophenyl)-3-methoxy-6-oxopyridazine-1(6H)-yl)acetate was obtained (yield: 69.0%). LCMS: RT = 3.35 min, $[M+H]^+$ = 393.06.

**Step B: tert-butyl 2-(4-(2-acetyl-5-chlorophenyl)-3-methoxy-6-oxopyridazin-1(6H)-yl)-3-(4-fluorophenyl)prop anoate**

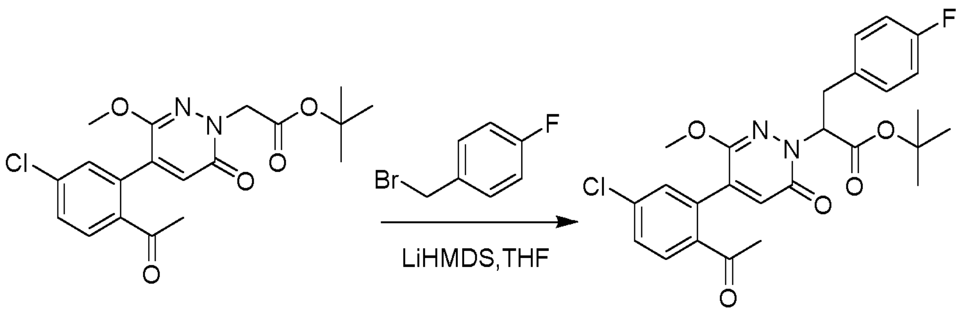


Tert-butyl 2-(4-(2-acetyl-5-chlorophenyl)-3-methoxy-6-oxopyridazin-1(6*H*)-yl)acetate (70 mg, 0.17 mmol) and 1-(bromomethyl)-4-fluorobenzene (40 mg, 0.20 mmol) were dissolved in tetrahydrofuran (5.0 mL). Subsequently, lithium bis(trimethylsilyl)amide (0.3 mL, 0.30 mmol) was added to the above solution. It was stirred at -50 °C for 2 hours.

Water (10 mL) was added to the reaction solution to dilute the reaction solution. The mixed solution was extracted with ethyl acetate (10 mL × 3 times). The organic phases were combined. The combined organic phase was washed with saturated brine (30 ml × 3 times), then dried with anhydrous sodium sulfate, and concentrated under reduced pressure. The resulting residue was purified by silica gel column chromatography (eluent: ethyl acetate/petroleum ether=1/5). 50 mg of yellow solid tert-butyl 2-(4-(2-acetyl-5-chlorophenyl)-3-methoxy-6-oxopyridazin-1(6*H*)-yl)-3-(4-fluorophenyl)propanoate was obtained (yield: 56.0%). LCMS: RT = 3.87 min, $[M+H]^+$ = 501.02.

**Step C: 2-(4-(2-acetyl-5-chlorophenyl)-3-methoxy-6-oxopyridazin-1(6*H*)-yl)-3-(4-fluorophenyl)prop anic acid**

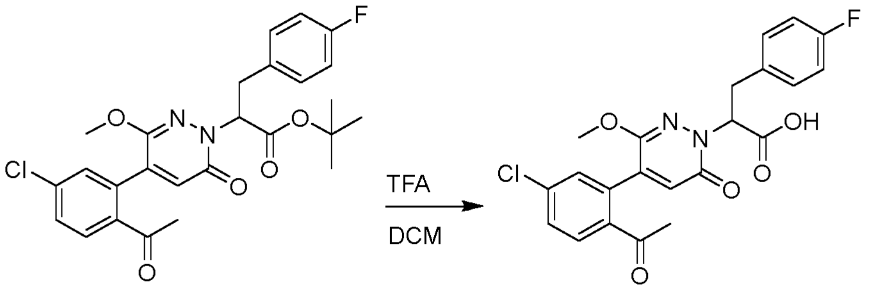


Tert-butyl 2-(4-(2-acetyl-5-chlorophenyl)-3-methoxy-6-oxopyridazin-1(6*H*)-yl)-3-(4-fluorophenyl)propano ate (50 mg, 0.1 mmol) was dissolved in dichloromethane (4.0 mL). Subsequently, trifluoroacetic acid (1.0 ml) was added to the above solution. It was stirred at room temperature for 2 hours.

The reaction solution was concentrated under reduced pressure. 40 mg of yellow solid 2-(4-(2-acetyl-5-chlorophenyl)-3-methoxy-6-oxopyridazin-1(6*H*)-yl) -3-(4-fluorophenyl)propanic acid was obtained (yield: 90.0%). LCMS: RT = 3.29 min, $[M+H]^+$ = 445.01.

**Step D: Synthesis of tert-butyl 4-(2-(4-(2-acetyl-5-chlorophenyl)-3-methoxy-6-oxopyridazin-1(6*H*)-yl)-3-4-fluorophenyl)pr opanamido)benzoate**

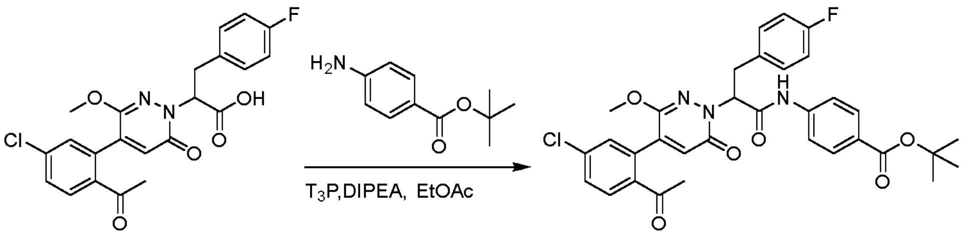


2-(4-(2-acetyl-5-chlorophenyl)-3-methoxy-6-oxopyridazin-1(6*H*)-yl)-3-(4-fluorophenyl)pro panic acid (40 mg, 0.10 mmol) and tert-butyl 4-aminobenzoate (22 mg, 0.12 mmol) were dissolved in ethyl acetate (10.0 mL). Subsequently, 1-propyl phosphoric anhydride (151 mg, 0.50 mmol) and N,N-diisopropylethylamine (37 mg, 0.30 mmol) were added to the above solution. It was stirred at 50 °C for 3 hours.

Water (30 mL) was added to the reaction solution to dilute the reaction solution. The mixed solution was extracted with ethyl acetate (10 mL × 3 times). The organic phases were combined. The combined organic phase was washed with saturated brine (30 ml × 3 times), then dried with anhydrous sodium sulfate, and concentrated under reduced pressure. The resulting residue was purified by silica gel column chromatography (eluent: dichloromethane/methanol=10/1). 35 mg of yellow solid tert-butyl 4-(2-(4-(2-acetyl-5-chlorophenyl)-3 -methoxy-6-oxopyridazin-1(6*H*)-yl)-3-4-fluorophenyl)propanamido)benzoate was obtained (yield: 58.0%). LCMS: RT = 4.14 min, $[M+H]^+$ = 620.14.

**Step E: Synthesis of 4-(2-(4-(2-acetyl-5-chlorophenyl)-3-methoxy-6-oxopyridazin-1(6*H*)-yl)-3-4-fluorophenyl)propanamido)benzoic acid**

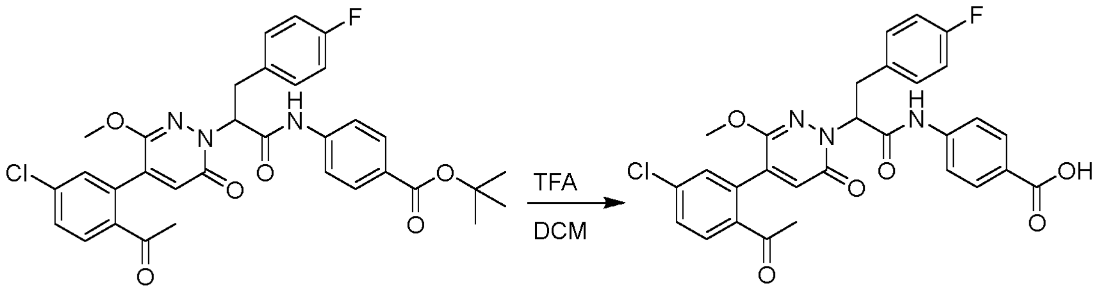


tert-butyl 4-(2-(4-(2-acetyl-5-chlorophenyl)-3-methoxy-6-oxopyridazin-1(6*H*)-yl)-3-4-fluorophenyl)propa namido)benzoate (40 mg, 0.060 mmol) was dissolved in dichloromethane (4.0 mL). Subsequently, trifluoroacetic acid (1.0 ml) was added to the above solution. It was stirred at room temperature for 2 hours.

The reaction solution was concentrated under reduced pressure, and the resulting residue was purified by preparative high performance liquid phase. 18 mg of white solid **4-(2-(**4-(2-acetyl-5-chlorophenyl)-3-methoxy-6 -oxopyridazin-1(6*H*)-yl)-3-4-fluorophenyl)propanamido)benzoic acid was obtained (yield: 50.0%). LCMS: RT = 3.98 min, $[M-H]^-$ = 562.08. $^1H$ NMR (500 MHz, DMSO) δ 10.51 (s, 1H), 8.02 (d, J = 8.4 Hz, 1H), 7.92 (d, J = 8.8 Hz , 2H), 7.73 (d, J = 8.7 Hz, 2H), 7.71 (dd, J = 8.4, 2.2 Hz, 1H), 7.52 (d, J = 2.2 Hz, 1H), 7.35 (dd, J = 8.6, 5.6 Hz, 2H), 7.11 (dd, J = 12.3, 5.5 Hz, 2H), 6.92 (s, 1H), 5.72 (dd, J = 10.2, 5.0 Hz, 1H), 3.68 (s, 3H), 3.51 ( dd, J = 14.1, 10.2 Hz, 1H), 3.42 (dd, J = 14.0, 4.7 Hz, 1H), 2.55 (s, 3H).

**Example 3**

**Synthesis of 4-(2-(4-(2-acetyl-5-chlorophenyl)-3-methoxy-6-oxopyridazin-1(6*H*)-yl)-3-(4-bromophenyl)p ropanamido)benzoic acid**

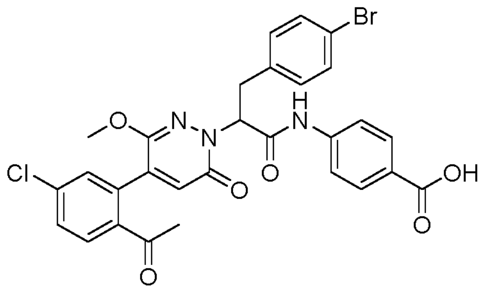

The specific synthetic route was as follows:

**Step A: Synthesis of tert-butyl 2-(4-(2-acetyl-5-chlorophenyl)-3-methoxy-6-oxopyridazin-1(6*H*)-yl)-3-(4-bromophenyl)pro panoate**

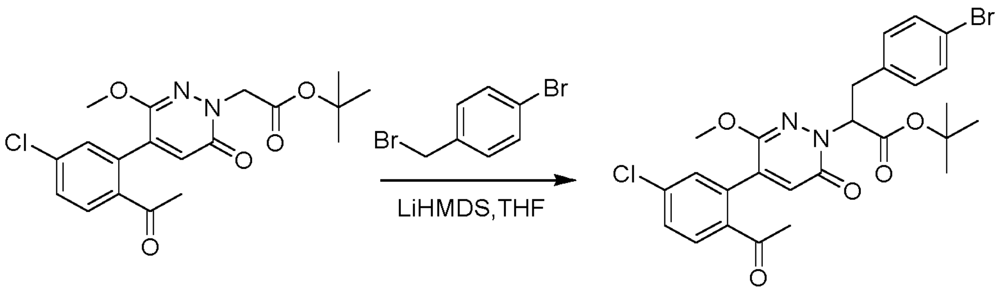


Tert-butyl 2-(4-(2-acetyl-5-chlorophenyl)-3-methoxy-6-oxopyridazin-1(6*H*)-yl)acetate (150 mg, 0.38 mmol ) and 1-bromo-4-(bromomethyl)benzene (114.7 mg, 0.46 mmol) were dissolved in tetrahydrofuran (5.0 mL). Subsequently, lithium bis(trimethylsilyl)amide (0.5 mL, 0.50 mmol) was added to the above solution. It was stirred at 50 °C for 2 hours.

Water (10 mL) was added to the reaction solution to dilute the reaction solution. The mixed solution was extracted with ethyl acetate (10 mL × 3 times). The organic phases were combined. The combined organic phase was washed with saturated brine (30 ml × 3 times), then dried with anhydrous sodium sulfate, and concentrated under reduced pressure. The residue was purified by silica gel column chromatography (eluent: ethyl acetate/petroleum ether=1/5). 120 mg of yellow solid tert-butyl 2-(4-(2-acetyl-5-chlorophenyl)-3-methoxy-6-oxopyridazin-1(6*H*)-yl)-3-(4-bromophenyl)propanoate was obtained (yield: 56.0%). LCMS: RT = 3.89 min, $[M+H]^+$ = 561.14.

**Step B: Synthesis of 2-(4-(2-acetyl-5-chlorophenyl)-3-methoxy-6-oxopyridazin-1(6*H*)-yl)-3-(4-bromophenyl)pro panic acid**

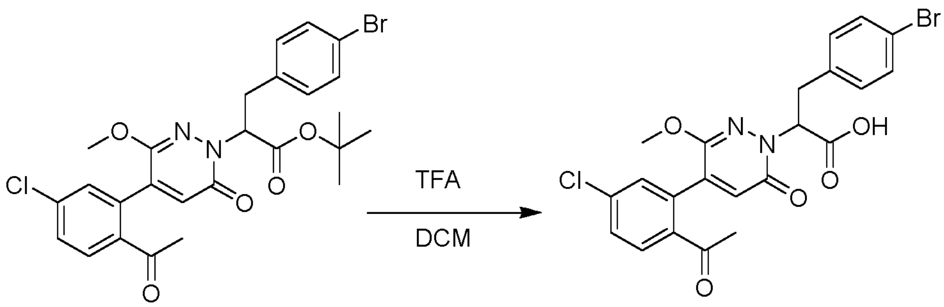


Tert-butyl 2-(4-(2-acetyl-5-chlorophenyl)-3-methoxy-6-oxopyridazin-1(6*H*)-yl)-3-(4-bromophenyl)propano ate (30 mg, 0.050 mmol) was dissolved in dichloromethane (4.0 mL). Subsequently, trifluoroacetic acid (1.0 ml) was added to the above solution. It was stirred at room temperature for 2 hours.

The reaction solution was concentrated under reduced pressure. 24 mg of yellow solid 2-(4-(2-acetyl-5-chlorophenyl)-3-methoxy-6-oxopyridazin-1(6*H*)-yl) -3-(4-bromophenyl)propanic acid was obtained (yield: 88.8%). LCMS: RT = 3.35 min, $[M+H]^+$ = 505.02.

**Step C: Synthesis of tert-butyl 4-(2-(4-(2-acetyl-5-chlorophenyl)-3-methoxy-6-oxopyridazin-1(6*H*)-yl)-3-(4-bromophenyl)p ropanamido)benzoate**

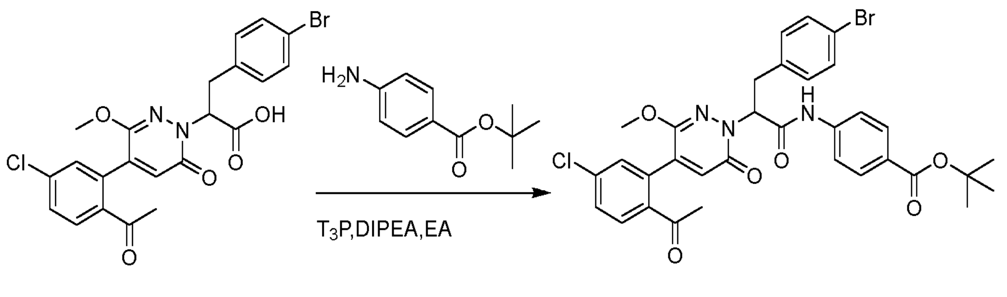


2-(4-(2-acetyl-5-chlorophenyl)-3-methoxy-6-oxopyridazin-1(6*H*)-yl)-3-(4-bromophenyl)pro pane acid (24 mg, 0.047 mmol) and tert-butyl 4-aminobenzoate (11 mg, 0.057 mmol) were dissolved in ethyl acetate (5.0 mL). Subsequently, 1-propyl phosphoric anhydride (59 mg, 0.19 mmol) and N,N-diisopropylethylamine (18.4 mg, 0.14 mmol) were added to the above solution. It was stirred at 50 °C for 3 hours.

Water (30 mL) was added to the reaction solution to dilute the reaction solution. The mixed solution was extracted with ethyl acetate (10 mL × 3 times). The organic phases were combined. The combined organic phase was washed with saturated brine (30 ml × 3 times), then dried with anhydrous sodium sulfate, and concentrated under reduced pressure. The resulting residue was purified by silica gel column chromatography (eluent: dichloromethane/methanol=10/1). 28 mg of yellow solid tert-butyl 4-(2-(4-(2-acetyl-5-chlorophenyl)-3-methoxy-6-oxopyridazin-1(6*H*)-yl)-3-(4-bromophenyl)propanamido)benzoate was obtained (yield: 87.5%). LCMS: RT = 4.27 min, $[M+H]^+$ = 680.10.

**Step D: Synthesis of 4-(2-(4-(2-acetyl-5-chlorophenyl)-3-methoxy-6-oxopyridazin-1(6*H*)-yl)-3-(4 -bromophenyl)propanamido)benzoic acid**

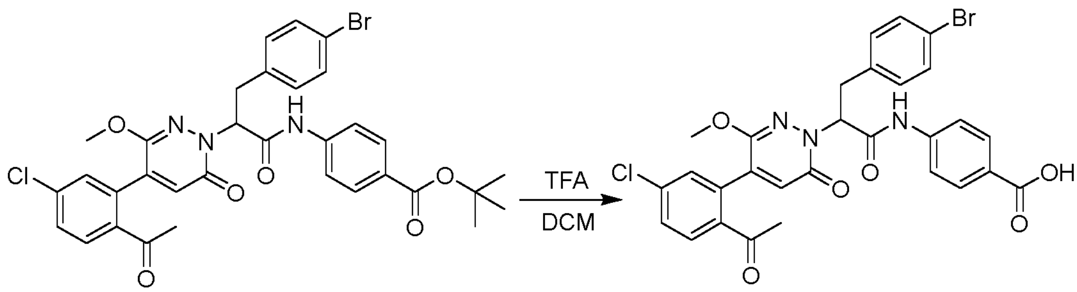


tert-butyl 4-(2-(4-(2-acetyl-5-chlorophenyl)-3-methoxy-6-oxopyridazin-1(6*H*)-yl)-3-(4-bromophenyl))prop anamido)benzoate (28 mg, 0.040 mmol) was dissolved in dichloromethane (4.0 mL). Subsequently, trifluoroacetic acid (1.0 ml) was added to the above solution. It was stirred at room temperature for 2 hours.

The reaction solution was concentrated under reduced pressure, and the resulting residue was purified by preparative high performance liquid phase. 12 mg of white solid 4-(2-(4-(2-acetyl-5-chlorophenyl)-3-methoxy-6 -oxopyridazin-1(6*H*)-yl)-3-(4-bromophenyl)propanamido)benzoic acid was obtained (yield: 48%). LCMS: RT = 4.15 min, $[M-H]^-$ = 623.94. $^1H$ NMR (400 MHz, DMSO) δ 12.76 (s, 1H), 10.51 (s, 1H), 7.96 (dd, J = 46.8, 7.5 Hz, 3H) , 7.73 (d, J = 7.7 Hz, 3H), 7.55 - 7.42 (m, 3H), 7.28 (s, 2H), 6.92 (s, 1H), 5.73 (s, 1H), 3.66 (s, 3H), 3.48 (d, J = 10.9 Hz, 2H), 2.55 (s, 3H).

**Example 4**

**Synthesis of 4-((2-(4-(2-acetyl-5-chlorophenyl)-3-methoxy-6-oxopyridazin-1(6*H*)-yl)-3-(4-(4-isopropyl-2-oxopiperazin-1-yl)phenyl)propanyl)oxy)benzoic acid**

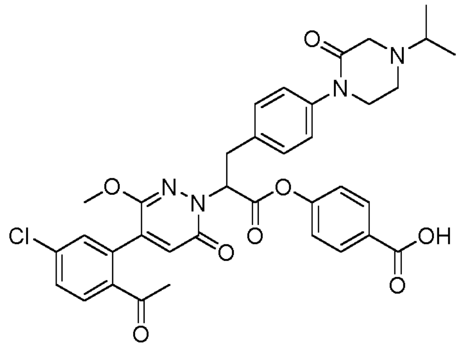

The specific synthetic route was as follows:

Step A: Synthesis of (4-bromophenyl)methanol

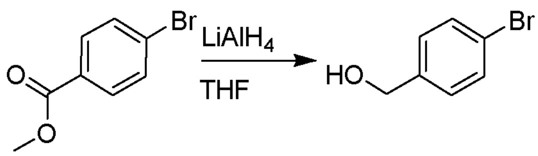


Methyl 4-bromobenzoate (2.0 g, 9.3 mmol) was dissolved in tetrahydrofuran (100.0 mL). Subsequently, lithium tetrahydroaluminum (700 mg, 18.6 mmol) was added to the above solution. It was stirred at room temperature for 18 hours.

Methanol (100 mL) was added to the reaction solution to quench the reaction, and then concentrated under reduced pressure. The resulting residue was purified by silica gel column chromatography (eluent: dichloromethane/methanol=10/1). 1.3 g of white oil (4-bromophenyl)methanol was obtained (yield: 87.5%). LCMS: RT=4.16 min, $[M+H]^+$=187.02.

**Step B: Synthesis of 4-isopropylpiperazin-2-one**

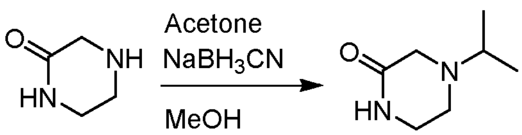


Piperazin-2-one (2.0 g, 20 mmol) was dissolved in methanol (50.0 mL). Subsequently, acetone (5.8 mg, 150 mmol) and sodium cyanoborohydride (2.6 g, 40 mmol) were added to the above solution. It was stirred at room temperature for 18 hours.

Methanol (50 mL) was added to the reaction solution to quench the reaction, and then concentrated under reduced pressure. The resulting residue was purified by silica gel column chromatography (eluent: dichloromethane/methanol=10/1). 2.8 g of white oil 4-isopropylpiperazin-2-one was obtained (yield: 100%). LCMS: RT = 0.82 min, $[M+H]^+$ = 143.04.

**Step C: Synthesis of 1-(4-(hydroxymethyl)phenyl)-4-isopropyl-2-one**

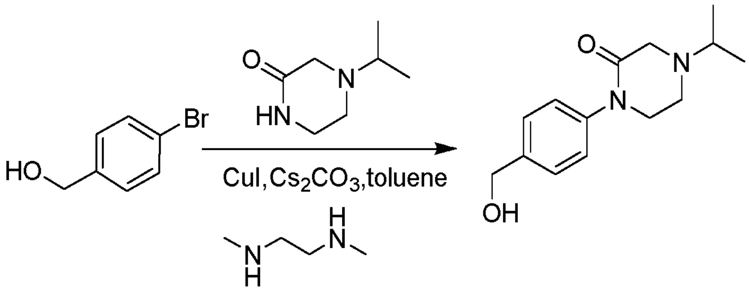


(4-bromophenyl)methanol (700 mg, 3.7 mmol) and 4-isopropylpiperazin-2-one (1 g, 7.4 mmol) were dissolved in toluene (20.0 mL). Subsequently, cesium carbonate (2.4 g, 7.4 mmol), N,N-dimethylethane-1,2-diamine (659 mg, 7.4 mmol) and cuprous iodide (712 mg, 3.7 mmol) were added to the above solution. The system was replaced with nitrogen for three times, and stirred at 100 °C for 18 hours.

Water (100 mL) was added to the reaction solution to dilute the reaction solution. The mixed solution was extracted with ethyl acetate (20 mL × 3 times). The organic phases were combined. The combined organic phase was washed with saturated brine (20 ml × 3 times), then dried with anhydrous sodium sulfate, and concentrated under reduced pressure. The resulting residue was purified by silica gel column chromatography (eluent: dichloromethane/methanol=10/1). 750 mg of white solid 1-(4-(hydroxymethyl)phenyl)-4-isopropyl-2-one was obtained (yield: 80.8%). LCMS: RT = 3.16 min, $[M+H]^+$ = 249.16.

**Step D: Synthesis of 1-(4-(bromomethyl)phenyl)-4-isopropyl-2-one**

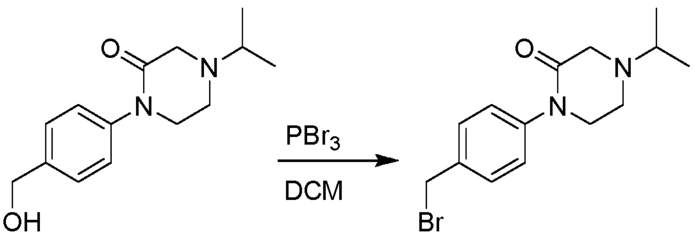


1-(4-(hydroxymethyl)phenyl)-4-isopropyl-2-one (150 mg, 0.6 mmol) was dissolved in dichloromethane (10.0 mL). Subsequently, phosphorus tribromide (324 mg, 1.2 mmol) was added to the above solution. It was stirred at room temperature for 1 hours.

Water (10 mL) was added to the reaction solution to dilute the reaction solution. The mixed solution was extracted with ethyl acetate (10 mL × 3 times). The organic phases were combined. The combined organic phase was washed with saturated brine (20 ml × 3 times), then dried with anhydrous sodium sulfate, and concentrated under reduced pressure. 90 mg of white oil 1-(4-(bromomethyl)phenyl)-4-isopropyl-2-one was obtained (yield: 80.8%). LCMS: RT = 3.46 min, $[M+H]^+$ = 311.12.

**Step E: Synthesis of tert-butyl 2-(4-(2-acetyl-5-chlorophenyl)-3-methoxy-6-oxopyridazin-1(6*H*)-yl)-3-(4-(4 -isopropyl-2-oxopiperazine-1-)tert-butyl)phenyl)propanoate**

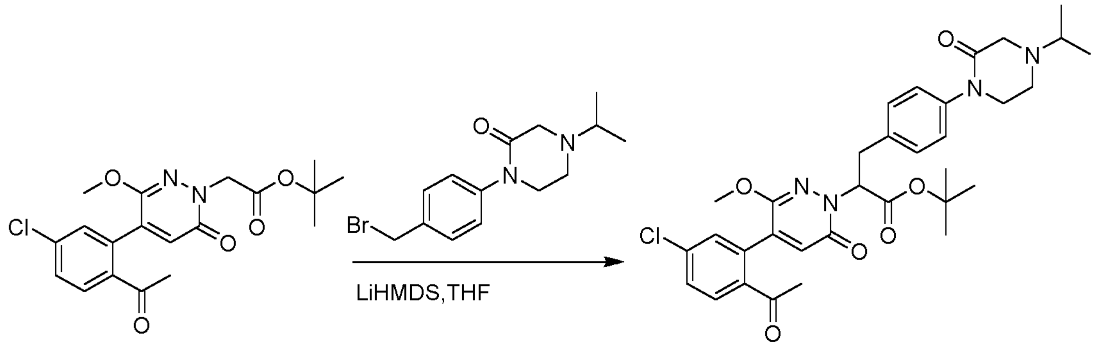


Tert-butyl 2-(4-(2-acetyl-5-chlorophenyl)-3-methoxy-6-oxopyridazin-1(6*H*)-yl)acetate (50 mg, 0.12 mmol) and 1-(4-(bromomethyl)phenyl)-4-isopropyl-2-one (47 mg, 0.15 mmol) were dissolved in tetrahydrofuran (5.0 mL). Subsequently, lithium bis(trimethylsilyl)amide (0.19 mL, 0.19 mmol) was added to the above solution. It was stirred at -50 °C for 2 hours.

Water (10 mL) was added to the reaction solution to dilute the reaction solution. The mixed solution was extracted with ethyl acetate (10 mL × 3 times). The organic phases were combined. The combined organic phase was washed with saturated brine (30 ml × 3 times), then dried with anhydrous sodium sulfate, and concentrated under reduced pressure. The residue was purified by silica gel column chromatography (eluent: dichloromethane/methanol=10/1). 20 mg of yellow oil tert-butyl 2-(4-(2-acetyl-5-chlorophenyl)-3-methoxy-6-oxopyridazin-1(6*H*)-yl)-3-(4-(4-isopropyl-2-oxopip erazine-1-)tert-butyl)phenyl)propanoate was obtained (yield: 25.0%). LCMS: RT = 3.26 min, $[M+H]^+$ = 623.10.

**Step F: Synthesis of 2-(4-(2-acetyl-5-chlorophenyl)-3-methoxy-6-oxopyridazin-1(6*H*)-yl)-3-(4-(4 -isopropyl-2-oxopiperazine-1-)tert-butyl)phenyl)propanic acid**

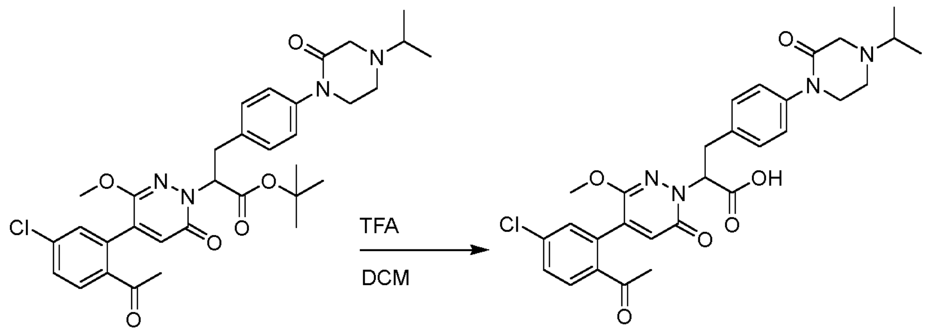


tert-butyl 2-(4-(2-acetyl-5-chlorophenyl)-3-methoxy-6-oxopyridazin-1(6*H*)-yl)-3-(4-(4-isopropyl)-2-oxopi perazine-1-)tert-butyl)phenyl)propanoate (20 mg, 0.030 mmol) was dissolved in dichloromethane (4.0 mL). Subsequently, trifluoroacetic acid (1.0 ml) was added to the above solution. It was stirred at room temperature for 2 hours.

The reaction solution was concentrated under reduced pressure, and the resulting residue was purified by preparative high performance liquid phase. 17 mg of white solid 2-(4-(2-acetyl-5-chlorophenyl)-3-methoxy-6-oxopyridazine-1(6*H*)-yl)-3-(4-(4-isopropyl-2-oxopi perazine-1-)tert-butyl)phenyl)propanic acid was obtained (yield: 93.0%) . LC-MS: RT = 2.56 min, $[M+H]^+$ = 568.25.

**Step G: Synthesis of tert-butyl 4-((2-(4-(2-acetyl-5-chlorophenyl)-3-methoxy-6-oxopyridazin-1(6*H*)-yl)-3-(4-(4-isopropyl-2-oxopiperazine)tert-butyl-1-yl)phenyl)propanamido)benzoate**

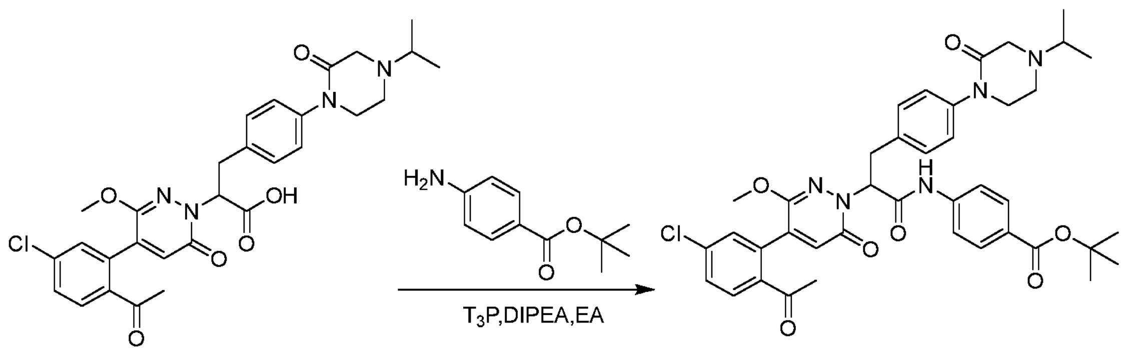


2-(4-(2-acetyl-5-chlorophenyl)-3-methoxy-6-oxopyridazin-1(6*H*)-yl)-3-(4-(4-isopropyl)-2-o xopiperazine-1-)tert-butyl)phenyl)propanic acid (17 mg, 0.029 mmol) and tert-butyl 4-aminobenzoate (7 mg, 0.035 mmol) were dissolved in ethyl acetate (5.0 ml). Subsequently, 1-propyl phosphoric anhydride (45 mg, 0.14 mmol) and N,N-diisopropylethylamine (11 mg, 0.087 mmol) were added to the above solution. It was stirred at 50 °C for 3 hours.

Water (30 mL) was added to the reaction solution to dilute the reaction solution. The mixed solution was extracted with ethyl acetate (10 mL × 3 times). The organic phases were combined. The combined organic phase was washed with saturated brine (30 ml × 3 times), then dried with anhydrous sodium sulfate, and concentrated under reduced pressure. The resulting residue was purified by silica gel column chromatography (eluent: dichloromethane/methanol=10/1). 8 mg of yellow solid tert-butyl 4-((2-(4-(2-acetyl-5-chlorophenyl) -3-methoxy-6-oxopyridazin-1(6*H*)-yl)-3-(4-(4-isopropyl-2-oxopiperazine)tert-butyl-1-yl)phenyl) propanamido)benzoate was obtained (yield: 87.5%). LCMS: RT = 3.42 min, $[M+H]^+$ = 742.16.

**Step H: Synthesis of 4-((2-(4-(2-acetyl-5-chlorophenyl)-3-methoxy-6-oxopyridazin-1(6*H*)-yl)-3-(4-(4-isopropyl-2-oxopiperazine)tert-butyl-1-yl)phenyl)propanamido)benzoic acid**

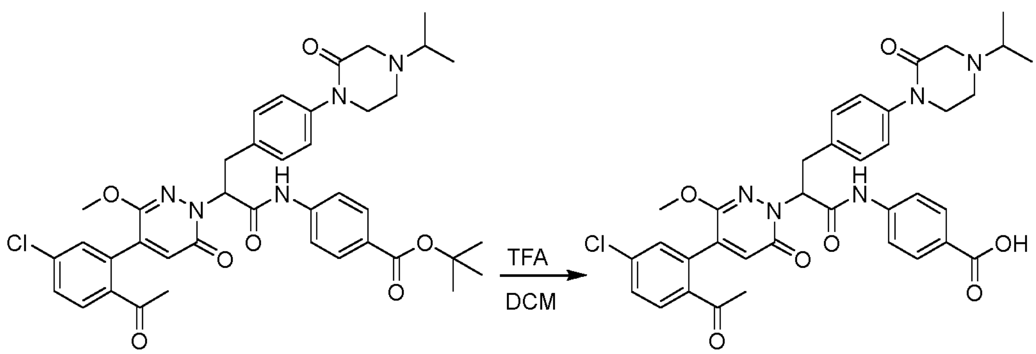


Tert-butyl 4-((2-(4-(2-acetyl-5-chlorophenyl)-3-methoxy-6-oxopyridazin-1(6*H*)-yl)-3-(4-(4-isopropyl-2-ox opiperazine)tert-butyl-1-yl)phenyl)propanamido)benzoate (8 mg, 0.01 mmol) was dissolved in dichloromethane (4.0 mL). Subsequently, trifluoroacetic acid (1.0 ml) was added to the above solution. It was stirred at room temperature for 2 hours.

The reaction solution was concentrated under reduced pressure, and the resulting residue was purified by preparative high performance liquid phase. 1.7 mg of white solid 4-((2-(4-(2-acetyl-5-chlorophenyl)-3-methoxy-6-oxopyridazin-1(6*H*)-yl)-3-(4-(4-isopropyl-2-ox opiperazine)tert-butyl-1-yl)phenyl)propanamido)benzoic acid was obtained (yield: 23.0%). LCMS: RT = 2.88 min, $[M-H]^-$=684.21.

**Example 5**

**Synthesis of 4-(2-(4-(2-acetyl-5-chlorophenyl)-3-methoxy-6-oxopyrrolidine-1(6*H*)-yl)-3-(4-(4-(ethoxyethy l)-2-oxopyridine-1-yl)phenyl)propanamido)benzoic acid**

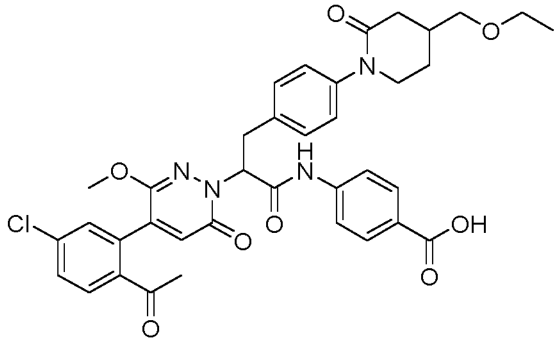

The specific synthesis route was as follows:

**Step A: Synthesis of 4-(hydroxymethyl)piperidine-2-one**

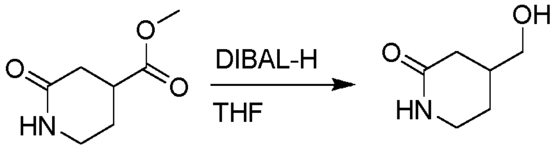


Methyl 2-oxopiperidine-4-carboxylate (1.0 g, 6.3 mmol) was dissolved in tetrahydrofuran/ methanol = 1:1 (100.0 ml). Subsequently, diisobutyl aluminum hydride (550 mg, 25.0 mmol) was added to the above solution. It was stirred at room temperature for 18 hours.

Methanol (100 ml) was added to the reaction solution to quench the reaction, and then concentrated under reduced pressure. The residue was purified by silica gel column chromatography (eluent: dichloromethane/methanol = 10/1) to obtain 500 mg of white oil 4-(hydroxymethyl)piperidine-2-one (yield: 61.0%). LCMS: RT = 1.53 min,$[M+H]^+$ = 130.10.

**Step B: Synthesis of (2-oxopyridine-4-yl)methyl 4-methylbenzene sulfonate**

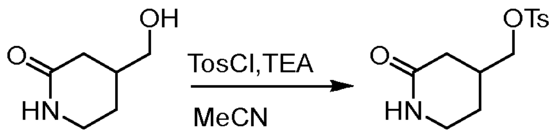


4-(hydroxymethyl)piperidine-2-one (500 mg, 3.8 mmol) was dissolved in acetonitrile (20.0 ml). Subsequently, 4-methylbenzenesulfonyl chloride (1.46 g, 7.7 mmol) and triethylamine (959 mg, 9.5 mmol) were added to the above solution. It was stirred at 50 °C for 3 hours.

The reaction solution was concentrated under reduced pressure, and the residue was purified by silica gel column chromatography (eluent: dichloromethane/methanol = 10/1) to obtain 530 mg of white solid (2-oxopyridine-4-yl)methyl 4-methylbenzene sulfonate (yield:48.0%). LCMS: RT = 3.04 min, $[M+H]^+$ = 284.15.

**Step C: Synthesis of 4-(ethoxymethyl)piperidine-2-one**

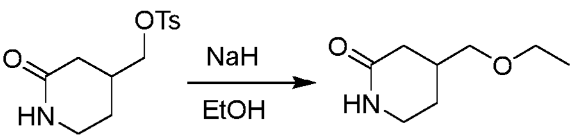

(2-oxopyridine-4-yl)methyl 4-methylbenzenesulfonate (380 mg, 1.3 mmol) was dissolved in ethanol (10.0 ml). Subsequently, sodium hydride (107 mg, 2.6 mmol) was added to the above solution. It was stirred at 50 °C for 3 hours.
The reaction solution was concentrated under reduced pressure, and the residue was purified by silica gel column chromatography (eluent: dichloromethane/methanol = 10/1) to obtain 180 mg of white solid 4-(ethoxymethyl)piperidine-2-one (yield: 85.0%). LCMS: RT = 2.95 min, $[M+H]^+$ = 158.06.

**Step D: Synthesis of 3-(4-bromophenyl)-2-((tert-butoxycarbonyl)amino)propanic acid**

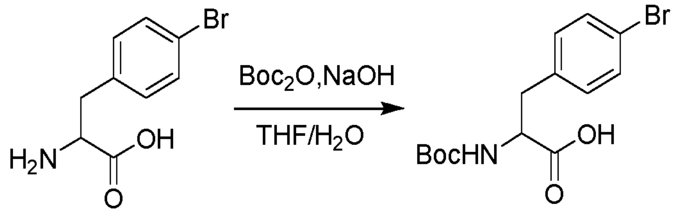


2-amino-3-(4-bromophenyl)propanic acid (4.0 g, 16 mmol) was dissolved in tetrahydrofuran/water = 2:1 (60.0 ml). Subsequently, sodium hydride (1.3 g, 32 mmol) and ditert-butyl dicarbonate (5.36 g, 24.5 mmol) were added to the above solution. It was stirred at room temperature for 3 hours.
Dilute hydrochloric acid solution (1.0 mol/L) was slowly added dropwise to the reaction solution to adjust the pH value to 3-4. White solid was precipitated. The mixed solution was extracted with ethyl acetate (30 ml × 3). The organic phases were combined. The combined organic phase was washed with saturated brine (30 ml × 3), then dried with anhydrous sodium sulfate, and concentrated under reduced pressure. The residue was purified by silica gel column chromatography (eluent: dichloromethane/methanol=20/1) to obtain 6.3 g of white solid 3-(4-bromophenyl)-2-((tert-butoxycarbonyl)amino)propanic acid (yield: 112.0%). LCMS: RT = 3.73min, $[M+H]^+$ = 345.04.

**Step E: Synthesis of tert-butyl 4-(3-(4-bromophenyl)-2-((tert-butoxycarbonyl)amino)propanamido)benzoate**

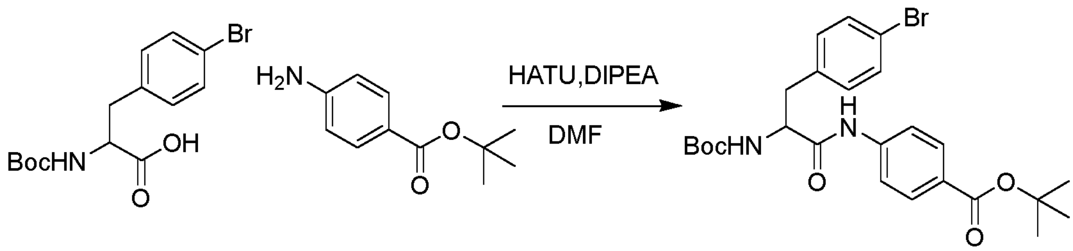


3-(4-bromophenyl)-2-((tert-butoxycarbonyl)amino)propanic acid (6.3 g, 18.3 mmol) was dissolved in N,N-dimethylformamide (60.0 ml). Subsequently, tert-butyl 4-aminobenzoate (3.9 g, 20.1 mmol), N,N-diisopropyl ethylamine (4.7 g, 36.6 mmol) and 2-(7-oxybenzotriazole)-N,N,N',N'-tetramethylurea hexafluorophosphate (10.4 g, 27.4 mmol) were added to the above solution. Nitrogen replacement was performed for three times, and the system was stirred at room temperature for 4 hours.
Saturated ammonium chloride solution was added to the reaction solution to quench the reaction. The mixed solution was extracted with ethyl acetate (100 ml × 3). The organic phases were combined. The organic phase was washed with saturated brine (100 ml), then dried with anhydrous sodium sulfate, and concentrated under reduced pressure. The residue was purified by silica gel column chromatography (eluent: ethyl acetate/petroleum ether=1/10). 7.8 g of white solid tert-butyl 4-(3-(4-bromophenyl)-2-((tert-butoxycarbonyl)amino)propanamido)benzoate was obtained (yield: 82.0%). LCMS: RT = 4.26 min, $[M+H]^+$ = 520.23.

**Step F: Synthesis of tert-butyl 4-(2-((tert-butoxycarbonyl) amino)-3-(4-(4-(ethoxymethyl)-2-oxopyridine-1-yl)phenyl)propanamido)benzoate**

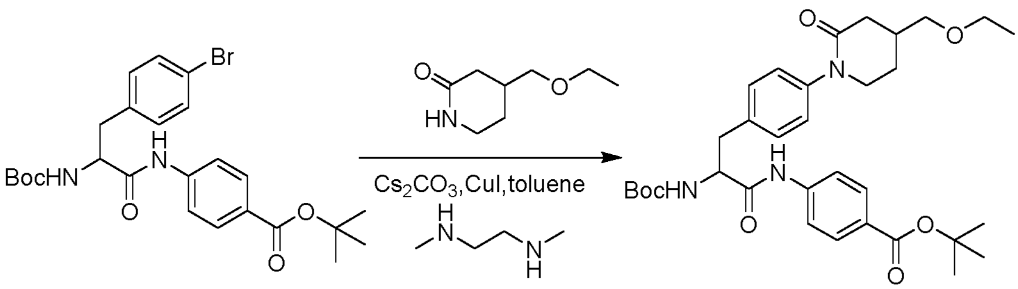


Tert-butyl 4-(3-(4-bromophenyl)-2-((tert-butoxycarbonyl)amino)propanamido)benzoate (2 g, 3.8 mmol) was dissolved in toluene (40.0 ml). Subsequently, 4-(ethoxymethyl)piperidine-2-one (302 mg, 1.9 mmol), cesium carbonate (1.25 g, 3.8 mmol), N,N-dimethylethyl-1,2-diamine (339 mg, 3.8 mmol), cuprous iodide (366 mg, 1.9 mmol) were added to the above solution, and nitrogen replacement was performed for three times. The system was stirred at 100 °C for 18 hours.

The reaction solution was diluted by adding water (100 ml). The mixed solution was extracted with ethyl acetate (20 ml × 3). The organic phases were combined. The combined organic phase was washed with saturated brine (20 ml × 3), then dried with anhydrous sodium sulfate, and concentrated under reduced pressure. The residue was purified by silica gel column chromatography (eluent: dichloromethane/methanol = 10/1) to obtain 330 mg of white solid tert-butyl 4-(2-((tert-butoxycarbonyl)amino)-3-(4-(4-(ethoxymethyl)-2-oxopyridine-1-yl)phenyl)propanam ido)benzoate (yield: 15.0%). LCMS: RT = 3.66min, $[M+H]^+$ = 596.11.

**Step G: Synthesis of 4-(2-amino-3-(4-(4-(ethoxymethyl)-2-oxopyridine-1-yl)phenyl)propanamido)benzoic acid**

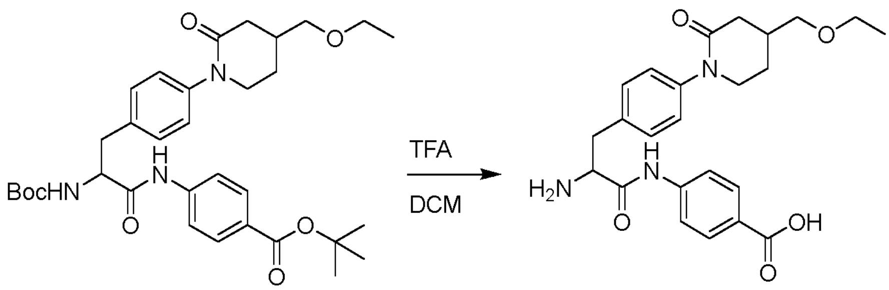


Tert-butyl 4-(2-((tert-butoxycarbonyl)amino)-3-(4-(4-(ethoxymethyl)-2-oxopyridine-1-yl)phenyl)propanam ido)benzoate (330 mg, 0.55 mmol) was dissolved in dichloromethane (4.0 ml). Subsequently, trifluoroacetic acid (1.0 ml) was added to the above solution and stirred at room temperature for 2 hours.

The reaction solution was concentrated under reduced pressure to obtain 230 mg of white solid 4-(2-amino-3-(4-(4-(ethoxymethyl)-2-oxopyridine-1-yl)phenyl)propanamido)benzoic acid (yield: 96.0%). LCMS: RT = 2.79 min, $[M+H]^+$ = 440.26.

**Step H: Synthesis of 4-(2-chloro-3-(4-(4-(ethoxymethyl)-2-oxopyridine-1-yl)phenyl) propanamido)benzoic acid**

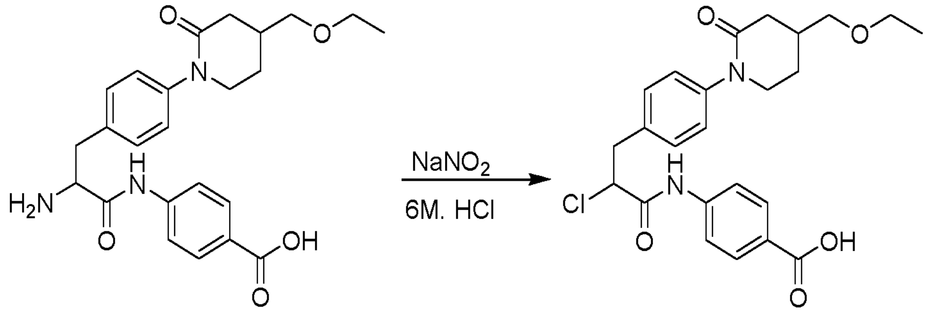


2-amino-3-(4-(4-(ethoxymethyl)-2-oxopyridine-1-yl)phenyl)propanamido)benzoic acid (230 mg, 0.52 mmol) was dissolved in hydrochloric acid (6 mol/L, 10 ml). Subsequently, sodium nitrite (80 mg, 1.10 mmol) was added to the above solution at 0 °C and stirred at 0 °C for 4 hours.

The reaction solution was extracted with dichloromethane (20 ml × 3). The organic phases were combined. The combined organic phase was washed with saturated brine (20 ml ), then dried with anhydrous sodium sulfate, and concentrated under reduced pressure. The residue was purified by silica gel column chromatography (eluent: dichloromethane/methanol = 10/1) to obtain 150 mg of white solid 4-(2-chloro-3-(4-(4-(ethoxymethyl)-2-oxopyridine-1-yl)phenyl) propanamido) benzoic acid(yield: 62.0%). LCMS: RT = 3.66 min, $[M+H]^+$ = 459.15.

**Step I: Synthesis of 4-(2-(4-(2-acetyl-5-chlorophenyl)-3-methoxy-6-oxopyrrolidine-1(6*H*)-yl)-3-(4-(4-(ethoxyethy l)-2-oxopyridine-1-yl)phenyl)propanamido)benzoic acid**

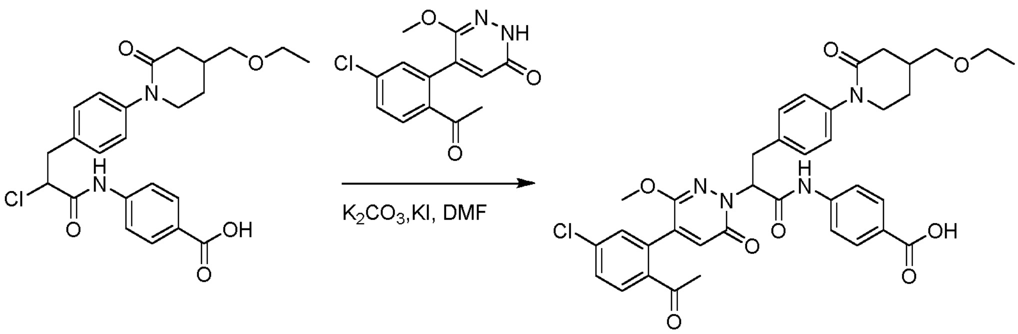


4-(2-chloro-3-(4-(4-(ethoxymethyl)-2-oxopyridine-1-yl)phenyl)propanamido)benzoic acid (150 mg, 0.33 mmol) was dissolved in N,N-dimethylformamide (10 ml). Subsequently, 5-(2-acetyl-5-chlorophenyl)-6-methylpyridine-3(2H)-one (139 mg, 0.50 mmol), potassium carbonate (92 mg, 0.66 mmol) and potassium iodide (5 mg, 0.030 mmol) were added to the above solution and stirred at 70 °C for 2 hours.

The reaction solution was diluted by adding saturated ammonium chloride aqueous solution (20 ml). The mixed solution was extracted with ethyl acetate (20 ml × 3). The organic phases were combined. The combined organic phase was washed with saturated brine (20 ml), then dried with anhydrous sodium sulfate, and concentrated under reduced pressure. The resulting residue was purified by HPLC to obtain 5 mg of 4-(2-(4-(2-acetyl-5-chlorophenyl)-3-methoxy-6-oxopyrrolidine-1(6*H*)-yl)-3-(4-(4-(ethoxyethyl)-2-oxopyridine-1-yl)phenyl)propanamido)benzoic acid (yield: 2.0%). LC-MS: RT = 3.78 min, $[M+H]^+$ = 701.37.

**Example 6**

**Synthesis of (S)-4-(2-(4-(2-acetyl-5-chlorophenyl)-3-(2-hydroxyethoxy)-6-oxopyridazine-1(6*H*)-yl)-3-phe nylpropanamido)benzoic acid**

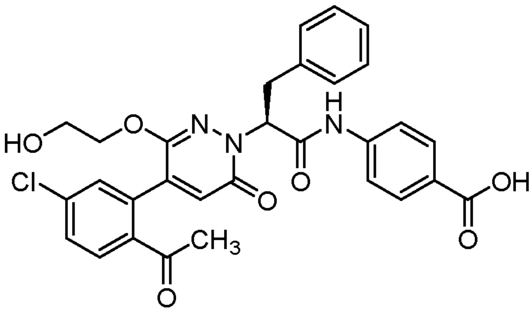

The specific synthesis route was as follows:

**Step A: Synthesis of 5-bromo-6-(2-((tert-butyl dimethylsilyl)oxy)ethoxy)-2-(4-methoxybenzyl)pyridazine-3(2*H*)-one**

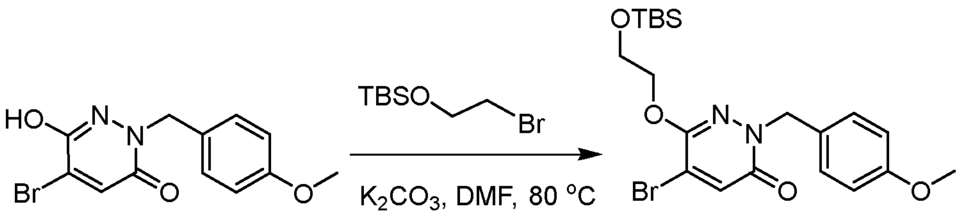


5-bromo-6-hydroxy-2-(4-methoxybenzyl)pyridazine-3(2*H*)-one (402 mg, 1.29 mmol), (2-bromoethoxy)(tert-butyl)dimethylsilane (1.24 g, 5.17 mmol) and potassium carbonate (714 mg, 5.17 mmol) were dissolved in N,N-dimethylformamide (5 ml), under nitrogen protection and stirred at 80 °C for 3 hours.

After the reaction solution was cooled, it was diluted with ethyl acetate (100 ml) and washed with water (20 ml × 2) and saturated brine (20ml), respectively. Then the organic layer was dried with the anhydrous sodium sulfate, filtered and concentrated, and the crude product was purified by silica gel column chromatography (petroleum ether: ethyl acetate = 5/1). 200 mg of white solid 5-bromo-6-(2-((tert-butyl dimethylsilyl)oxy)ethoxy)-2-(4-methoxybenzyl) pyridazine-3(2*H*)-one was obtained (yield: 33.0%). LC-MS: RT = 5.13 min, $[M+H]^+$ = 469.10.

**Step B: Synthesis of 5-(2-acetyl-5-chlorophenyl)-6-(2-((tert-butyl dimethylsilyl)oxy)ethoxy)-2-(4-methoxybenzyl)pyridazine-3(2*H*)-one**

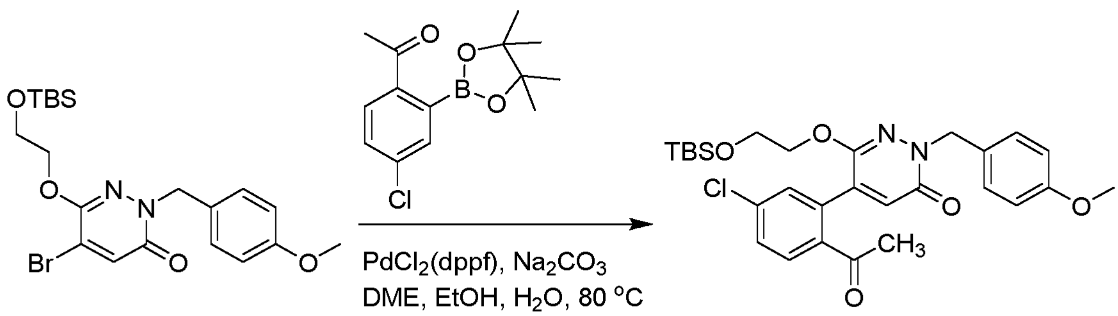


1-(4-chloro-2-(4,4,5,5-tetramethyl-1,3,2-dioxaborolan-2-yl)phenyl)ethan-1-one (156 mg, 0.55 mmol), 5-bromo-6-(2-((tert butyl dimethylsilyl)oxy)ethoxy)-2-(4-methoxybenzyl) pyridazine-3(2*H*)-one (200 mg, 0.43 mmol), [1,1'-bis(diphenylphosphino)ferrocene]palladium chloride (35 mg, 0.043 mmol) and sodium carbonate (92 mg, 0.86 mmol) were dissolved in a mixed solvent of ethylene glycol dimethyl ether (3 ml)/ethanol (0.4 ml)/water (0.4 ml) under nitrogen protection, reacted at 90 °C for 2.5 hours.

After the reaction solution was cooled, it was diluted with ethyl acetate (100 ml) and washed with water (20 ml × 2) and saturated brine (20ml), respectively. Then the organic layer was dried with the anhydrous sodium sulfate,

filtered and concentrated, and the crude product was purified by silica gel column chromatography (petroleum ether: ethyl acetate = 5/2). 85 mg of white solid 5-(2-acetyl-5-chlorphenyl)-6-(2-((tert-butyl dimethylsilyl)oxy)ethoxy)-2-(4-methoxybenzyl) pyridazine-3(2*H*)-one was obtained (yield: 36.0%). LC-MS: RT = 4.93 min, $[M+H]^+$ = 543.20.

**Step C: Synthesis of 5-(2-acetyl-5-chlorophenyl)-6-(2-hydroxyethoxy)pyridazine-3 (2*H*)-one**

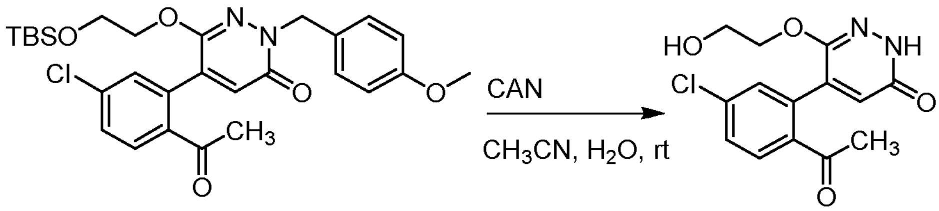


5-(2-acetyl-5-chlorophenyl)-6-(2-((tert-butyl dimethylsilyl)oxy)ethoxy)-2-(4-methoxybenzyl) pyridazine-3(2*H*)-one (85 mg, 0.156 mmol), ceric ammonium nitrate (258 mg, 0.470 mmol) were dissolved in a mixed solvent of acetonitrile (3.0 ml)/water (1.0 ml), and additional ceric ammonium nitrate (602 mg, 1.10 mmol) was added after three hours, the reaction was continued stirred at room temperature for 2.5 hours.

The reaction was quenched with water, diluted with ethyl acetate (100 ml) and washed with water (10 ml×2) and saturated brine (20ml) respectively. Then the organic layer was dried with the anhydrous sodium sulfate, filtered and concentrated, and the crude product was purified by silica gel column chromatography (petroleum ether: ethyl acetate = 2/1). 30 mg of white solid 5-(2-acetyl-5-chlorophenyl)-6-(2-hydroxyethoxy)pyridazine-3(2*H*)-one was obtained (yield: 63.0%). LC-MS: RT = 2.80 min, $[M+H]^+$ = 309.10.

**Step D: Synthesis of tert-butyl (S)-4-(2-(4-(2-acetyl-5-chlorophenyl)-3-(2-hydroxyethoxy)-6-oxopyridazine-1(6*H*)-yl)-3-phe nylpropanamido)benzoate**

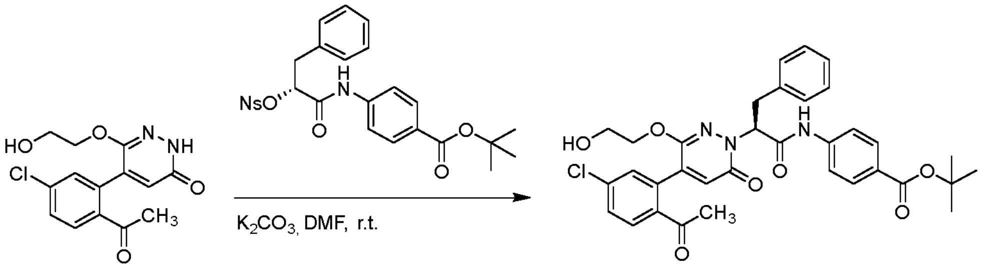


5-(2-acetyl-5-chlorophenyl)-6-(2-hydroxyethoxy)pyridazine-3(2*H*)-one (44 mg, 0.143 mmol), tert-butyl (R)-4-(2-((4-nitrophenyl)sulfonyl)oxy)-3-phenylpropanamido)benzoate (83 mg, 0.157 mmol) and potassium carbonate (40 mg, 0.286 mmol) were dissolved in N,N-dimethylformamide (3 ml) and stirred at 45 °C for 6.5 hours.

After the reaction solution was cooled, it was diluted with ethyl acetate (50 ml) and washed with water (10 ml × 2) and saturated brine (10ml) respectively. Then the organic layer was dried with the anhydrous sodium sulfate, filtered and concentrated, and the crude product was purified by silica gel column chromatography (petroleum ether: ethyl acetate = 1/1). 31 mg of white solid tert-butyl (S)-4-(2-(4-(2-acetyl-5-chlorophenyl)-3-(2-hydroxyethoxy)-6-oxopyridazine-1 (6*H*)-yl)-3-phenylpropanamido)benzoate was obtained (yield: 34.0%). LC-MS: RT = 4.19 min, $[M+H]^+$ = 632.16.

**Step E: Synthesis of (S)-4-(2-(4-(2-acetyl-5-chlorophenyl)-3-(2-hydroxyethoxy)-6-oxopyridazine-1(6*H*)-yl)-3-phenylpropanamido)benzoic acid**

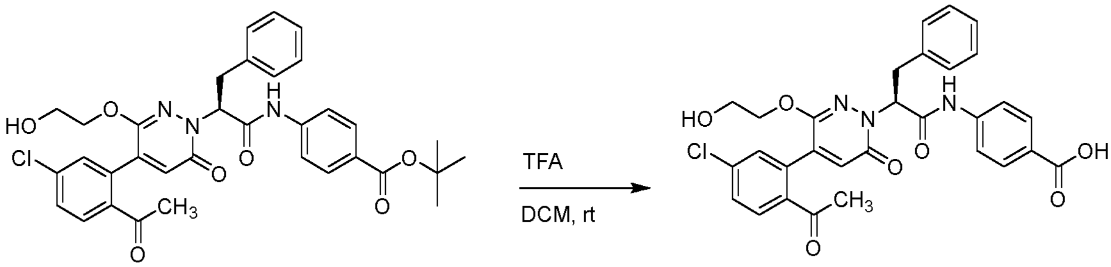


tert-butyl (S)-4-(2-(4-(2-acetyl-5-chlorophenyl)-3-(2-hydroxyethoxy)-6-oxopyridazine-1(6*H*)-yl)-3-phenyl propanamido)benzoate (31 mg, 0.049 mmol) was dissolved in dichloromethane (5.0 ml), trifluoroacetic acid (1.0 ml) was added dropwise at room temperature, and the reaction was continued at room temperature for 2.5 hours.

The reaction solution was concentrated by a rotary evaporator, vacuumed by an oil pump, and dissolved in methanol. Then n-hexane was added dropwise to the solution system, a large amount of solid was precipitated, stirred at room temperature for 1 hour and filtered to obtain 10 mg of white solid (S)-4-(2-(4-(2-acetyl-5-chlorophenyl)-3-(2-hydroxyethoxy)-6-oxypyridazine-1 (6*H*)-yl)-3-phenylpropanamido)benzoic acid (yield: 35.0%). LC-MS: RT = 3.64 min, $[M-H]^-$ = 574.10. $^1H$ NMR (500 MHz, DMSO) δ 12.73 (s, 1H), 10.51 (s, 1H), 8.01 (d, *J* = 8.4 Hz, 1H), 7.91 (d, *J* = 8.6 Hz, 2H), 7.72 (d, *J* = 8.6 Hz, 2H), 7.69 (dd, *J* = 8.4, 1.9 Hz, 1H), 7.50 (d, *J* = 1.8 Hz, 1H), 7.34 - 7.25 (m, 5H), 7.19 (t, *J* = 6.8 Hz, 1H), 6.89 (s, 1H), 5.75 (dd, *J* = 10.0, 5.0 Hz, 1H), 4.69 (t, *J* = 5.3 Hz, 1H), 4.08 (s, 1H), 4.06 - 3.94 (m, 2H), 3.52 (ddd, *J* = 24.4, 12.4, 7.8 Hz, 3H), 3.40 (dd, *J*= 14.1, 4.8 Hz, 1H), 2.55 (s, 3H).

**Example 7**

**Synthesis of 4-(2-(4-(2-acetyl-5-chlorophenyl)-3-methoxy-6-oxopyridazine-1(6*H*)-yl)-3-(p-tolyl)propana mido)benzoic acid**

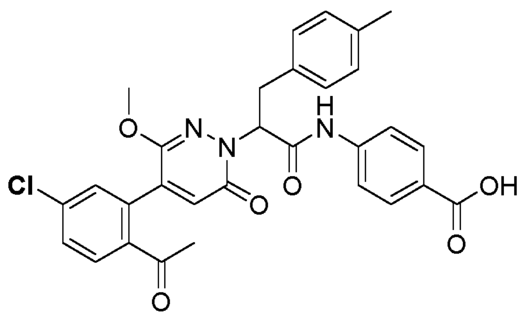

The specific synthesis route was as follows:

**Step A: Synthesis of tert-butyl 2-(4-(2-acetyl-5-chlorophenyl)-3-methoxy-6-oxopyridazine-1(6*H*)-yl)acetate**

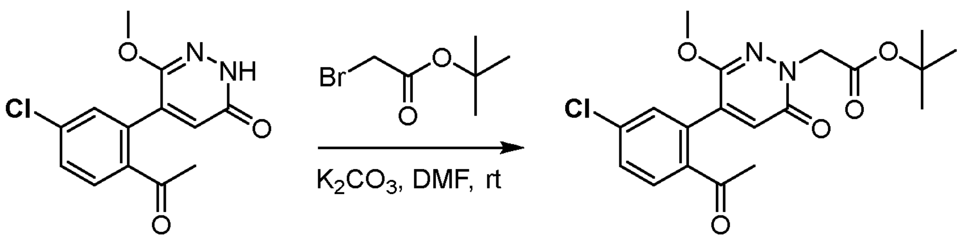


5-(2-acetyl-5-chlorophenyl)-6-methoxypyridazine-3(2*H*)-one (1.1 g, 3.9 mmol) and potassium carbonate (1.1 g, 7.9 mmol) were dissolved in N,N-dimethylformamide (10 ml), under nitrogen protection, and tert-butyl 2-bromoacetate (922 mg, 4.7 mmol) was added at room temperature, and stirred for 4 hours.

The reaction solution was quenched with water and extracted with ethyl acetate (50 ml × 3), the organic phase was washed with saturated brine (20 ml × 2), dried with anhydrous sodium sulfate, filtered and concentrated. The crude product was purified by silica gel column chromatography (petroleum ether: ethyl acetate = 2/1). 1.3 g of tert-butyl 2-(4-(2-acetyl-5-chlorophenyl)-3-methoxy-6-oxopyridazine-1(6*H*)-yl)acetate was obtained (yield: 85.0%). MS (ESI) M/Z: 393.3 $[M+H]^+$

**Step B: Synthesis of tert-butyl 2-(4-(2-acetyl-5-chlorophenyl)-3-methoxy-6-oxopyridazine-1(6*H*)-yl)-3-(p-tolyl)propanoate**

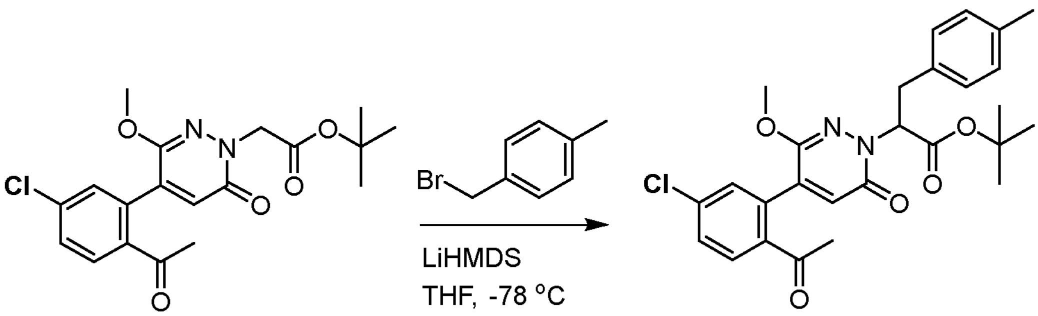


tert-butyl 2-(4-(2-acetyl-5-chlorophenyl)-3-methoxy-6-oxopyridazine-1(6*H*)-yl)acetate (70 mg, 0.18 mmol) and 1-(bromomethyl)-4-methylbenzene (66 mg, 0.35 mmol) were dissolved in anhydrous tetrahydrofuran (4.0 ml), cooled to -78 °C, stirred for 10 minutes, bis(trimethylsilyl)aminolithium (700 μL, 1.0 mol/L, dissolved in tetrahydrofuran) was added dropwise and stirred at -78 °C for 2 hours.

The reaction solution was quenched with water and extracted with ethyl acetate (20 ml × 3), the organic phase was washed with saturated brine (10 ml × 2), dried with anhydrous sodium sulfate, filtered and concentrated. The crude product was purified by silica gel column chromatography (petroleum ether: ethyl acetate = 2/1). 80 g of tert-butyl 2-(4-(2-acetyl-5-chlorophenyl)-3-methoxy-6-oxopyridazine-1(6*H*)-yl)-3-(p-tolyl)propanoate was obtained (yield: 89.0%).

**Step C: Synthesis of 2-(4-(2-acetyl-5-chlorophenyl)-3-methoxy-6-oxopyridazine-1 (6*H*)-yl)-3-(p-tolyl)propanic acid**

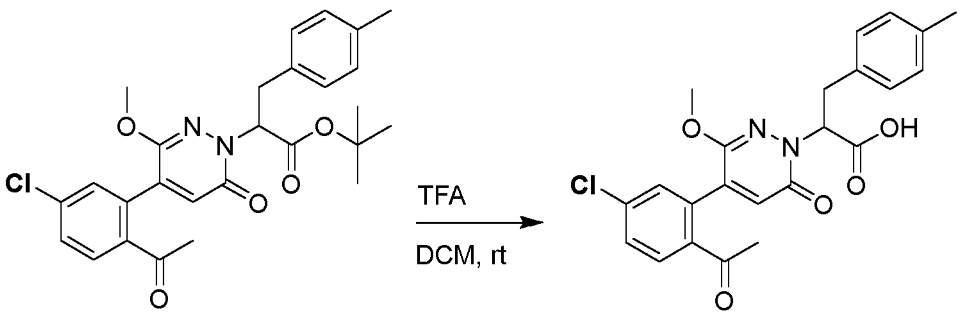


Tert-butyl 2-(4-(2-acetyl-5-chlorophenyl)-3-methoxy-6-oxopyridazine-1(6*H*)-yl)-3-(p-tolyl) propanoate (80 mg, 0.16 mmol) was dissolved in dichloromethane (1.5 ml), trifluoroacetic acid (0.5 ml) was added dropwise at room temperature, and the reaction was continued at room temperature for 4 hours.

The reaction solution was concentrated by a rotary evaporator and further dried by an oil pump to obtain 70 mg of 2-(4-(2-acetyl-5-chlorophenyl)-3-methoxy-6-oxopyridazine-1 (6*H*)-yl)-3-(p-tolyl)propanic acid (yield: 99.0%).

**Step D: Synthesis of tert-butyl 4-(2-(4-(2-acetyl-5-chlorophenyl)-3-methoxy-6-oxopyridazine-1(6*H*)-yl)-3-(p-tolyl)propanamido)benzoate**

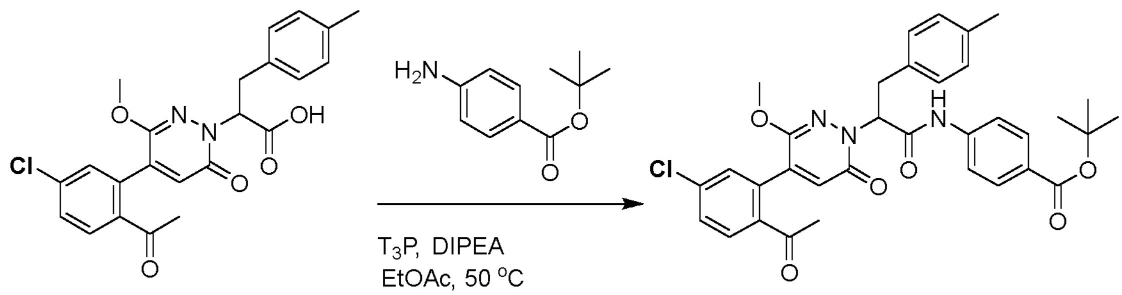


-(4-(2-acetyl-5-chlorophenyl)-3-methoxy-6-oxopyridazine-1(6*H*)-yl)-3-(p-tolyl)propanic acid (25 mg, 0.057 mmol), tert-butyl 4-aminobenzoate (11 mg, 0.057 mmol), 1-propyl phosphorous anhydride (91 mg, 0.285 mmol) and N,N-diisopropyl ethyl amine (29 μL, 0.171 mmol) were dissolved in ethyl acetate (2.0 ml), stirred at 50 ° C for 3 hours.

After the reaction solution was cooled, it was diluted with ethyl acetate (50 ml) and washed with water (10 ml × 2) and saturated brine (10ml), respectively. Then the organic layer was dried with the anhydrous sodium sulfate, filtered and concentrated, and the crude product was purified by silica gel column chromatography (petroleum ether: ethyl acetate = 3/1). 11 mg of tert-butyl 4-(2-(4-(2-acetyl-5-chlorophenyl)-3-methoxy-6-oxypyridazine-1(6*H*)-yl)-3-(p-tolyl)propanamid o) benzoate was obtained (yield: 32.0%). LC-MS: RT = 4.67 min, $[M+H]^+$ = 616.19.

**Step E: Synthesis of 4-(2-(4-(2-acetyl-5-chlorophenyl)-3-methoxy-6-oxopyridazine-1(6*H*) -yl)-3-(p-tolyl)propanamido)benzoic acid**

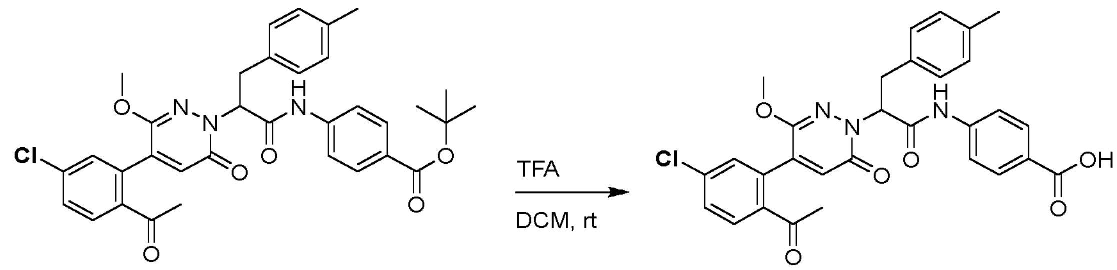


Tert-butyl 4-(2-(4-(2-acetyl-5-chlorophenyl)-3-methoxy-6-oxopyridazine-1(6H)-yl)-3-(p-tolyl)propanamid o) benzoate (11 mg) was dissolved in dichloromethane (2.5 ml), trifluoroacetic acid (0.5 ml) was added dropwise at room temperature, and the reaction was stirred at room temperature for 50 minutes.

The reaction solution was concentrated by a rotary evaporator, vacuumed by an oil pump, and dissolved in methanol. Then n-hexane was added dropwise to the solution system, a large amount of solid was precipitated, and stirred at room temperature for 1 hour and filtered to obtain 7.6 mg of white solid 4-(2-(4-(2-acetyl-5-chlorophenyl)-3-methoxy-6-oxopyridazine-1(6*H*)-yl)-3-(p-tolyl)propanamid o) benzoic acid (yield: 76.0%). LC-MS: RT = 4.08 min, $[M+H]^+$ = 560.10. $^1$H NMR (500 MHz, DMSO-$d_6$, ppm) δ 12.72 (s, 1H), 10.52 (s, 1H), 7.99 (d, *J* = 8.4 Hz, 1H), 7.91 (d, *J* = 8.7 Hz, 2H), 7.73 (d, *J* = 8.8 Hz, 2H), 7.69 (dd, *J* = 8.3, 2.1 Hz, 1H), 7.51 (d, *J* = 2.1 Hz, 1H), 7.20 (d, *J* = 8.0 Hz, 2H), 7.09 (d, *J* = 7.9 Hz, 2H), 6.91 (s, 1H), 5.71 (dd, *J* = 10.3, 4.7 Hz, 1H), 3.67 (s, 3H), 3.48 (dd, *J* = 14.1, 10.4 Hz, 1H), 3.37-3.32 (m, 1H), 2.53 (s, 3H), 2.24 (s, 3H).

**Example 8**

**Synthesis of 4 (2-(4-(2-acetyl-5-chlorophenyl)-3-methoxy-6-oxopyridazine-1(6*H*) -yl)-3-(m-tolyl)propanamido)benzoic acid**

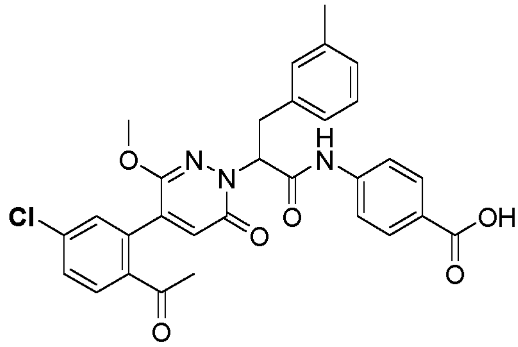

The specific synthesis route was as follows:

**Step A: Synthesis of tert-butyl 2-(4-(2-acetyl-5-chlorophenyl)-3-methoxy-6-oxopyridazine-1(6*H*)-yl)-3-(m-tolyl)propanoate**

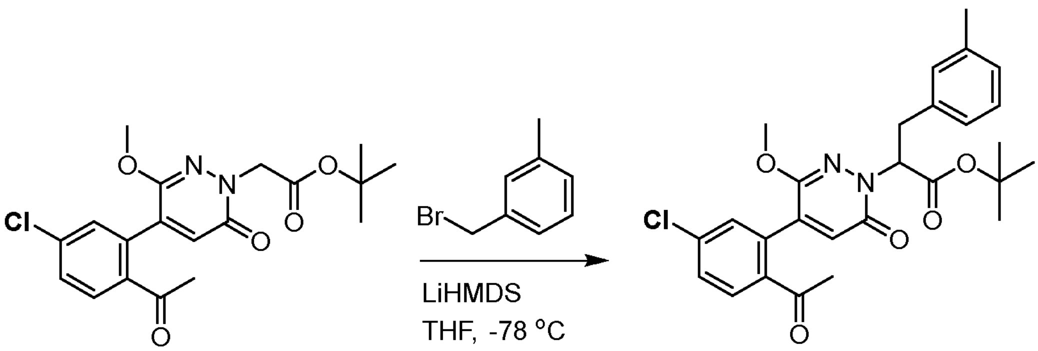


Tert-butyl 2-(4-(2-acetyl-5-chlorophenyl)-3-methoxy-6-oxopyridazine-1(6*H*)-yl)acetate (70 mg, 0.18 mmol) and 1-(bromomethyl)-3-methylbenzene (66 mg, 0.35 mmol) were dissolved in anhydrous tetrahydrofuran (4 ml), cooled to -78 °C, stirred for 10 minutes, bis (trimethylsilyl)aminolithium (700 μL, 1.0 mol/L, dissolved in tetrahydrofuran) was added dropwise and stirred at -78 °C for 2 hours.

The reaction solution was quenched with water and extracted with ethyl acetate (20 ml × 3), the organic phase was washed with saturated brine (10 ml × 2), dried with anhydrous sodium sulfate, filtered and concentrated. The crude product was purified by silica gel column chromatography (petroleum ether: ethyl acetate = 2/1). 80 g of tert-butyl 2-(4-(2-acetyl-5-chlorophenyl)-3-methoxy-6-oxopyridazine-1(6*H*)-yl)-3-(m-tolyl)propanoate was obtained (yield: 89.0%).

**Step B: Synthesis of 2-(4-(2-acetyl-5-chlorophenyl)-3-methoxy-6-oxopyridazine-1(6*H*) -yl)-3-(m-tolyl)propanic acid**

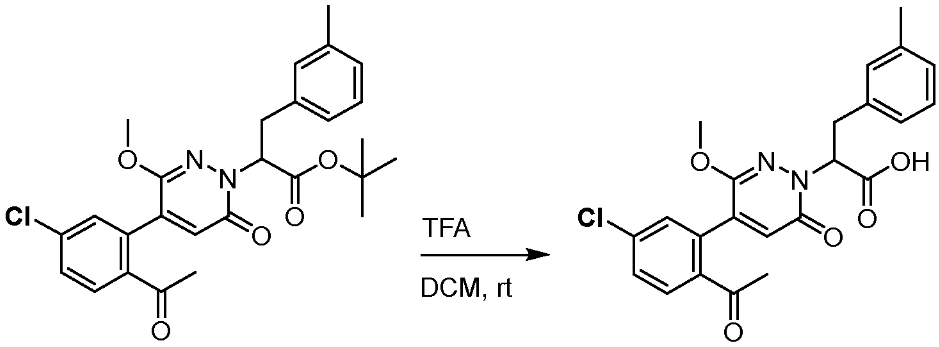


Tert-butyl 2-(4-(2-acetyl-5-chlorophenyl)-3-methoxy-6-oxopyridazine-1(6*H*)-yl)-3-(m-tolyl) propanoate (80 mg, 0.16 mmol) was dissolved in dichloromethane (1.5 ml), trifluoroacetic acid (0.5 ml) was added dropwise at room temperature, and the reaction was continued at room temperature for 4 hours.

The reaction solution was concentrated by a rotary evaporator and dried by an oil pump to obtain 70 mg of 2-(4-(2-acetyl-5-chlorophenyl)-3-methoxy-6-oxopyridazine-1(6*H*)-yl)-3-(m-tolyl) propanic acid (yield: 99.0%).

**Step C: Synthesis of tert-butyl 4-(2-(4-(2-acetyl-5-chlorophenyl)-3-methoxy-6-oxopyridazine-1(6*H*)-yl)-3-(m-tolyl)propanamido)benzoate**

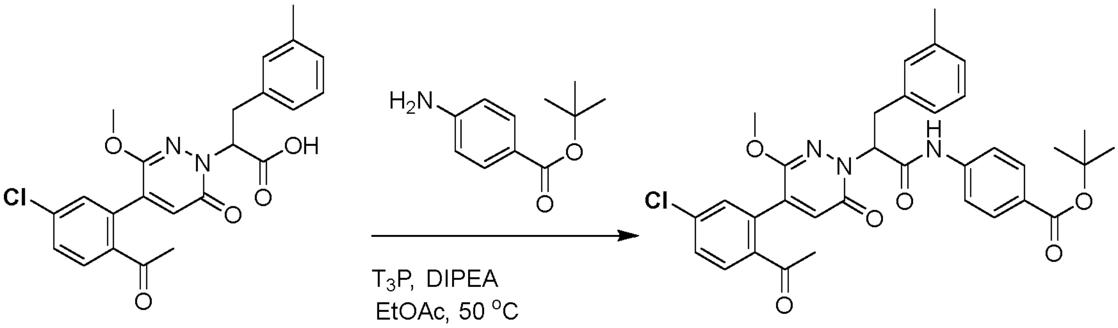


2-(4-(2-acetyl-5-chlorophenyl)-3-methoxy-6-oxopyridazine-1(6*H*)-yl)-3-(m-tolyl)propanic acid (38 mg, 0.086 mmol), tert-butyl 4-aminobenzoate (17 mg, 0.086 mmol), 1-propyl phosphorous anhydride (137 mg, 0.430 mmol) and N,N-diisopropyl ethyl amine (43 μL, 0.258 mmol) were dissolved in ethyl acetate (2.0 ml), stirred at 50 °C for 3 hours.

After the reaction solution was cooled, it was diluted with ethyl acetate (50 ml) and washed with water (10 ml × 2) and saturated brine (10ml), respectively. Then the organic layer was dried with the anhydrous sodium sulfate, filtered and concentrated, and the crude product was purified by silica gel column chromatography (petroleum ether: ethyl acetate = 3/1). 19 mg of tert-butyl 4-(2-(4-(2-acetyl-5-chlorophenyl)-3-methoxy-6-oxypyridazine-1(6*H*)-yl)-3-(m-tolyl)propanamid o)benzoate was obtained (yield: 36.0%). LC-MS: RT = 4.67 min, $[M+H]^+$ = 616.19.

**Step D: Synthesis of 4-(2-(4-(2-acetyl-5-chlorophenyl)-3-methoxy-6-oxopyridazine-1 (6*H*)-yl)-3-(m-tolyl)propanamido)benzoic acid**

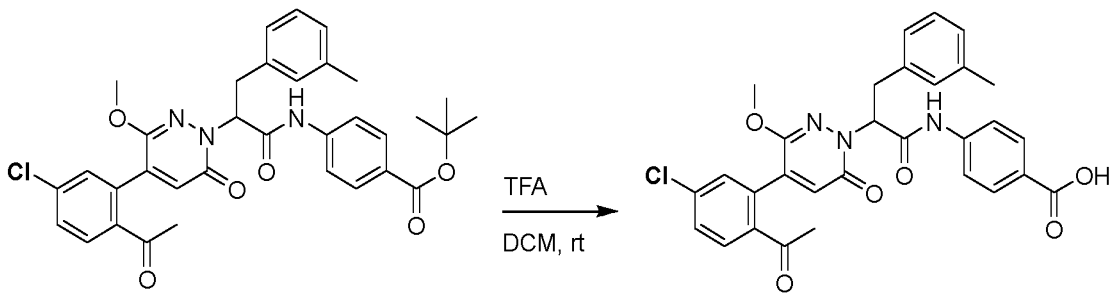


Tert-butyl 4-(2-(4-(2-acetyl-5-chlorophenyl)-3-methoxy-6-oxopyridazine-1(6*H*)-yl)-3-(m-tolyl)propanamido)benzoate (19 mg) was dissolved in dichloromethane (2.5 ml), trifluoroacetic acid (0.5 ml) was added dropwise at room temperature, and the reaction was stirred at room temperature for 2 hours.

The reaction solution was concentrated by a rotary evaporator, vacuumed by an oil pump, and dissolved in methanol. Then n-hexane was added dropwise to the solution system, a large amount of solid was precipitated, and stirred at room temperature for 1 hour and filtered to obtain 9.5 mg of white solid 4-(2-(4-(2-acetyl-5-chlorophenyl)-3-methoxy-6-oxopyridazine-1 (6*H*)-yl)-3-(m-tolyl)propanamido)benzoic acid (yield: 55.0%). LC-MS: RT = 4.08 min, $[M+H]^+$ = 560.12. $^1H$ NMR (400 MHz, DMSO-$d_6$, ppm) δ12.73 (s, 1H), 10.53 (s, 1H), 7.99 (d, *J* = 8.3 Hz, 1H), 7.91 (d, *J* = 8.6 Hz, 2H), 7.71 (dd, *J* = 12.2, 8.7 Hz, 3H), 7.51 (s, 1H), 7.21 - 7.09 (m, 3H), 7.00 (d, *J* = 7.2 Hz, 1H), 6.91 (s, 1H), 5.71 (dd, *J* = 10.3, 4.6 Hz, 1H), 3.69 (s, 3H), 3.49 (dd, *J* = 13.9, 10.4, 1H), 3.38-3.32 (m, 1H), 2.53 (s, 3H), 2.26 (s, 3H).

**Example 9**

**Synthesis of (S)-2-(4-(2-acetyl-5-chlorophenyl)-3-methoxy-6-oxopyridazine-1 (6*H*)-yl)-3-phenyl-N-(quinoxaline-6-yl)propanamide**

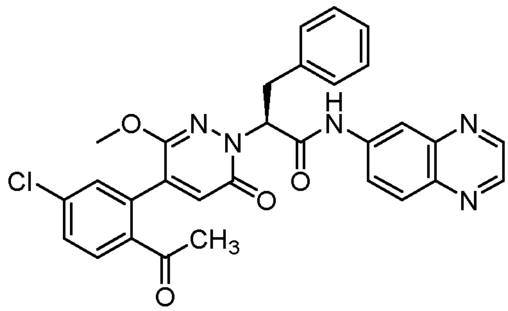

The specific synthesis route was as follows:

**Step A: Synthesis of methyl (R)-3-phenyl-2-((trifluoromethyl)sulfonyl)oxy)propanoate**

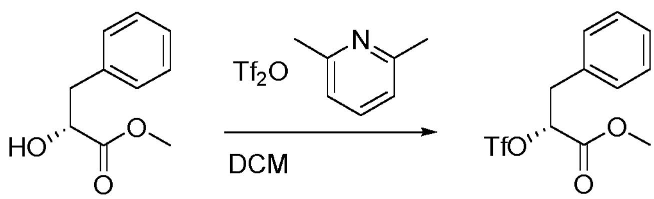


Methyl (R)-2-hydroxy-3-phenylpropanoate (5.00 g, 27.8 mmol) was dissolved in dichloromethane (30.0 ml), 2,6-dimethylpyridine (3.47 g, 33.3 mmol) was added, then trifluoromethylsulfonic anhydride (5.4 ml, 33.3 mmol) was added slowly at -10 °C, and stirred for 30 minutes.

Water (20 ml) was added to the reaction solution to quench the reaction, ethyl acetate (100 ml) was added to

the reaction solution, and the mixture was washed with saturated brine (20 ml × 3), then dried with anhydrous sodium sulfate, and concentrated under reduced pressure. The residue was purified by silica gel column chromatography (eluent: ethyl acetate/petroleum ether=100/5). 5.1 g of colorless oil methyl (R)-3-phenyl-2-((trifluoromethyl)sulfonyl)oxy) propanoate was obtained (yield: 59.0%). LCMS: RT = 3.24 min, $[M+H]^+$ = 313.28.

**Step B: Synthesis of methyl (S)-2-(4-(2-acetyl-5-chlorophenyl)-3-methoxy-6-oxopyridazine-1(6*H*)-yl)-3-phenylpropanoa** te

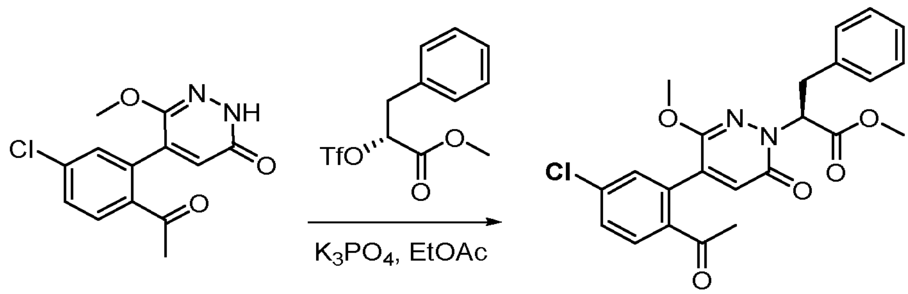


5-(2-acetyl-5-chlorophenyl)-6-methoxypyridine-3(2H)-one (1.2 g, 4.3 mmol) and potassium phosphate (5.4 g, 25.8 mmol) were dissolved in ethyl acetate (20.0 ml). Subsequently, methyl (R)-3-phenyl-2-((trifluoromethyl)sulfonyl)oxy)propanoate (6.7 g, 21.6 mmol) was added to the above solution and stirred at room temperature for 3 hours.

Water (5 ml) was added to the reaction solution to quench the reaction. Ethyl acetate (100 ml) was added to the solution and the mixture was washed with saturated brine (20 ml × 3), then dried with anhydrous sodium sulfate, and concentrated under reduced pressure. The residue was purified by silica gel column chromatography (eluent: ethyl acetate/petroleum ether = 1/1). 1.2 g of yellow solid methyl (S)-2-(4-(2-acetyl-5-chlorophenyl)- 3-methoxy-6-oxopyridazine-1(6*H*)-yl) -3-phenylpropanoate was obtained (yield: 63.0%). LCMS: RT = 4.08 min, $[M+H]^+$ = 441.11.

**Step C: Synthesis of methyl (S)-2-(4-(2-acetyl-5-chlorophenyl)-3-methoxy-6-oxopyridazine-1(6*H*)-yl)-3-phenylpropanic acid**

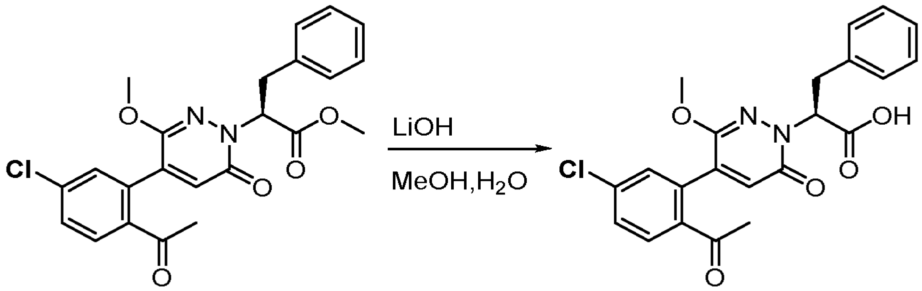


Methyl (S)-2-(4-(2-acetyl-5-chlorophenyl)-3-methoxy-6-oxopyridazine-1(6*H*)-yl)-3-phenylpropanoate (1.20 g, 2.72 mmol) was dissolved in methanol (15.0 ml) and water (6.0 ml). Subsequently, Lithium hydroxide monohydrate (217 mg, 5.44 mmol) was added to the above solution, stirred at room temperature for 2 hours.

Dilute hydrochloric acid solution (1.0 mol/L) was slowly added dropwise to the reaction solution to adjust the pH value to 4-5. Ethyl acetate (100 ml) was added to the solution and the mixture was washed with saturated brine (20 ml × 3), then dried with anhydrous sodium sulfate, and concentrated under reduced pressure. The residue was purified by silica gel column chromatography (eluent: ethyl acetate/petroleum ether = 1/10). 460 g of yellow solid (S)-2-(4-(2-acetyl-5-chlorophenyl)-3-methoxy-6-oxopyridazine-1(6*H*)-yl)-3-phenylpropanic acid was obtained (yield: 39.6%). LCMS: RT = 3.88 min, $[M+H]^+$ = 427.09.

**Step D: Synthesis of (S)-2-(4-(2-acetyl-5-chlorophenyl)-3-methoxy-6-oxopyridazine-1 (6H)-yl)-3-phenyl-N-(quinoxaline-6-yl)propanamide**

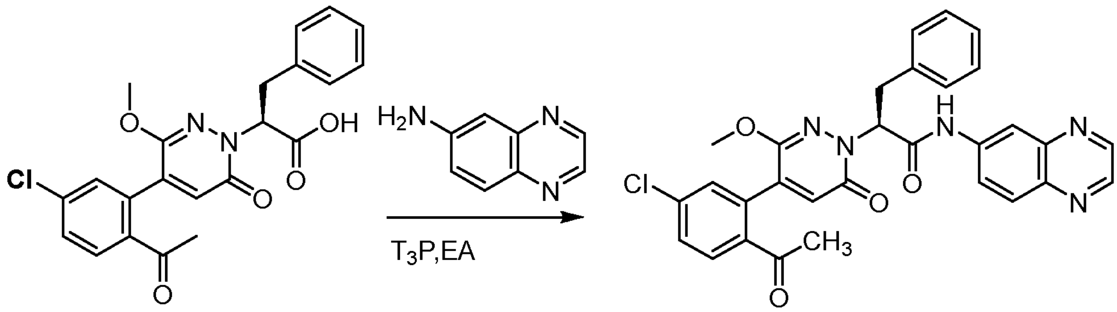


(S)-2-(4-(2-acetyl-5-chlorophenyl)-3-methoxy-6-oxopyridazine-1(6H)-yl)-3-phenylpropani c acid (59 mg, 0.138 mmol) and quinoxaline-6-amine (24 mg, 0.166 mmol) were dissolved in ethyl acetate solution (5.0 ml), N,N-Diisopropylethylamine (89 mg, 0.690 mmol) was added. Subsequently, 1-propylphosphorous anhydride (175 mg, 0.552 mmol)was added to the above solution. The reaction solution was heated to 60 ° C and stirred for 8 hours.

The reaction solution was cooled to room temperature and concentrated under reduced pressure. The residue was purified by TLC plate (methanol / dichloromethane = 1:16) to obtain 80 mg of crude product, which was purified by preparative high performance liquid chromatography. The separation conditions are as follows: chromatographic column: X select C18 19 mm * 150 mm; Mobile phase: water (containing 0.05% trifluoroacetic acid) and acetonitrile; Flow rate: 25 ml / min; Gradient: acetonitrile rises from 5% to 100% in 7 minutes; Detection wavelength: 254 nm. After purification, 9 mg of yellow solid (S)-2-(4-(2-acetyl-5-chlorophenyl)-3-methoxy-6-oxypyridazine-1(6H)-yl)-3-phenyl-N-(quinoxal ine-6-yl) Propanamide was purified (yield: 11.8%). LCMS: RT = 3.96 min, $[M+H]^+$ = 554.15. $^1$H NMR (500 MHz, DMSO) 8 10.71 (s, 1H), 8.88 (d,J= 1.8 Hz, 1H), 8.82 (d, *J* = 1.8 Hz, 1H), 8.49 (d, *J* = 2.2 Hz, 1H), 7.98 (ddd, *J* = 18.2, 11.4, 5.7 Hz, 3H), 7.68 (dd, *J* = 8.3, 2.1 Hz, 1H), 7.50 (d, *J* = 2.1 Hz, 1H), 7.31 (t, *J* = 6.9 Hz, 2H), 7.23 (dt, *J* = 36.4, 7.1 Hz, 3H), 6.93 (d, *J* = 7.2 Hz, 1H), 5.77 (dd, *J* = 9.8, 5.1 Hz, 1H), 3.66 (s, 3H), 3.51 (dd, *J* = 14.0, 10.2 Hz, 1H), 3.41 (dd, *J* = 14.1, 4.8 Hz, 1H) 2.52 (s, 3H).

**Example 10**

**Synthesis of (S)-2-(4-(2-acetyl-5-chlorophenyl)-3-methoxy-6-oxopyridazine-1 (6H)-yl)-N-(2-methyl-2h-indazole-5-yl)-3-phenylpropanamide**

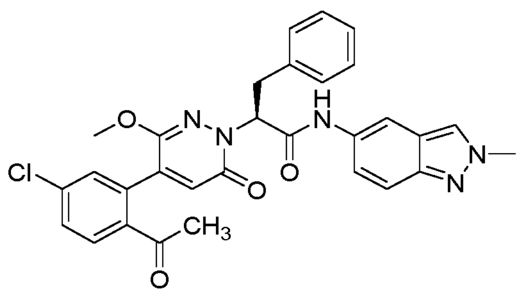

**Step A: Synthesis of (S)-2-(4-(2-acetyl-5-chlorophenyl)-3-methoxy-6-oxopyridazine-1(6H)-yl)-N-(2-methyl-2h-in dazole-5-yl)-3-phenylpropanamide**

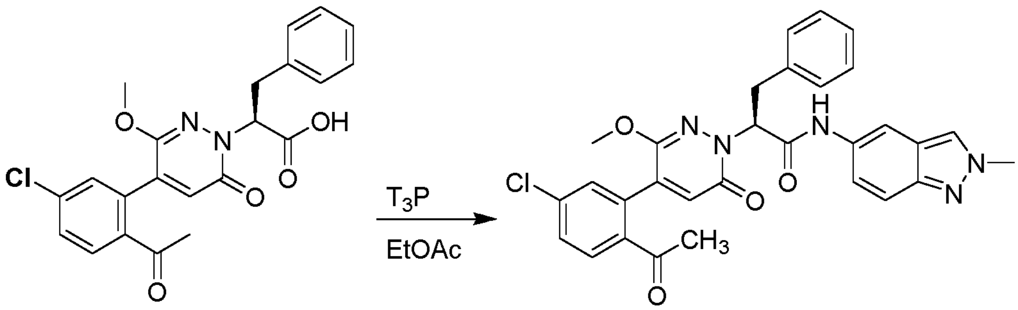


(S)-2-(4-(2-acetyl-5-chlorophenyl)-3-methoxy-6-oxopyridazine-1 (6H)-yl)-3-phenylpropanic acid (83 mg, 0.195 mmol) and quinoxaline-6-amine (28.6 mg, 0.195 mmol) were dissolved in ethyl acetate solution (3.0 ml), N,N-Diisopropylethylamine (8251 mg, 1.95 mmol) was added. Subsequently, 1-propylphosphorous anhydride (248 mg, 0.78 mmol)was added to the above solution. The reaction solution was heated to 60 ° C and stirred for 18 hours.

The reaction solution was cooled to room temperature and concentrated under reduced pressure. The residue was purified by TLC plate (methanol / dichloromethane = 1:15 AM) to obtain 80 mg of crude product, which was purified by preparative high performance liquid chromatography. The separation conditions are as follows: chromatographic column: X select C18 19 mm * 150 mm; Mobile phase: water (containing 0.05% trifluoroacetic acid) and acetonitrile; Flow rate: 25 ml / min; Gradient: acetonitrile rises from 5% to 100% in 7 minutes; Detection wavelength: 254 nm. After purification, 50 mg of yellow solid (S)-2-(4-(2-acetyl-5-chlorophenyl)-3-methoxy-6-oxopyridazine-1(6H)-yl)-N-(2-methyl-2h-indaz ole-5-yl)-3-phenylpropanamide was purified (yield: 46.3%). LCMS: RT= 3.87 min, $[M+H]^+$ = 556.16. $^1H$ NMR (400 MHz, DMSO) $\delta$ 10.08 (s, 1H), 8.24 (s, 1H), 8.07 (s, 1H), 7.98 (d, $J$ = 8.4 Hz, 1H), 7.67 (dd, $J$ = 8.3, 2.2 Hz, 1H), 7.51 (dd, $J$ = 18.5, 5.6 Hz, 2H), 7.24 (ddt, $J$ = 32.0, 30.0, 6.9 Hz, 6H), 6.89 (s, 1H), 5.73 (dd, $J$ = 10.2, 4.8 Hz, 1H), 4.11 (s, 3H), 3.67 (s, 3H), 3.51 (dd, $J$ = 14.0, 10.2 Hz, 1H), 3.41 (dd, $J$ = 14.1, 4.8 Hz, 1H), 2.53 - 2.50 (m, 3H).

**Example 11**

**Synthesis of (S)-2-(4-(2-acetyl-5-chlorophenyl)-3-methoxy-6-oxopyridazin-1(6*H*)-yl)-*N*-(1H-benzo[d]imi dazol-5-yl)-3-phenylpropanamide**

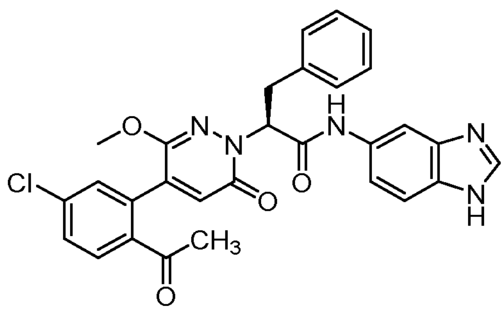

The specific synthetic route was as follows.

**Step A: Synthesis of 1*H*-benzo[d]imidazol-5-amine**

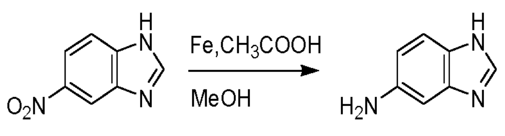


5-nitro-1***H***-benzo[d]imidazole (200 mg, 1.23 mmol) was dissolved in methanol (10 ml), added with acetic acid (1 ml) and iron powder (687 mg, 12.3 mmol), heated to 80°C and reacted for 4 hours.

Saturated sodium bicarbonate was added to the reaction solution until pH=7-8. The mixed solution was extracted

with ethyl acetate (100 ml × 3) and concentrated. 150 mg of yellow solid 1***H***-benzo[d]imidazol-5-amine was obtained (yield: 92.0%). LCMS:RT= 0.60 min, $[M+H]^+$ = 134.06.

**Step B: Synthesis of (S)-2-(4-(2-acetyl-5-chlorophenyl)-3-methoxy-6-oxopyridazin-1(6*H*)-yl)-*N*-(1H-benzo[d]imi dazol-5-yl)-3-phenylpropanamide**

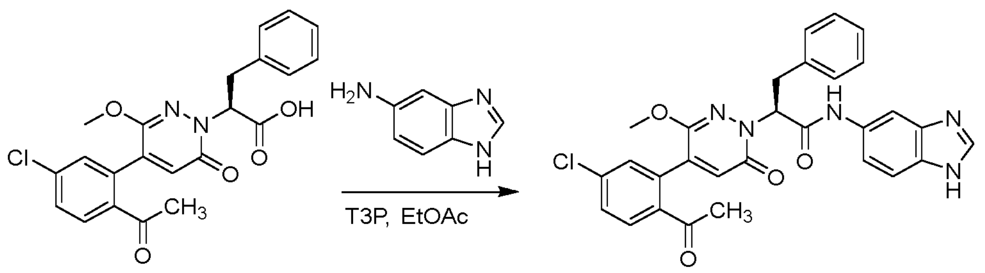


(S)-2-(4-(2-acetyl-5-chlorophenyl)-3-methoxy-6-oxopyridazin-1(6*H*)-yl)-3-phenylpropanic acid (65 mg, 0.150 mmol) and 1***H***-benzo[d]imidazol-5-amine (22 mg, 0.167mmol) were dissolved in ethyl acetate (3.0 ml), and N, N-diisopropylethylamine (193 mg, 1.5 mmol) was added. Subsequently, 1-propyl phosphonic anhydride (190 mg, 0.6 mmol) was added to the above solution. The reaction solution was heated to 60°C and stirred for 4 hours.

The reaction solution was cooled to room temperature and concentrated under reduced pressure. The crude product was purified by preparative high performance liquid chromatography. Separation conditions were as follows: chromatographic column: X select C18 19 mm * 150 mm; mobile phase: water (comprising 0.05% trifluoroacetic acid) and acetonitrile; flow rate: 25 ml/min; gradient: acetonitrile increasing from 5% to 100% in 7 minutes; detection wavelength: 254 nm. Upon purification, 6.05 mg of yellow solid (S)-2-(4-(2-acetyl-5-chlorophenyl)-3-methoxy-6-oxopyridazin-1(6*H*)-yl)-*N*-(1H-benzo[d]imidaz ol-5-yl)-3-phenylpropanamide was obtained (yield: 7.0%). LCMS: RT= 3.07 min, $[M+H]^+$ = 542.15.

**Example 12**

**Synthesis of (S)-2-(4-(2-acetyl-5-chlorophenyl)-3-methoxy-6-oxopyridazin-1(6*H*)-yl)-*N*-(2-oxodihydroind ol-5-yl)-3-phenylpropanamide**

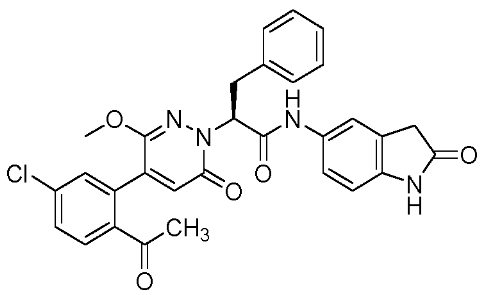

The specific synthetic route was as follows.

**Step A: Synthesis of 5-amino-2,3-dihydro-1*H*-indol-2-one**

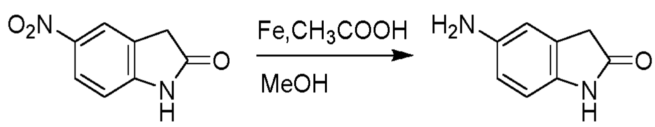

5-nitroindol-2-one (200 mg, 1.12 mmol) was dissolved in methanol (10 ml), added with acetic acid (1 ml) and iron powder (629 mg, 11.2 mmol), heated to 80°C and reacted for 4 hours.

Saturated sodium bicarbonate was added to the reaction solution until pH=7-8. The mixed solution was extracted with ethyl acetate (100 ml × 3) and concentrated. 150 mg of yellow solid 5-amino-2,3-dihydro-1***H***-indol-2-one was obtained (yield: 88.0%). LCMS: RT= 0.61 min, $[M+H]^+$ = 165.09.

**Step B: (S)-2-(4-(2-acetyl-5-chlorophenyl)-3-methoxy-6-oxopyridazin-1(6*H*)-yl)-*N*-(2-oxodihydroind ol-5-yl)-3-phenylpropanamide**

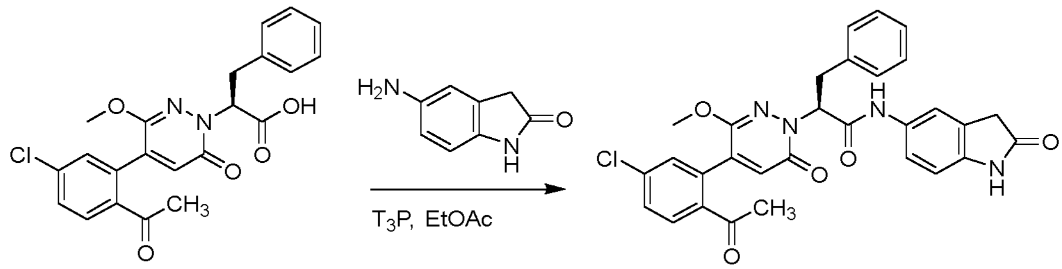


(S)-2-(4-(2-acetyl-5-chlorophenyl)-3-methoxy-6-oxopyridazin-1(6*H*)-yl)-3-phenylpropanic acid (65 mg, 0.150 mmol) and 5-amino-2,3-dihydro-1***H***-indol-2-one (25 mg, 0.167 mmol) were dissolved in ethyl acetate (3.0 ml), and N,N-diisopropylethylamine (193 mg, 1.5 mmol) was added. Subsequently, 1-propyl phosphonic anhydride (190 mg, 0.6 mmol) was added to the above solution. The reaction solution was heated to 60°C and stirred for 3 hours.

The reaction solution was cooled to room temperature and concentrated under reduced pressure. The crude product was purified by preparative high performance liquid chromatography. Separation conditions were as follows: chromatographic column: X select C18 19 mm * 150 mm; mobile phase: water (comprising 0.05% trifluoroacetic acid) and acetonitrile; flow rate: 25 ml/min; gradient: acetonitrile increasing from 5% to 100% in 7 minutes; detection wavelength: 254 nm. Upon purification, 2.42 mg of yellow solid (S)-2-(4-(2-acetyl-5-chlorophenyl)-3-methoxy-6-oxopyridazin-1(6*H*)-yl)-*N*-(2-oxodihydroindol-5-yl)-3-phenylpropanamide was obtained (yield: 2.8%). LCMS: RT= 3.72 min, $[M+H]^+$ = 557.15.

**Example 13**

**Synthesis of (S)-4-(2-(4-(2-acetyl-5-chlorophenyl)-3-methoxy-6-oxopyridazin-1(6*H*)-yl)-3-phenylpropana mido)-2-fluorobenzamide**

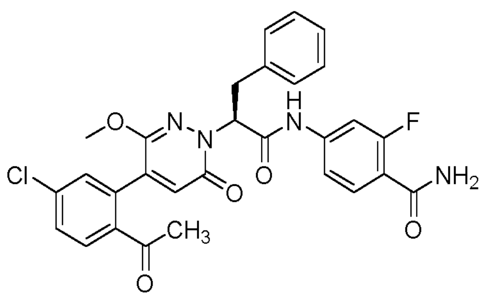

The specific synthetic route was as follows.

**Step A: Synthesis of 2-fluoro-4-nitrobenzamide**

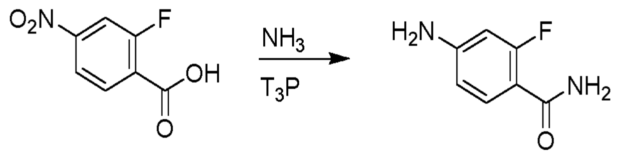


2-fluoro-4-nitrobenzoic acid (200 mg, 1.08 mmol) and ammonia (4.3 ml, 0.5 mol tetrahydrofuran solution) were dissolved in ethyl acetate (5.0 ml). 1-propyl phosphonic anhydride (1.37 g, 4.32 mmol) was added to the above solution. It was stirred at room temperature for 3 hours.

The reaction solution was cooled to room temperature and concentrated under reduced pressure. 180 mg of off-white solid 2-fluoro-4-nitrobenzamide was obtained (yield: 90.0%) by silica gel column purification. LCMS: RT= 3.45 min, $[M+H]^+$ = 185.02.

**Step B: Synthesis of 4-amino-2-fluorobenzamide**

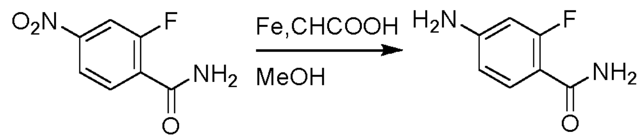


2-fluoro-4-nitrobenzamide (180 mg, 0.97 mmol) was dissolved in methanol (10 ml), added with acetic acid (1 ml) and iron powder (669 mg, 9.7 mmol), heated to 80°C and reacted for 4 hours.

Saturated sodium bicarbonate was added to the reaction solution until pH=7-8. The mixed solution was extracted with ethyl acetate (100 ml × 3) and concentrated. 140 mg of yellow solid 4-amino-2-fluorobenzamide was obtained (yield: 93.0%). LCMS: RT= 1.15 min, $[M+H]^+$ = 155.05.

**Step C: Synthesis of (S)-4-(2-(4-(2-acetyl-5-chlorophenyl)-3-methoxy-6-oxopyridazin-1(6*H*)-yl)-3-phenylpropana mido)-2-fluorobenzamide**

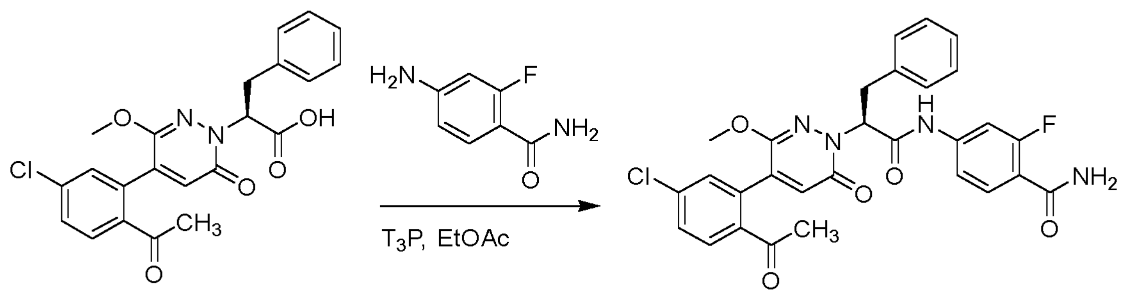


(S)-2-(4-(2-acetyl-5-chlorophenyl)-3-methoxy-6-oxopyridazin-1(6*H*)-yl)-3-phenylpropanic acid (40 mg, 0.09 mmol) and 4-amino-2-fluorobenzamide (15.9 mg, 0.1 mmol) were dissolved in ethyl acetate (3.0 ml), and N,N-diisopropylethylamine (116 mg, 0.9 mmol) was added. Subsequently, 1-propyl phosphonic anhydride (114 mg, 0.36 mmol) was added to the above solution. The reaction solution was heated to 60°C and stirred for 3 hours.

The reaction solution was cooled to room temperature and concentrated under reduced pressure. The crude product was purified by preparative high performance liquid chromatography. Separation conditions were as follows: chromatographic column: X select C18 19 mm * 150 mm; mobile phase: water (comprising 0.05% trifluoroacetic acid) and acetonitrile; flow rate: 25 ml/min; gradient: acetonitrile increasing from 5% to 100% in 7 minutes; detection wavelength: 254 nm. Upon purification, 2.47 mg of yellow solid (S)-4-(2-(4-(2-acetyl-5-chlorophenyl)-3-methoxy-6-oxopyridazin-1(6***H***)-yl)-3-phenylpropanami do)-2-fluorobenzamide was obtained (yield: 4.8%). LCMS: RT= 3.83 min, $[M+H]^+$ = 563.14.

**Example 14**

**Synthesis of (S)-4-(2-(4-(2-acetyl-5-chlorophenyl)-3-methoxy-6-oxopyridazin-1(6*H*)-yl)-3-phenylpropana mido)-2-fluorobenzoic acid**

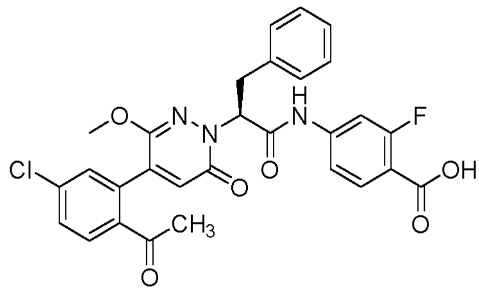

The specific synthetic route was as follows.

**Step A: Synthesis of 4-amino-2-fluorobenzoic acid**

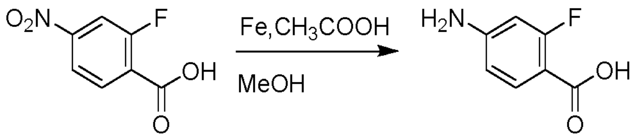


2-fluoro-4-nitrobenzoic acid (200 mg, 1.12 mmol) was dissolved in methanol (10 ml), added with acetic acid (1 ml) and iron powder (629 mg, 11.2 mmol), heated to 80°C and reacted for 4 hours.

Saturated sodium bicarbonate was added to the reaction solution until pH=7-8. The mixed solution was extracted with ethyl acetate (100 ml × 3) and concentrated. 150 mg of yellow solid 4-amino-2-fluorobenzoic acid was obtained (yield: 89.0%). LCMS: RT= 1.66 min, $[M+H]^+$ = 156.03.

**Step B: Synthesis of (S)-4-(2-(4-(2-acetyl-5-chlorophenyl)-3-methoxy-6-oxopyridazin-1(6*H*)-yl)-3-phenylpropana mido)-2-fluorobenzoic acid**

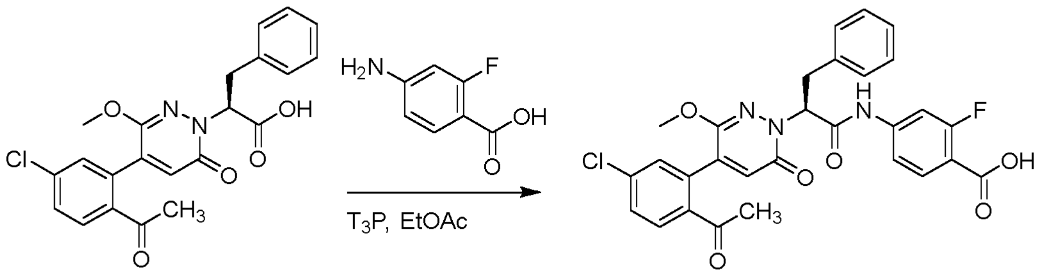


(S)-2-(4-(2-acetyl-5-chlorophenyl)-3-methoxy-6-oxopyridazin-1(6*H*)-yl)-3-phenylpropanic acid (40 mg, 0.09 mmol) and 4-amino-2-fluorobenzoic acid (16 mg, 0.10 mmol) were dissolved in ethyl acetate (3.0 ml), and N,N-diisopropylethylamine (116 mg, 0.9 mmol) was added. Subsequently, 1-propyl phosphonic anhydride (114 mg, 0.36 mmol) was added to the above solution. The reaction solution was heated to 60°C and stirred for 3 hours.

The reaction solution was cooled to room temperature and concentrated under reduced pressure. The crude product was purified by preparative high performance liquid chromatography. Separation conditions were as follows: chromatographic column: X select C18 19 mm * 150 mm; mobile phase: water (comprising 0.05% trifluoroacetic acid)

and acetonitrile; flow rate: 25 ml/min; gradient: acetonitrile increasing from 5% to 100% in 7 minutes; detection wavelength: 254 nm. Upon purification, 1.28 mg of yellow solid, (S)-4-(2-(4-(2-acetyl-5-chlorophenyl)-3-methoxy-6-oxopyridazin-1(6*H*)-yl)-3-phenylpropanami do)-2-fluorobenzoic acid was obtained (yield: 2.4%). LCMS: RT= 3.99 min, $[M+H]^+$ = 564.12.

**Example 15**

**Synthesis of (S)-5-(2-(4-(2-acetyl-5-chlorophenyl)-3-methoxy-6-oxopyridazin-1(6*H*)-yl)-3-phenylpropana mido)-1*H*-indol-2-formic acid**

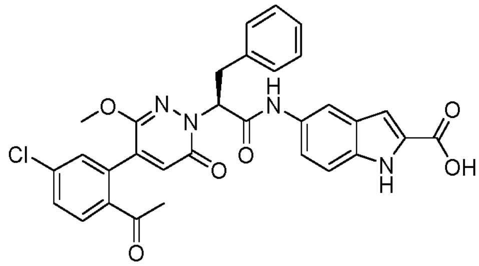

The specific synthetic route was as follows.

**Step A: Synthesis of di-tert butyl (R)-5-(2-hydroxy-3-phenylpropanamido)-1*H*-indol-1,2-diformate**

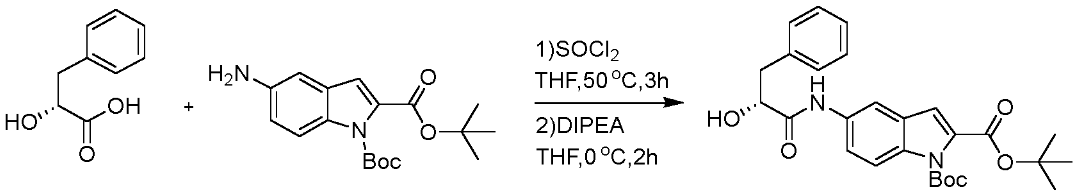


D-phenyl lactic acid (1.00 g, 6.0 mmol) was dissolved in dry tetrahydrofuran (40.0 ml) in a dry three-necked flask, and was stirred under ice bath for 15 minutes under the protection of nitrogen. Sulfoxide chloride (0.7 ml, 9.0 mmol) was slowly added to the reaction solution dropwise in 30 minutes. The reaction solution was heated to 50°C, stirred for 3 hours at constant temperature, cooled to room temperature, and spun dried. The residues was vacuumed by an oil pump for 15 minutes and dissolved in THF to obtain a solution A. Di-tert butyl 5-amino-1***H***-indol-1,2-dicarboxylate (1.6 g, 4.8 mmol) and diisopropylethylamine (3.0 ml, 18 mmol) were dissolved in dry tetrahydrofuran (20.0 ml) in a dry three-necked flask and were stirred under ice bath for 15 minutes under the protection of nitrogen. The solution A was slowly added to the mixed solution dropwise and stirred for 1 hour under ice bath. It was monitored by LCMS until the reaction was completed.

It was quenched by adding water to the reaction solution, and the mixed solution was extracted with ethyl acetate (200 ml × 3). The organic phases were combined. The combined organic phase was washed with saturated brine (100 ml × 3), then dried with anhydrous sodium sulfate, and concentrated under reduced pressure. The residue was purified by silica gel column chromatography (eluent: ethyl acetate/petroleum ether = 1/4). 1.0 g of yellow solid di-tert butyl (R)-5-(2-hydroxy-3-phenylpropanamido)-1***H***-indol-1,2-diformate was obtained (yield: 34.7 %). LCMS: RT= 4.53 min, $[M+H]^+$ = 481.38.

**Step B: Synthesis of di-tert butyl (R)-5-(2-(((4-nitrophenyl)sulfonyl)oxo)-3-phenylpropanamido)-1*H*-indol-1,2-diformate**

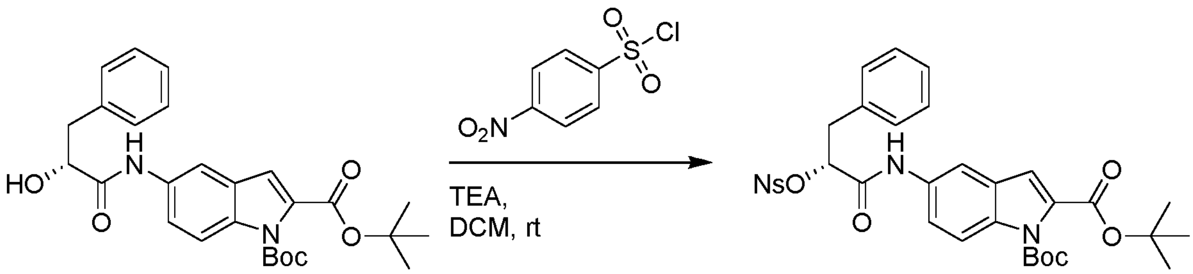


di-*tert*-butyl (*R*)-5-(2-(((4-nitrophenyl)sulfonyl)oxy)-3-phenylpropanamido)-1*H*-indole-1,2-dicarboxylate

di-tert butyl (R)-5-(2-hydroxy-3-phenylpropanamido)-1***H***-indol-1,2-diformate (330 mg, 0.7 mmol) and triethylamine (0.3 ml, 2.1 mmol) were dissolved in dichloromethane (10.0 ml). 4-nitrobenzene sulfonyl chloride (221 mg, 1.0 mmol) was added to the reaction solution under ice bath and stirred at room temperature for 2 hours.

It was quenched by adding saturated sodium bicarbonate solution (10 ml) to the reaction solution. The mixed solution was extracted with ethyl acetate (30 ml × 3). The organic phases were combined. The combined organic phase was washed with saturated brine (10 ml × 3), then dried with anhydrous sodium sulfate, and concentrated under reduced pressure. The residue was dissolved in dichloromethane (4 ml), which was then added to n-hexane (60 ml) dropwise under stirring. A large amount of white solid was precipitated and filtered. The filter cake was collected to obtain 440 mg of white solid di-tert butyl (R)-5-(2-(((4-nitrophenyl)sulfonyl)oxo)-3-phenylpropanamido)-1***H***-indol-1,2-diformate (yield: 96.7%). LCMS: RT= 4.75 min, $[M+H]^+$ = 688.09.

**Step C: Synthesis of di-tert butyl (S)-5-(2-(4-(2-acetyl-5-chlorophenyl)-3-methoxy-6-oxopyridazin-1(6*H*)-yl)-3-phenylpropana mido)-1H-indol-1,2-diformate**

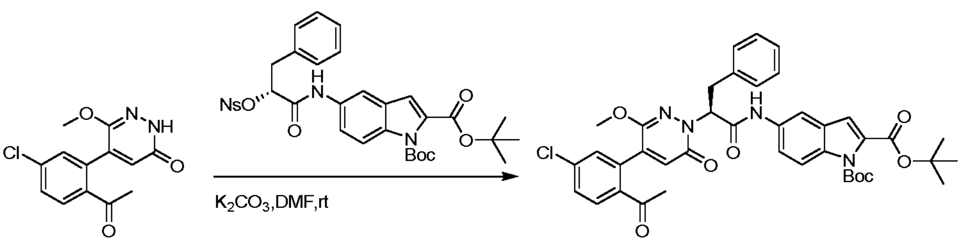


5-(2-acetyl-5-chlorophenyl)-6-methoxypyridazin-3(2*H*)-one (100 mg, 0.36 mmol) was dissolved in N,N-dimethylformamide (2.0 ml). Subsequently, potassium carbonate (100 mg, 0.72 mmol) and di-tert butyl (R)-5-(2-(((4-nitrophenyl)sulfonyl)oxo)-3-phenylpropanamido)-1***H***-indol-1,2-diformate (240.0 mg, 0.36 mmol) were added to the above solution. It was stirred at room temperature for 12 hours.

The reaction was quenched by adding water to the reaction solution. The mixed solution was extracted with ethyl acetate (20 ml × 3). The organic phases were combined, and the combined organic phase was washed with saturated brine (10 ml × 3), then dried with anhydrous sodium sulfate, and concentrated under reduced pressure. The residue was purified by silica gel column chromatography (eluent: ethyl acetate/petroleum ether = 1/1). 65 mg of yellow solid di-tert butyl (S)-5-(2-(4-(2-acetyl-5-chlorophenyl)-3-methoxy-6-oxopyridazin-1(6*H*)-yl)-3-phenylpropanami do)-1*H*-indol-1,2-diformate was obtained (yield: 24.4%). LCMS: RT= 4.92 min, $[M+H]^+$ = 741.20.

**Step D: Synthesis of (S)-5-(2-(4-(2-acetyl-5-chlorophenyl)-3-methoxy-6-oxopyridazin-1(6*H*)-yl)-3-phenylpropana mido)-1*H*-indol-2-formic acid**

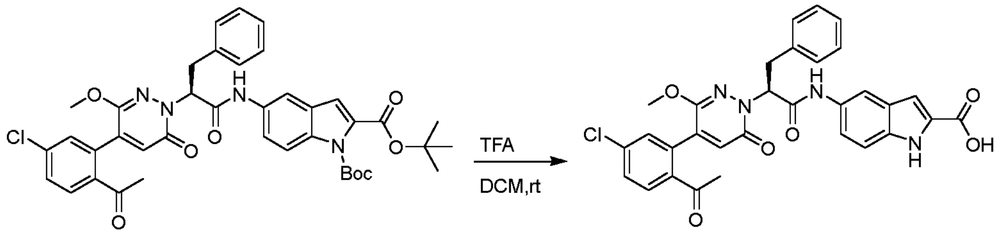


di-tert butyl (S)-5-(2-(4-(2-acetyl-5-chlorophenyl)-3-methoxy-6-oxopyridazin-1(6*H*)-yl)-3-phenylpropanami do)-1*H*-indol-1,2-diformate (65 mg, 0.09 mmol) was dissolved in dichloromethane (2.0 ml). Subsequently, trifluoroacetic acid (1.0 ml) was added to the above solution and was stirred at room temperature for 1 hour.

The reaction solution was concentrated under reduced pressure under air bath. The residue was purified by preparative high performance liquid chromatography, and 7 mg of yellow solid (S)-5-(2-(4-(2-acetyl-5-chlorophenyl)-3-methoxy-6-oxopyridazin-1(6*H*)-yl)-3-phenylpropanami do)-1*H*-indol-2-formic acid was obtained (yield: 11.0%). LCMS: RT= 3.88 min, $[M+H]^+$ = 585.10. $^1H$ NMR (500 MHz, DMSO) δ 12.85 (s, 1H), 11.70 (s, 1H), 10.03 (s, 1H), 7.98 (d, *J* = 8.3 Hz, 2H), 7.68 (dd, *J* = 8.3, 2.1 Hz, 1H), 7.49 (d, *J* = 2.1 Hz, 1H), 7.40 - 7.24 (m, 6H), 7.19 (dd, *J* = 13.7, 6.6 Hz, 1H), 7.04 (d, *J* = 1.8 Hz, 1H), 6.89 (s, 1H), 5.74 (dd, *J* = 10.1, 4.9 Hz, 1H), 3.68 (s, 3H), 3.52 (dd, *J* = 14.1, 10.4 Hz, 1H), 3.42 (dd, *J* = 14.0, 4.8 Hz, 1H), 2.52 (s, 3H).

**Example 16**

**Synthesis of (S)-4-(2-(4-(2-acetyl-5-chlorophenyl)-3-ethoxy-6-oxopyridazin-1(6*H*)-yl)-3-phenylpropanam ido)benzoic acid**

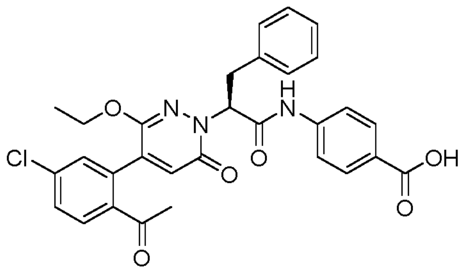

The specific synthetic route was as follows.

**Step A: Synthesis of 5-bromo-6-ethoxy-2-(4-methoxybenzyl)pyridazin-3(2*H*)-one**

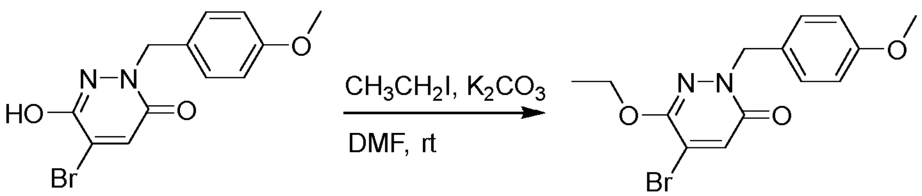


5-bromo-6-hydroxy-2-(4-methoxybenzyl)pyridazin-3(2***H***)-one (0.5 g, 1.6 mmol) was dissolved in N,N-dimethylformamide (2.0 ml) at room temperature. Subsequently, potassium carbonate (442 mg, 3.2 mmol) and iodoethane (380mg, 2.4 mmol) were added in the above solution. It was stirred at room temperature for 3 hours.

It was quenched by adding water to the reaction solution, and the mixed solution was extracted with ethyl acetate (30 ml × 3). The organic phases were combined. The combined organic phase was washed with saturated brine (20 ml × 3), then dried with anhydrous sodium sulfate, and concentrated under reduced pressure. The obtained residue was purified by silica gel column chromatography (eluent: ethyl acetate/petroleum ether = 1/4), and 280 mg of white

solid 5-bromo-6-ethoxy-2-(4-methoxybenzyl)pyridazin-3(2***H***)-one was obtained (yield: 51.3%). LCMS: RT= 4.06 min, $[M+H]^+$ = 341/339.

**Step B: Synthesis of 5-(2-acetyl-5-chlorophenyl)-6-ethoxy-2-(4-methoxybenzyl)pyridazin-3(2*H*)-one**

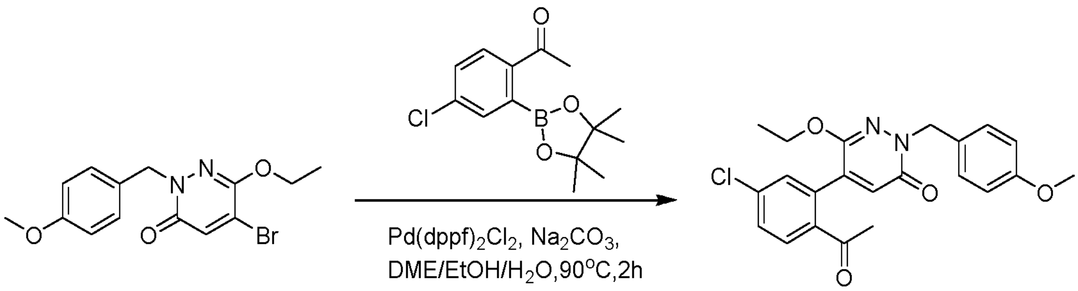


5-bromo-6-ethoxy-2-(4-methoxybenzyl)pyridazin-3(2***H***)-one (280 mg, 0.82 mmol), 1-(4-chloro-2-(4,4,5,5-tetramethyl-1,3,2-dioxaborolane-2-yl)phenyl)ethan-1-one (280 mg, 1.0 mmol) and sodium carbonate (180 mg, 1.7 mmol) were added to a three-necked bottle at room temperature and nitrogen replacement was performed. A mixed solvent (10 ml, DME: EtOH: $H_2O$ = 8:1:1) was added and nitrogen replacement was performed. [1,1'-bis(diphenylphosphino)ferrocene]palladium dichloride dichloromethane complex (59 mg, 0.07 mmol) was added and nitrogen replacement was performed. It was heated to 90°C and reacted for 2 hours.

The reaction solution was cooled to room temperature and filtered with diatomite. The filter cake was washed with ethyl acetate (30 ml × 2), and the filtrate and washing solution were combined and concentrated under reduced pressure. The obtained residue was added with water (50 ml), and the mixed solution was extracted with ethyl acetate (50 ml × 3). The organic phases were combined. The combined organic phase was washed with saturated brine (20 ml × 3), then dried with anhydrous sodium sulfate, and concentrated under reduced pressure. The residue was purified by silica gel column chromatography (eluent: ethyl acetate/petroleum ether = 1/2). 120 mg of yellow solid 5-(2-acetyl-5-chlorophenyl)-6-ethoxy-2-(4-methoxybenzyl)pyridazin-3(2*H*)-one, was obtained (yield: 50.0%). LCMS: RT= 4.12 min, $[M+H]^+$ = 413.12.

**Step C: Synthesis of 5-(2-acetyl-5-chlorophenyl)-6-ethoxypyridazin-3(2*H*)-one**

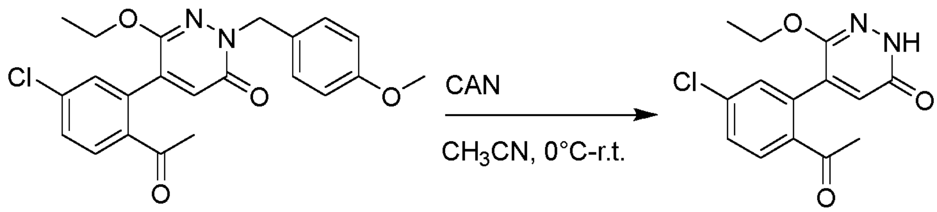


5-(2-acetyl-5-chlorophenyl)-6-ethoxy-2-(4-methoxybenzyl)pyridazin-3(2*H*)-one (120 mg, 0.29 mmol) was added to a mixed solvent (4 ml, acetonitrile: water = 3:1) at 0°C, and then ceric ammonium nitrate (1.5 g, 2.9 mmol) was added slowly, followed by reacting for 30 minutes at room temperature.

After the reaction was completed, it was quenched by adding water, and the mixed solution was extracted with ethyl acetate (30 ml × 3). The organic phases were combined. The combined organic phase was washed with saturated brine (30 ml × 2), then dried with anhydrous sodium sulfate, and concentrated under reduced pressure. The residue was purified by silica gel column chromatography (eluent: ethyl acetate/n-hexane = 1/1). 64 mg of yellow solid 5-(2-acetyl-5-chlorophenyl)-6-ethoxypyridazin-3(2*H*)-one was obtained (yield: 75.6%). LCMS: RT= 3.41 min, $[M+H]^+$ = 293.09.

**Step D: Synthesis of tert-butyl (S)-4-(2-(4-(2-acetyl-5-chlorophenyl)-3-ethoxy-6-oxopyridazin-1(6*H*)-yl)-3-phenylpropanam ido)benzoate**

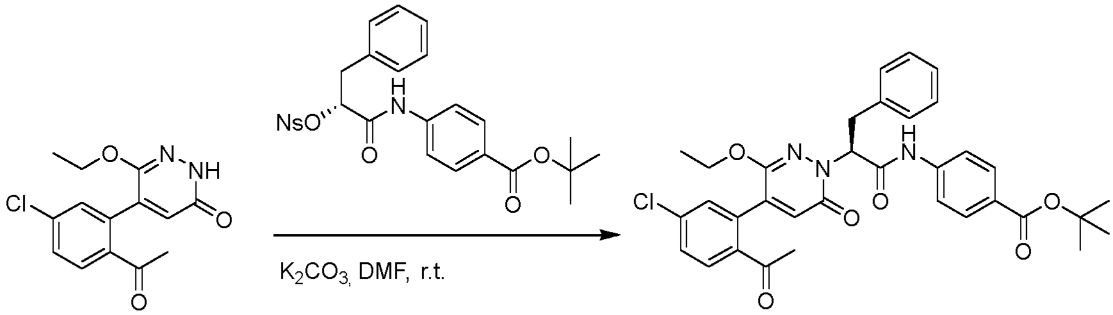


5-(2-acetyl-5-chlorophenyl)-6-ethoxypyridazin-3(2***H***)-one (64 mg, 0.22 mmol), tert-butyl (R)-4-(2-(((4-nitrophenyl)sulfonyl)oxo)-3-phenylpropanamido)benzoate (170 mg, 0.33 mmol), and potassium carbonate (61 mg, 0.44 mmol) were added in N,N-dimethylformamide (2.0 ml) at room temperature and reacted overnight at room temperature.

After the reaction was completed, it was quenched by adding water, and the mixed solution was extracted with ethyl acetate (10 ml × 3). The organic phases were combined. The combined organic phase was washed with saturated brine (10 ml × 2), then dried with anhydrous sodium sulfate, and concentrated under reduced pressure. The residue was purified by silica gel column chromatography (eluent: ethyl acetate/n-hexane = 1/2). 58 mg of pale yellow solid tert-butyl (S)-4-(2-(4-(2-acetyl-5-chlorophenyl)-3-ethoxy-6-oxopyridazin-1(6***H***)-yl)-3-phenylpropanamido )benzoate was obtained (yield: 43.0%). LCMS: RT= 4.67 min, $[M+H]^+$ = 616.18.

**Step G: Synthesis of (S)-4-(2-(4-(2-acetyl-5-chlorophenyl)-3-ethoxy-6-oxopyridazin-1(6*H*)-yl)-3-phenylpropanam ido)benzoic acid**

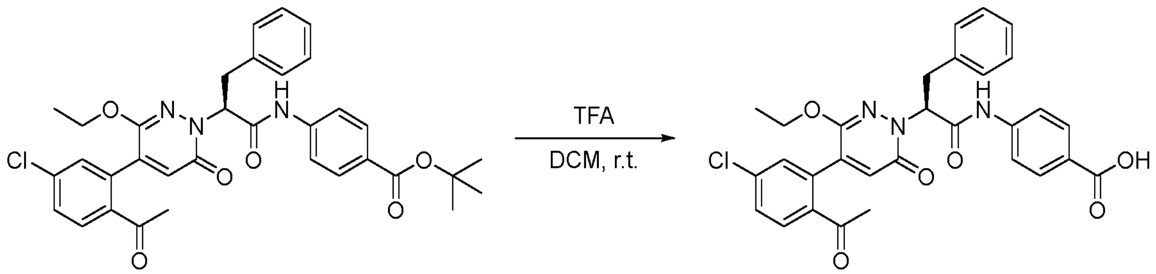


Tert-butyl (S)-4-(2-(4-(2-acetyl-5-chlorophenyl)-3-ethoxy-6-oxopyridazin-1(6***H***)-yl)-3-phenylpropanamido )benzoate (58 mg, 0.09 mmol) was dissolved in dichloromethane (2.0 ml). Subsequently, trifluoroacetic acid (0.5 ml) was added to the above solution and was stirred at room temperature for 1 hour.

The reaction solution was concentrated under reduced pressure under air bath. The obtained residue was purified by slurrying with dichloromethane and n-hexane, and 19 mg of yellow solid (S)-4-(2-(4-(2-acetyl-5-chlorophenyl)-3-ethoxy-6-oxopyridazin-1(6***H***)-yl)-3-phenylpropanamido )benzoic acid was obtained (yield: 36.0%). LCMS: RT= 4.08 min, $[M+H]^+$ = 560.08. $^1$H NMR (500 MHz, DMSO) δ 12.76 (s, 1H), 10.52 (s, 1H), 8.02 (d, *J* = 8.4 Hz, 1H), 7.92 (d, *J* = 8.7 Hz, 2H), 7.79 - 7.67 (m, 3H), 7.51 (d, *J*= 2.1 Hz, 1H), 7.38 - 7.25 (m, 4H), 7.21 (d, *J*= 6.4 Hz, 1H), 6.91 (s, 1H), 5.77 (dd, *J* = 9.8, 5.1 Hz, 1H), 4.10 (dd, *J* = 17.0, 7.0 Hz, 2H), 3.44 (ddd, *J* = 22.3, 18.9, 11.2 Hz, 2H), 2.55 (s, 3H), 1.15 (t, *J* = 7.0 Hz, 3H).

**Example 17**

**Synthesis of (S)-2-(4-(2-acetyl-5-chlorophenyl)-3-methoxy-6-oxopyridazin-1(6*H*)-yl)-3-phenyl-*N*-(4-amin osulfonylphenyl)propanamide**

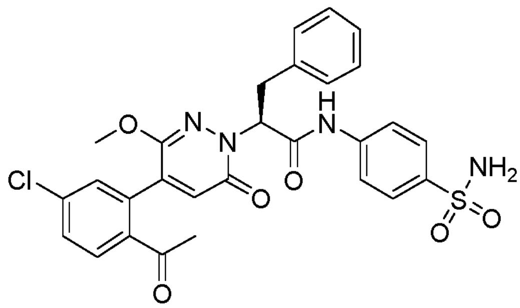

The specific synthetic route was as follows.

**Step A: Synthesis of methyl (S)-2-(4-(2-acetyl-5-chlorophenyl)-3-methoxy-6-oxopyridazin-1(6*H*)-yl)-3-phenylpropanoat** e

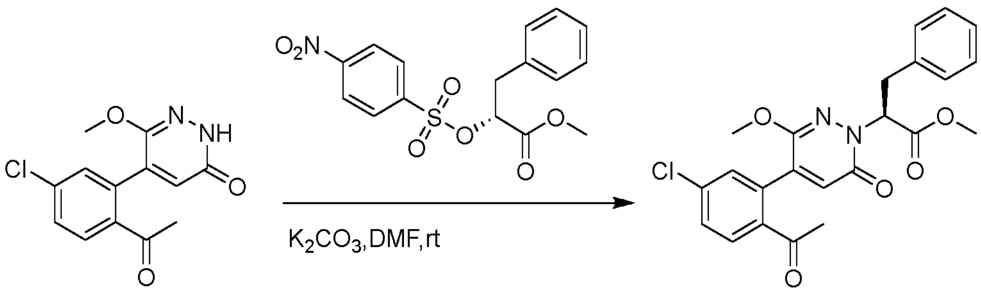


5-(2-acetyl-5-chlorophenyl)-6-ethoxypyridazin-3(2H)-one (230 mg, 0.83 mmol), methyl (R)-2-(2-(((4-nitrophenyl)sulfonyl)oxo)-3-phenylpropanoate (450 mg, 1.2 mmol), and potassium carbonate (230 mg, 1.67 mmol) were added in N,N-dimethylformamide (4.0 ml) at room temperature and reacted at room temperature for 6 hours.

After the reaction was completed, it was quenched by adding water, and the mixed solution was extracted with ethyl acetate (40 ml × 3). The organic phases were combined. The combined organic phase was washed with saturated brine (20 ml × 2), then dried with anhydrous sodium sulfate, and concentrated under reduced pressure. The residue was purified by silica gel column chromatography (eluent: ethyl acetate/n-hexane = 1/2). 270 mg of pale yellow solid methyl (S)-2-(4-(2-acetyl-5-chlorophenyl)-3-methoxy-6-oxopyridazin-1(6*H*)-yl)-3-phenylpropanoate was obtained (yield: 74.0%). LCMS: RT= 4.08 min, $[M+H]^+$ = 441.09.

**Step B: Synthesis of (S)-2-(4-(2-acetyl-5-chlorophenyl)-3-methoxy-6-oxopyridazin-1(6*H*)-yl)-3-phenylpropanic acid**

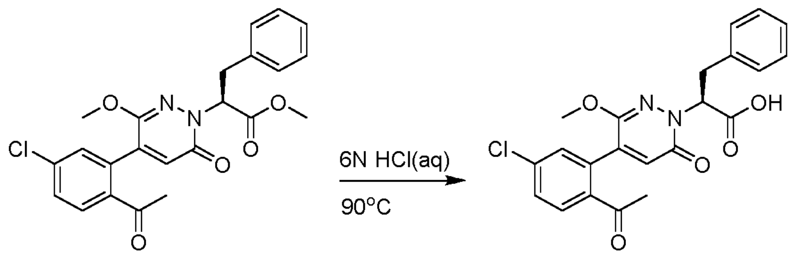


Methyl (S)-2-(4-(2-acetyl-5-chlorophenyl)-3-methoxy-6-oxopyridazin-1(6***H***)-yl)-3-phenylpropanoate (270 mg, 0.61 mmol) was dissolved in 6N hydrochloric acid solution (10.0 ml). It was heated to 50°C and stirred for 12 hours at constant temperature.

The reaction was quenched by adding saturated sodium bicarbonate solution to the reaction solution, and the

pH of which was adjusted to weak acidity. The mixed solution was extracted with ethyl acetate (50 ml × 3). The organic phases were combined. The combined organic phase was washed with saturated brine (20 ml × 3), then dried with anhydrous sodium sulfate, and concentrated under reduced pressure. 240 mg of yellow solid (S)-2-(4-(2-acetyl-5-chlorophenyl)-3-methoxy-6-oxopyridazin-1(6***H***)-yl)-3-phenylpropanic acid was obtained (yield: 92.0%). LCMS: RT= 3.87 min, $[M+H]^+$ = 427.05.

**Step C: Synthesis of (S)-2-(4-(2-acetyl-5-chlorophenyl)-3-methoxy-6-oxopyridazin-1(6*H*)-yl)-3-phenyl-*N*-(4-amin osulfonylphenyl)propanamide**

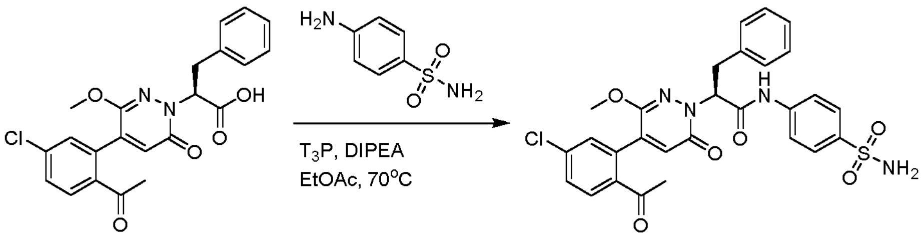


(S)-2-(4-(2-acetyl-5-chlorophenyl)-3-methoxy-6-oxopyridazin-1(6***H***)-yl)-3-phenylpropanic acid (33 mg, 0.12 mmol), sulfanilamide (52 mg, 0.35 mmol) and N,N-diisopropylethylamine (0.2 ml) were dissolved in ethyl acetate (2.0 ml). Subsequently, 1-propyl phosphonic anhydride (0.2 ml, 50% ethyl acetate solution) was added to the above solution. It was heated to 70°C and stirred for 15 hours at constant temperature.

The reaction was quenched by adding water (10 ml) to the reaction solution. The mixed solution was extracted with ethyl acetate (20 ml × 3). The organic phases were combined. The combined organic phase was washed with saturated brine (10 ml × 3), then dried with anhydrous sodium sulfate, and concentrated under reduced pressure. The residue was purified by preparative high performance liquid chromatography, and 6 mg of white solid (S)-2-(4-(2-acetyl-5-chlorophenyl)-3-methoxy-6-oxopyridazin-1(6***H***)-yl)-3-phenyl-*N*-(4-aminos ulfonylphenyl)propanamide was obtained (yield: 15.0%). LCMS: RT= 3.81 min, $[M+H]^+$ = 581.06.

**Example 18**

**Synthesis of (S)-4-(2-(4-(2-acetyl-5-chlorophenyl)-3-methoxy-6-oxopyridazin-1(6*H*)-yl)-3-phenylpropana mido)phenylsulfonic acid**

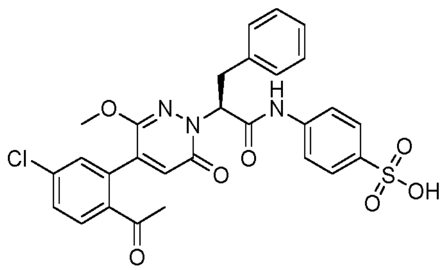

The specific synthetic route was as follows.

**Step A: Synthesis of (2R, 2'S)-N,N'-(dithio(-4,1-phenylene))bis(2-chloro-3-phenylpropanamide)**

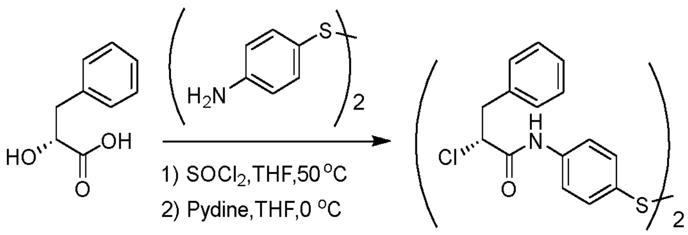


D-phenyl lactic acid (0.6 g, 3.6 mmol) was dissolved in dry tetrahydrofuran (40.0 ml) in a dry three-necked flask, and was stirred under ice bath for 15 minutes under the protection of nitrogen. Sulfoxide chloride (0.8 ml, 10.8 mmol) was slowly added to the reaction solution dropwise in 30 minutes. The reaction solution was heated to 70°C, stirred for 5 hours at constant temperature, cooled to room temperature, and spun dried. The residues vacuumed by an oil pump for 15 minutes and dissolved in THF to obtain a solution A. 4,4-dithiodianiline (890 mg, 3.6 mmol) and diisopropylethylamine (2 ml, 10.8 mmol) were dissolved in dry tetrahydrofuran (20.0 ml) in a dry three-necked flask and were stirred under ice bath for 15 minutes under the protection of nitrogen. The solution A was slowly added to the mixed solution dropwise and stirred for 1 hour under ice bath. It was monitored by LCMS until the reaction was completed.

It was quenched by adding water to the reaction solution, and the mixed solution was extracted with ethyl acetate (40 ml × 3). The organic phases were combined. The combined organic phase was washed with saturated brine (20 ml × 3), then dried with anhydrous sodium sulfate, and concentrated under reduced pressure. The residue was purified by silica gel column chromatography (eluent: ethyl acetate/petroleum ether = 1/4). 1.2 g of yellow solid (2R, 2'S)-N,N'-(dithio(-4,1-phenylene))bis(2-chloro-3-phenylpropanamide) was obtained (yield: 38.0%). LCMS: RT= 4.72 min, $[M+H]^+$ = 581.08.

**Step B: Synthesis of (R)-4-(2-chloro-3-phenylpropanamido)phenylsulfonic acid**

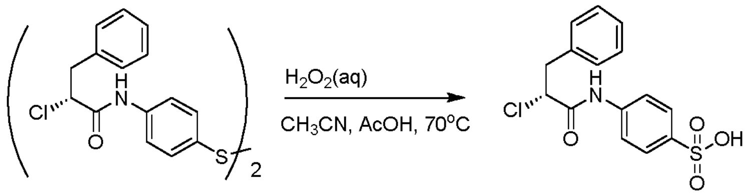


(2R, 2'S)-N,N'-(dithio(-4,1-phenylene))bis(2-chloro-3-phenylpropanamide) (300 mg, 0.52 mmol) was dissolved in acetonitrile (10.0 ml). Subsequently, 30% hydrogen peroxide solution (2.0 ml) was added to the above solution. It was heated to 50°C and stirred for 5 hours at constant temperature.

The reaction was quenched by adding saturated sodium bicarbonate solution to the reaction solution, and the pH of which was then adjusted to weak acidity. The mixed solution was extracted with ethyl acetate (30 ml × 3). The organic phases were combined. The combined organic phase was washed with saturated brine (10 ml × 3), then dried with anhydrous sodium sulfate, and concentrated under reduced pressure. 300 mg of yellow solid (R)-4-(2-chloro-3-phenylpropanamido)phenylsulfonic acid was obtained (yield: 86.0%). LCMS: RT = 6.83 min, $[M-H]^-$ = 337.95.

**Step C: Synthesis of (S)-4-(2-(4-(2-acetyl-5-chlorophenyl)-3-methoxy-6-oxopyridazin-1(6*H*)-yl)-3-phenylpropana mido)phenylsulfonic acid**

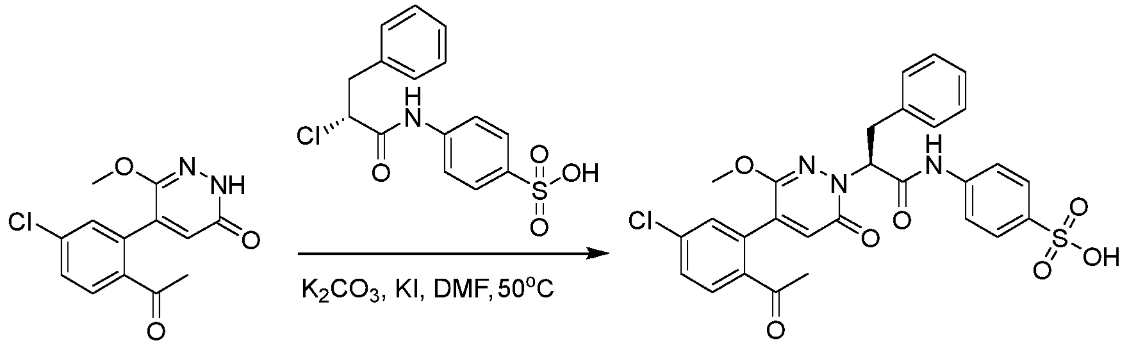


H)-one (33 mg, 0.12 mmol) and potassium carbonate (33 mg, 0.24 mmol) were dissolved in N,N-dimethylformamide (2.0 ml). Subsequently, potassium iodide (40 mg, 0.24 mmol) and (R)-4-(2-chloro-3-phenylpropanamido)phenylsulfonic acid (40 mg, 0.18 mmol) were added to the above solution. It was heated to 50°C and stirred for 40 hours at constant temperature.

The reaction was quenched by adding water (10 ml) to the reaction solution. The mixed solution was extracted with ethyl acetate (20 ml × 3). The organic phases were combined. The combined organic phase was washed with saturated brine (10 ml × 3), then dried with anhydrous sodium sulfate, and concentrated under reduced pressure. The residue was purified by preparative high performance liquid chromatography, and 16 mg of yellow solid (S)-4-(2-(4-(2-acetyl-5-chlorophenyl)-3-methoxy-6-oxopyridazin-1(6*H*)-yl)-3-phenylpropanami do)phenylsulfonic acid was obtained (yield: 23.0%). LCMS: RT= 7.96 min, $[M+H]^+$ = 582.12.

## Example 19

**Synthesis of (S)-4-(2-(4-(2-acetyl-5-chlorophenyl)-3-methoxy-6-oxopyridazin-1(6*H*)-yl)-3-phenylpropana mido)benzoic acid**

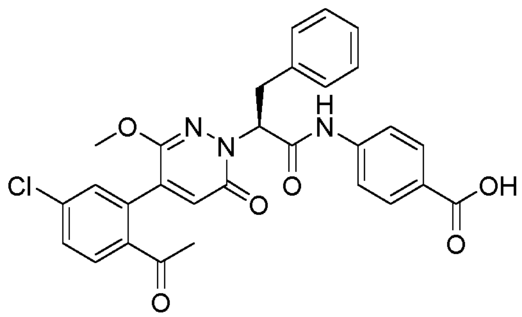

The specific synthetic route was as follows.

**Step A: Synthesis of 5-bromo-6-hydroxy-2-(4-methoxybenzyl)pyridazin-3(2*H*)-one**

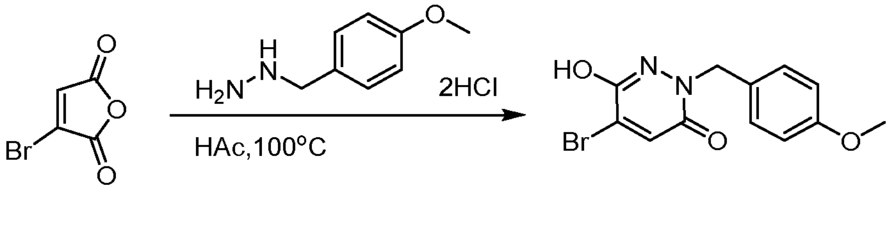


Bromomaleic anhydride (2.00 g, 11.3 mmol) and (4-methoxybenzyl)hydrazine hydrochloride (2.13 g, 11.3 mmol) were added in glacial acetic acid (50.0 ml) and reacted at 100°C for 3 hours.

The reaction solution was cooled to room temperature after the reaction was completed, and was poured into water. A large amount of solid was precipitated, which was stirred for a while and filtered by suction. The filter cake was washed with water and dried. 1.50 g of yale yellow solid 5-bromo-6-hydroxy-2-(4-methoxybenzyl)pyridazin-3(2***H***)-one was obtained, which was used directly for the next reaction without purification. LCMS: RT= 3.44 min, $[M+H]^+$ = 311.03.

**Step B: Synthesis of 5-bromo-6-methoxy-2-(4-methoxybenzyl)pyridazin-3(2*H*)-one**

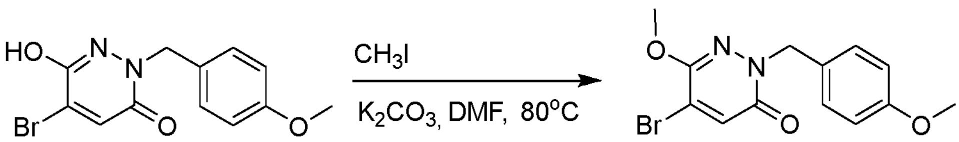


5-bromo-6-hydroxy-2-(4-methoxybenzyl)pyridazin-3(2***H***)-one (1.50 g, 4.82 mmol) and potassium carbonate (2.66 g, 19.29 mmol) were added in *N,N*-dimethylformamide (15.0 ml) at room temperature, and were stirred at 80°C for 15 minutes. Iodomethane (1.2 ml) was added to the solution at this temperature and reacted for another 30 minutes.

After the reaction was completed, it was quenched by adding water, and the mixed solution was extracted with ethyl acetate (50 ml × 3). The organic phases were combined. The combined organic phase was washed with saturated brine (50 ml × 2), then dried with anhydrous sodium sulfate, and concentrated under reduced pressure. The residue was purified by silica gel column chromatography (eluent: ethyl acetate/n-hexane = 1/3). 1.10 g of white solid 5-bromo-6-methoxy-2-(4-methoxybenzyl)pyridazin-3(2***H***)-one was obtained (yield: 70.3%). LCMS: RT= 3.87 min, $[M+H]^+$ = 325.01.

**Step C: Synthesis of 6-acetyl-3-chlorophenylboric acid pinacol ester**

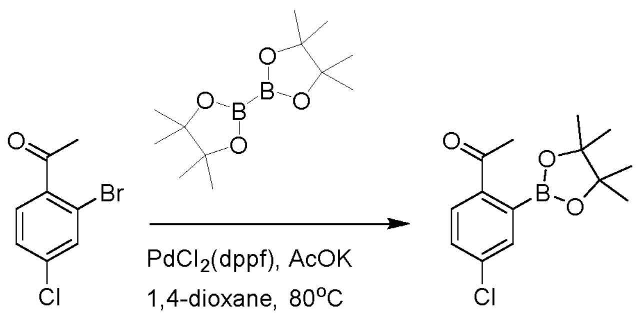


2-bromo-4-chloroacetophenone (5.00 g, 21.41 mmol), bis(pinacolato)diborone (8.16 g, 32.12 mmol) and potassium acetate (4.20 g, 42.82 mmol) were added in a three-necked bottle at room temperature, and nitrogen replacement was performed. 1,4-dioxane (60.0 ml) was added, and nitrogen replacement was performed. [1,1'-bis(diphenylphosphino)ferrocene]palladium chloride (1.75 g, 2.14 mmol) was added and nitrogen replacement was performed. It was heated to 80°C and reacted for 3 hours.

After the reaction was completed, it was quenched by adding water and filtered by suction with diatomite. The filter cake was washed with ethyl acetate, and the filtrate was extracted with ethyl acetate (80 ml × 3). The organic phases were combined. The combined organic phase was washed with saturated brine (50 ml × 2), then dried with anhydrous sodium sulfate, and concentrated under reduced pressure. The residue was purified by silica gel column chromatography (eluent: ethyl acetate/n-hexane = 1/50). 2.1 g of yellow solid 6-acetyl-3-chlorophenylboric acid pinacol ester was obtained (yield: 35.0%). LCMS: RT = 4.26 min, $[M-H]^-$ = 279.08.

**Step D: Synthesis of 5-(2-acetyl-5-chlorophenyl)-6-methoxy-2-(4-methoxybenzyl)pyridazin-3(2*H*)-one**

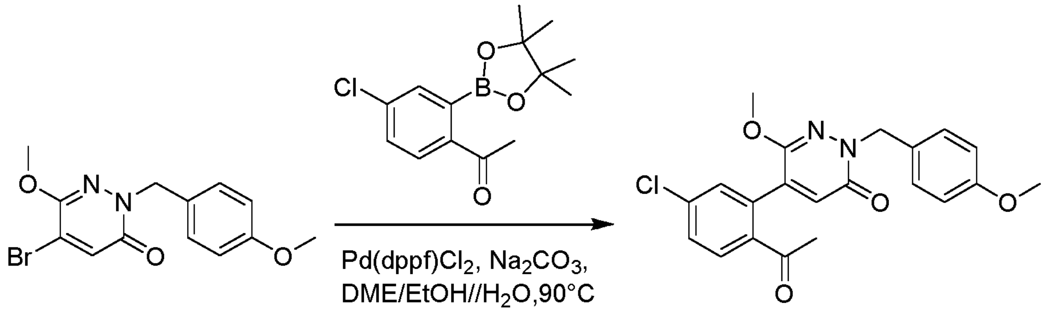


5-bromo-6-methoxy-2-(4-methoxybenzyl)pyridazin-3(2H)-one (1.10 g, 3.39 mmol), 6-acetyl-3-chlorophenylboric acid pinacol ester (949 mg, 3.39 mmol) and sodium carbonate (718 mg, 6.78 mmol) were added to a three-necked bottle at room temperature and nitrogen replacement was performed. A mixed solvent (10 ml, 1, 2-dimethoxyethane: EtOH: $H_2O$ = 8:1:1) was added and nitrogen replacement was performed. [1,1'-bis(diphenylphosphino)ferrocene]palladium chloride (249 mg, 0.34 mmol) was added and nitrogen replacement was performed. It was heated to 90°C and reacted for 1 hour.

After the reaction was completed, it was quenched by adding water, and the mixed solution was extracted with ethyl acetate (50 ml × 3). The organic phases were combined. The combined organic phase was washed with saturated brine (50 ml × 2), then dried with anhydrous sodium sulfate, and concentrated under reduced pressure. The residue was purified by silica gel column chromatography (eluent: ethyl acetate/n-hexane = 1/2). 676 mg of yellow solid 5-(2-acetyl-5-chlorophenyl)-6-methoxy-2-(4-methoxybenzyl)pyridazin-3(2*H*)-one was obtained (yield: 50.2%). LCMS: RT= 3.99 min, $[M+H]^+$ = 399.07.

**Step E: Synthesis of 5-(2-acetyl-5-chlorophenyl)-6-methoxypyridazin-3(2*H*)-one**

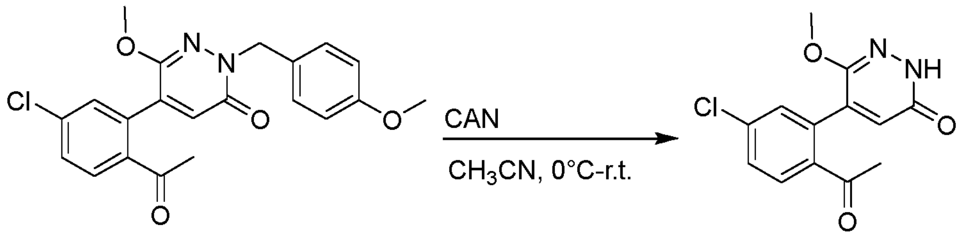


5-(2-acetyl-5-chlorophenyl)-6-methoxy-2-(4-methoxybenzyl)pyridazin-3(2H)-one (676 mg, 1.70 mmol) was added to a mixed solvent (4 ml, acetonitrile: water = 3:1) at 0°C, and then ceric ammonium nitrate (7.46 g, 13.60 mmol) was added slowly, followed by reacting for 30 minutes at room temperature.

After the reaction was completed, it was quenched by adding water, and the mixed solution was extracted with ethyl acetate (30 ml × 3). The organic phases were combined. The combined organic phase was washed with saturated brine (30 ml × 2), then dried with anhydrous sodium sulfate, and concentrated under reduced pressure. The residue was purified by silica gel column chromatography (eluent: ethyl acetate/n-hexane = 1/1). 238 mg of yellow solid 5-(2-acetyl-5-chlorophenyl)-6-methoxypyridazin-3(2*H*)-one was obtained (yield: 50.0%). LCMS: RT= 3.23 min, $[M+H]^+$ = 279.08.

**Step E: Synthesis of tert-butyl (S)-4-(2-(4-(2-acetyl-5-chlorophenyl)-3-methoxy-6-oxopyridazin-1(6*H*)-yl)-3-phenylpropana mido)benzoate**

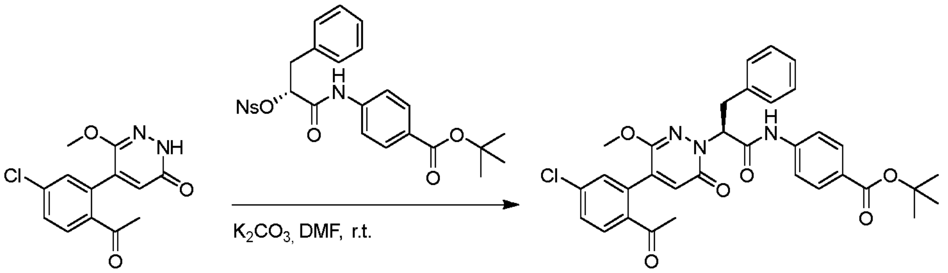


5-(2-acetyl-5-chlorophenyl)-6-methoxypyridazin-3(2H)-one (50 mg, 0.18 mmol), tert-butyl (R)-4-(2-(((4-nitrophenyl)sulfonyl)oxo)-3-phenylpropanamido)benzoate (113 mg, 0.22 mmol), and potassium carbonate (50 mg, 0.36 mmol) were added in N,N-dimethylformamide (2.0 ml) at room temperature and reacted overnight at room temperature.

After the reaction was completed, it was quenched by adding water, and the mixed solution was extracted with ethyl acetate (10 ml × 3). The organic phases were combined. The combined organic phase was washed with saturated brine (10 ml × 2), then dried with anhydrous sodium sulfate, and concentrated under reduced pressure. The residue was purified by silica gel column chromatography (eluent: ethyl acetate/n-hexane = 1/2). 75 mg of yale yellow solid tert-butyl (S)-4-(2-(4-(2-acetyl-5-chlorophenyl)-3-methoxy-6-oxopyridazin-1(6*H*)-yl)-3-phenylpropanami do)benzoate was obtained (yield: 66.7%). LCMS: RT= 4.53 min, $[M+H]^+$ = 602.13.

**Step G: Synthesis of (S)-4-(2-(4-(2-acetyl-5-chlorophenyl)-3-methoxy-6-oxopyridazin-1(6*H*)-yl)-3-phenylpropana mido)benzoic acid**

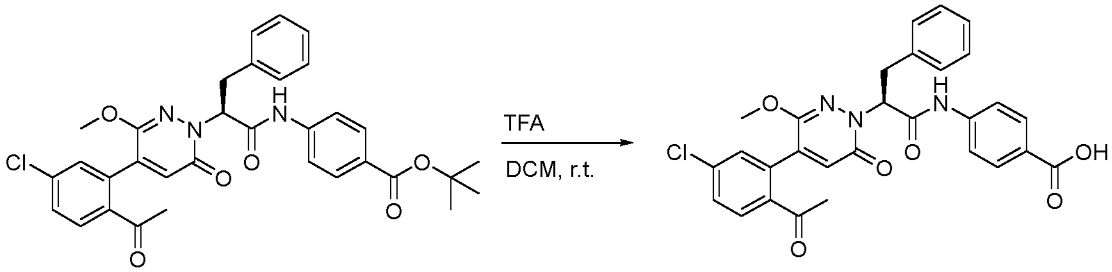


Tert-butyl (S)-4-(2-(4-(2-acetyl-5-chlorophenyl)-3-methoxy-6-oxopyridazin-1(6*H*)-yl)-3-phenylpropanami do)benzoate (75 mg, 0.12 mmol) was dissolved in dichloromethane (2.0 ml) at room temperature. Trifluoroacetic acid (0.25 ml) was added dropwise and reacted at room temperature for 3 hours.

After the reaction was completed, dichloromethane was evaporated and trifluoroacetic acid was removed by oil pump. The residue was dissolved in dichloromethane (1.0 ml) and added in n-hexane (10.0 ml) dropwise. A white solid was precipitated and filtered by suction. The filter cake was washed with n-hexane and dried. 50 mg of white solid (S)-4-(2-(4-(2-acetyl-5-chlorophenyl)-3-methoxy-6-oxopyridazin-1(6***H***)-yl)-3-phenylpropanami do)benzoic acid was obtained (yield: 76.5%). LCMS: RT = 3.98 min, $[M-H]^-$ = 544.10. $^1H$ NMR (500 MHz, DMSO) δ 12.79 (s, 1H), 10.52 (s, 1H), 7.99 (d, *J* = 8.4 Hz, 1H), 7.91 (d, *J* = 8.7 Hz, 2H), 7.72 (d, *J*= 8.7 Hz, 2H), 7.69 (dd, *J*= 8.3, 2.1 Hz, 1H), 7.50 (d, *J*= 2.1 Hz, 1H), 7.37-7.23 (m, 4H), 7.19 (t, *J*= 7.1 Hz, 1H), 6.91 (s, 1H), 5.74 (dd, *J*= 10.2, 4.9 Hz, 1H), 3.67 (s, 3H), 3.52 (dd, *J* = 14.1, 10.3 Hz, 1H), 3.41 (dd, *J* = 14.1, 4.7 Hz, 1H), 2.53 (s, 3H).

**Example 20**

**Synthesis of (S)-4-(2-(4-(2-acetyl-5-chlorophenyl)-3-(cyclopropylmethoxy)-6-oxopyridazin-1(6*H*)-yl)-3-p henylpropanamido)benzoic acid**

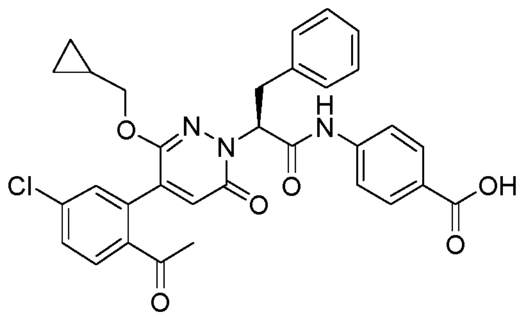

The specific synthetic route was as follows.

**Step A: Synthesis of 5-bromo-6-(cyclopropylmethoxy)-2-(4-methoxybenzyl)pyridazin-3(2*H*)-one**

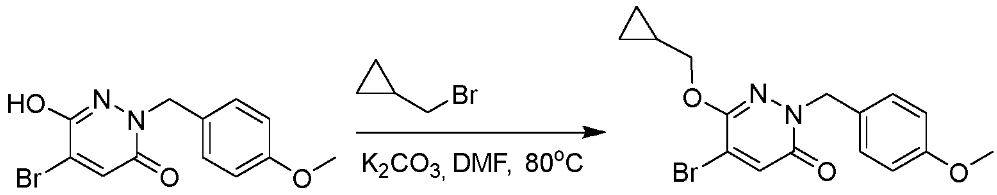


5-bromo-6-hydroxy-2-(4-methoxybenzyl)pyridazin-3(2H)-one (1.50 g, 4.82 mmol) and potassium carbonate (2.66 g, 19.29 mmol) were added in N,N-dimethylformamide (15.0 ml) at room temperature, and were stirred at 80°C for 15 minutes. Cyclopropylmethyl bromide (1.8 ml) was added to the solution at this temperature and reacted for another 30 minutes.

After the reaction was completed, it was quenched by adding water, and the mixed solution was extracted with ethyl acetate (50 ml × 3). The organic phases were combined. The combined organic phase was washed with saturated brine (50 ml × 2), then dried with anhydrous sodium sulfate, and concentrated under reduced pressure. The residue was purified by silica gel column chromatography (eluent: ethyl acetate/n-hexane = 1/3). 1.32 g of white solid 5-bromo-6-(cyclopropylmethoxy)-2-(4-methoxybenzyl)pyridazin-3(2H)-one was obtained (yield: 74.9%). LCMS: RT= 4.20 min, $[M+H]^+$ = 365.39.

**Step B: Synthesis of 5-(2-acetyl-5-chlorophenyl)-6-(cyclopropylmethoxy)-2-(4-methoxybenzyl)pyridazin-3(2*H*)-one**

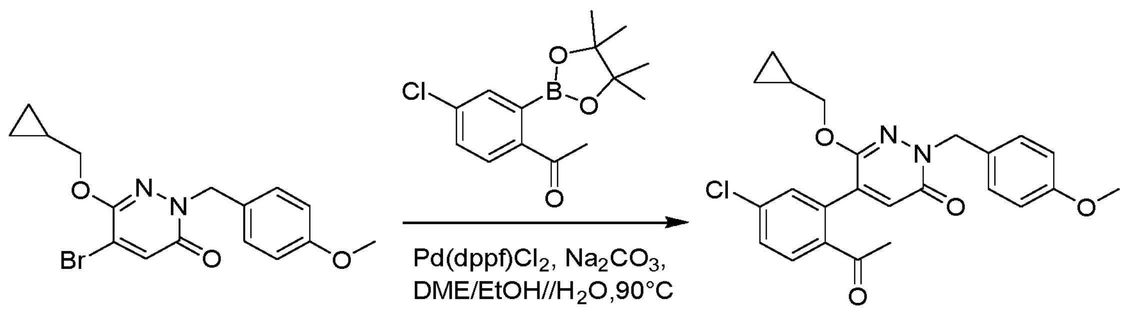


5-bromo-6-(cyclopropylmethoxy)-2-(4-methoxybenzyl)pyridazin-3(2H)-one (1.32 g, 3.61 mmol), 6-acetyl-3-chlorophenylboric acid pinacol ester (1.01 g, 3.61 mmol) and sodium carbonate (765 mg, 7.22 mmol) were added to a three-necked bottle at room temperature and nitrogen replacement was performed. A mixed solvent (10 ml, 1,2-dimethoxyethane: EtOH: $H_2O$ = 8:1:1) was added and nitrogen replacement was performed. [1,1'-bis(diphenylphosphino)ferrocene]palladium chloride (263.2 mg, 0.36 mmol) was added and nitrogen replacement was performed. It was heated to 90°C and reacted for 1 hour.

After the reaction was completed, it was quenched by adding water, and the mixed solution was extracted with ethyl acetate (50 ml × 3). The organic phases were combined. The combined organic phase was washed with saturated brine (50 ml × 2), then dried with anhydrous sodium sulfate, and concentrated under reduced pressure. The residue was purified by silica gel column chromatography (eluent: ethyl acetate/n-hexane = 1/2). 685 mg of yellow solid 5-(2-acetyl-5-chlorophenyl)-6-(cyclopropylmethoxy)-2-(4-methoxybenzyl)pyridazin-3(2*H*)-one was obtained (yield: 43.2%). LCMS: RT= 4.26 min, $[M+H]^+$ = 439.13.

**Step C: Synthesis of 5-(2-acetyl-5-chlorophenyl)-6-(cyclopropylmethoxy)pyridazin-3(2*H*)-one**

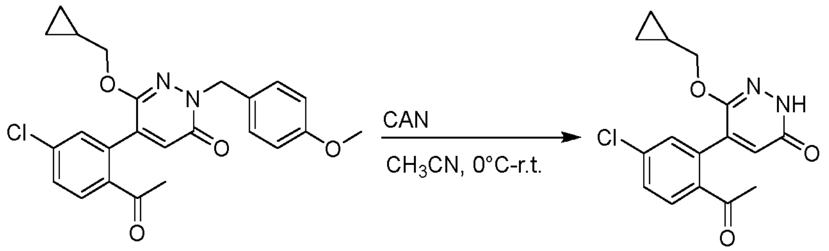


5-(2-acetyl-5-chlorophenyl)-6-(cyclopropylmethoxy)-2-(4-methoxybenzyl)pyridazin-3(2H)-one (685 mg, 1.56 mmol) was added to a mixed solvent (4 ml, acetonitrile: water = 3:1) at 0°C, and then ceric ammonium nitrate (6.85 g, 12.59 mmol) was added slowly, followed by reacting for 30 minutes at room temperature.

After the reaction was completed, it was quenched by adding water, and the mixed solution was extracted with ethyl acetate (30 ml × 3). The organic phases were combined. The combined organic phase was washed with saturated brine (30 ml × 2), then dried with anhydrous sodium sulfate, and concentrated under reduced pressure. The residue was purified by silica gel column chromatography (eluent: ethyl acetate/n-hexane = 1/1). 223 mg of yellow solid 5-(2-acetyl-5-chlorophenyl)-6-(cyclopropylmethoxy)pyridazin-3(2*H*)-one was obtained (yield: 44.8%). LCMS: RT= 3.59 min, $[M+H]^+$ = 319.04.

**Step D: Synthesis of tert-butyl (S)-4-(2-(4-(2-acetyl-5-chlorophenyl)-3-(cyclopropylmethoxy)-6-oxopyridazin-1(6*H*)-yl)-3-p henylpropanamido)benzoate**

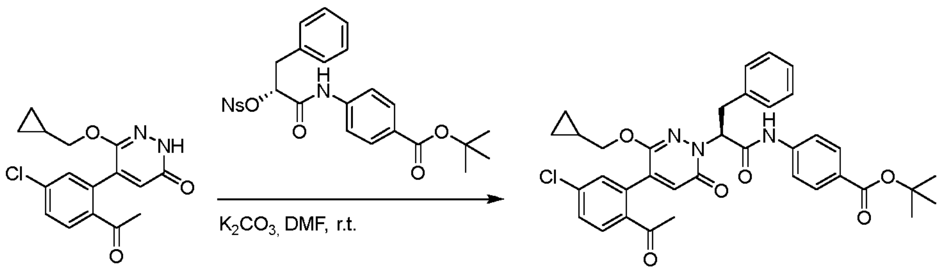


5-(2-acetyl-5-chlorophenyl)-6-(cyclopropylmethoxy)pyridazin-3(2H)-one (50 mg, 0.16 mmol), tert-butyl (R)-4-(2-(((4-nitrophenyl)sulfonyl)oxo)-3-phenylpropanamido)benzoate (100 mg, 0.19 mmol), and potassium carbonate (44 mg, 0.32 mmol) were added in N,N-dimethylformamide (2.0 ml) at room temperature and reacted overnight at room temperature.

After the reaction was completed, it was quenched by adding water, and the mixed solution was extracted with ethyl acetate (10 ml × 3). The organic phases were combined. The combined organic phase was washed with saturated brine (10 ml × 2), then dried with anhydrous sodium sulfate, and concentrated under reduced pressure. The residue was purified by silica gel column chromatography (eluent: ethyl acetate/n-hexane = 1/2). 63 mg of a light yellow solid, tert-butyl (S)-4-(2-(4-(2-acetyl-5-chlorophenyl)-3-(cyclopropylmethoxy)-6-oxopyridazin-1(6*H*)-yl)-3-phen ylpropanamido)benzoate, was obtained (yield: 62.5%). LCMS:RT= 4.77 min, $[M+H]^+$ = 642.19.

**Step E: Synthesis of (S)-4-(2-(4-(2-acetyl-5-chlorophenyl)-3-(cyclopropylmethoxy)-6-oxopyridazin-1(6*H*)-yl)-3-p henylpropanamido)benzoic acid**

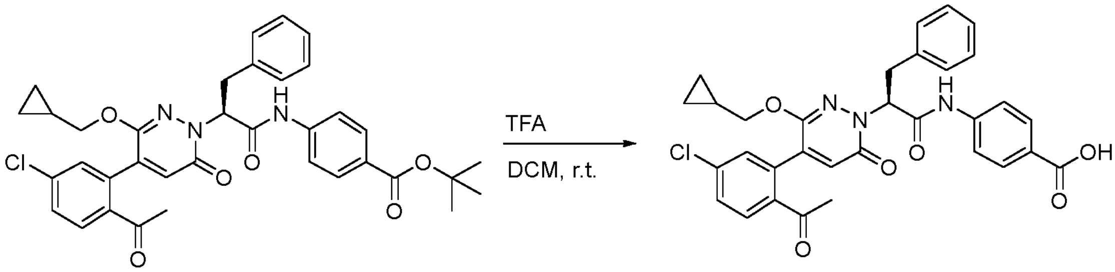


Tert-butyl (S)-4-(2-(4-(2-acetyl-5-chlorophenyl)-3-(cyclopropylmethoxy)-6-oxopyridazin-1(6H)-yl)-3-phen ylpropanamido)benzoate (63 mg, 0.10 mmol) was dissolved in dichloromethane (2.0 ml) at room temperature. Trifluoroacetic acid (0.25 ml) was added dropwise and reacted at room temperature for 3 hours.

After the reaction was completed, dichloromethane was evaporated and trifluoroacetic acid was pumped out with an oil pump. The residue was dissolved in dichloromethane (1.0 ml) and added in n-hexane (10.0 ml) dropwise. A white solid was precipitated and suction filtrated. The filter cake was washed with n-hexane and dried. 50 mg of a white solid, (S)-4-(2-(4-(2-acetyl-5-chlorophenyl)-3-(cyclopropylmethoxy)-6-oxopyridazin-1(6H)-yl)-3-phen ylpropanamido)benzoic acid, was obtained (yield: 85.3%). LCMS:RT = 4.20 min, $[M-H]^-$ = 584.14.

## Example 21

**Synthesis of (S)-4-(2-(4-(2-acetyl-5-chlorophenyl)-3-(2-methoxyethoxy)-6-oxopyridazin-1(6*H*)-yl)-3-phen ylpropanamido)benzoic acid**

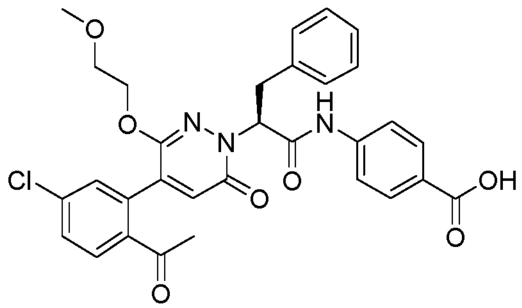

The specific synthetic route was as follows.

**Step A: Synthesis of 5-bromo-2-(4-methoxybenzyl)-6-(2-methoxyethoxy)pyridazin-3(2*H*)-one**

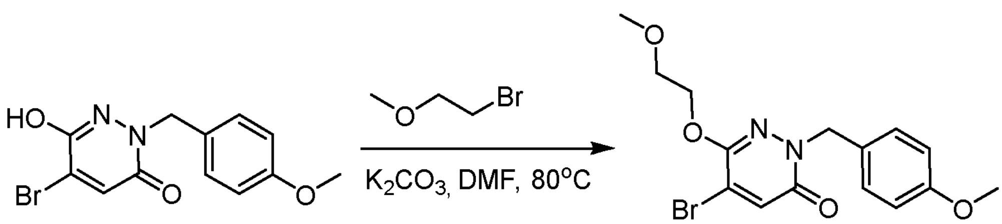


5-bromo-6-hydroxy-2-(4-methoxybenzyl)pyridazin-3(2H)-one (500 mg, 1.61 mmol) and potassium carbonate (900 mg, 6.52 mmol) were added in N,N-dimethylformamide (5.0 ml) at room temperature, and were stirred at 80°C for

15 minutes. 1-bromo-2-methoxyethane (0.6 ml) was added to the solution at this temperature and reacted for another 30 minutes.

After the reaction was completed, it was quenched by adding water, and the mixed solution was extracted with ethyl acetate (50 ml × 3). The organic phases were combined. The combined organic phase was washed with saturated brine (50 ml × 2), then dried with anhydrous sodium sulfate, and concentrated under reduced pressure. The residue was purified by silica gel column chromatography (eluent: ethyl acetate/n-hexane = 1/3). 333 mg of white solid 5-bromo-2-(4-methoxybenzyl)-6-(2-methoxyethoxy)pyridazin-3(2***H***)-one was obtained (yield: 55.9%). LCMS:RT= 3.81 min, $[M+H]^+$ = 369.03.

**Step B: Synthesis of 5-(2-acetyl-5-chlorophenyl)-2-(4-methoxybenzyl)-6-(2-methoxyethoxy)pyridazin-3(2*H*)-one**

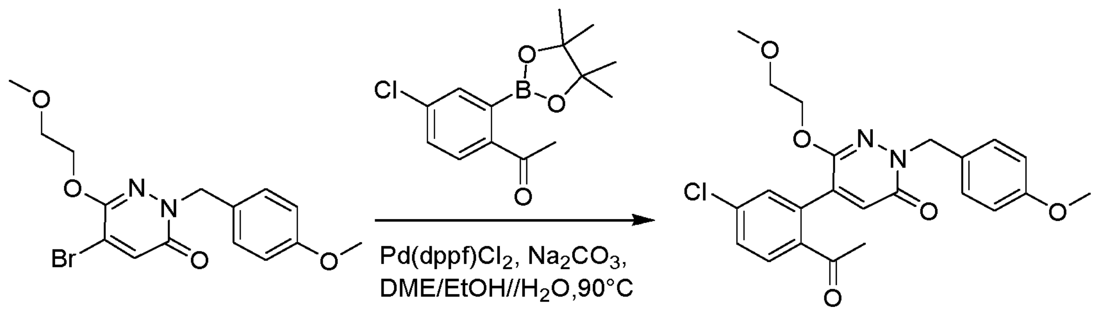


5-bromo-2-(4-methoxybenzyl)-6-(2-methoxyethoxy)pyridazin-3(2***H***)-one (333 mg, 0.90 mmol), 6-acetyl-3-chlorophenylboric acid pinacol ester (253 mg, 0.90 mmol) and sodium carbonate (192 mg, 1.81 mmol) were added to a three-necked bottle at room temperature and nitrogen replacement was performed. A mixed solvent (10 ml, 1, 2-dimethoxyethane:EtOH:$H_2O$ = 8:1:1) was added therein and nitrogen replacement was performed. [1,1'-bis(diphenylphosphino)ferrocene]dichloropalladium (74 mg, 0.09 mmol) was added therein and nitrogen replacement was performed. It was heated to 90°C and reacted for 1 hour.

After the reaction was completed, it was quenched by adding water, and the mixed solution was extracted with ethyl acetate (50 ml × 3). The organic phases were combined. The combined organic phase was washed with saturated brine (50 ml × 2), then dried with anhydrous sodium sulfate, and concentrated under reduced pressure. The residue was purified by silica gel column chromatography (eluent: ethyl acetate/n-hexane = 1/2). 186 mg of yellow solid 5-(2-acetyl-5-chlorophenyl)-2-(4-methoxybenzyl)-6-(2-methoxyethoxy)pyridazin-3(2*H*)-one, was obtained (yield: 46.7%). LC-MS:RT= 3.97 min, $[M+H]^+$ = 443.15.

**Step C: Synthesis of 5-(2-acetyl-5-chlorophenyl)-6-(2-methoxyethoxy)pyridazin-3(2*H*)-one**

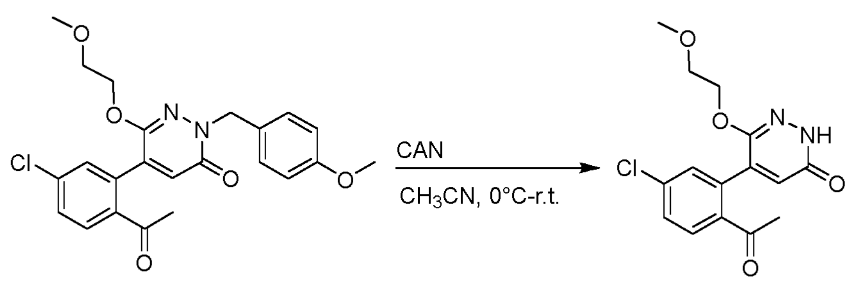


5-(2-acety1-5-chloropheny1)-2-(4-methoxybenzyl)-6-(2-methoxyethoxy)pyridazin-3(2***H***)-on e (186 mg, 0.42 mmol) was added to a mixed solvent (4 ml, acetonitrile: water = 3:1) at 0°C, and then ceric ammonium nitrate (2.30 g, 4.20 mmol) was added slowly, followed by reacting for 30 minutes at room temperature.

After the reaction was completed, it was quenched by adding water, and the mixed solution was extracted with ethyl acetate (30 ml × 3). The organic phases were combined. The combined organic phase was washed with saturated brine (30 ml × 2), then dried with anhydrous sodium sulfate, and concentrated under reduced pressure. The residue was purified by silica gel column chromatography (eluent: ethyl acetate/n-hexane = 1/1). 89 mg of yellow solid 5-(2-acetyl-5-chlorophenyl)-6-(2-methoxyethoxy)pyridazin-3(2***H***)-one was obtained (yield: 66.7%). LCMS:RT= 3.25 min, $[M+H]^+$ = 323.10.

**Step D: Synthesis of tert-butyl (S)-4-(2-(4-(2-acetyl-5-chlorophenyl)-3-(2-methoxyethoxy)-6-oxopyridazin-1(6*H*)-yl)-3-phen ylpropanamido)benzoate**

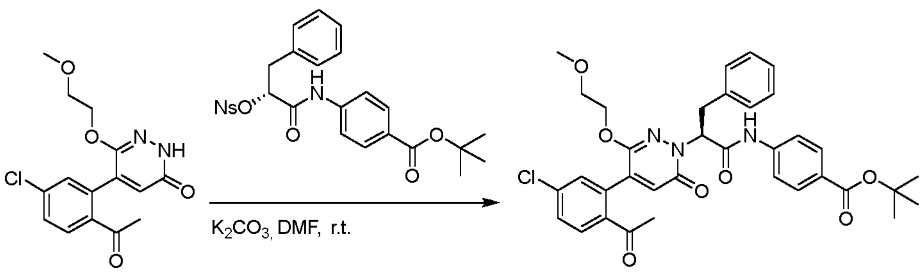


5-(2-acetyl-5-chlorophenyl)-6-(2-methoxyethoxy)pyridazin-3(2H)-one (89 mg, 0.28 mmol), tert-butyl (R)-4-(2-(((4-nitrophenyl)sulfonyl)oxo)-3-phenylpropanamido)benzoate (160 mg, 0.30 mmol), and potassium carbonate (77 mg, 0.56 mmol) were added in N,N-dimethylformamide (2.0 ml) at room temperature and reacted overnight at room temperature.

After the reaction was completed, it was quenched by adding water, and the mixed solution was extracted with ethyl acetate (10 ml × 3). The organic phases were combined. The combined organic phase was washed with saturated brine (10 ml × 2), then dried with anhydrous sodium sulfate, and concentrated under reduced pressure. The residue was purified by silica gel column chromatography (eluent: ethyl acetate/n-hexane = 1/2). 140 mg of pale yellow solid tert-butyl (S)-4-(2-(4-(2-acetyl-5-chlorophenyl)-3-(2-methoxyethoxy)-6-oxopyridazin-1(6H)-yl)-3-phenylp ropanamido)benzoate was obtained (yield: 78.6%). LCMS:RT= 4.52 min, $[M+H]^+$ = 646.20.

**Step E: Synthesis of (S)-4-(2-(4-(2-acetyl-5-chlorophenyl)-3-(2-methoxyethoxy)-6-oxopyridazin-1(6*H*)-yl)-3-phen ylpropanamido)benzoic acid**

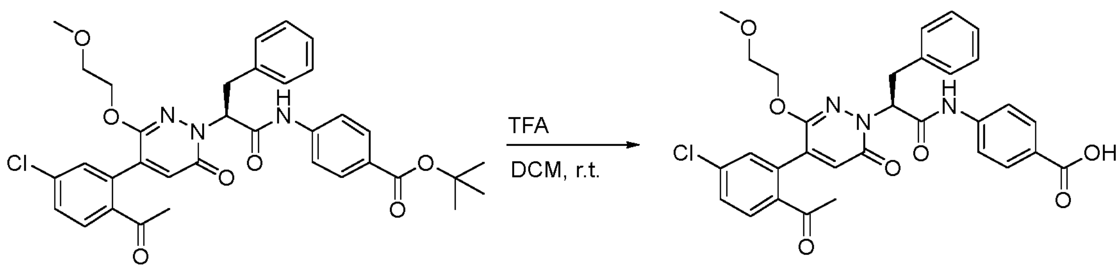


Tert-butyl (S)-4-(2-(4-(2-acetyl-5-chlorophenyl)-3-(2-methoxyethoxy)-6-oxopyridazin-1(6H)-yl)-3-phenylp ropanamido)benzoate (140 mg, 0.22 mmol) was dissolved in dichloromethane (2.0 ml) at room temperature. Trifluoroacetic acid (0.25 ml) was added dropwise and reacted at room temperature for 3 hours.

After the reaction was completed, dichloromethane was evaporated and trifluoroacetic acid was pumped out with an oil pump. The residue was dissolved in dichloromethane (1.0 ml) and added in n-hexane (10.0 ml) dropwise. A white solid was precipitated and suction filtrated. The filter cake was washed with n-hexane and dried. 68 mg of white solid (S)-4-(2-(4-(2-acetyl-5-chlorophenyl)-3-(2-methoxyethoxy)-6-oxopyridazin-1(6H)-yl)-3-phenylp ropanamido)benzoic acid, was obtained (yield: 52.4%). LCMS:RT = 3.96 min, $[M-H]^-$ = 588.10. $^1H$ NMR (500 MHz, DMSO) δ 12.71 (s, 1H), 10.51 (s, 1H), 8.02 (d, *J* = 8.4 Hz, 1H), 7.90 (d, *J* = 7.0 Hz, 2H), 7.72 (d, *J* = 8.8 Hz, 2H), 7.69 (dd, *J* = 8.4, 2.1 Hz, 1H), 7.49 (d, *J* = 2.2 Hz, 1H), 7.32 - 7.25 (m, 4H), 7.23 - 7.16 (m, 1H), 6.89 (s, 1H), 5.75 (dd, *J*= 9.9, 5.1 Hz, 1H), 4.19 - 4.07 (m, 2H), 3.53 - 3.42 (m, 4H), 3.12 (s,3H), 2.54 (s, 3H).

**Example 22**

**Synthesis of (S)-4-(2-(4-(2-acetyl-5-chlorophenyl)-3-isopropoxy-6-oxopyridazin-1(6*H*)-yl)-3-phenylprop anamido)benzoic acid**

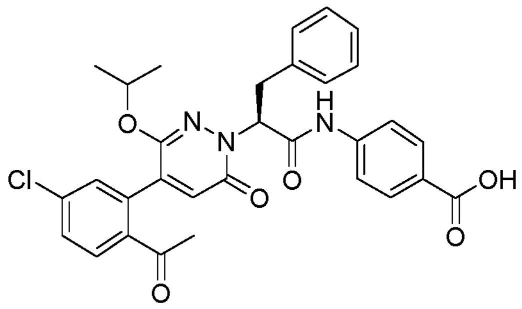

The specific synthetic route was as follows.

**Step A: Synthesis of 5-bromo-6-isopropyl-2-(4-methoxybenzyl)pyridazin-3(2*H*)-one**

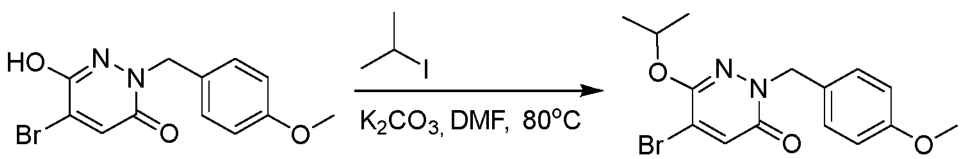


5-bromo-6-hydroxy-2-(4-methoxybenzyl)pyridazin-3(2***H***)-one (500 mg, 1.61 mmol) and potassium carbonate (900 mg, 6.52 mmol) were added to N,N-dimethylformamide (5.0 ml) at room temperature. It was stirred at 80°C for 15 minutes. At this temperature, 2-iodomethane (0.6 ml) was added, and the reaction was continued for 30 minutes.

The reaction was completed, quenched by adding water, and the mixture was extracted with ethyl acetate (50 ml × 3 times). The organic phases were combined, and the organic phase was first washed with saturated brine (50 ml × 2 times), then dried with anhydrous sodium sulfate, and finally concentrated under reduced pressure. The obtained residue was purified by silica gel column chromatography (eluent: ethyl acetate/n-hexane=1/3). 212 mg of white solid, 5-bromo-6-isopropyl-2-(4-methoxybenzyl)pyridazin-3(2*H*)-one, was obtained (yield: 37.3%). LCMS: RT = 4.21 min, $[M+H]^+$ = 353.02.

**Step B: Synthesis of 5-(2-acetyl-5-chlorophenyl)-6-isopropyl-2-(4-methoxybenzyl)pyridazin-3(2*H*)-one**

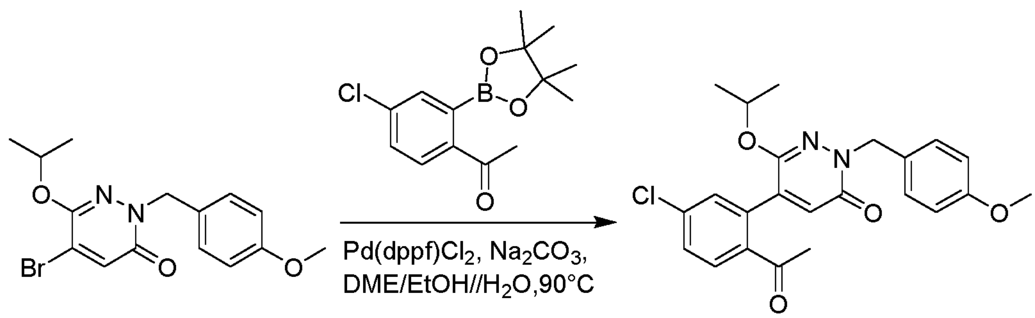


5-bromo-6-isopropyl-2-(4-methoxybenzyl)pyridazin-3(2*H*)-one (212 mg, 0.60 mmol), 6-acetyl-3-chlorophenylboronic acid pinacol ester (169 mg, 0.60 mmol) and sodium carbonate (128 mg, 1.20 mmol) were added to a three-necked flask at room temperature, and nitrogen replacement was performed. A mixed solvent (10 mL, 1,2-dimethoxyethane: ethanol: water=8:1:1) was added and nitrogen replacement was performed. 1,1'-bisdiphenylphosphinoferrocene palladium dichloride (49 mg, 0.06 mmol) was added and nitrogen replacement was performed. The mixture was heated to 90 °C to react for 1 hour.

The reaction was completed, quenched by adding water, and the mixture was extracted with ethyl acetate (50 ml × 3 times). The organic phases were combined, and the organic phase was first washed with saturated brine (50 ml × 2 times), then dried with anhydrous sodium sulfate, and finally concentrated under reduced pressure. The obtained residue was purified by silica gel column chromatography (eluent: ethyl acetate/n-hexane=1/2). 127 mg of yellow solid, 5-(2-acetyl-5-chlorophenyl)-6-isopropyl-2-(4-methoxybenzyl)pyridazin-3(2*H*)-one, was obtained (yield: 50.0%). LCMS: RT = 4.24 min.

**Step C: Synthesis of 5-(2-acetyl-5-chlorophenyl)-6-isopropylpyridazin-3(2*H*)-one**

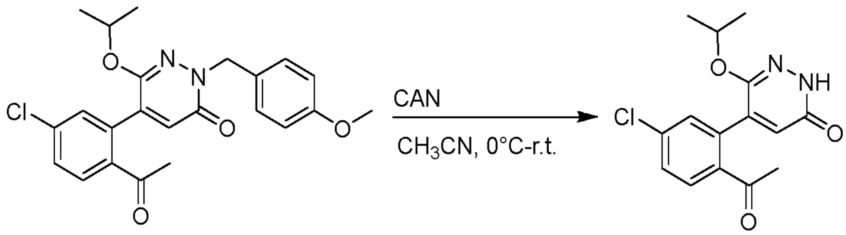


5-(2-acetyl-5-chlorophenyl)-6-isopropyl-2-(4-methoxybenzyl)pyridazin-3(2*H*)-one (127 mg, 0.30 mmol) was added to a mixed solvent (4 ml, acetonitrile: water = 3:1) at 0°C, and then ceric ammonium nitrate (1.64 g, 2.99 mmol) was slowly added. After the addition was completed, the reaction was performed at room temperature for 30 minutes.

The reaction was completed, quenched by adding water, and the mixture was extracted with ethyl acetate (30 ml × 3 times). The organic phases were combined, and the organic phase was first washed with saturated brine (30 ml × 2 times), then dried with anhydrous sodium sulfate, and finally concentrated under reduced pressure. The obtained residue was purified by silica gel column chromatography (eluent: ethyl acetate/n-hexane=1/1). 47 mg of yellow solid, 5-(2-acetyl-5-chlorophenyl)-6-isopropylpyridazin-3(2*H*)-one, was obtained (yield: 50.0%). LCMS: RT = 3.55 min, $[M+H]^+$ = 307.08.

**Step D: Synthesis of tert-butyl (S)-4-(2-(4-(2-acetyl-5-chlorophenyl)-3-isopropoxy-6-oxopyridazin-1(6*H*)-yl)-3-phenylprop anamido)benzoate**

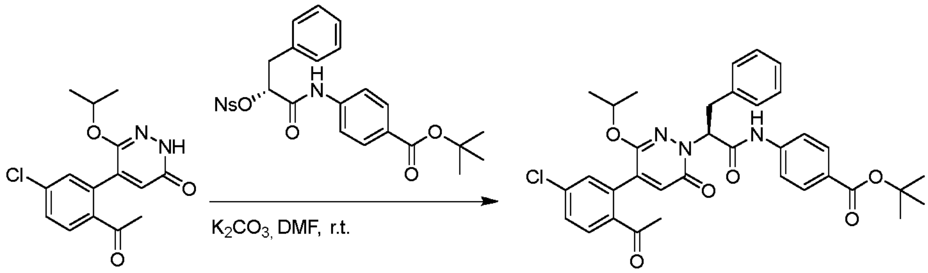


5-(2-acetyl-5-chlorophenyl)-6-isopropylpyridazin-3(2*H*)-one (47 mg, 0.15 mmol), tert-butyl (R)-4-(2-(((4-nitrophenyl)sulfonyl)oxy)-3-phenylpropanamido)benzoate (89 mg, 0.17 mmol) and potassium carbonate (43 mg, 0.31 mmol) were added to N,N-dimethylformamide (2.0 mL) at room temperature, and the reaction was performed at room temperature overnight.

The reaction was completed, quenched by adding water, and the mixture was extracted with ethyl acetate (10 ml × 3 times). The organic phases were combined, and the organic phase was first washed with saturated brine (10 ml × 2 times), then dried with anhydrous sodium sulfate, and finally concentrated under reduced pressure. The obtained residue was purified by silica gel column chromatography (eluent: ethyl acetate/n-hexane=1/2). 80 mg of pale yellow solid, tert-butyl (S)-4-(2-(4-(2-acetyl-5-chlorophenyl)-3-isopropoxy-6-oxopyridazin-1(6*H*)-yl)-3-phenylpropana mido)benzoate, was obtained (yield: 86.7%). LCMS: RT = 4.85 min, $[M+H]^+$ = 630.15.

**Step E: Synthesis of (S)-4-(2-(4-(2-acetyl-5-chlorophenyl)-3-isopropoxy-6-oxopyridazin-1(6*H*)-yl)-3-phenylprop anamido)benzoic acid**

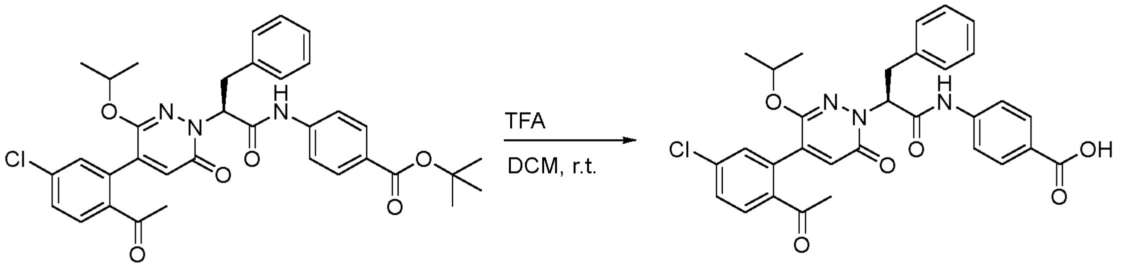


Tert-butyl (S)-4-(2-(4-(2-acetyl-5-chlorophenyl)-3-isopropoxy-6-oxopyridazin-1(6*H*)-yl)-3-phenylpropana mido)benzoate (80 mg, 0.13 mmol) was added to dichloromethane (2.0 mL) at room temperature, and trifluoroacetic acid (0.25 mL) was added dropwise. The reaction was performed at room temperature for 3 hours.

After the reaction was completed, dichloromethane was evaporated to dryness and trifluoroacetic acid was removed by an oil pump. The obtained residue was dissolved in dichloromethane (1.0 mL), and it was added dropwise to n-hexane (10.0 mL) to precipitate a white solid, which was filtered off by suction. The filter cake was washed with n-hexane and dried to give 40 mg of white solid, (S)-4-(2-(4-(2-acetyl-5-chlorophenyl)-3-isopropoxy-6-oxopyridazin-1(6*H*)-yl)-3-phenylpropana mido)benzoic acid (yield: 53.6%). LCMS: RT = 4.16 min, $[M-H]^-$ = 572.07. $^1H$ NMR (400 MHz, DMSO) δ 12.72 (s, 1H), 10.50 (s, 1H), 8.01 (d, *J*= 8.4 Hz, 1H), 7.90 (d, *J*= 8.7 Hz, 2H), 7.72 (d, *J* = 8.8 Hz, 2H), 7.69 (dd, *J* = 8.4, 2.2 Hz, 1H), 7.47 (d, *J* = 2.1 Hz, 1H), 7.28 (d, *J* = 4.4 Hz, 4H), 7.19 (dt, *J* = 8.7, 4.4 Hz, 1H), 6.88 (s, 1H), 5.77 (dd, *J* = 8.7, 6.2 Hz, 1H), 4.85 (dt, *J* = 12.4, 6.1 Hz, 1H), 3.43 (dd, *J* = 7.4, 3.2 Hz, 2H), 2.55 (s, 3H), 1.10 (dd, *J* = 9.9, 6.2 Hz, 6H).

**Example 23**

**Synthesis of (S)-4-(2-(4-(2-acetyl-5-chlorophenyl)-6-oxo-3-(2,2,2-trifluoroethoxy)pyridazine-1(6*H*)-yl)-3-phenylpropanamido)benzoic acid**

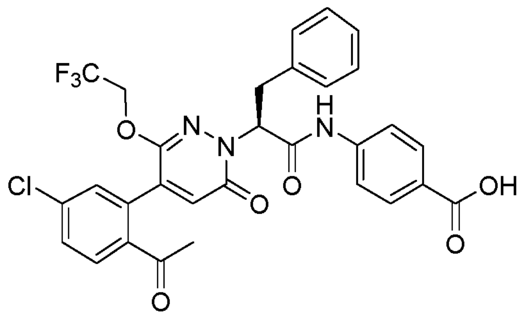

The specific synthetic route was as follows.

**Step A: Synthesis of 5-bromo-2-(4-methoxybenzyl)-6-(2,2,2-trifluoroethoxy)pyridazin-3(2*H*)-one**

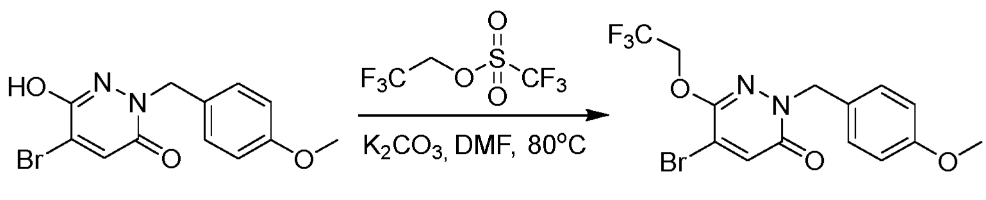

5-bromo-6-hydroxy-2-(4-methoxybenzyl)pyridazin-3(2***H***)-one (350 mg, 1.13 mmol) and potassium carbonate (535 mg, 3.88 mmol) were added to N,N-dimethylformamide (5.0 ml) at room temperature. It was stirred at 80°C for 15 minutes. At this temperature, 2,2,2-trifluoroethyl trifluoromethanesulfonate (0.6 ml) was added, and the reaction was continued for 30 minutes.

The reaction was completed, quenched by adding water, and the mixture was extracted with ethyl acetate (50 ml × 3 times). The organic phases were combined, and the organic phase was first washed with saturated brine (50 ml × 2 times), then dried with anhydrous sodium sulfate, and finally concentrated under reduced pressure. The obtained residue was purified by silica gel column chromatography (eluent: ethyl acetate/n-hexane=2/5). 123 mg of white solid, 5-bromo-2-(4-methoxybenzyl)-6-(2,2,2-trifluoroethoxy)pyridazin-3(2*H*)-one, was obtained (yield: 27.4%). LCMS: RT = 4.04 min, $[M+H]^+$ = 392.97.

**Step B: Synthesis of 5-(2-acetyl-5-chlorophenyl)-2-(4-methoxybenzyl)-6-(2,2,2-trifluoroethoxy)pyridazine-3(2*H*) -one**

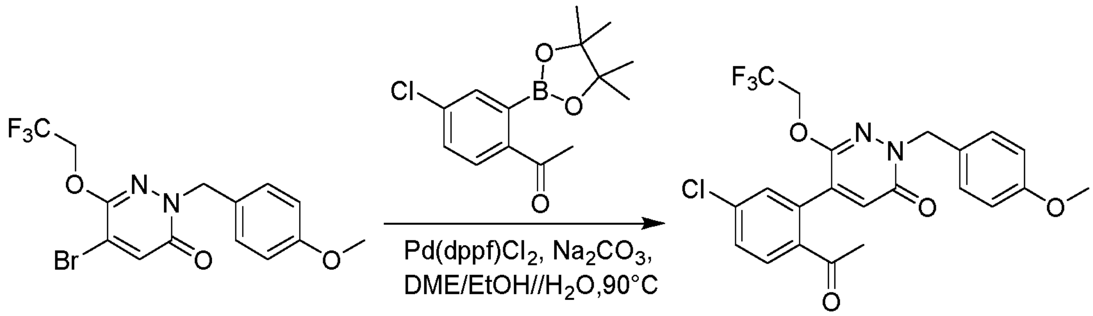


5-bromo-2-(4-methoxybenzyl)-6-(2,2,2-trifluoroethoxy)pyridazin-3(2*H*)-one (123 mg, 0.31 mmol), 6-acetyl-3-chlorophenylboronic acid pinacol ester (88 mg, 0.32 mmol) and sodium carbonate (66 mg, 0.62 mmol) were added to a three-necked flask at room temperature, and nitrogen replacement was performed. A mixed solvent (10 mL, 1,2-dimethoxyethane: ethanol: water=8:1:1) was added and nitrogen replacement was performed. 1,1'-bisdiphenylphosphinoferrocene palladium dichloride (22 mg, 0.03 mmol) was added and nitrogen replacement was performed. The mixture was heated to 90 °C to react for 1 hour.

The reaction was completed, quenched by adding water, and the mixture was extracted with ethyl acetate (50 ml × 3 times). The organic phases were combined, and the organic phase was first washed with saturated brine (50 ml × 2 times), then dried with anhydrous sodium sulfate, and finally concentrated under reduced pressure. The obtained residue was purified by silica gel column chromatography (eluent: ethyl acetate/n-hexane=1/3). 64 mg of yellow solid 5-(2-acetyl-5-chlorophenyl)-2-(4-methoxybenzyl)-6-(2,2,2-trifluoroethoxy)pyridazine-3(2*H*)-one was obtained (yield: 45.2%). LCMS: RT = 4.15 min, $[M+H]^+$ = 467.09.

**Step C: Synthesis of 5-(2-acetyl-5-chlorophenyl)-6-(2,2,2-trifluoroethoxy)pyridazin-3(2*H*)-one**

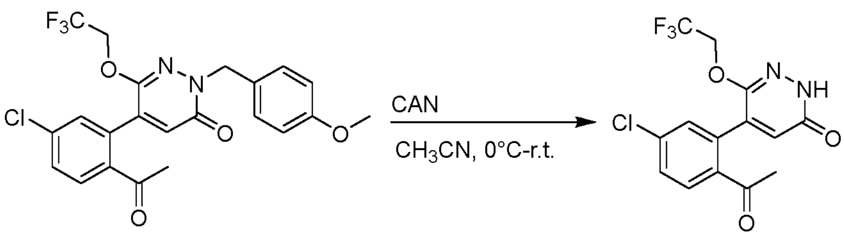


5-(2-acetyl-5-chlorophenyl)-2-(4-methoxybenzyl)-6-(2,2,2-trifluoroethoxy)pyridazine-3(2*H* )-one (64 mg, 0.14 mmol) was added to a mixed solvent (4 ml, acetonitrile: water = 3:1) at 0°C, and then ceric ammonium nitrate (751 mg, 1.37 mmol) was slowly added. After the addition was completed, the reaction was performed at room temperature for 30 minutes.

The reaction was completed, quenched by adding water, and the mixture was extracted with ethyl acetate (30 ml × 3 times). The organic phases were combined, and the organic phase was first washed with saturated brine (30 ml × 2 times), then dried with anhydrous sodium sulfate, and finally concentrated under reduced pressure. The obtained residue was purified by silica gel column chromatography (eluent: ethyl acetate/n-hexane=1/1). 30 mg of yellow solid, 5-(2-acetyl-5-chlorophenyl)-6-(2,2,2-trifluoroethoxy)pyridazin-3(2*H*)-one, was obtained (yield: 64.3%). LCMS: RT = 3.57 min, $[M+H]^+$ = 347.04.

**Step D: Synthesis of tert-butyl (S)-4-(2-(4-(2-acetyl-5-chlorophenyl)-6-oxo-3-(2,2,2-trifluoroethoxy)pyridazine-1(6*H*)-yl)-3-phenylpropanamido)benzoate**

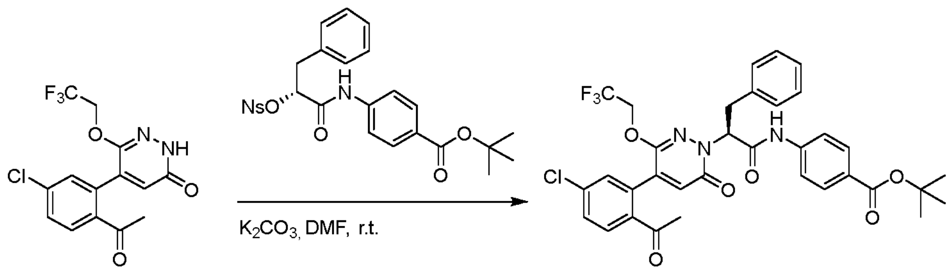


5-(2-acetyl-5-chlorophenyl)-6-(2,2,2-trifluoroethoxy)pyridazin-3(2*H*)-one (30 mg, 0.09 mmol), tert-butyl (R)-4-(2-(((4-nitrophenyl)sulfonyl)oxy)-3-phenylpropanamido)benzoate (69 mg, 0.13 mmol) and potassium carbonate (24 mg, 0.18 mmol) were added to N,N-dimethylformamide (2.0 mL) at room temperature, and the reaction was performed at room temperature overnight.

The reaction was completed, quenched by adding water, and the mixture was extracted with ethyl acetate (10 ml × 3 times). The organic phases were combined, and the organic phase was first washed with saturated brine (10 ml × 2 times), then dried with anhydrous sodium sulfate, and finally concentrated under reduced pressure. The obtained residue was purified by silica gel column chromatography (eluent: ethyl acetate/n-hexane=1/2). 80 mg of pale yellow solid, tert-butyl (S)-4-(2-(4-(2-acetyl-5-chlorophenyl)-6-oxo-3-(2,2,2-trifluoroethoxy)pyridazine-1(6*H*)-yl)-3-phe nylpropanamido)benzoate, was obtained (yield: 55.6%). LCMS: RT = 4.60 min, $[M+H]^+$ = 670.18.

**Step E: Synthesis of (S)-4-2-4-(2-acetyl-5-chlorophenyl)-6-oxo-3-(2,2,2-trifluoroethoxy)pyridazine-1(6*H*)-yl)-3-p henylpropanamido)benzoic acid**

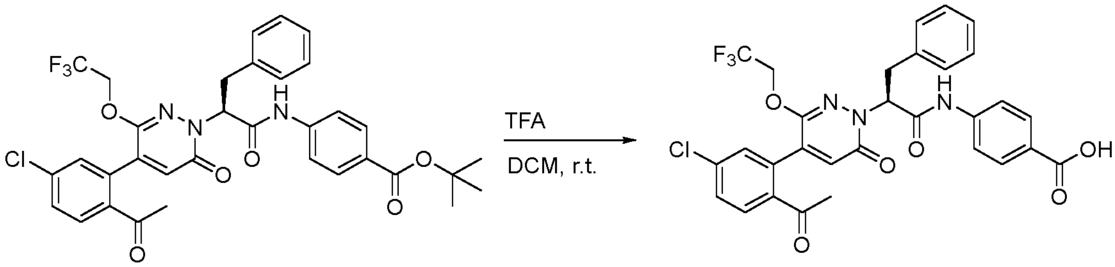


Tert-butyl (S)-4-(2-(4-(2-acetyl-5-chlorophenyl)-6-oxo-3-(2,2,2-trifluoroethoxy)pyridazine-1(6*H*)-yl)-3-phe nylpropanamido)benzoate (30 mg, 0.05 mmol) was added to dichloromethane (2.0 mL) at room temperature, and trifluoroacetic acid (0.25 mL) was added dropwise. The reaction was performed at room temperature for 3 hours.

After the reaction was completed, dichloromethane was evaporated to dryness and trifluoroacetic acid was removed by an oil pump. The obtained residue was dissolved in dichloromethane (1.0 mL), and it was added dropwise to n-hexane (10.0 mL) to precipitate a white solid, which was filtered off by suction. The filter cake was washed with n-hexane and dried to give 14 mg of white solid, (S)-4-2-4-(2-acetyl-5-chlorophenyl)-6-oxo-3-(2,2,2-trifluoroethoxy)pyridazine-1(6*H*)-yl)-3-phen ylpropanamido)benzoic acid (yield: 45.7%). LCMS: RT = 4.08 min, $[M-H]^-$ = 612.06. $^1H$ NMR (400 MHz, DMSO) δ 12.71 (s, 1H), 10.53 (s, 1H), 8.10 (d, *J*= 8.4 Hz, 1H), 7.91 (d, *J*= 8.8 Hz, 2H), 7.78 - 7.67 (m, 3H), 7.52 (d, *J* = 2.1 Hz, 1H), 7.37 - 7.24 (m, 4H), 7.19 (t, *J* = 6.3 Hz, 1H), 6.95 (s, 1H), 5.75 (dd, *J* = 9.8, 5.3 Hz, 1H), 4.70

(q, $J$ = 8.8 Hz, 2H), 3.55 (dd, $J$ = 14.2, 10.2 Hz, 1H), 3.42 (dd, $J$ = 14.2, 4.9 Hz, 1H), 2.53 (s, 3H).

**Example 24**

**Synthesis of (S)-4-(2-(4-(2-acetyl-5-chlorophenyl)-6-oxo-3-(oxetan-3-yloxy)-pyridazine-1(6*H*)-yl)-3-phen ylpropanamido)benzoic acid**

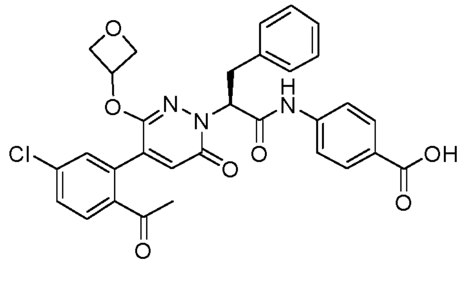

The specific synthetic route was as follows.

**Step A: Synthesis of 5-bromo-2-(4-methoxybenzyl)-6-(oxetan-3-yloxy)pyridazin-3(2*H*)-one**

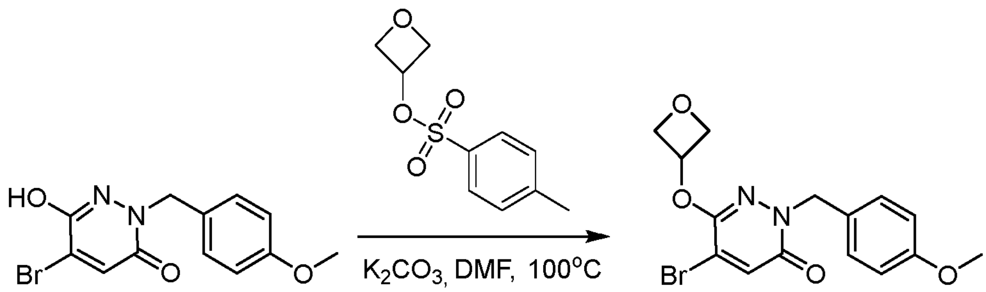


5-bromo-6-hydroxy-2-(4-methoxybenzyl)pyridazin-3(2***H***)-one (500 mg, 1.61 mmol) and potassium carbonate (890 mg, 6.45 mmol) were added to N,N-dimethylformamide (5.0 ml) at room temperature. It was stirred at 100°C for 15 minutes. At this temperature, 3-p-tosyloxyoxetane (1.47 g, 6.44 mmol) was added, and the reaction was continued overnight.

The reaction was completed, quenched by adding water, and the mixture was extracted with ethyl acetate (50 ml × 3 times). The organic phases were combined, and the organic phase was first washed with saturated brine (50 ml × 2 times), then dried with anhydrous sodium sulfate, and finally concentrated under reduced pressure. The obtained residue was purified by silica gel column chromatography (eluent: ethyl acetate/n-hexane=1/2). 330 mg of white solid, 5-bromo-2-(4-methoxybenzyl)-6-(oxetan-3-yloxy)pyridazin-3(2*H*)-one, was obtained (yield: 55.9%). LCMS: RT = 3.62 min, $[M+H]^+$ = 367.03.

**Step B: Synthesis of 5-(2-acetyl-5-chlorophenyl)-2-(4-methoxybenzyl)-6-(oxetan-3-yloxy)pyridazine-3(2*H*)-one**

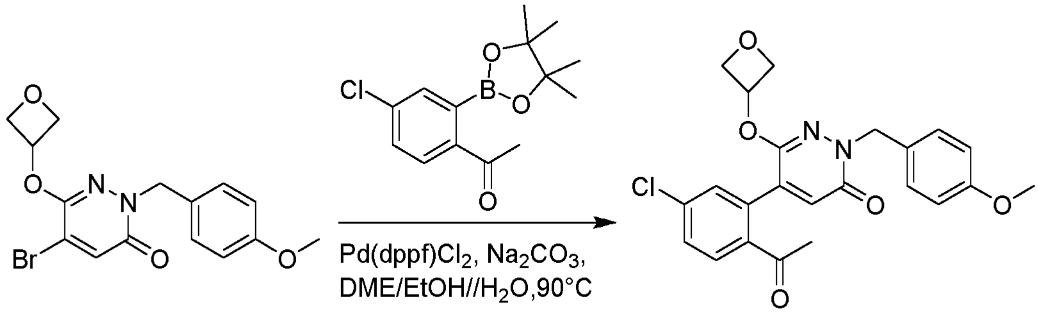


5-bromo-2-(4-methoxybenzyl)-6-(oxetan-3-yloxy)pyridazin-3(2*H*)-one (330 mg, 0.90 mmol), 6-acetyl-3-chlorophenylboronic acid pinacol ester (252 mg, 0.90 mmol) and sodium carbonate (191 mg, 1.80 mmol) were added to a three-necked flask at room temperature, and nitrogen replacement was performed. A mixed solvent (10 mL, 1,2-dimethoxyethane: ethanol: water=8:1:1) was added and nitrogen replacement was performed. 1,1'-bisdiphenylphosphinoferrocene palladium dichloride (66 mg, 0.09 mmol) was added and nitrogen replacement was performed. The mixture was heated to 90 °C to react for 1 hour.

The reaction was completed, quenched by adding water, and the mixture was extracted with ethyl acetate (50 ml × 3 times). The organic phases were combined, and the organic phase was first washed with saturated brine (50 ml × 2 times), then dried with anhydrous sodium sulfate, and finally concentrated under reduced pressure. The obtained residue was purified by silica gel column chromatography (eluent: ethyl acetate/n-hexane=1/3). 80 mg of yellow solid, 5-(2-acetyl-5-chlorophenyl)-2-(4-methoxybenzyl)-6-(oxetan-3-yloxy)pyridazine-3(2*H*)-one, was obtained (yield: 20.0%). LCMS: RT = 3.82 min, $[M+H]^+$ = 441.08.

**Step C: Synthesis of 5-(2-acetyl-5-chlorophenyl)-6-(oxetan-3-yloxy)pyridazin-3(2*H*)-one**

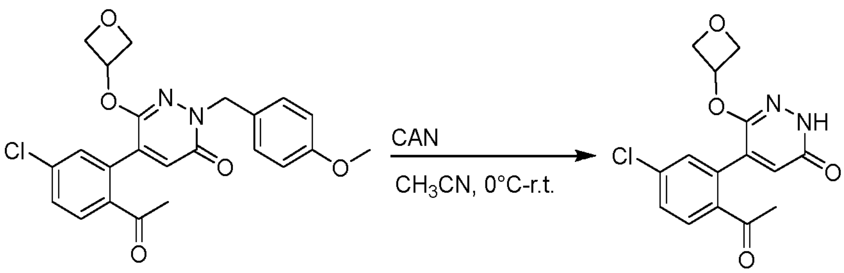


5-(2-acetyl-5-chlorophenyl)-2-(4-methoxybenzyl)-6-(oxetan-3-yloxy)pyridazine-3(2*H*)-one (80 mg, 0.18 mmol) was added to a mixed solvent (4 ml, acetonitrile: water = 3:1) at 0°C, and then ceric ammonium nitrate (790 mg, 1.44 mmol) was slowly added. After the addition was completed, the reaction was performed at room temperature for 30 minutes.

The reaction was completed, quenched by adding water, and the mixture was extracted with ethyl acetate (30 ml × 3 times). The organic phases were combined, and the organic phase was first washed with saturated brine (30 ml × 2 times), then dried with anhydrous sodium sulfate, and finally concentrated under reduced pressure. The obtained residue was purified by silica gel column chromatography (eluent: ethyl acetate/n-hexane=1/1). 30 mg of yellow solid, 5-(2-acetyl-5-chlorophenyl)-6-(oxetan-3-yloxy)pyridazin-3(2*H*)-one, was obtained (yield: 50.0%). LCMS: RT = 3.09 min, $[M+H]^+$ = 321.07.

**Step D: Synthesis of tert-butyl (S)-4-(2-(4-(2-acetyl-5-chlorophenyl)-6-oxo-3-(oxetan-3-yloxy)-pyridazine-1(6*H*)-yl)-3-phen ylpropanamido)benzoate**

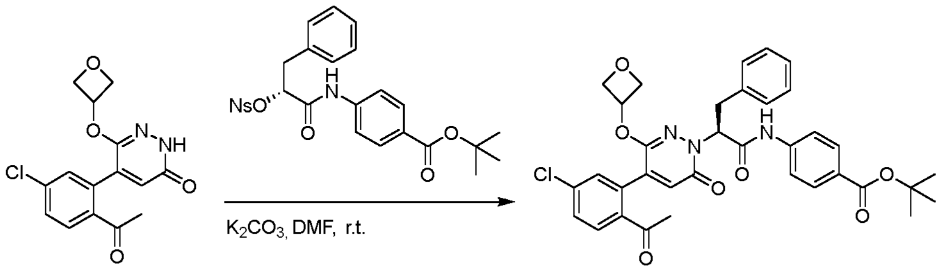


5-(2-acetyl-5-chlorophenyl)-6-(oxetan-3-yloxy)pyridazin-3(2H)-one (30 mg, 0.09 mmol), tert-butyl (R)-4-(2-(((4-nitrophenyl)sulfonyl)oxy)-3-phenylpropanamido)benzoate (69 mg, 0.13 mmol) and potassium carbonate (24 mg, 0.18 mmol) were added to N,N-dimethylformamide (2.0 mL) at room temperature, and the reaction was performed at room temperature overnight.

The reaction was completed, quenched by adding water, and the mixture was extracted with ethyl acetate (10 ml × 3 times). The organic phases were combined, and the organic phase was first washed with saturated brine (10 ml × 2 times), then dried with anhydrous sodium sulfate, and finally concentrated under reduced pressure. The obtained residue was purified by silica gel column chromatography (eluent: ethyl acetate/n-hexane=1/2). 80 mg of pale yellow solid, tert-butyl (S)-4-(2-(4-(2-acetyl-5-chlorophenyl)-6-oxo-3-(oxetan-3-yloxy)-pyridazine-1(6*H*)-yl)-3-phenylp ropanamido)benzoate was obtained (yield: 77.8%). LCMS: RT = 4.38 min, $[M+H]^+$ = 644.16.

**Step E: Synthesis of (S)-4-(2-(4-(2-acetyl-5-chlorophenyl)-6-oxo-3-(oxetan-3-yloxy)-pyridazine-1(6*H*)-yl)-3-phen ylpropanamido)benzoic acid**

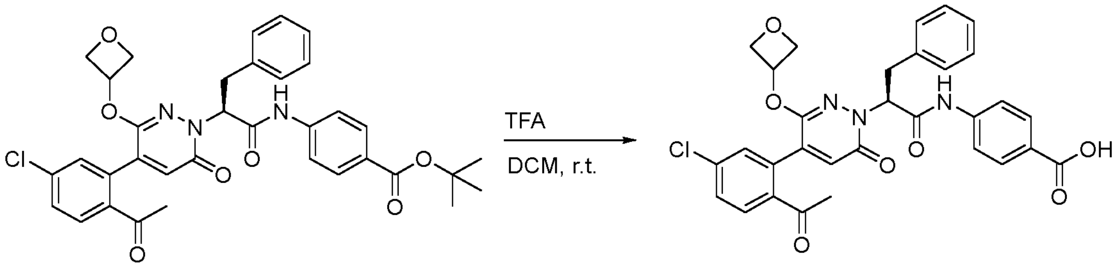


Tert-butyl (S)-4-(2-(4-(2-acetyl-5-chlorophenyl)-6-oxo-3-(oxetan-3-yloxy)-pyridazin-1(6*H*)-yl)-3-phenylpr opanamido)benzoate (45 mg, 0.07 mmol) was added to dichloromethane (2.0 mL) at room temperature, and trifluoroacetic acid (0.25 mL) was added dropwise. The reaction was performed at room temperature for 3 hours.

After the reaction was completed, dichloromethane was evaporated to dryness and trifluoroacetic acid was removed by an oil pump. The obtained residue was dissolved in dichloromethane (1.0 mL), and it was added dropwise to n-hexane (10.0 mL) to precipitate a white solid, which was filtered off by suction. The filter cake was washed with n-hexane and dried to give 11 mg of white solid, (S)-4-(2-(4-(2-acetyl-5-chlorophenyl)-6-oxo-3-(oxetan-3-yloxy)-pyridazine-1(6*H*)-yl)-3-phenylp ropanamido)benzoic acid (yield: 26.7%). LCMS: RT = 3.82 min, $[M-H]^-$ = 586.07. $^1$H NMR (400 MHz, DMSO) δ 12.70 (s, 1H), 10.48 (s, 1H), 8.05 (d, *J* = 8.4 Hz, 1H), 7.90 (d, *J* = 8.7 Hz, 2H), 7.76 - 7.70 (m, 2H), 7.55 (d, *J* = 2.0 Hz, 1H), 7.31 - 7.23 (m, 4H), 7.23 - 7.16 (m, 1H), 6.98 (s, 1H), 5.73 (dd, *J* = 8.9, 6.3 Hz, 1H), 5.25 - 5.21 (m, 1H), 4.72 (t, *J* = 6.6 Hz, 2H), 4.30 (dd, *J*= 7.4, 5.2 Hz, 2H), 3.41 - 3.35 (m, 2H), 2.60 (s, 3H).

**Example 25**

**Synthesis of (S)-4-(2-(4-(2-acetyl-5-chlorophenyl)-3-(difluoromethoxy)-6-oxopyridazin-1(6*H*)-yl)-3-phen ylpropanamido)benzoic acid**

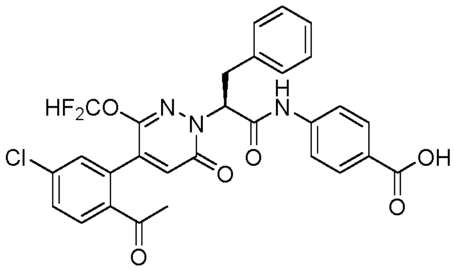

The specific synthetic route was as follows.

**Step A: Synthesis of 5-bromo-6-(difluoromethoxy)-2-(4-methoxybenzyl)pyridazin-3(2*H*)-one**

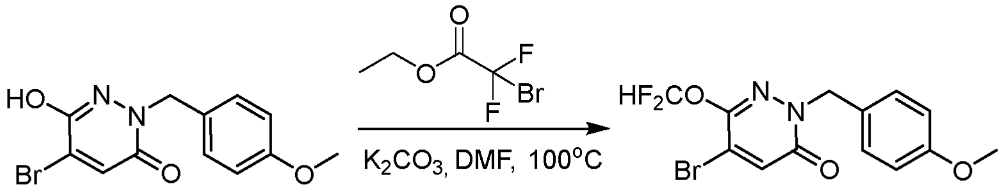


5-bromo-6-hydroxy-2-(4-methoxybenzyl)pyridazin-3(2*H*)-one (600 mg, 1.93 mmol) and potassium carbonate (1.07 g, 7.72 mmol) were added to N,N-dimethylformamide (5.0 ml) at room temperature. It was stirred at 100°C for 15 minutes. At this temperature, ethyl difluorobromoacetate (1.0 ml) was added, and the reaction was continued for 1 hour.

The reaction was completed, quenched by adding water, and the mixture was extracted with ethyl acetate (50 ml × 3 times). The organic phases were combined, and the organic phase was first washed with saturated brine (50 ml × 2 times), then dried with anhydrous sodium sulfate, and finally concentrated under reduced pressure. The obtained residue was purified by silica gel column chromatography (eluent: ethyl acetate/n-hexane=1/2). 270 mg of white solid, 5-bromo-6-(difluoromethoxy)-2-(4-methoxybenzyl)pyridazin-3(2*H*)-one, was obtained (yield: 38.9%). LCMS: RT = 3.92 min, $[M+H]^+$ = 360.97.

**Step B: Synthesis of 5-(2-acetyl-5-chlorophenyl)-6-(difluoromethoxy)-2-(4-methoxybenzyl)pyridazin-3(2*H*)-one**

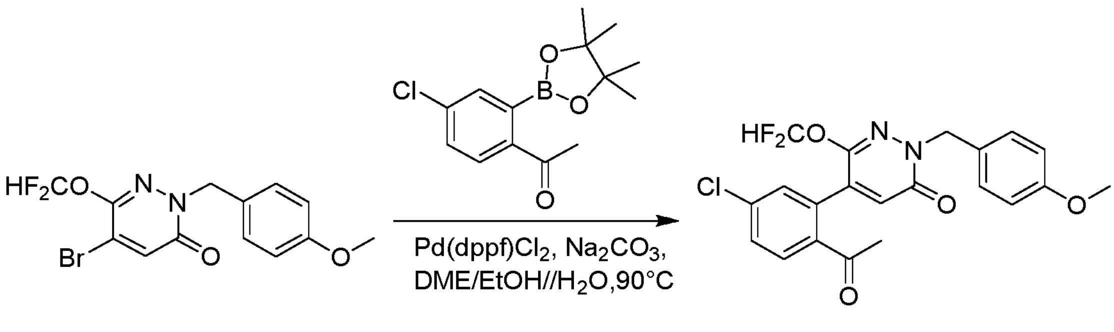


5-bromo-6-(difluoromethoxy)-2-(4-methoxybenzyl)pyridazin-3(2*H*)-one (270 mg, 0.75 mmol), 6-acetyl-3-chlorophenylboronic acid pinacol ester (210 mg, 0.75 mmol) and sodium carbonate (160 mg, 1.50 mmol) were added to a three-necked flask at room temperature, and nitrogen replacement was performed. A mixed solvent (10 mL, 1,2-dimethoxyethane: ethanol: water=8:1:1) was added and nitrogen replacement was performed. 1,1'-bisdiphenylphosphinoferrocene palladium dichloride (59 mg, 0.08 mmol) was added and nitrogen replacement was performed. The mixture was heated to 90 °C to react for 1 hour.

The reaction was completed, quenched by adding water, and the mixture was extracted with ethyl acetate (50 ml × 3 times). The organic phases were combined, and the organic phase was first washed with saturated brine (50 ml × 2 times), then dried with anhydrous sodium sulfate, and finally concentrated under reduced pressure. The obtained

residue was purified by silica gel column chromatography (eluent: ethyl acetate/n-hexane=1/3). 110 mg of yellow solid, 5-(2-acetyl-5-chlorophenyl)-6-(difluoromethoxy)-2-(4-methoxybenzyl)pyridazin-3(2*H*)-one, was obtained (yield: 33.3%). LCMS: RT = 4.08 min, $[M+H]^+$ = 435.07.

**Step C: Synthesis of 5-(2-acetyl-5-chlorophenyl)-6-(difluoromethoxy)-pyridazin-3(2*H*)-one**

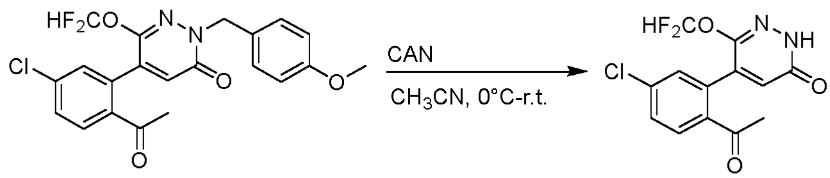


5-(2-acetyl-5-chlorophenyl)-6-(difluoromethoxy)-2-(4-methoxybenzyl)pyridazin-3(2*H*)-one (110 mg, 0.25 mmol) was added to a mixed solvent (4 ml, acetonitrile: water = 3:1) at 0°C, and then ceric ammonium nitrate (1.10 g, 2.00 mmol) was slowly added. After the addition was completed, the reaction was performed at room temperature for 30 minutes.

The reaction was completed, quenched by adding water, and the mixture was extracted with ethyl acetate (30 ml × 3 times). The organic phases were combined, and the organic phase was first washed with saturated brine (30 ml × 2 times), then dried with anhydrous sodium sulfate, and finally concentrated under reduced pressure. The obtained residue was purified by silica gel column chromatography (eluent: ethyl acetate/n-hexane=1/1). 50 mg of yellow solid, 5-(2-acetyl-5-chlorophenyl)-6-(difluoromethoxy)-pyridazin-3(2*H*)-one, was obtained (yield: 64.0%). LCMS: RT = 3.47 min, $[M+H]^+$ = 315.02.

**Step D: Synthesis of tert-butyl (S)-4-(2-(4-(2-acetyl-5-chlorophenyl)-3-(difluoromethoxy)-6-oxopyridazin-1(6*H*)-yl)-3-phen ylpropanamido)benzoate**

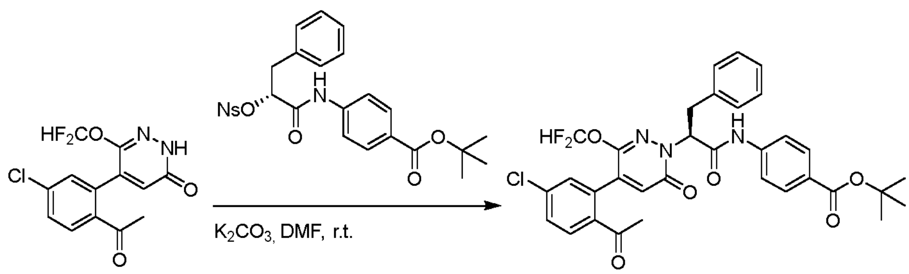


Solid 5-(2-acetyl-5-chlorophenyl)-6-(difluoromethoxy)pyridazin-3(2*H*)-one (50 mg, 0.16 mmol), tert-butyl (R)-4-(2-(((4-nitrophenyl)sulfonyl)oxy)-3-phenylpropanamido)benzoate (92 mg, 0.18 mmol) and potassium carbonate (44 mg, 0.32 mmol) were added to N,N-dimethylformamide (2.0 mL) at room temperature, and the reaction was performed at room temperature overnight.

The reaction was completed, quenched by adding water, and the mixture was extracted with ethyl acetate (10 ml × 3 times). The organic phases were combined, and the organic phase was first washed with saturated brine (10 ml × 2 times), then dried with anhydrous sodium sulfate, and finally concentrated under reduced pressure. The obtained residue was purified by silica gel column chromatography (eluent: ethyl acetate/n-hexane=1/2). 55 mg of pale yellow solid, tert-butyl (S)-4-(2-(4-(2-acetyl-5-chlorophenyl)-3-(difluoromethoxy)-6-oxopyridazin-1(6*H*)-yl)-3-phenylpr opanamido)benzoate, was obtained (yield: 56.3%). LCMS: RT = 4.54 min, $[M+H]^+$ = 638.14.

**Step E: Synthesis of (S)-4-(2-(4-(2-acetyl-5-chlorophenyl)-6-oxo-3-(oxetan-3-yloxy)-pyridazine-1(6*H*)-yl)-3-phen ylpropanamido)benzoic acid**

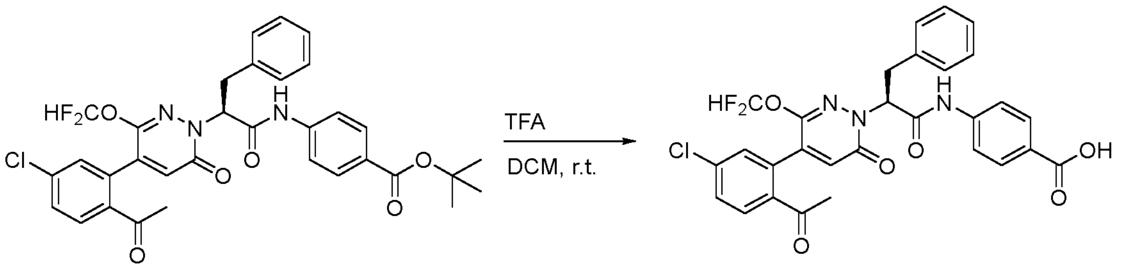


Tert-butyl (S)-4-(2-(4-(2-acetyl-5-chlorophenyl)-3-(difluoromethoxy)-6-oxopyridazin-1(6*H*)-yl)-3-phenylpr opanamido)benzoate (55 mg, 0.09 mmol) was added to dichloromethane (2.0 mL) at room temperature, and trifluoroacetic acid (0.25 mL) was added dropwise. The reaction was performed at room temperature for 3 hours.

After the reaction was completed, dichloromethane was evaporated to dryness and trifluoroacetic acid was removed by an oil pump. The obtained residue was dissolved in dichloromethane (1.0 mL), and it was added dropwise to n-hexane (10.0 mL) to precipitate a white solid, which was filtered off by suction. The filter cake was washed with n-hexane and dried to give 20 mg of white solid, (S)-4-(2-(4-(2-acetyl-5-chlorophenyl)-6-oxo-3-(oxetan-3-yloxy)-pyridazine-1(6*H*)-yl)-3-phenylp ropanamido)benzoic acid (yield: 38.3%). LCMS: RT = 4.04 min, $[M-H]^-$ = 580.04. $^1H$ NMR (400 MHz, DMSO) δ 12.74 (s, 1H), 10.59 (s, 1H), 8.12 (d, *J*= 8.4 Hz, 1H), 7.91 (d, *J*= 8.7 Hz, 2H), 7.76 (dd, *J* = 8.4, 2.2 Hz, 1H), 7.72 (d, *J* = 8.7 Hz, 2H), 7.57 (d, *J* = 1.9 Hz, 1H), 7.36 (t, *J* = 62.5 Hz, 1H), 7.35 - 7.25 (m, 4H), 7.19 (dd, *J* = 12.2, 5.1 Hz, 1H), 7.03 (s, 1H), 5.75 (dd, *J* = 10.2, 5.0 Hz, 1H), 3.52 (dd, *J* = 14.1, 10.5 Hz, 1H), 3.41 (dd, *J* = 14.3, 4.8 Hz, 1H), 2.57 (s, 3H).

**Example 26**

**Synthesis of (S)-2-(4-(2-acetyl-5-chlorophenyl)-3-methoxy-6-oxopyridazin-1(6*H*)-yl)-3-phenyl-N-(quinox alin-6-yl)propanamide**

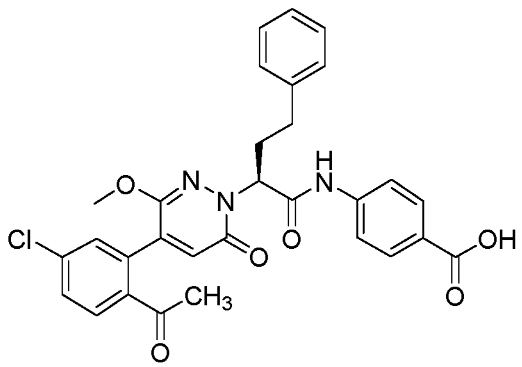

The specific synthetic route was as follows.

**Step A: Methyl (R)-3-phenyl-2-(((trifluoromethyl)sulfonyl)oxy)butyrate**

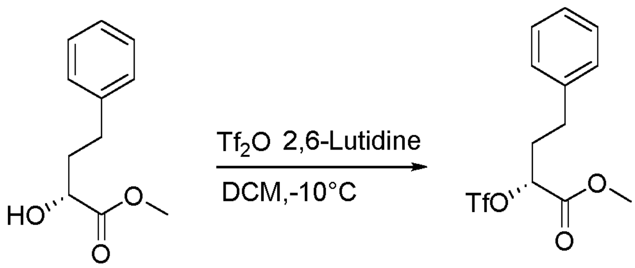

Methyl (R)-2-hydroxy-3-phenylbutyrate (3.00 g, 14.41 mmol) was dissolved in dichloromethane (25.0 mL), 2,6-dimethyl pyridine (2.0 mL) was added, and then trifluoromethanesulfonic anhydride (3.0 mL) was slowly added at -10°C. The reaction was stirred for 30 minutes.

The reaction solution was added with water (30 mL) to quench the reaction. Ethyl acetate (100 mL) was added to the reaction solution. The mixed solution was washed with saturated brine (30 mL × 3 times), then dried with anhydrous sodium sulfate, and finally concentrated under reduced pressure. The obtained residue was purified by silica gel column chromatography (eluent: ethyl acetate/petroleum ether=20/1). 2.0 g of colorless oily methyl (R)-3-phenyl-2-(((trifluoromethyl)sulfonyl)oxy)butyrate was obtained (yield: 42.4%). LCMS: RT = 4.43 min.

**Step B: Synthesis of methyl (S)-2-(4-(2-acetyl-5-chlorophenyl)-3-methoxy-6-oxopyridazin-1(6*H*)-yl)-3-phenylbutyrate**

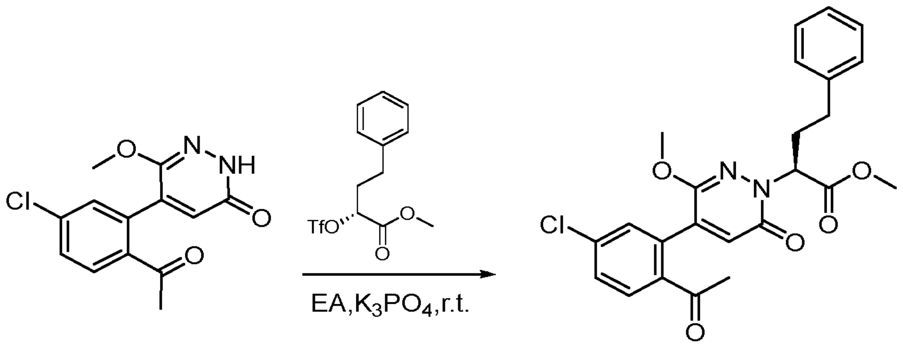


5-(2-acetyl-5-chlorophenyl)-6-methoxypyridazin-3(2*H*)-one (200 mg, 0.50 mmol) and potassium phosphate (425 mg, 2.00 mmol) were dissolved in ethyl acetate (6.0 mL). Subsequently, methyl (R)-3-phenyl-2-(((trifluoromethyl)sulfonyl)oxy)butyrate (204 mg, 0.60 mmol) was added to the above solution, and the solution was stirred at room temperature for 3 hours.

Water (1 mL) was added to the reaction solution to quench the reaction. Ethyl acetate (50 mL) was added, and the mixed solution was washed with saturated brine (10 mL×3 times), then dried with anhydrous sodium sulfate, and finally concentrated under reduced pressure. The obtained residue was purified by silica gel column chromatography (eluent: ethyl acetate/petroleum ethet=1/1). 110 mg of yellow solid, methyl (S)-2-(4-(2-acetyl-5-chlorophenyl)-3-methoxy-6-oxopyridazin-1(6*H*)-yl)-3-phenylbutyrate, was obtained (yield: 46.0%). LCMS: RT = 4.29 min, $[M+H]^+$ = 469.06.

**Step C: Synthesis of (S)-2-(4-(2-acetyl-5-chlorophenyl)-3-methoxy-6-oxopyridazin-1(6*H*)-yl)-3-phenylbutyric acid**

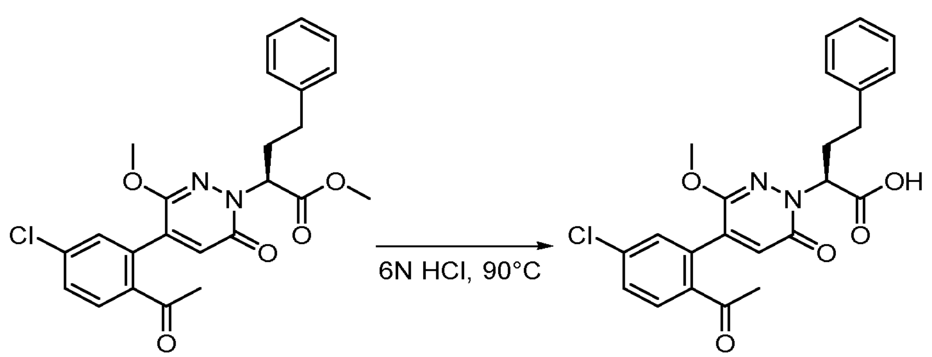


Methyl (S)-2-(4-(2-acetyl-5-chlorophenyl)-3-methoxy-6-oxopyridazin-1(6*H*)-yl)-3-phenylbutyrate (110 mg, 0.23 mmol) was dissolved in 6N HCl (4.0 mL). The reaction was performed at 90°C overnight.

After the reaction was completed, it was cooled to -10°C, and the pH value was adjusted to 3-4 with 6N NaOH. Ethyl acetate (30 mL) was added, and the mixed solution was washed with saturated brine (10 mL×3 times), then dried

with anhydrous sodium sulfate, and finally concentrated under reduced pressure. 100 mg of yellow solid, (S)-2-(4-(2-acetyl-5-chlorophenyl)-3-methoxy-6-oxopyridazin-1(6*H*)-yl)-3-phenylbutyric acid, was obtained. It was directly used in the next step without purification. LCMS: RT = 3.97 min, $[M+H]^+$ = 441.10.

**Step D: Synthesis of tert-butyl (S)-4-(2-(4-(2-acetyl-5-chlorophenyl)-3-methoxy-6-oxopyridazin-1(6*H*)-yl)-4-phenylbutana mido)benzoate**

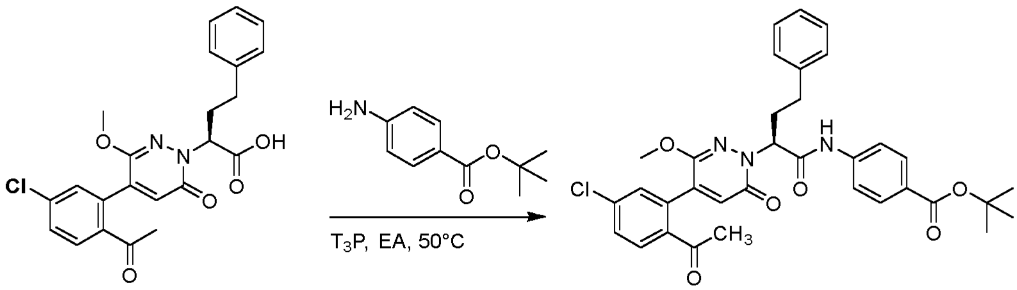


(S)-2-(4-(2-acetyl-5-chlorophenyl)-3-methoxy-6-oxopyridazin-1(6*H*)-yl)-3-phenylbutyric acid (100 mg, 0.23 mmol) and tert-butyl 4-amino-benzate (53 mg, 0.27 mmol) were dissolved in ethyl acetate (3.0 mL) at room temperature, and N,N-diisopropylethylamine (0.1 ml) was added. Subsequently, 1-propylphosphoric anhydride (0.5 ml) was added to the above solution. The reaction solution was heated to 50°C and stirred for 4 hours.

The reaction was completed, quenched by adding water, and the mixture was extracted with ethyl acetate (10 ml × 3 times). The organic phases were combined, and the organic phase was first washed with saturated brine (10 ml × 2 times), then dried with anhydrous sodium sulfate, and finally concentrated under reduced pressure. The obtained residue was purified by silica gel column chromatography (eluent: ethyl acetate/n-hexane=1/3). 100 mg of pale yellow solid, tert-butyl (S)-4-(2-(4-(2-acetyl-5-chlorophenyl)-3 -methoxy-6-oxopyridazin-1(6*H*)-yl)-4-phenylbutanamido )benzoate, was obtained (yield: 69.6%). LCMS: RT = 4.64 min, $[M+H]^+$ = 616.20.

**Step E: Synthesis of (S)-4-(2-(4-(2-acetyl-5-chlorophenyl)-3-methoxy-6-oxopyridazin-1(6*H*)-yl)-4-phenylbutana mido)benzoic acid**

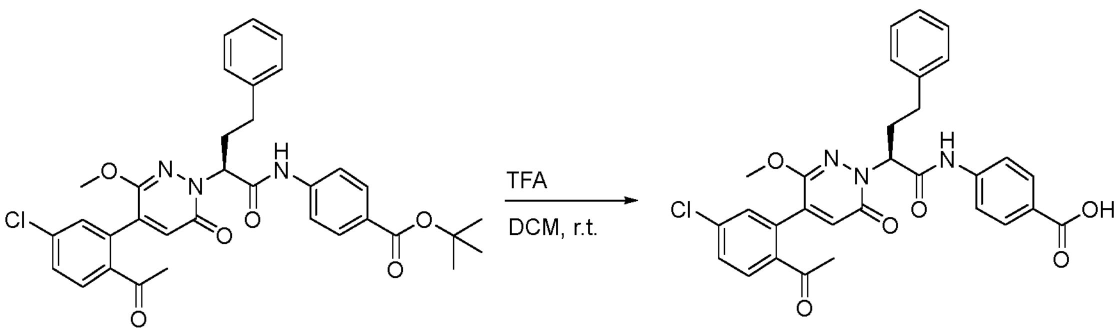


tert-Butyl (S)-4-(2-(4-(2-acetyl-5-chlorophenyl)-3-methoxy-6-oxopyridazin-1(6*H*)-yl)-4-phenylbutanamido )benzoate (100 mg, 0.16 mmol) was added to dichloromethane (2.0 mL) at room temperature, and trifluoroacetic acid (0.25 mL) was added dropwise. The reaction was performed at room temperature for 3 hours.

After the reaction was completed, dichloromethane was evaporated to dryness and trifluoroacetic acid was removed by an oil pump. The obtained residue was dissolved in dichloromethane (1.0 mL), and it was added dropwise to n-hexane (10.0 mL) to precipitate a white solid, which was filtered off by suction. The filter cake was washed with n-hexane and dried to give 70 mg of white solid, (S)-4-(2-(4-(2-acetyl-5-chlorophenyl)-3-methoxy-6-oxopyridazin-1(6*H*)-yl)-4-phenylbutanamido )benzoic acid (yield: 78.3%). LCMS: RT = 4.05 min, $[M-H]^-$ = 558.06. $^1$H NMR (500 MHz, DMSO) δ 12.72 (s, 1H), 10.49 (s, 1H), 8.03 (d, *J*= 8.4 Hz, 1H), 7.90 (d, *J*= 8.8 Hz, 2H), 7.76 - 7.68 (m, 3H), 7.58 (d, *J* = 2.1 Hz, 1H), 7.33 - 7.21 (m, 4H), 7.19 (t, *J* = 7.2 Hz, 1H), 7.00 (s, 1H), 5.38 (dd, *J* = 10.2, 4.4 Hz, 1H), 3.66 (s, 3H), 2.67 (t, *J* =

7.8 Hz, 2H), 2.57 (s, 3H), 2.45 - 2.14 (m, 2H).

**Example 27**

**Synthesis of (S)-4-(2-(4-(2-acetyl-5-chlorophenyl)-3-methoxy-6-oxopyridazin-1(6*H*)-yl)-3-phenyl propanamido)-2-chlorobenzoic acid**

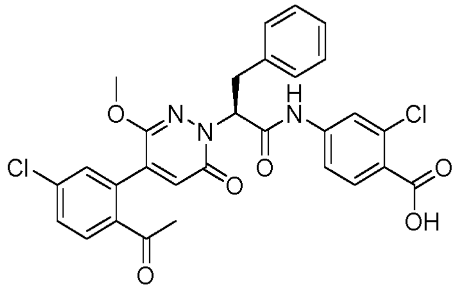

The specific synthetic route was as follows.

**Step A: Synthesis of methyl (S)-4-2-(4-(2-acetyl-5-chlorophenyl)-3-methoxy-6-oxopyridazin-1(6*H*)-yl)-3-phenyl-propana mido)-2-chlorobenzoate**

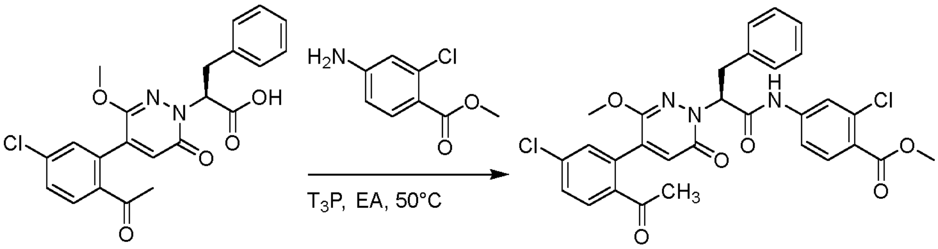


(S)-2-(4-(2-acetyl-5-chlorophenyl)-3-methoxy-6-oxopyridazin-1(6*H*)-yl)-3-phenylpropanic acid (40 mg, 0.09 mmol), methyl 2-chloro-4-amino-benzate (21 mg, 0.11 mmol) were dissolved in ethyl acetate (3.0 mL) at room temperature, and N,N-diisopropylethylamine (0.05 ml) was added. Subsequently, 1-propylphosphoric anhydride (0.2 ml) was added to the above solution. The reaction solution was heated to 50°C and stirred for 4 hours.

The reaction was completed, quenched by adding water, and the mixture was extracted with ethyl acetate (10 ml × 3 times). The organic phases were combined, and the organic phase was first washed with saturated brine (10 ml × 2 times), then dried with anhydrous sodium sulfate, and finally concentrated under reduced pressure. The obtained residue was purified by silica gel column chromatography (eluent: ethyl acetate/n-hexane=1/2). 35 mg of pale yellow solid, methyl (S)-4-2-(4-(2-acetyl-5-chlorophenyl)-3-methoxy-6-oxopyridazin-1(6*H*)-yl)-3-phenylpropanamid o)-2-chlorobenzoate, was obtained (yield: 66.7%). LCMS: RT = 4.31 min, $[M+H]^+$ = 594.05.

**Step B: Synthesis of (S)-4-(2-(4-(2-acetyl-5-chlorophenyl)-3-methoxy-6-oxopyridazin-1(6*H*)-yl)-3-phenyl propanamido)-2-chlorobenzoic acid**

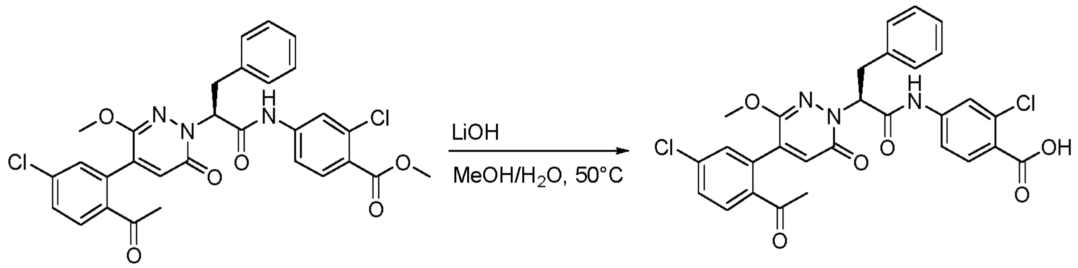


Methyl (S)-4-2-(4-(2-acetyl-5-chlorophenyl)-3-methoxy-6-oxopyridazin-1(6*H*)-yl)-3-phenylpropanamid o)-2-chlorobenzoate (35 mg, 0.06 mmol) was dissolved in methanol (2.0 mL). Subsequently, an aqueous solution (1.0 mL) of lithium hydroxide monohydrate (5 mg, 0.11 mmol) was added to the above solution. It was stirred at 50°C overnight.

Diluted hydrochloric acid solution (1.0 mol/L) was slowly added dropwise to the reaction solution to adjust the pH value to 3-4. Ethyl acetate (50 mL) was added, and the mixed solution was washed with saturated brine (10 mL×3 times), then dried with anhydrous sodium sulfate, and finally concentrated under reduced pressure. The obtained residue was purified by silica gel column chromatography (eluent: ethyl acetate/petroleum ether=2/1). 6 mg of white solid, (S)-4-(2-(4-(2-acetyl-5-chlorophenyl)-3-methoxy-6-oxopyridazin-1(6*H*)-yl)-3-phenylpropanami do)-2-chlorobenzoic acid, was obtained (yield: 17.3%). LCMS: RT = 4.08 min, $[M-H]^-$ = 578.04.

**Example 28**

**Synthesis of (S)-4-(2-(4-(2-acetyl-5-chlorophenyl)-3-methoxy-6-oxopyridazin-1(6*H*)-yl)-3-phenylpropan amido)-2-methoxybenzoic acid**

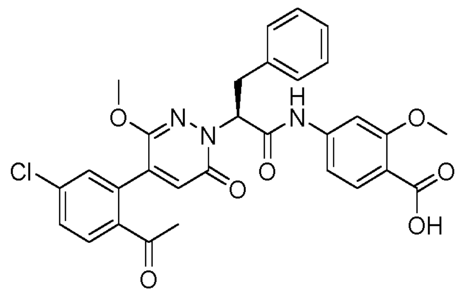

The specific synthetic route was as follows.

**Step A: Synthesis of methyl (S)-4-(2-(4-(2-acetyl-5-chlorophenyl)-3-methoxy-6-oxopyridazin-1(6*H*)-yl)-3-phenylpropan amido)-2-methoxybenzoate**

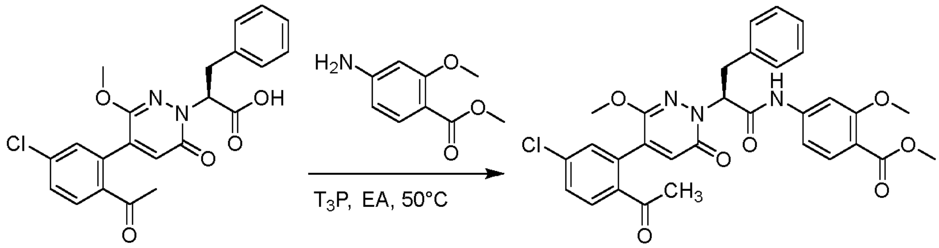


(S)-2-(4-(2-acetyl-5-chlorophenyl)-3-methoxy-6-oxopyridazin-1(6*H*)-yl)-3-phenylpropanic acid (40 mg, 0.09 mmol), methyl 2-methoxy-4-amino-benzate (22 mg, 0.12 mmol) were dissolved in ethyl acetate (3.0 mL) at room temperature, and N,N-diisopropylethylamine (0.05 ml) was added. Subsequently, 1-propylphosphoric anhydride (0.2 ml) was added to the above solution. The reaction solution was heated to 50°C and stirred for 4 hours.

The reaction was completed, quenched by adding water, and the mixture was extracted with ethyl acetate (10 ml × 3 times). The organic phases were combined, and the organic phase was first washed with saturated brine (10 ml × 2 times), then dried with anhydrous sodium sulfate, and finally concentrated under reduced pressure. The obtained residue was purified by silica gel column chromatography (eluent: ethyl acetate/n-hexane=1/2). 50 mg of pale yellow solid, methyl (S)-4-(2-(4-(2-acetyl-5-chlorophenyl)-3-methoxy-6-oxopyridazin-1(6*H*)-yl)-3-phenylpropanami do)-2-methoxybenzoate, was obtained (yield: 94.2%). LCMS: RT = 4.09 min, $[M+H]^+$ = 590.11.

**Step B: Synthesis of (S)-4-(2-(4-(2-acetyl-5-chlorophenyl)-3-methoxy-6-oxopyridazin-1(6*H*)-yl)-3-phenylpropan amido)-2-methoxybenzoic acid**

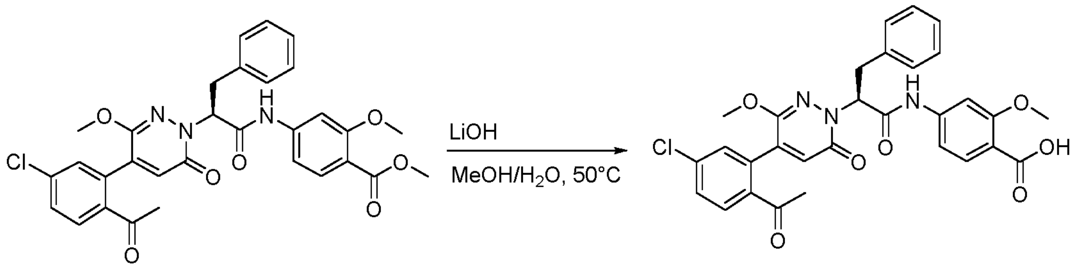


Methyl (S)-4-(2-(4-(2-acetyl-5-chlorophenyl)-3-methoxy-6-oxopyridazin-1(6*H*)-yl)-3-phenylpropanami do)-2-methoxybenzoate (50 mg, 0.08 mmol) was dissolved in methanol (2.0 mL). Subsequently, an aqueous solution (1.0 mL) of lithium hydroxide monohydrate (8.4 mg, 0.16 mmol) was added to the above solution. It was stirred at 50°C for 4 hours.

Diluted hydrochloric acid solution (1.0 mol/L) was slowly added dropwise to the reaction solution to adjust the pH value to 3-4. Ethyl acetate (50 mL) was added, and the mixed solution was washed with saturated brine (10 mL×3 times), then dried with anhydrous sodium sulfate, and finally concentrated under reduced pressure. The obtained residue was purified by silica gel column chromatography (eluent: ethyl acetate/petroleum ether=2/1). 6 mg of white solid, (S)-4-(2-(4-(2-acetyl-5-chlorophenyl)-3-methoxy-6-oxopyridazin-1(6*H*)-yl)-3-phenylpropanami do)-2-methoxybenzoic acid, was obtained (yield: 13.0%). LCMS: RT = 3.93 min, $[M-H]^-$ = 574.09.

**Example 29**

**Synthesis of (S)-4-(2-(4-(2-acetyl-5-chlorophenyl)-3-methoxy-6-oxopyridazin-1(6*H*)-yl)-3-phenylpropan amido)-2-hydroxybenzoic acid**

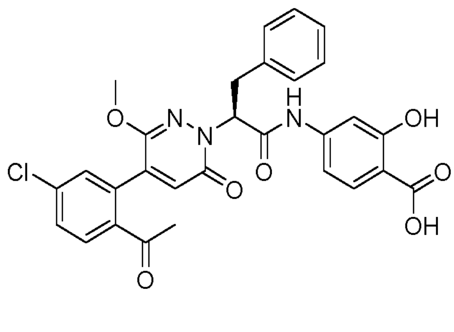

The specific synthetic route was as follows.

**Step A: Synthesis of methyl (S)-4-(2-(4-(2-acetyl-5-chlorophenyl)-3-methoxy-6-oxopyridazin-1(6*H*)-yl)-3-phenyl-propan amido)-2-hydroxybenzoate**

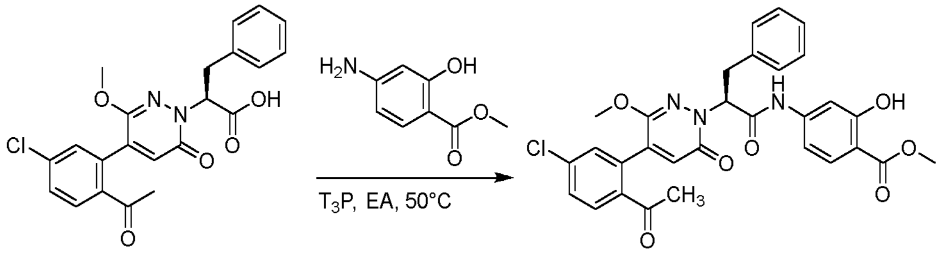


(S)-2-(4-(2-acetyl-5-chlorophenyl)-3-methoxy-6-oxopyridazin-1(6*H*)-yl)-3-phenylpropanic acid (40 mg, 0.09 mmol), methyl 2-hydroxy-4-amino-benzate (19 mg, 0.12 mmol) were dissolved in ethyl acetate (3.0 mL) at room temperature, and N,N-diisopropylethylamine (0.05 ml) was added. Subsequently, 1-propylphosphoric anhydride (0.2 ml) was added to the above solution. The reaction solution was heated to 50°C and stirred for 4 hours.

The reaction was completed, quenched by adding water, and the mixture was extracted with ethyl acetate (10 ml × 3 times). The organic phases were combined, and the organic phase was first washed with saturated brine (10 ml × 2 times), then dried with anhydrous sodium sulfate, and finally concentrated under reduced pressure. The obtained residue was purified by silica gel column chromatography (eluent: ethyl acetate/n-hexane=1/2). 6 mg of pale yellow solid, methyl (S)-4-(2-(4-(2-acetyl-5-chlorophenyl)-3-methoxy-6-oxopyridazin-1(6*H*)-yl)-3-phenylpropanami do)-2-hydroxybenzoate, was obtained (yield: 55.6%). LCMS: RT = 4.27 min, $[M-H]^-$ = 576.23.

**Step B: Synthesis of (S)-4-(2-(4-(2-acetyl-5-chlorophenyl)-3-methoxy-6-oxopyridazin-1(6*H*)-yl)-3-phenylpropan amido)-2-hydroxybenzoic acid**

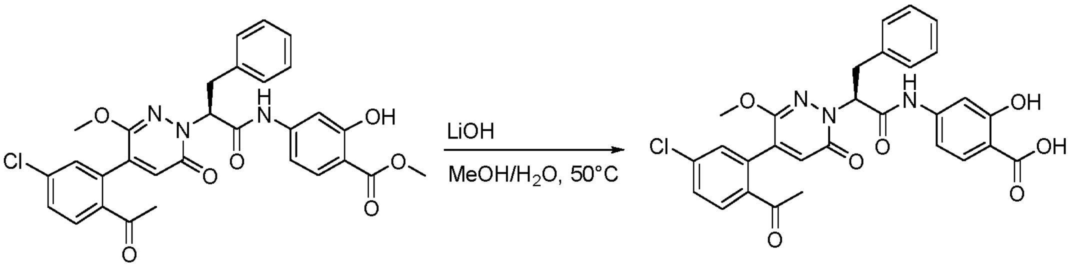


Methyl (S)-4-(2-(4-(2-acetyl-5-chlorophenyl)-3-methoxy-6-oxopyridazin-1(6*H*)-yl)-3-phenylpropanami do)-2-hydroxybenzoate (30 mg, 0.05 mmol) was dissolved in methanol (2.0 mL). Subsequently, an aqueous solution (1.0 mL) of lithium hydroxide monohydrate (5 mg, 0.11 mmol) was added to the above solution. It was stirred at 50°C for 4 hours.

Diluted hydrochloric acid solution (1.0 mol/L) was slowly added dropwise to the reaction solution to adjust the pH value to 3-4. Ethyl acetate (50 mL) was added, and the mixed solution was washed with saturated brine (10 mL×3 times), then dried with anhydrous sodium sulfate, and finally concentrated under reduced pressure. The obtained residue was purified by silica gel column chromatography (eluent: ethyl acetate/petroleum ether=2/1). 5 mg of white solid, (S)-4-(2-(4-(2-acetyl-5-chlorophenyl)-3-methoxy-6-oxopyridazin-1(6*H*)-yl)-3-phenylpropanami do)-2-hydroxybenzoic acid, was obtained (yield: 17.9%). LCMS: RT = 4.36 min, $[M-H]^-$ = 560.07.

**Example 30**

**Synthesis of (S)-2-(4-(2-acetyl-5-chlorophenyl)-3-methoxy-6-oxopyridazin-1(6*H*)-yl)-N-(1-oxo-1,2-dihyd roisoquinolin-6-yl)-3-phenylpropanamide**

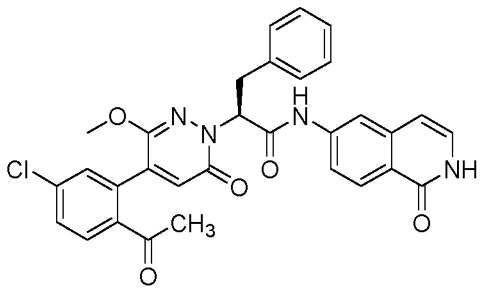

The specific synthetic route was as follows.

**Step A: Synthesis of (S)-2-(4-(2-acetyl-5-chlorophenyl)-3-methoxy-6-oxopyridazin-1(6*H*)-yl)-N-(1-oxo-1,2-dihyd roisoquinolin-6-yl)-3-phenylpropanamide**

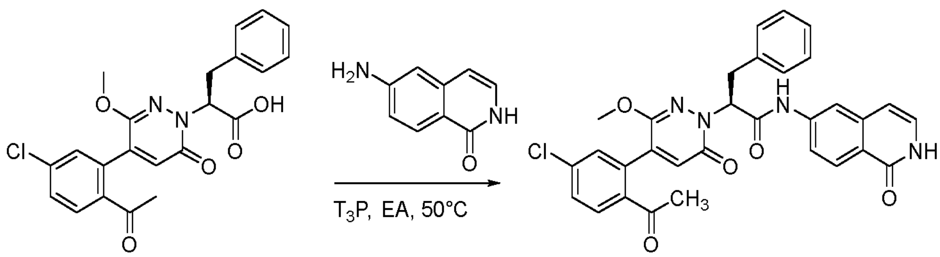


(S)-2-(4-(2-acetyl-5-chlorophenyl)-3-methoxy-6-oxopyridazin-1(6*H*)-yl)-3-phenylpropanic acid (40 mg, 0.09 mmol), 6-aminoisoquinolin-1(2H)-one (18 mg, 0.12 mmol) were dissolved in ethyl acetate (3.0 mL) at room temperature, and N,N-diisopropylethylamine (0.05 ml) was added. Subsequently, 1-propylphosphoric anhydride (0.2 ml) was added to the above solution. The reaction solution was heated to 50°C and stirred for 4 hours.

The reaction was completed, quenched by adding water, and the mixture was extracted with ethyl acetate (10 ml × 3 times). The organic phases were combined, and the organic phase was first washed with saturated brine (10 ml × 2 times), then dried with anhydrous sodium sulfate, and finally concentrated under reduced pressure. The obtained residue was purified by silica gel column chromatography (eluent: ethyl acetate/n-hexane=1/2). 10 mg of pale yellow solid, (S)-2-(4-(2-acetyl-5-chlorophenyl)-3-methoxy-6-oxopyridazin-1(6*H*)-yl)-1-oxo-1,2-dihydrois oquinolin-6-yl)-3-phenylpropanamide, was obtained (yield: 19.6%). LCMS: RT = 3.80 min, $[M-H]^-$ = 567.07. $^1$H NMR (400 MHz, DMSO) δ 13.15 (s, 1H), 10.59 (s, 1H), 8.00 (d, *J* = 8.4 Hz, 1H), 7.89 (d, *J*= 1.9 Hz, 1H), 7.84 (d, *J*= 8.6 Hz, 1H), 7.69 (dd, *J* = 8.3, 2.1 Hz, 1H), 7.57 (dd, *J* = 8.6, 20 Hz, 1H), 7.50 (d, *J* = 2.1 Hz, 1H), 7.34 - 7.25 (m, 4H), 7.19 (t, *J* = 7.5 Hz, 1H), 6.91 (s, 1H), 5.70 (dd, *J* = 9.8, 5.1 Hz, 1H), 3.66 (s, 3H), 3.52 - 3.38 (m, 2H), 2.53 (s, 3H).

**Example 31**

**Synthesis of (S)-2-(4-(2-acetyl-5-chlorophenyl)-3-methoxy-6-oxopyridazin-1(6*H*)-yl)-N-(2-oxo-1,2,3,4-tet rahydroquinolin-6-yl)-3-phenylpropanamide**

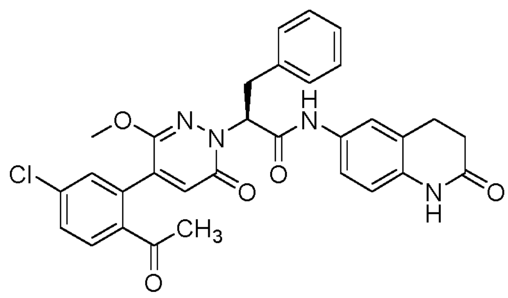

The specific synthetic route was as follows.

**Step A: Synthesis of (S)-2-(4-(2-acetyl-5-chlorophenyl)-3-methoxy-6-oxopyridazin-1(6*H*)-yl)-N-(2-oxo-1,2,3,4-tetrahydroquinolin-6-yl)-3-phenylpropanamide**

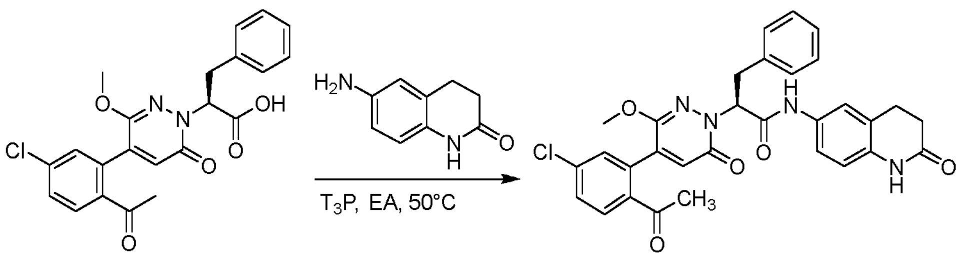


(S)-2-(4-(2-acetyl-5-chlorophenyl)-3-methoxy-6-oxopyridazin-1(6*H*)-yl)-3-phenylpropanic acid (40 mg, 0.09 mmol), 6-amino-3,4-dihydro-2(1H)-quinolinone (18 mg, 0.12 mmol) were dissolved in ethyl acetate (3.0 mL) at room temperature, and N,N-diisopropylethylamine (0.05 ml) was added. Subsequently, 1-propylphosphoric anhydride (0.2 ml) was added to the above solution. The reaction solution was heated to 50°C and stirred for 4 hours.

The reaction was completed, quenched by adding water, and the mixture was extracted with ethyl acetate (10 ml × 3 times). The organic phases were combined, and the organic phase was first washed with saturated brine (10 ml × 2 times), then dried with anhydrous sodium sulfate, and finally concentrated under reduced pressure. The obtained residue was purified by silica gel column chromatography (eluent: pure ethyl acetate). 15 mg of pale yellow solid, (S)-2-(4-(2-acetyl-5-chlorophenyl)-3-methoxy-6-oxopyridazin-1(6*H*)-yl)-N-(2-oxo-1,2,3,4-tetrah ydroquinolin-6-yl)-3-phenylpropanamide, was obtained (yield: 29.2%). LCMS: RT = 3.79 min, $[M-H]^-$ = 570.17. $^1H$ NMR (400 MHz, DMSO) δ 10.03 (s, 1H), 7.99 (d, *J* = 8.4 Hz, 1H), 7.69 (dd, *J* = 8.3, 2.2 Hz, 1H), 7.49 (d, *J* = 2.1 Hz, 1H), 7.45 (s, 1H), 7.33 - 7.25 (m, 5H), 7.18 (t, *J* = 6.7 Hz, 1H), 6.89 (s, 1H), 6.78 (d, *J*= 8.5 Hz, 1H), 5.69 (dd, *J* = 10.3, 4.8 Hz, 1H), 3.68 (s, 3H), 3.49 (dd, *J* = 13.9, 10.6 Hz, 1H), 3.38 (dd, *J* = 14.2, 4.7 Hz, 1H), 2.84 (t, *J* = 7.5 Hz, 2H), 2.53 (s, 3H), 2.45 - 2.39 (m, 2H).

**Example 32**

**Synthesis of (S)-4-(2-(4-(5-chloro-2-propanylphenyl)-3-methoxy-6-oxopyridazin-1(6*H*)-yl)-3-phenylprop anamido)benzoic acid**

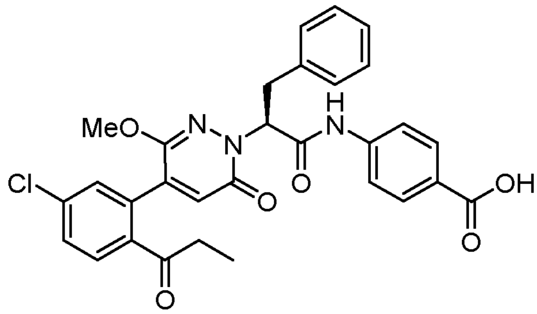

The specific synthetic route was as follows.

**Step A: Synthesis of 1-(2-bromo-4-chlorophenyl)propan-1-one**

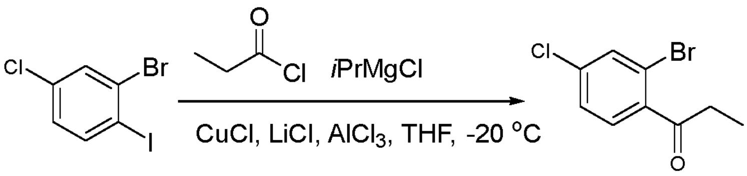


According to a well-known method (Angewandte Chemie, International Edition, 2010, 49(46), 8729-8732), 2-bromo-4-chloro-1-iodobenzene (2.00 g, 6.30 mmol) was dissolved in 2 mL of tetrahydrofuran, and cooled to -20°C. A solution of isopropylmagnesium chloride in n-hexane (2 M concentration) (4.1 mL, 8.2 mmol) was added dropwise, and the mixture was stirred at this temperature for 1 hour.

Propanyl chloride (716 μl, 8.20 mmol), lithium chloride (23 mg, 378 μmol), cuprous chloride (19 mg, 189 μmol) and aluminum trichloride (25 mg, 189 μmol) were added to 2 mL of tetrahydrofuran. It was stirred homogeneously at room temperature, and cooled in an ice-water bath. The above reaction mixture previously reacted for one hour was slowly added dropwise into the above mixed solution. The reaction was performed at room temperature for two hours. The reaction solution was quenched by adding with 40 mL of saturated ammonium chloride solution, extracted with dichloromethane (40 mL × 3 times), and organic phases were combined. The organic phase was washed with saturated brine (50 mL), then dried with anhydrous sodium sulfate, and finally concentrated under reduced pressure. The obtained residue was purified by silica gel column chromatography (eluent: petroleum ether/ethyl acetate=20/1). 1.37 g of colorless transparent liquid, 1-(2-bromo-4-chlorophenyl)propan-1-one, was obtained (yield: 87.8%). LCMS: RT = 4.30 min, without molecular ion peak.

**Step B: Synthesis of 1-(4-chloro-2-(4,4,5,5-tetramethyl-1,3,2-dioxaborolan-2-yl)phenyl)propan-1-one**

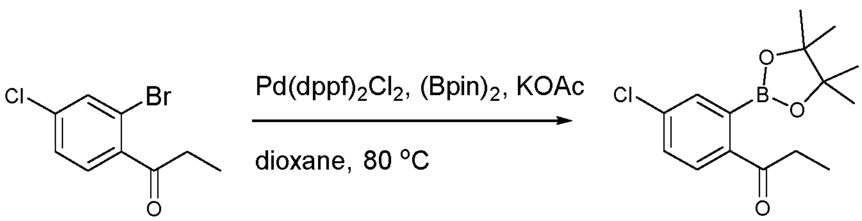


A solution of 1-(2-bromo-4-chlorophenyl)propan-1-one (1.37 g, 5.53 mmol), bis(pinacolato)diboron (2.83 g, 11.1 mmol), potassium acetate (1.09 g, 11.1 mmol) in 1,4-dioxane (21 mL) was added with $Pd(dppf)_2Cl_2 \cdot 2CH_2Cl_2$ (227 mg, 0.28 mmol) under nitrogen protection. The mixed solution was reacted at 80°C for 3 hours. After the reaction solution was cooled to room temperature, it was extracted with ethyl acetate (50 mL×2 times). Organic phases were combined and washed with water (50 mL) and saturated brine (50 mL) successively. It was then dried with anhydrous sodium sulfate, and finally concentrated under reduced pressure. The obtained residue was purified by silica gel column chromatography (eluent: petroleum ether/ethyl acetate=10/1). 1.0 g of pale yellow solid, 1-(4-chloro-2-(4,4,5,5-tetramethyl-1,3,2-dioxaborolan-2-yl)phenyl)propan-1-one, was obtained (yield: 61.3%). LCMS: RT = 4.46 min, $[M+H]^+$ = 293.13.

**Step C: Synthesis of 5-(5-chloro-2-propanylphenyl)-6-methoxy-2-(4-methoxybenzyl)pyridazin-3(2*H*)-one**

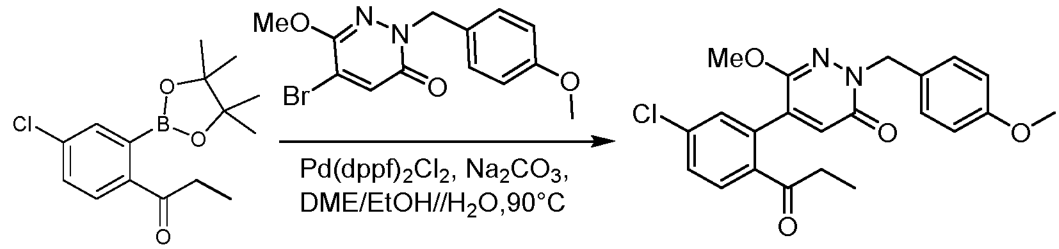


A solution of 1-(4-chloro-2-(4,4,5,5-tetramethyl-1,3,2-dioxaborolan-2-yl)phenyl)propan-1-one (268 mg, 0.91 mmol), 5-bromo-6-methoxy-2-(4-methoxybenzyl)pyridazin-3(2H)-one (269 mg, 0.83 mmol), sodium carbonate (176 mg, 1.66 mmol) in ethylene glycol dimethyl ether (5.6 mL) was added with ethanol (0.7 mL), water (0.7 mL) and $Pd(dppf)_2Cl_2$ (30 mg, 0.040 mmol) under nitrogen protection. The mixed solution was reacted at 90°C for 1 hour. After the reaction solution was cooled to room temperature, it was extracted with ethyl acetate (30 mL×2 times). Organic phases were combined and washed with water (40 mL) and saturated brine (40 mL) successively. It was then dried with anhydrous sodium sulfate, and finally concentrated under reduced pressure. The obtained residue was purified by silica gel column chromatography (eluent: petroleum ether/ethyl acetate=10/1-2/1). 321 mg of pale yellow oily product, 5-(5-chloro-2-propanylphenyl)-6-methoxy-2-(4-methoxybenzyl)pyridazin-3(2*H*)-one, was obtained (yield: 94.0%). LCMS: RT = 4.11 min, $[M+H]^+$ = 413.11.

**Step D: Synthesis of 5-(5-chloro-2-propanylphenyl)-6-methoxypyridazin-3(2*H*)-one**

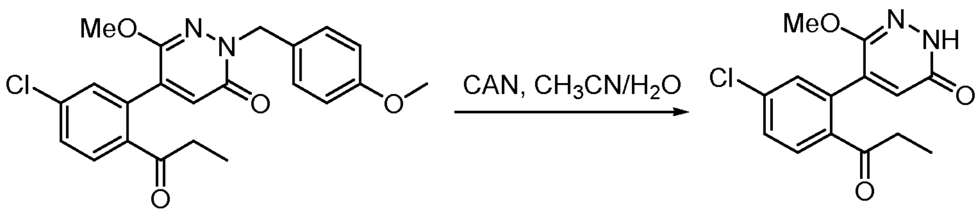


A solution of 5-(5-chloro-2-propanylphenyl)-6-methoxy-2-(4-methoxybenzyl)pyridazin-3(2H)-one (290 mg, 0.70 mmol) in acetonitrile/water (4.7 mL/1.6 mL) was added with ceric ammonium nitrate (3.09 g, 5.63 mmol) under an ice-water bath. After the addition was completed, the ice-water bath was removed, and the reaction was performed at room temperature for 0.5 hour. The reaction solution was extracted with ethyl acetate (50 mL×2 times). Organic phases were combined and washed with water (50 mL×2 times) and saturated brine (50 mL) successively. It was then dried with anhydrous sodium sulfate, and finally concentrated under reduced pressure. The obtained residue was purified by silica gel column chromatography (eluent: petroleum ether/ethyl acetate=1.5/1-1/1.5). 65 mg of white solid, 5-(5-chloro-2-propanylphenyl)-6-methoxypyiidazin-3(2*H*)-one, was obtained (yield: 31.8%). LCMS: RT = 3.42 min, $[M+H]^+$ = 293.01.

**Step E: Synthesis of tert-butyl (S)-4-(2-(4-(5-chloro-2-propanylphenyl)-3-methoxy-6-oxopyridazin-1(6*H*)-yl)-3-phenylprop anamido)benzoate**

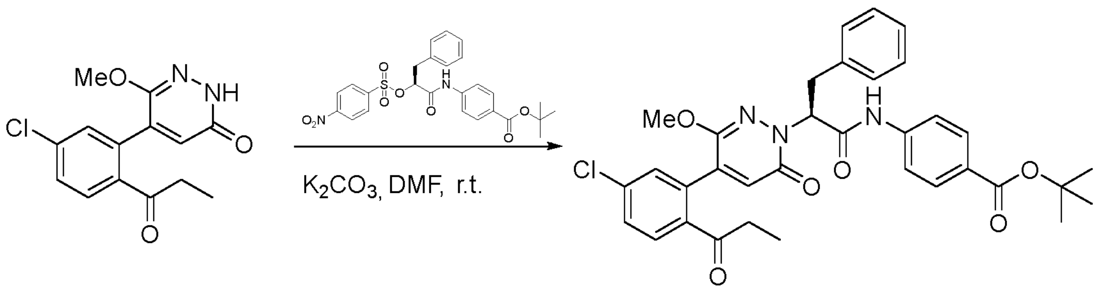


A solution of 5-(5-chloro-2-propanylphenyl)-6-methoxypyridazin-3(2*H*)-one (65 mg, 0.22 mmol) in N,N-dimethylmethaneamide (2.2 mL) was added with potassium carbonate (61 mg, 0.44 mmol) and tert-butyl (R)-4-(2-(((4-nitrophenyl)sulfonyl)oxy)-3-phenylpropanamido)benzoate (128 mg, 0.24 mmol) at room temperature. After the addition was completed, the mixture was heated to 40°C to react overnight.

The reaction solution was extracted with ethyl acetate (50 mL×2 times). Organic phases were combined and washed with water (50 mL×2 times) and saturated brine (50 mL) successively. It was then dried with anhydrous sodium sulfate, and finally concentrated under reduced pressure. The obtained residue was purified by silica gel column chromatography (eluent: petroleum ether/ethyl acetate=3/1). 127 mg of colorless oily tert-butyl (S)-4-(2-(4-(5-chloro-2-propanylphenyl)-3-methoxy-6-oxopyridazin-1(6*H*)-yl)-3-phenylpropana mido)benzoate was obtained (yield: 94.0%). LCMS: RT = 4.68 min, $[M-H]^-$ = 614.14.

**Step F: Synthesis of (S)-4-(2-(4-(5-chloro-2-propanylphenyl)-3-methoxy-6-oxopyridazin-1(6*H*)-yl)-3-phenylprop anamido)benzoic acid**

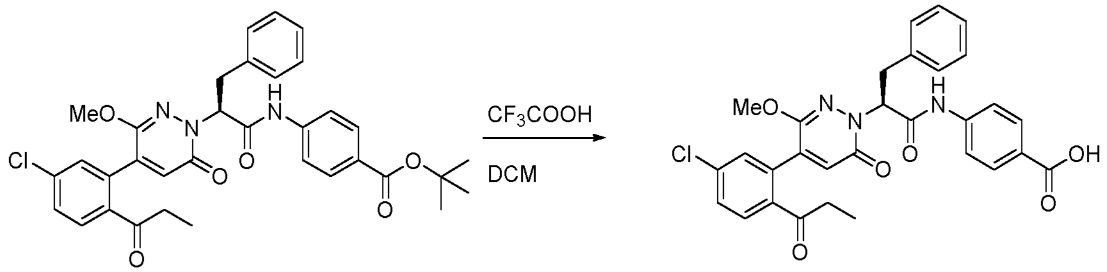


A solution of tert-butyl (S)-4-(2-(4-(5-chloro-2-propanylphenyl)-3-methoxy-6-oxopyridazin-1(6*H*)-yl)-3-phenylpropana mido)benzoate (170 mg, 0.28 mmol) in dichloromethane (3.0 mL) was added with trifluoroacetic acid (0.5 mL) at room temperature. After the addition was completed, the reaction was performed at room temperature for 0.5 hour, and the solvent was evaporated under reduced pressure. The residue was dissolved in absolute ethanol (2.0 ml), the solution was slowly added dropwise with 40 ml of n-hexane, and a large amount of solid was precipitated. It was filtrated under reduced pressure to give 110 mg of white solid, (S)-4-(2-(4-(5-chloro-2-propanylphenyl)-3-methoxy-6-oxopyridazine-1(6*H*)-yl)-3-phenylpropan amido)benzoic acid (yield: 70.0%). LCMS: RT = 4.09 min, $[M-H]^-$ = 559.15. $^1H$ NMR(500 MHz, DMSO) $^1H$ NMR (500 MHz, DMSO) δ 12.75 (br s, 1H), 10.53 (s, 1H), 7.97 (d, *J* = 8.4 Hz, 1H), 7.91 (d, *J* = 8.7 Hz, 2H), 7.73 (d, *J* = 8.7 Hz, 2H), 7.67 (dd, *J* = 8.3, 2.1 Hz, 1H), 7.51 (d, *J*= 2.0 Hz, 1H), 7.36-7.26 (m, 4H), 7.21 (t, *J*= 7.1 Hz, 1H), 6.91 (s, 1H), 5.72 (dd, *J*= 10.3, 4.8 Hz, 1H), 3.64 (s, 3H), 3.55 (dd, *J* = 14.0, 10.3 Hz, 1H), 3.40 (dd, *J* = 14.0, 4.6 Hz, 1H), 2.99 (brs, 2H), 1.03 (t, *J* = 7.1 Hz, 3H).

**Example 33**

**Synthesis** of **4-(2-(4-(2-acetyl-5-chlorophenyl)-3-methoxy-6-oxopyridazin-1(6H)-yl)-3-(4-(cyclopropanef ormamido)phenyl)propanamido)benzoic acid**

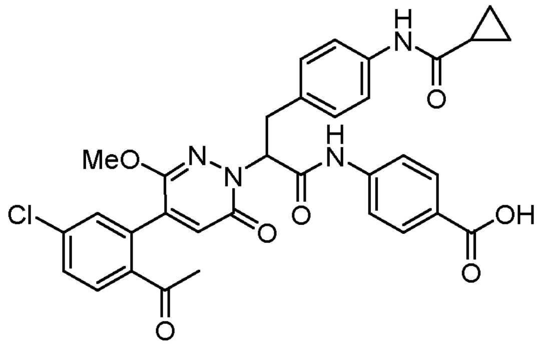

The specific synthetic route is as follows.

**Step A: Synthesis of 2-hydroxy-3-(4-nitrophenyl)propanic acid**

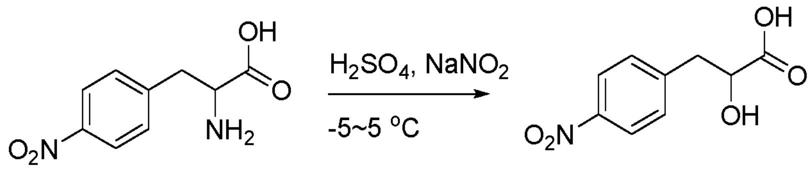


2-Amino-3-(4-nitrophenyl)propanic acid (4.2 g, 20.0 mmol) was dissolved in 80 mL of 0.5 M sulfuric acid, cooled with an ice-water bath, and sodium nitrite solution (5.52 g dissolved in 18 ml of water, 80.0 mmol) was slowly added dropwise to the solution. Keeping the temperature not higher than 5°C, the reaction was carried out for 3.5 hours. The reaction solution was extracted with ethyl acetate (100 mL×2), and the organic phases were combined and washed with saturated brine (80 mL). It was then dried with anhydrous sodium sulfate and concentrated under reduced pressure. The obtained crude product 2-hydroxy-3-(4-nitrophenyl)propanic acid was directly used in the next reaction (yield: 61.0%). LCMS: RT = 2.99 min, [M-H]- = 209.98.

**Step B: Synthesis of tert-butyl 4-(2-hydroxy-3-(4-nitrophenyl)propanamido)benzoate**

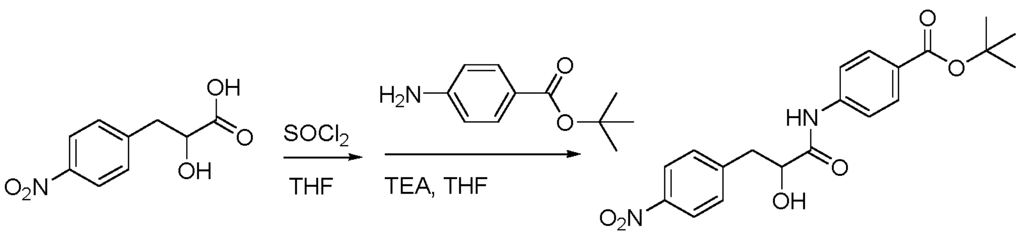


Under nitrogen protection, thionyl chloride (1.8 mL, 24.5 mmol) was slowly added dropwise to a solution of 2-hydroxy-3-(4-nitrophenyl) propanic acid (2.59 g, 12.3 mmol) in tetrahydrofuran (61.5 mL). After the addition was completed, the mixture was heated to 45 °C to react overnight. The solvent was evaporated to dryness under reduced pressure, and the obtained residue was directly used in the next step of reaction.

The above crude product was dissolved in 30 mL of tetrahydrofuran, to which triethylamine (4.1 mL, 29.5 mmol) was added, and then a solution of tert-butyl 4-aminobenzoate (1.9 g in 19 mL THF, 9.84 mmol) was slowly added dropwise. The reaction was carried out at room temperature for 0.5 hour. The reaction solution was quenched with water (50 mL), extracted with ethyl acetate (100 mL×2 times), and the organic phases were combined and washed with saturated brine (50 mL). It was then dried with anhydrous sodium sulfate and concentrated under reduced pressure. The obtained residue was purified by silica gel column chromatography (eluent: petroleum ether/ethyl acetate=3/1-2/1). 1.1 g of yellow solid, tert-butyl 4-(2-hydroxy-3-(4-nitrophenyl)propanamido)benzoate, was obtained (yield: 23.0%). LCMS:

RT = 4.11 min, without molecular ion peak.

**Step C: Synthesis of tert-butyl 4-(3-(4-nitrophenyl)-2-((4-nitrophenyl)sulfonyl)oxy)propanamido)benzoate**

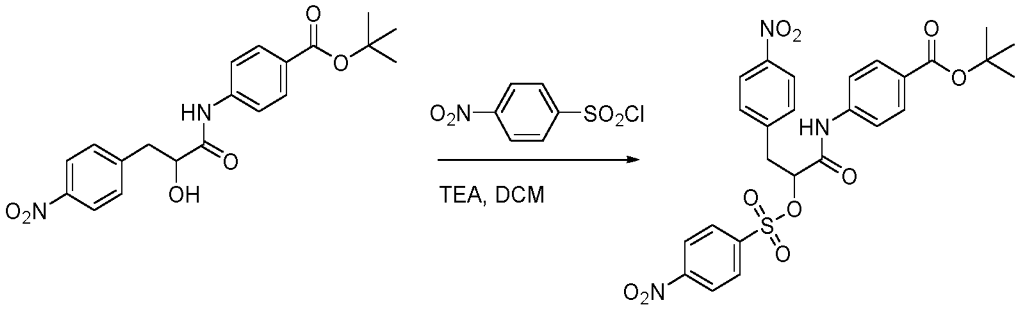


Triethylamine (1.2 mL, 8.6 mmol) and 4-nitrobenzenesulfonyl chloride (947 mg, 4.3 mmol) were added sequentially to a solution of tert-butyl 4-(2-hydroxy-3-(4-nitrophenyl)propanamido)benzoate (1.1 g, 2.85 mmol) in dichloromethane (28.5 mL) at room temperature. After the addition was completed, the reaction was carried out at room temperature for 3 hours.

The reaction solution was diluted with dichloromethane (100 mL), washed with water (50 mL) and saturated brine (50 mL) successively. It was then dried with anhydrous sodium sulfate and concentrated under reduced pressure. The obtained residue was purified by silica gel column chromatography (eluent: petroleum ether/ethyl acetate = 4/1-2/1). 1.02 g of a yellow solid, tert-butyl 4-(3-(4-nitrophenyl)-2-((4-nitrophenyl)sulfonyl)oxy)propanamido)benzoate, was obtained (yield: 63.0 %). LCMS: RT = 4.29 min, without molecular ion peak.

**Step D: Synthesis of tert-butyl 4-(2-(4-(2-acetyl-5-chlorophenyl)-3-methoxy-6-oxopyridazin-1(6H)-yl)-3-4-nitrophenyl)pro panamido)benzoate**

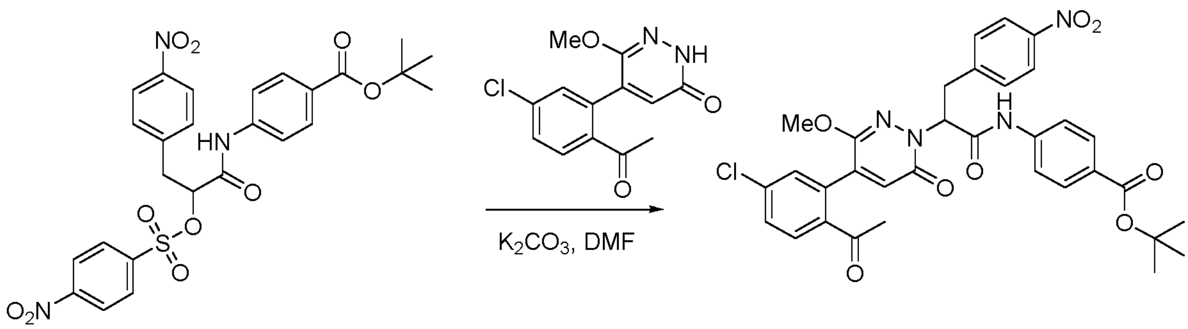


5-(2-acetyl-5-chlorophenyl)-6-methoxypyridazin-3(2H)-one (219 mg, 0.79 mmol) and potassium carbonate (218 mg, 1.58 mmol) were added to a solution of tert-butyl 4-(3-(4-nitrophenyl)-2-((4-nitrophenyl)sulfonyl)oxy)propanamido)benzoate (585 mg, 1.02 mol) in N,N-dimethylformamide (4.0 mL) at room temperature, and the reaction was carried out at room temperature overnight.

The reaction solution was extracted with ethyl acetate (50 mL×2), and the organic phases were combined and washed with water (40 mL×2) and saturated brine (40 mL) successively. It was then dried with anhydrous sodium sulfate and concentrated under reduced pressure. The obtained residue was purified by silica gel column chromatography (eluent: petroleum ether/ethyl acetate = 4/1-2/1). 500 mg of a yellow solid, tert-butyl 4-(2-(4-(2-acetyl-5-chlorophenyl)-3-methoxy-6-oxopyridazin-1(6H)-yl)-3-4-nitrophenyl)propana mido)benzoate, was obtained (yield: 63.0%). LCMS: RT = 4.46 min, [M-H]- = 645.05.

**Step E: Synthesis of tert-butyl 4-(2-(4-(2-acetyl-5-chlorophenyl)-3-methoxy-6-oxopyridazin-1(6H)-yl)-3-(4-aminophenyl)p ropanamido)benzoate**

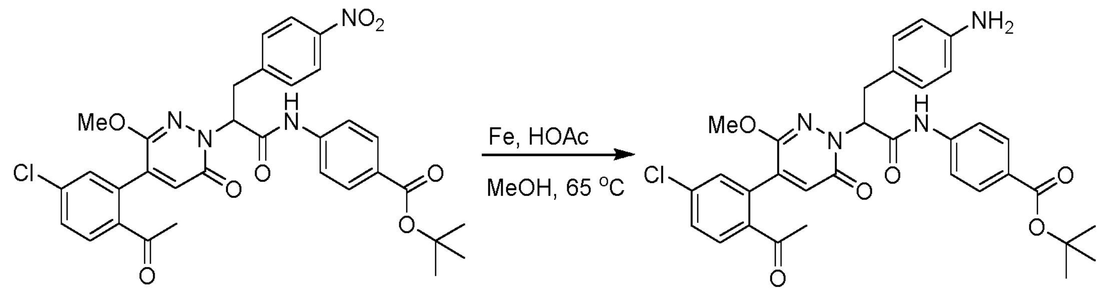


Under nitrogen protection, acetic acid (0.78 mL) and reduced iron powder (438 mg, 7.8 mmol) were successively added to a solution of tert-butyl 4-(2-(4-(2-acetyl-5-chlorophenyl)-3-methoxy-6-oxopyridazin-1(6H)-yl)-3-4-nitrophenyl)propana mido)benzoate (505 mg, 0.78 mmol) in methanol (7.8 mL), and the reaction was heated to 65°C and carried out for 1 hour.

The reaction solution was adjusted to pH=8 with saturated sodium bicarbonate, diluted with ethyl acetate (100 mL), filtered through diatomite, and the organic phase was washed with water (40 mL) and saturated brine (40 mL) successively. It was then dried with anhydrous sodium sulfate and concentrated under reduced pressure. The obtained residue was a crude product of tert-butyl 4-(2-(4-(2-acetyl-5-chlorophenyl)-3-methoxy-6-oxopyridazin-1(6H)-yl)-3-(4-aminophenyl)propa namido)benzoate, which was used directly in the next step of reaction. LCMS: RT = 4.00 min, [M-H]-= 615.16.

**Step F: Synthesis of 4-(2-(4-(2-acetyl-5-chlorophenyl)-3-methoxy-6-oxopyridazin-1(6H)-yl)-3-(4-(cyclopropanef ormamido)phenyl)propanamido)benzoic acid**

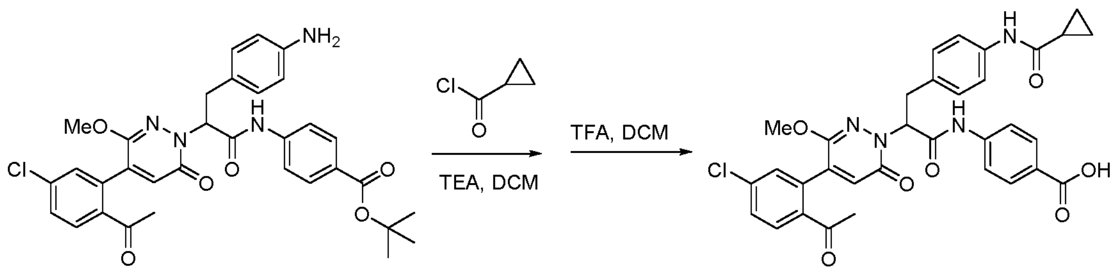


Under nitrogen protection, triethylamine (46 μl, 0.33 mmol) and cyclopropanecarbonyl chloride (20 μl, 0.22 mmol) were added to a solution of tert-butyl 4-(2-(4-(2-acetyl-5-chlorophenyl)-3-methoxy-6-oxopyridazin-1(6H)-yl)-3-(4-aminophenyl)propa namido)benzoate (65 mg, 0.11 mmol) in dichloromethane (1.1 mL), and the reaction was carried out at room temperature for 0.5 hour. The reaction solution was quenched with water (1 mL), diluted with dichloromethane (50 mL), and washed with water (30 mL) and saturated brine (30 mL) successively. It was then dried with anhydrous sodium sulfate and concentrated under reduced pressure. The obtained crude product was directly used in the next reaction. LCMS: RT = 4.28 min, [M+H]+= 685.23.

The above crude product was dissolved in dichloromethane (1.0 ml), and trifluoroacetic acid (0.2 ml) was added. The reaction was carried out at room temperature for 45 minutes. The solvent was evaporated under reduced pressure, and the obtained residue was purified by preparative HPLC to give 20 mg of a pale yellow solid, 4-(2-(4-(2-acetyl-5-chlorophenyl)-3-methoxy-6-oxopyridazin-1(6H)-yl)-3-(4-(cyclopropaneform amido)phenyl)propanamido)benzoic acid (yield: 29.0%). LCMS: RT = 3.72 min, $[M+H]^+$= 629.18. $^1$H NMR (500 MHz, DMSO) δ 12.71 (br s, 1H), 10.48 (s, 1H), 10.10 (s, 1H), 8.00 (d , J = 8.4 Hz, 1H), 7.90 (d, J = 8.7 Hz, 2H), 7.72 (d, J = 8.7 Hz, 2H), 7.69 (dd, J = 8.4, 2.1 Hz, 1H), 7.51 (d , J = 2.0 Hz, 1H), 7.49 (d, J = 8.4 Hz, 2H), 7.21 (d, J = 8.4 Hz, 2H), 6.90 (s, 1H), 5.69 (dd, J = 10.2, 4.8 Hz , 1H), 3.68 (s, 3H), 3.46 (dd, J = 14.0, 10.4 Hz, 1H), 3.38 - 3.34 (m, 1H), 2.54 (s, 3H), 1.77 - 1.71 (m, 1H), 0.88 - 0.82 (m, 2H), 0.79 - 0.72 (m, 2H).

**Example 34**

**Synthesis of S)-4-(2-(4-(2-acetyl-5-chlorophenyl)-3-hydroxy-6-oxopyridazin-1(6H)-yl)-3-phenylpropana mido)benzoic acid**

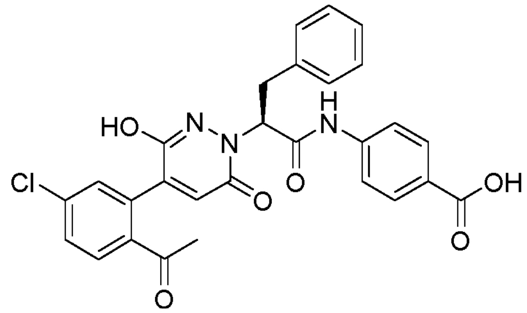

The specific synthetic route is as follows.

**Step A: Synthesis of 5-(2-acetyl-5-chlorophenyl)-6-hydroxy-2-(4-methoxybenzyl)pyridazin-3(2H)-one**

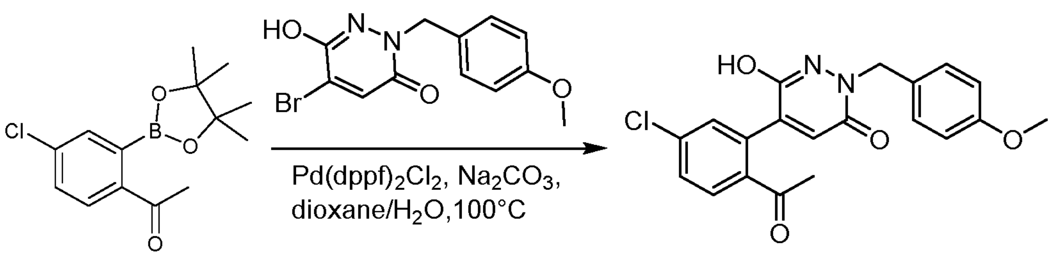


Under nitrogen protection, water (5 mL) and Pd(dppf)$_2$Cl$_2$ (370 mg, 0.48 mmol) were added to a solution of 1-(4-chloro-2-(4,4,5,5-tetramethyl-1,3,2-dioxaborolan-2-yl)phenyl)ethan-1-one (1.6 g, 5.7 mmol), 5-bromo-6-hydroxy-2-(4-methoxybenzyl)pyridazin-3(2H)-one (1.5 g, 4.8 mmol), potassium carbonate (1.3 g, 9.6 mmol) in 1,4-dioxane (20 mL), and the mixture was reacted at 100°C for 3 hours.

After the reaction solution was cooled to room temperature, it was extracted with ethyl acetate (100 mL×2). The organic phases were combined and washed with water (100 mL) and saturated brine (100 mL) successively. It was then dried with anhydrous sodium sulfate and concentrated under reduced pressure. The obtained residue was purified by silica gel column chromatography (eluent: petroleum ether/ethyl acetate=2/1). 500 mg of a yellow solid, 5-(2-acetyl-5-chlorophenyl)-6-hydroxy-2-(4-methoxybenzyl)pyridazin-3(2H)-one, was obtained (yield: 27.0%). LCMS: RT = 3.72 min, [M+H]+ = 383.03.

**Step B: Synthesis of 5-(2-acetyl-5-chlorophenyl)-6-(allyloxy)-2-(4-methoxybenzyl)pyridazin-3(2H)-one**

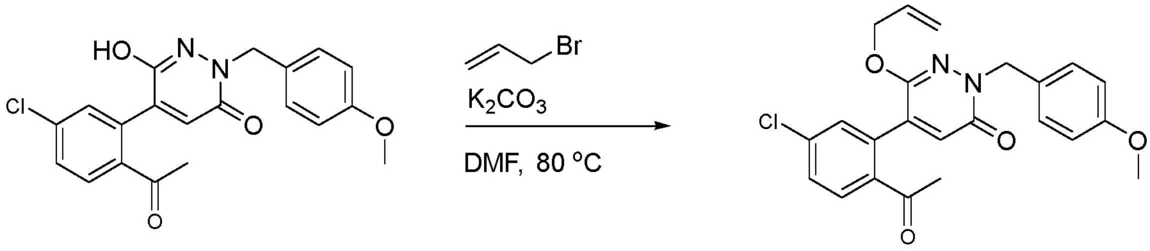


Potassium carbonate (205 mg, 1.49 mmol) was added to a solution of 5-(2-acetyl-5-chlorophenyl)-6-hydroxy-2-(4-methoxybenzyl)pyridazin-3(2H)-one (200 mg, 0.52 mmol) in N,N-dimethylformamide (2.5 mL) at room temperature. The mixture was heated to 80°C and stirred for 15 minutes, then allyl bromide (180 μl, 2.1 mmol) was added and the reaction was carried out at this temperature for 0.5 h.

After cooling to room temperature, the reaction solution was extracted with ethyl acetate (50 mL×2 times), and the organic phases were combined and washed with water (40 mL×2 times) and saturated brine (40 mL) successively. It was then dried with anhydrous sodium sulfate and concentrated under reduced pressure. The obtained residue was purified by silica gel column chromatography (eluent: petroleum ether/ethyl acetate = 3/1). 150 mg of an yellow oily product, 5-(2-acetyl-5-chlorophenyl)-6-(allyloxy)-2-(4-methoxybenzyl)pyridazin-3(2H)-one, was obtained (yield: 68.0%). LCMS: RT = 4.17 min, [M+H]+= 425.10.

**Step C: Synthesis of 5-(2-acetyl-5-chlorophenyl)-6-(allyloxy)pyridazin-3(2H)-one**

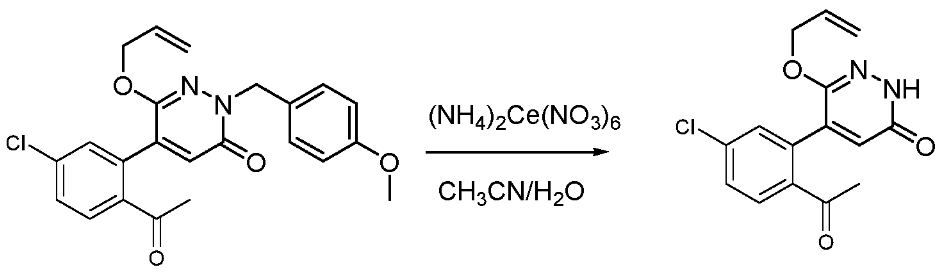


Under ice-water bath, ceric ammonium nitrate (903 mg, 1.65 mmol) was added to a solution of 5-(2-acetyl-5-chlorophenyl)-6-(allyloxy)-2-(4-methoxybenzyl)pyridazin-3(2H)-one (140 mg, 0.33 mmol) in acetonitrile/water (2.4 mL/0.8 mL). After the addition was completed, the ice-water bath was removed, and the reaction was carried out at room temperature for 0.5 hour. The reaction solution was extracted with ethyl acetate (50 mL×2 times), and the organic phases were combined and washed with water (50 mL×2 times) and saturated brine (50 mL) successively. It was then dried with anhydrous sodium sulfate and finally concentrated under reduced pressure. The obtained residue was purified by silica gel column chromatography (eluent: petroleum ether/ethyl acetate=1/1-1/1.5). 85 mg of an yellow oily product, 5-(2-acetyl-5-chlorophenyl)-6-(allyloxy)pyridazin-3(2H)-one, was obtained (yield: 85.0%). LCMS: RT = 3.48 min, [M+H]+ = 305.10.

**Step D: Synthesis of tert-butyl (S)-4-(2-(4-(2-acetyl-5-chlorophenyl)-3-(allyloxy)-6-oxopyridazine-1(6H)-yl)-3-phenylpropa namido)benzoate**

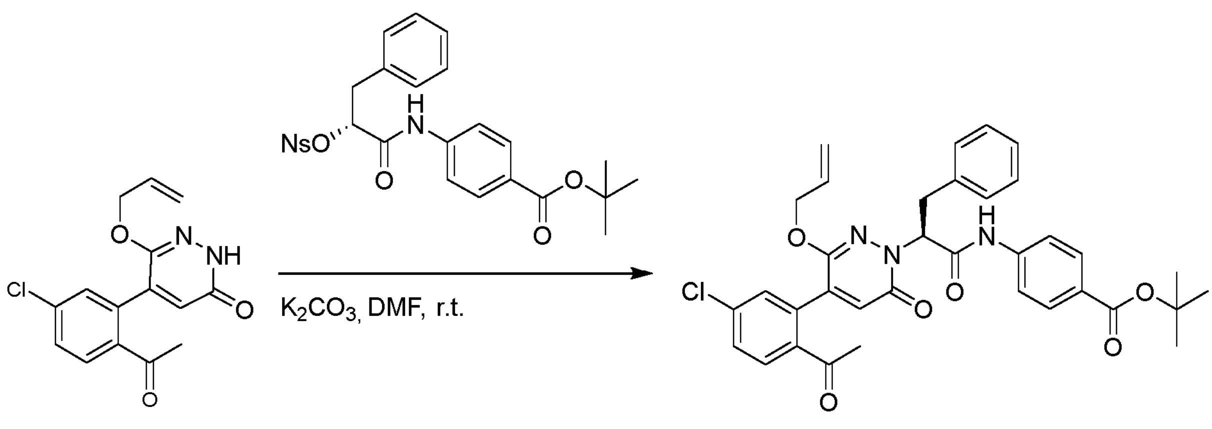


Potassium carbonate (77 mg, 0.56 mmol) and tert-butyl (R)-4-(2-(((4-nitrophenyl)sulfonyl)oxy)-3-phenylpropanamido)benzoate (161 mg, 0.31 mmol) were added to a solution of 5-(2-acetyl-5-chlorophenyl)-6-(allyloxy)pyridazin-3(2H)-one (85 mg, 0.28 mmol) in N,N-dimethylformamide (3.0 mL) at room temperature, and the reaction was carried out at room temperature overnight.

The reaction solution was extracted with ethyl acetate (50 mL×2 times), and the organic phases were combined and washed with water (30 mL×2 times) and saturated brine (30 mL) successively. It was then dried with anhydrous sodium sulfate and concentrated under reduced pressure. The obtained residue was purified by silica gel column chromatography (eluent: petroleum ether/ethyl acetate=4/1-2/1) to obtain 104 mg of a pale yellow oily product, tert-butyl (S)-4-(2-(4-(2-acetyl-5-chlorophenyl)-3-(allyloxy)-6-oxopyridazin-1(6H)-yl)-3-phenylpropanami do)benzoate (yield: 59.0%). LCMS: RT = 4.69 min, [M-H]- = 626.13.

**Step E: Synthesis of tert-butyl (S)-4-(2-(4-(2-acetyl-5-chlorophenyl)-3-hydroxy-6-oxopyridazin-l(6H)-yl)-3-phenylpropana mido)benzoate**

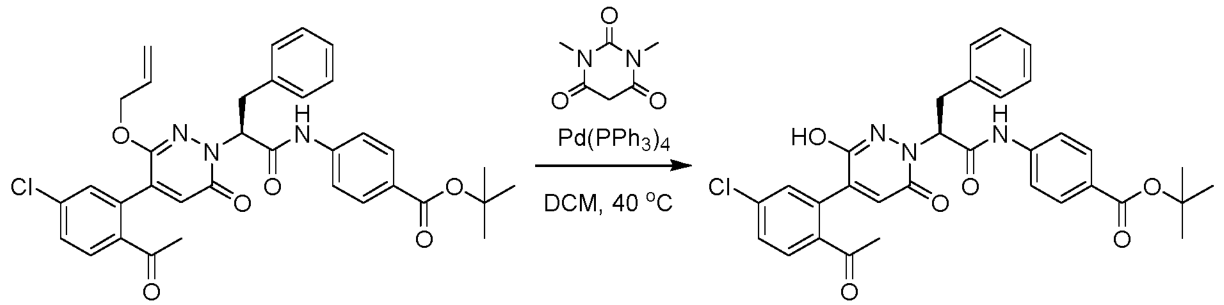


Under nitrogen protection, 1,3-dimethylbarbituric acid (149 mg, 0.95 mmol) and tetrakis(triphenylphosphine)palladium (7 mg, 0.006 mmol) were added to a solution of tert-butyl (S)-4-(2-(4-(2-acetyl-5-chlorophenyl)-3-(allyloxy)-6-oxopyridazin-1(6H)-yl)-3-phenylpropanami do)benzoate (100 mg, 0.16 mmol) in dichloromethane (4.0 mL), and the reaction solution was heated to 40°C to react for 1.5 hours.

The reaction solution was diluted with dichloromethane (100 mL), and washed with saturated sodium bicarbonate solution (40 mL), water (40 mL), and saturated brine (30 mL) successively. It was then dried with anhydrous sodium sulfate and concentrated under reduced pressure. The obtained residue was purified by silica gel column chromatography (eluent: petroleum ether/ethyl acetate = 4/1 - 2/1) to obtain 52 mg of a colorless oily product, tert-butyl (S)-4-(2-(4-(2-acetyl-5-chlorophenyl)-3-hydroxy-6-oxopyridazin-1(6H)-yl)-3-phenylpropanamid o)benzoate (yield: 55.0% ). LCMS: RT = 4.31 min, [M+H]+ = 588.16.

**Step F: Synthesis of (S)-4-(2-(4-(2-acetyl-5-chlorophenyl)-3-hydroxy-6-oxopyridazin-1(6H)-yl)-3-phenylpropana mido)benzoic acid**

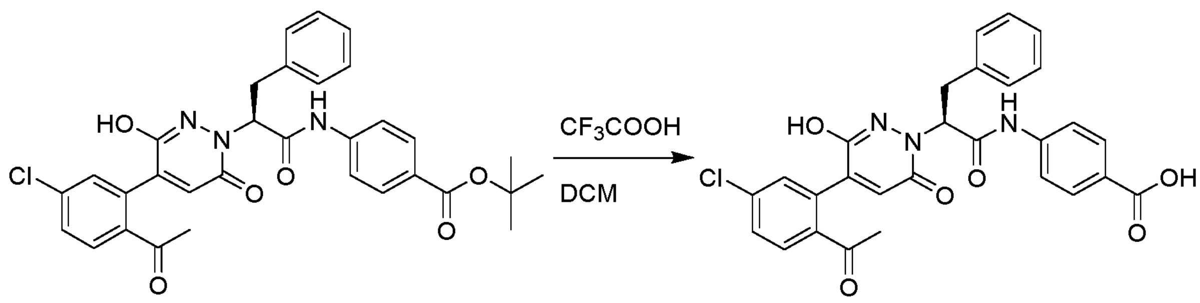


At room temperature, trifluoroacetic acid (0.5 mL) was added to a solution of tert-butyl (S)-4-(2-(4-(2-acetyl-5-chlorophenyl)-3-hydroxy-6-oxopyridazin-1(6H)-yl)-3-phenylpropanamid e yl)benzoate (52 mg, 0.088 mmol) in dichloromethane (2.0 mL). After the addition was completed, the reaction was carried out at room temperature for 0.5 hours, and the solvent was evaporated under reduced pressure. The obtained residue was purified by silica gel column chromatography (eluent: petroleum ether/ethyl acetate=2/1-1/1) to obtain 30 mg of a white solid, (S)-4-(2-(4-(2-acetyl-5-chlorophenyl)-3-hydroxy-6-oxopyridazin-1(6H)-yl)-3-phenylpropanamido)benzoic acid (yield: 64.0%). LCMS: RT = 3.78 min, [M+H]+= 532.09.

**Example 35**

**Synthesis** of **(S)-4-(2-(4-(5-chloro-2-(2-hydroxyacetyl)phenyl)-3-methoxy-6-oxopyridazin-1(6H)-yl)-3-ph enylpropanamido)benzoic acid**

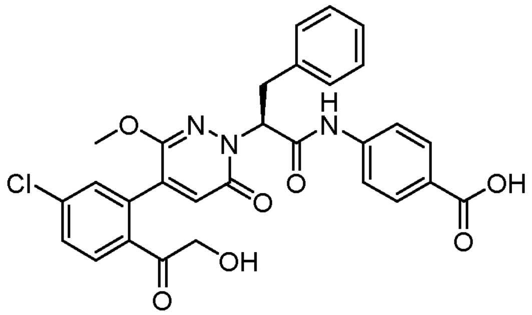

The specific synthetic route is as follows.

**Step A: Synthesis of (S)-4-(2-(4-(5-chloro-2-(2-hydroxyacetyl)phenyl)-3-methoxy-6-oxopyridazine-1(6H)-yl)-3-p henylpropanamido)benzoic acid**

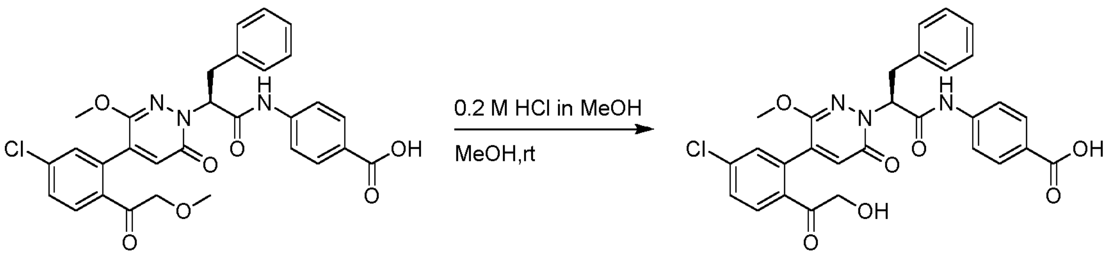


(S)-4-(2-(4-(5-chloro-2-(2-methoxyacetyl)phenyl)-3-methoxy-6-oxopyridazine-1(6H)-yl)-3-phenylpropanamido)benzoic acid (17 mg, 0.03 mmol) was dissolved in methanol (4.0 mL), and a methanol solution in hydrochloric acid (0.5 mL) was added. The mixture was stirred at room temperature for 1 hour.

The reaction solution was concentrated under reduced pressure, and the obtained residue was purified by preparative high performance liquid chromatography to obtain 8 mg of a white solid, (S)-4-(2-(4-(5-chloro-2-(2-hydroxyacetyl))phenyl)-3-methoxy-6-oxopyridazin-1(6H)-yl)-3-phen ylpropanamido)benzoic acid (yield: 47.0%). LCMS: RT = 3.69 min, [MH]- = 560.13. $^1$H NMR (400 MHz, DMSO) δ 12.87 - 12.61 (m, 1H), 10.49 (s, 1H), 7.91 (dd, J = 13.3, 8.6 Hz, 2H), 7.70 (d, J = 8.7 Hz, 2H), 7.65 (dd, J = 8.3, 2.1 Hz, 1H), 7.52 (d, J = 2.1 Hz, 1H), 7.34 - 7.25 (m, 3H), 7.18 (t, J = 6.7 Hz, 1H), 6.91 (s, 1H), 5.71 (dd, J = 9.7, 5.2 Hz, 1H), 4.65 (s, 2H), 3.66 (s, 3H), 3.55 - 3.45 (m, 1H), 3.40 (dd, J = 14.0, 5.3 Hz, 1H), 1.22 (s, 2H).

**Example 36**

**Synthesis of (S)-4-(2-(4-(5-chloro-2-(2,2-difluoroacetyl)phenyl)-3-methoxy-6-oxopyridazine-1(6H)-yl)-3-phenylpropanamido)benzoic acid**

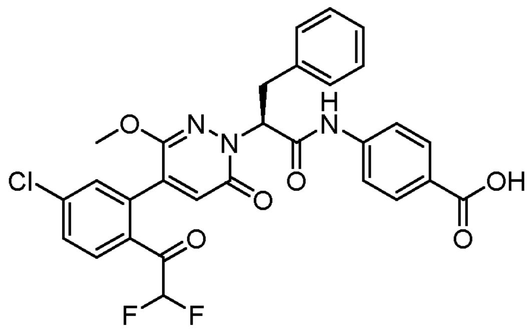

The specific synthetic route is as follows.

**Step A: Synthesis of (Z)-5-(2-(1-(butylimino)ethyl)-5-chlorophenyl)-6-methoxy-2-(4-methoxybenzyl)pyridazin-3( 2H)-one**

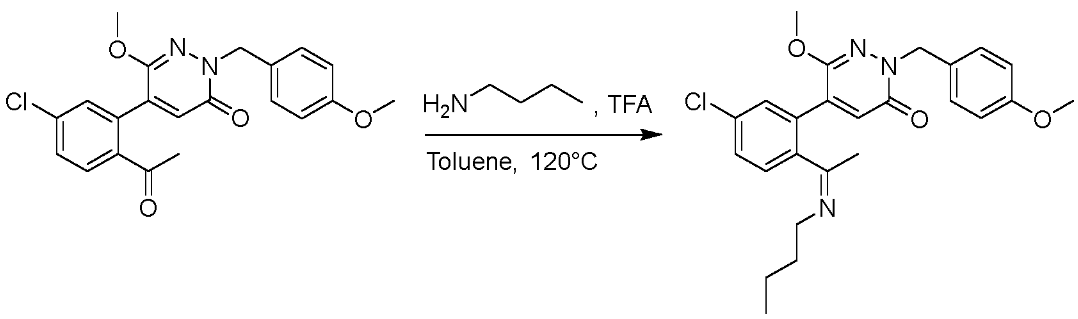


5-(2-acetyl-5-chlorophenyl)-6-methoxy-2-(4-methoxybenzyl)pyridazin-3(2H)-one (200 mg, 0.50 mmol), n-butylamine (2.0 ml) were added to toluene (2.0 ml), and trifluoroacetic acid (8.0 μl) was added dropwise. A water separator was installed, and the reaction was carried out at 120 °C for 8 hours.

After the reaction was completed, the toluene was evaporated to dryness, and the obtained residue was dissolved in methyl tert-butyl ether (30 mL), washed with saturated brine (10 mL × 3 times), and then dried with anhydrous sodium sulfate and concentrated under reduced pressure. 220 mg of a yellow oily product, (Z)-5-(2-(1-(butylimino)ethyl)-5-chlorophenyl)-6-methoxy-2-(4-methoxybenzyl)pyridazin-3(2H) -one was obtained, which was directly used in the next step without purification. LCMS: RT = 3.10 min, [M+H]+ = 454.17.

**Step B: Synthesis of 5-(5-chloro-2-(2,2-difluoroacetyl)phenyl)-6-methoxy-2-(4-methoxybenzyl)pyridazin-3(2H)-one**

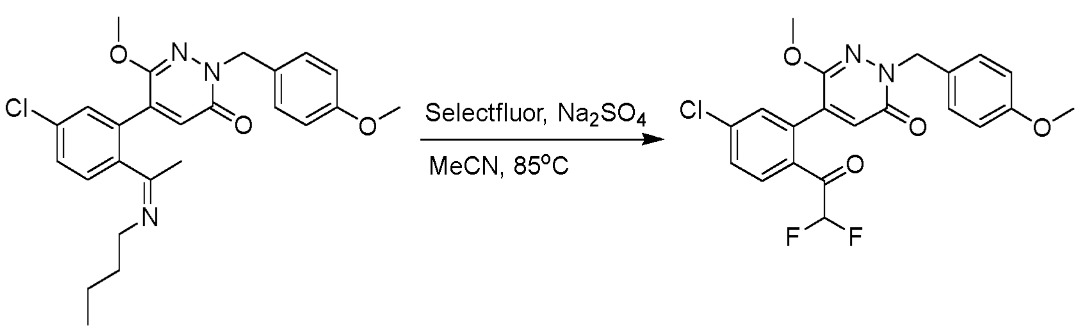


(Z)-5-(2-(1-(butylimino)ethyl)-5-chlorophenyl)-6-methoxy-2-(4-methoxybenzyl)pyridazin-3(2H)-one (220 mg, 0.49 mmol), selective fluorine reagent (206 mg, 0.58 mmol) and a small amount of anhydrous sodium sulfate were added

to anhydrous acetonitrile (2.0 mL) at room temperature, and reacted at 85°C overnight.

After the reaction was completed, it was quenched by adding water, and the pH was adjusted to 3-4 with 1 M dilute hydrochloric acid. The mixture was extracted with ethyl acetate (30 ml × 3 times). The organic phases were combined, and the organic phase was first washed with saturated brine (20 ml × 3), then dried with anhydrous sodium sulfate and concentrated under reduced pressure. The obtained residue was purified by silica gel column chromatography (eluent: ethyl acetate/n-hexane=2/5). 60 mg of a yellow solid, 5-(5-chloro-2-(2,2-difluoroacetyl)phenyl)-6-methoxy-2-(4-methoxybenzyl)pyridazin-3(2H)-one, was obtained (yield: 28.6%). LCMS: RT = 4.06 min, [M+H]+ = 435.04.

**Step C: Synthesis of 5-(5-chloro-2-(2,2-difluoroacetyl)phenyl)-6-methoxypyridazin-3(2H)-one**

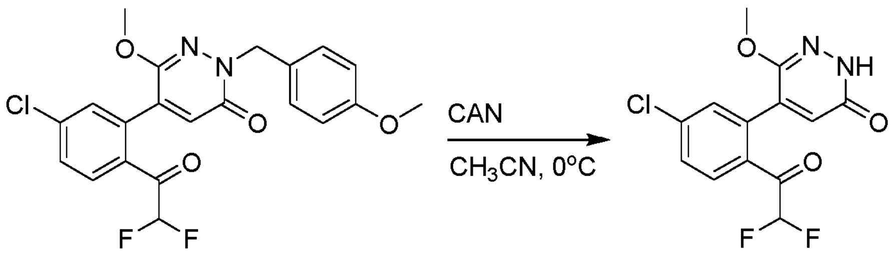


5-(5-chloro-2-(2,2-difluoroacetyl)phenyl)-6-methoxy-2-(4-methoxybenzyl)pyridazin-3(2H)-one (60 mg, 0.14 mmol) was added to a mixed solvent (4 mL, acetonitrile: water = 3:1) at 0°C, and then ceric ammonium nitrate (605 mg, 1.10 mmol) was slowly added. After the addition was completed, the reaction was carried out at room temperature 30 minutes.

The reaction was completed, quenched by adding water, and the mixture was extracted with ethyl acetate (20 ml × 3). The organic phases were combined, and the organic phase was first washed with saturated brine (10 ml × 2), then dried with anhydrous sodium sulfate and concentrate under reduced pressure. The obtained residue was purified by silica gel column chromatography (eluent: ethyl acetate/n-hexane=1/1). 25 mg of a yellow solid, 5-(5-chloro-2-(2,2-difluoroacetyl)phenyl)-6-methoxypyridazin-3(2H)-one, was obtained(yield: 57.2%). LCMS: RT = 3.42 min, [M+H]+ = 315.03.

**Step D: Synthesis of tert-butyl (S)-4-(2-(4-(5-chloro-2-(2,2-difluoroacetyl)phenyl)-3-methoxy-6-oxopyridazine-1(6H)-yl)-3-phenylpropanamido)benzoate**

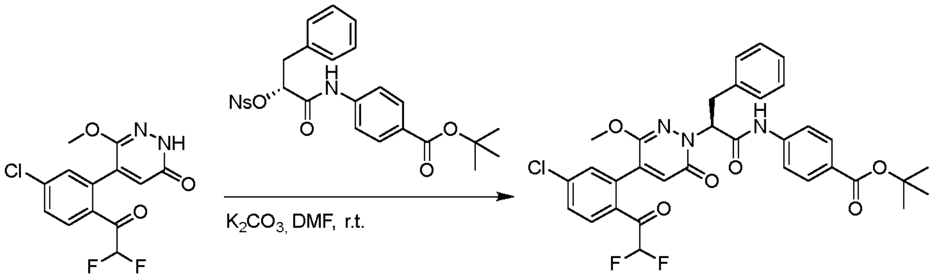


5-(5-chloro-2-(2,2-difluoroacetyl)phenyl)-6-methoxypyridazin-3(2H)-one (25 mg, 0.08 mmol), tert-butyl (R)-4-(2-(((4-nitrophenyl)sulfonyl)oxy)-3-phenylpropanamido)benzoate (46 mg, 0.08 mmol) and potassium carbonate (22 mg, 0.16 mmol) were added to N,N-dimethylformamide (2.0 mL) at room temperature, and the reaction was carried out at room temperature overnight.

The reaction was completed, quenched by adding water, and the mixture was extracted with ethyl acetate (10 ml × 3 ). The organic phases were combined, and the organic phase was first washed with saturated brine (10 ml × 2), then dried with anhydrous sodium sulfate and concentrate under reduced pressure. The obtained residue was purified by silica gel column chromatography (eluent: ethyl acetate/n-hexane=1/2). 32 mg of a pale yellow solid, tert-butyl (S)-4-(2-(4-(5-chloro-2-(2,2-difluoroacetyl)phenyl)-3-methoxy-6-oxopyridazine-1(6H)-yl)-3-phe nylpropanamido)benzoate, was obtained (yield: 62.5%). LCMS: RT = 4.13 min, [M+H]+ = 638.15.

**Step E: Synthesis of (S)-4-(2-(4-(5-chloro-2-(2,2-difluoroacetyl(phenyl)-3-methoxy-6-oxopyridazine-1(6H)-yl)-3-phenylpropanamido)benzoic acid**

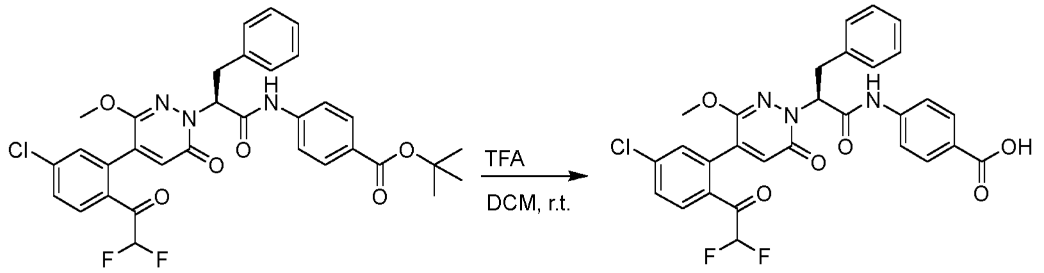


tert-Butyl (S)-4-(2-(4-(5-chloro-2-(2,2-difluoroacetyl)phenyl)-3-methoxy-6-oxopyridazine-1(6H)-yl)-3-phe nylpropanamido)benzoate (32 mg, 0.05 mmol) was added to dichloromethane (2.0 mL) at room temperature, and trifluoroacetic acid (0.5 mL) was added dropwise. The reaction was carried out at room temperature for 3 hours.

After the reaction was completed, dichloromethane was evaporated to dryness and trifluoroacetic acid was sucked dry with an oil pump. The obtained residue was dissolved in dichloromethane (1.0 mL), and it was added dropwise to n-hexane (10.0 mL) to precipitate a white solid, which was filtered off with suction. The filter cake was washed with n-hexane and dried to give 18 mg of a pale yellow solid, (S)-4-(2-(4-(5-chloro-2-(2,2-difluoroacetyl(phenyl)-3-methoxy-6-oxopyridazine-1(6H)-yl)-3-phe nylpropanamido)benzoic acid (yield: 62.0%). LCMS: RT = 4.00 min, [MH]- = 580.07. $^1$H NMR (500 MHz, DMSO) δ 12.71 (s, 1H), 10.53 (s, 1H), 8.03 (d, J = 8.4 Hz, 1H), 7.91 (d, J = 8.7 Hz, 2H), 7.80 (dd, J = 8.4, 2.1 Hz, 1H), 7.73 (d, J = 8.7 Hz, 2H), 7.66 (d, J = 2.0 Hz, 1H), 7.37 -7.25 (m, 4H), 7.19 (t, J = 7.2 Hz, 1H), 7.12 (t, J = 52.4 Hz, 1H), 7.01 (s, 1H), 5.78-5.71 (m, 1H), 3.63 ( s, 3H), 3.53 (dd, J = 14.1, 10.3 Hz, 1H), 3.42 (dd, J = 14.2, 4.6 Hz, 1H).

**Example 37**

**Synthesis of (S)-4-(2-(4-(5-chloro-2-(2-fluoroacetyl)phenyl)-3-methoxy-6-oxopyridazin-1(6H)-yl)-3-phen ylpropanamido)benzoic acid**

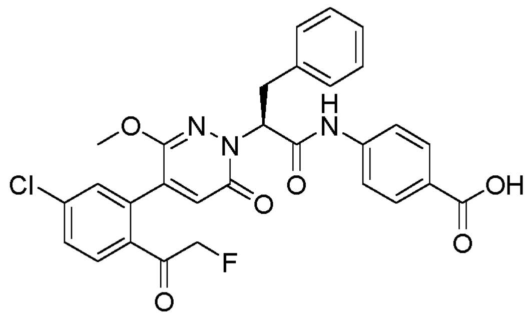

The specific synthetic route is as follows.

**Step A: Synthesis of 5-(5-Chloro-2-(2-methoxyacetyl)phenyl)-6-methoxy-2-(4-methoxybenzyl)pyridazin-3(2H)-one**

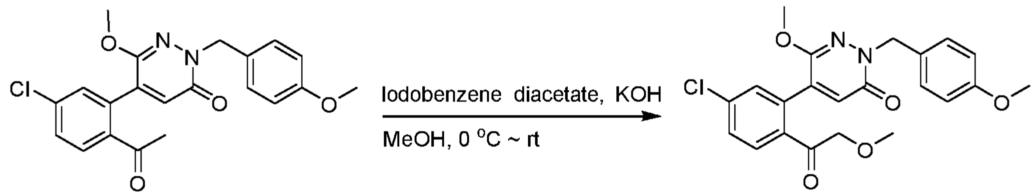


5-(2-acetyl-5-chlorophenyl)-6-methoxy-2-(4-methoxybenzyl)pyridazin-3(2H)-one (300 mg, 0.75 mmol) and potassium hydroxide (210 mg, 3.75 mmol) were added to methanol (5.0 ml) at room temperature, stirred at 0°C for 5 min, and iodobenzene diacetate (364 mg, 1.13 mmol) was added dropwise. The reaction was carried out at this temperature for 15 minutes.

After the reaction was completed, sodium sulfite solution and ammonium chloride solution were added under ice bath, and the mixture was extracted with ethyl acetate (50 ml × 3). The organic phases were combined, and the organic phase was first washed with saturated brine (30 ml × 3), then dried with anhydrous sodium sulfate and concentrated under reduced pressure. The obtained residue was purified by silica gel column chromatography (eluent: ethyl acetate/n-hexane=1/2). 230 mg of a yellow solid, 5-(5-chloro-2-(2-methoxyacetyl)phenyl)-6-methoxy-2-(4-methoxybenzyl)pyridazin-3(2H)-one, was obtained (yield: 72.0%). LCMS: RT = 3.99 min, [M+H]+ = 429.34.

**Step B: Synthesis of 5-(5-chloro-2-(2-hydroxyacetyl)phenyl)-6-methoxy-2-(4-methoxybenzyl)pyridazin-3(2H)-on e**

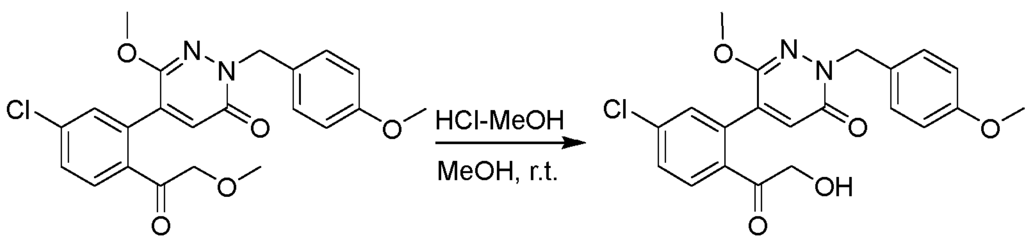


5-(5-chloro-2-(2-methoxyacetyl)phenyl)-6-methoxy-2-(4-methoxybenzyl)pyridazin-3(2H)-one (230 mg, 0.54 mmol) was added to methanol (8.0 mL) at room temperature, to which a methanol solution in hydrochloric acid (0.8 mL, 3.20 mmol, 4.0 mol/L) was added dropwise.

After the reaction was completed, it was quenched by adding water, and the pH was adjusted to neutral with saturated sodium bicarbonate. The mixture was extracted with ethyl acetate (30 ml × 3). The organic phases were combined, and the organic phase was first washed with saturated brine (20 ml × 3), then dried with anhydrous sodium sulfate and concentrated under reduced pressure. 200 mg of a yellow solid, 5-(5-chloro-2-(2-hydroxyacetyl)phenyl)-6-methoxy-2-(4-methoxybenzyl)pyridazin-3(2H)-one, was obtained, which was directly used in the next step without purification. LCMS: RT = 3.69 min, [M+H]+ = 415.07.

**Step C: Synthesis of 5-(5-chloro-2-(2-hydroxyacetyl)phenyl)-6-methoxy-2-(4-methoxybenzyl)pyridazin-3(2H)-on e**

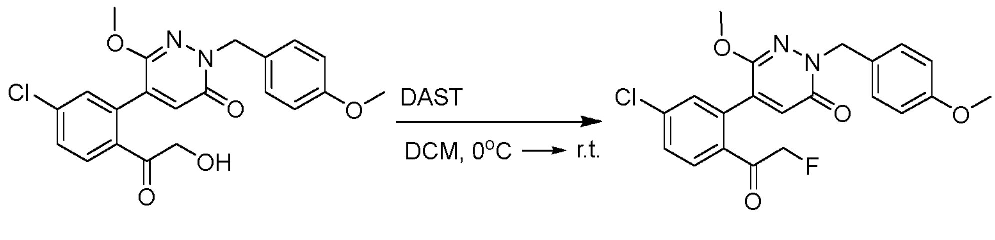


5-(5-chloro-2-(2-hydroxyacetyl)phenyl)-6-methoxy-2-(4-methoxybenzyl)pyridazin-3(2H)-o ne (200 mg, 0.48

mmol) was added to dichloromethane (10.0 mL) at 0°C, and then diethylaminosulfur trifluoride (90 μL, 0.72 mmol) was slowly added dropwise to the above solution. The reaction was carried out at room temperature overnight.

After the reaction was completed, it was quenched by adding water, and the pH was adjusted to neutral with saturated sodium bicarbonate. The mixture was extracted with ethyl acetate (20 mL × 3). The organic phases were combined, and the organic phase was first washed with saturated brine (10 mL × 2), then dried with anhydrous sodium sulfate and concentrated under reduced pressure. The obtained residue was purified by silica gel column chromatography (eluent: ethyl acetate/n-hexane=1/2). 70 mg of a yellow solid, 5-(5-chloro-2-(2-fluoroacetyl)phenyl)-6-methoxy-2-(4-methoxybenzyl)pyridazin-3(2H)-one was obtained (yield: 35.4%). LCMS: RT = 3.94 min, [M+H]+ = 417.11.

**Step D: Synthesis of 5-(5-chloro-2-(2-fluoroacetyl)phenyl)-6-methoxypyridazin-3(2H)-one**

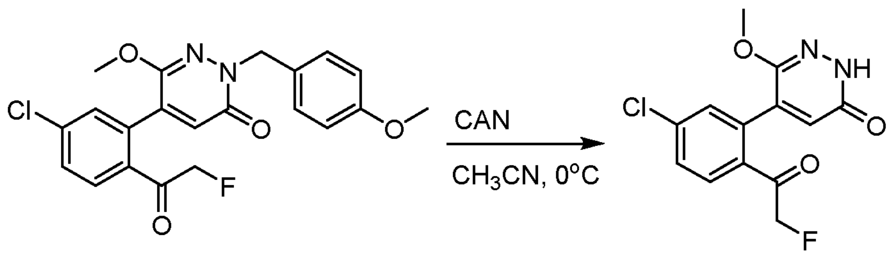


5-(5-chloro-2-(2-fluoroacetyl)phenyl)-6-methoxy-2-(4-methoxybenzyl)pyridazin-3(2H)-one (70 mg, 0.17 mmol) was added to a mixed solvent (4 mL, acetonitrile: water = 3:1) at 0°C, and then ceric ammonium nitrate (595 mg, 1.09 mmol) was slowly added. After the addition was completed, the reaction was carried out at room temperature for 30 minutes.

The reaction was completed, quenched by adding water, and the mixture was extracted with ethyl acetate (20 ml × 3). The organic phases were combined, and the organic phase was first washed with saturated brine (10 ml × 2), then dried with anhydrous sodium sulfate and concentrated under reduced pressure. The obtained residue was purified by silica gel column chromatography (eluent: ethyl acetate/n-hexane=1/1). 30 mg of a yellow solid, 5-(5-chloro-2-(2-fluoroacetyl)phenyl)-6-methoxypyridazin-3(2H)-one, was obtained (yield: 47.1%). LCMS: RT = 3.19 min, [M+H]+ = 297.02.

**Step E: Synthesis of tert-butyl (S)-4-(2-(4-(5-chloro-2-(2-fluoroacetyl)phenyl)-3-methoxy-6-oxopyridazine-1(6H)-yl)-3-phe nylpropanamido)benzoate**

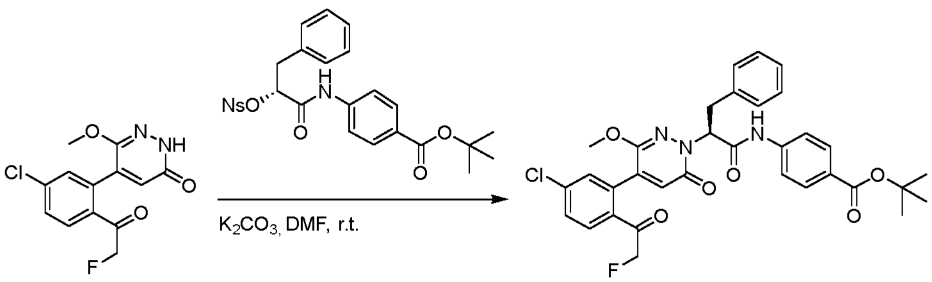


5-(5-chloro-2-(2-fluoroacetyl)phenyl)-6-methoxypyridazin-3(2H)-one (30 mg, 0.08 mmol), tert-butyl (R)-4-(2-(((4-nitrophenyl)sulfonyl)oxy)-3-phenylpropanamido)benzoate (46 mg, 0.08 mmol) and potassium carbonate (20 mg , 0.16 mmol) were added to N,N-dimethylformamide (2.0 mL) at room temperature, and the reaction was carried out overnight at room temperature.

The reaction was completed, quenched by adding water, and the mixture was extracted with ethyl acetate (10 ml × 3). The organic phases were combined, the organic phase was first washed with saturated brine (10 ml × 2), then dried with anhydrous sodium sulfate and concentrate under reduced pressure. The obtained residue was purified by silica gel column chromatography (eluent: ethyl acetate/n-hexane=1/1). 22 mg of a pale yellow solid, tert-butyl (S)-4-(2-(4-(5-chloro-2-(2-fluoroacetyl)phenyl)-3-methoxy-6-oxopyridazin-1(6H)-yl)-3-phenylp ropanamido)benzoate, was

obtained (yield: 50.0%). LCMS: RT = 4.53 min, [M-H]- = 618.13.

**Step F: Synthesis of (S)-4-(2-(4-(5-chloro-2-(2-fluoroacetyl)phenyl)-3-methoxy-6-oxopyridazine-1(6H)-yl)-3-phe nylpropanamido)benzoic acid**

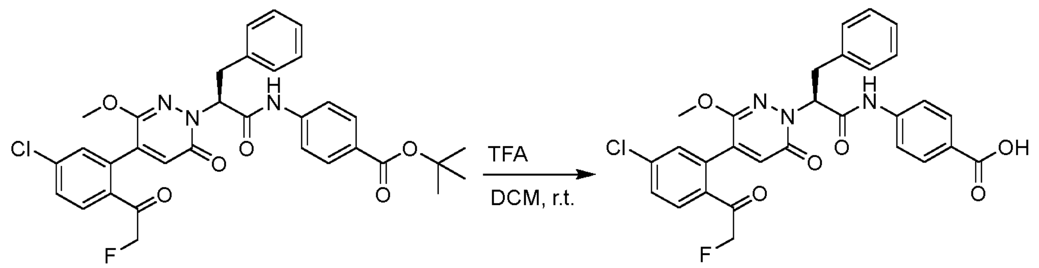


tert-Butyl (S)-4-(2-(4-(5-chloro-2-(2-difluoroacetyl)phenyl)-3-methoxy-6-oxopyridazin-1(6H)-yl)-3-phenyl propanamido)benzoate (22 mg, 0.04 mmol) was added to dichloromethane (2.0 mL) at room temperature, and trifluoroacetic acid (0.5 mL) was added dropwise. The reaction was carried out at room temperature for 3 hours.

After the reaction was completed, dichloromethane was evaporated to dryness and trifluoroacetic acid was dried with an oil pump. The obtained residue was dissolved in dichloromethane (1.0 mL), and it was added dropwise to n-hexane (10.0 mL) to precipitate a white solid, which was filtered off with suction. The filter cake was washed with n-hexane and dried to give 5 mg of a pale yellow solid, (S)-4-(2-(4-(5-chloro-2-(2-fluoroacetyl)phenyl)-3-methoxy-6-oxopyridazin-1(6H)-yl)-3-phenylp ropanamido)benzoic acid (yield: 22.2%). LCMS: RT = 3.96 min, [M-H]- = 562.06.

**Example 38**

**Synthesis** of **(S)-4-(2-(4-(2-acetyl-5-chlorophenyl)-3-methoxy-6-oxopyridazin-1(6H)-yl)-3-phenylpropan amido)-3-fluorobenzoic acid**

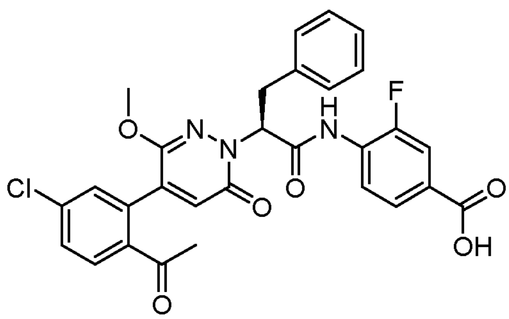

The specific synthetic route is as follows.

**Step A: Synthesis of methyl (S)-4-2-(4-(2-acetyl-5-chlorophenyl)-3-methoxy-6-oxopyridazin-1(6H)-yl)-3-phenyl-propana mido)-3-fluorobenzoate**

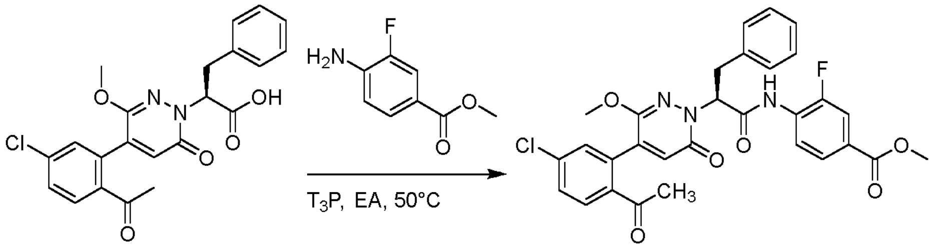


(S)-2-(4-(2-Acetyl-5-chlorophenyl)-3-methoxy-6-oxopyridazin-1(6H)-yl)-3-phenylpropanic acid (80 mg, 0.19 mmol), methyl 3-fluoro-4-amino-benzoate (38 mg, 0.22 mmol) were dissolved in ethyl acetate (2.0 mL) at room temperature, and N,N-diisopropylethylamine (0.09 mL) was added. Subsequently, 1-propylphosphoric anhydride (0.4 ml) was added to the above solution. The reaction solution was heated to 50 °C and stirred for 3 hours.

The reaction was completed, quenched by adding water, and the mixture was extracted with ethyl acetate (20 ml × 3). The organic phases were combined, and the organic phase was first washed with saturated brine (10 ml × 2), then dried with anhydrous sodium sulfate and concentrate under reduced pressure. The obtained residue was purified by silica gel column chromatography (eluent: ethyl acetate/n-hexane=1/2). 80 mg of a pale yellow solid, methyl (S)-4-2-(4-(2-acetyl-5-chlorophenyl)-3-methoxy-6-oxopyridazin-1(6H)-yl)-3-phenylpropanamid o)-3-fluorobenzoate, was obtained (yield: 73.7%). LCMS: RT = 4.28 min, [M+H]+ = 578.06.

**Step B: Synthesis of (S)-4-(2-(4-(2-acetyl-5-chlorophenyl)-3-methoxy-6-oxopyridazin-1(6H)-yl)-3-phenylpropan amido)-3-fluorobenzoic acid**

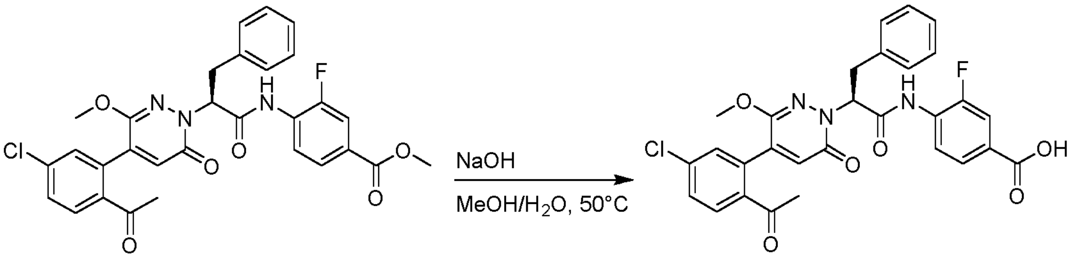


Methyl (S)-4-2-(4-(2-acetyl-5-chlorophenyl)-3-methoxy-6-oxopyridazin-1(6H)-yl)-3-phenylpropanamid o)-3-fluorobenzoate (80 mg, 0.14 mmol) was dissolved in methanol (2.0 mL). Subsequently, an aqueous solution (1.0 mL) of sodium hydroxide (11 mg, 0.28 mmol) was added to the above solution. The reaction solution was carried out at 50°C for 3 hours.

Dilute hydrochloric acid solution (1.0 mol/L) was slowly added dropwise to the reaction solution to adjust the pH value to 3-4. Ethyl acetate (30 mL) was added, and the mixture was washed with saturated brine (10 mL×3), dried with anhydrous sodium sulfate and concentrated under reduced pressure. The obtained residue was dissolved in dichloromethane (1.0 mL), and it was added dropwise to n-hexane (10.0 mL) to precipitate a white solid, which was filtered off with suction. The filter cake was washed with n-hexane and dried to obtain 38.42 mg of a white solid, (S)-4-(2-(4-(2-acetyl-5-chlorophenyl)-3-methoxy-6-oxopyridazin-1(6H)-yl)-3-phenylpropanami do)-3-fluorobenzoic acid (yield: 48.7%). LCMS: RT = 4.09 min, [MH]- = 562.07. $^1H$ NMR (500 MHz, DMSO) δ 13.13 (s, 1H), 10.28 (s, 1H), 7.99 (d, J = 8.4 Hz, 1H), 7.79 -7.73 (m, 2H), 7.72 (d, J = 1.8 Hz, 1H), 7.68 (dd, J = 8.3, 2.2 Hz, 1H), 7.49 (d, J = 2.1 Hz, 1H), 7.31 (dt, J = 15.2, 6.7 Hz, 5H), 7.19 (t, J = 7.3 Hz, 1H), 6.91 (s, 1H), 5.94 (dd, J = 10.6, 4.8 Hz, 1H), 3.68 (s, 3H), 3.42 (dd, J = 14.6, 4.7 Hz, 1H), 3.37-3.34 (m, 1H), 2.52 (s, 3H).

**Example 39**

**Synthesis of 4-(2-(4-(2-acetyl-5-chlorophenyl)-3-methoxy-6-oxopyridazin-1(6H)-yl)-3-(4-(cyclobutanefor mamido)phenyl)propanamido)benzoic acid**

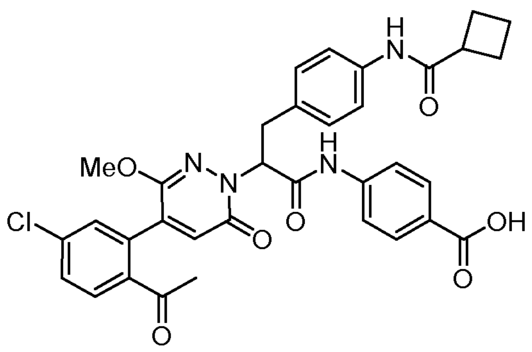

**Step A: Synthesis of 4-(2-(4-(2-acetyl-5-chlorophenyl)-3-methoxy-6-oxopyridazin-1(6H)-yl)-3-(4-(cyclobutaneformamido)phenyl)propanamido)benzoic acid**

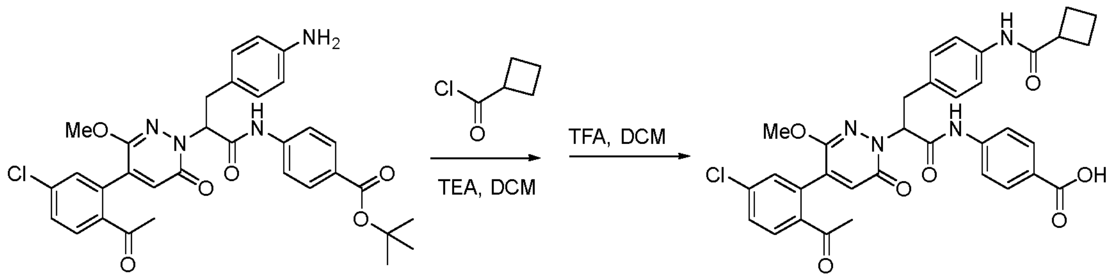


Under nitrogen protection, triethylamine (27 µl, 0.17 mmol) and cyclopropanecarbonyl chloride (15 µl, 0.12 mmol) were added to a solution of tert-butyl 4-(2-(4-(2-acetyl-5-chlorophenyl)-3-methoxy-6-oxopyridazin-1(6H)-yl)-3-(4-aminophenyl)propa namido)benzoate (40 mg, 0.065 mmol) in dichloromethane (1.0 mL), and the reaction was carried out at room temperature for 0.5 hour. The reaction solution was quenched with water (1 mL), diluted with dichloromethane (50 mL), and washed with water (30 mL) and saturated brine (30 mL) successively. It was then dried with anhydrous sodium sulfate and concentrated under reduced pressure. The obtained crude product was directly used in the next reaction. LCMS: RT = 4.36 min, [M+H]+ = 699.24.

The above crude product was dissolved in dichloromethane (1.0 ml), and trifluoroacetic acid (0.2 ml) was added. The reaction was carried out at room temperature for 45 minutes. The solvent was evaporated under reduced pressure, and the obtained residue was purified by preparative HPLC to give 20 mg of a pale yellow solid, 4-(2-(4-(2-acetyl-5-chlorophenyl)-3-methoxy-6-oxopyridazin-1(6H)-yl)-3-(4-(cyclobutaneforma mido)phenyl)propanamido)benzoic acid (yield: 48.0%). LCMS: RT = 3.82 min, [M+H]- = 641.15. $^1$H NMR (500 MHz, DMSO) δ 12.73 (s, 1H), 10.60 - 10.36 (m, 1H), 9.62 (s, 1H), 8.00 (d, J =8.4 Hz, 1H), 7.90 (d, J =8.7 Hz, 2H), 7.72 (d, J =8.8 Hz, 2H), 7.69 (dd, J =8.3, 2.1 Hz, 1H), 7.54 - 7.47 (m, 3H), 7.21 (d, J =8.5 Hz, 2H), 6.89 (s, 1H), 5.70 (dd, J =10.3, 4.8 Hz, 1H), 3.68 (s, 3H), 3.48 - 3.43 (m, 1H), 3.34 (dd, J =14.1, 4.7 Hz, 1H), 3.22 - 3.14 (m, 1H), 2.54 (s, 3H), 2.24 - 2.15 (m, 2H), 2.12 - 2.03 (m , 2H), 1.96 - 1.87 (m, 1H), 1.83 - 1.73 (m, 1H).

**Example 40**

**Synthesis of 4-(2-(4-(2-acetyl-5-chlorophenyl)-3-methoxy-6-oxopyridazin-1(6H)-yl)-3-(4-(cyclopentanefo rmamido)phenyl)propanamido)benzoic acid**

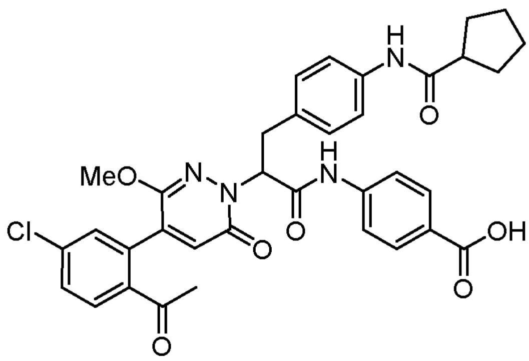

**Step A: Synthesis of 4-(2-(4-(2-acetyl-5-chlorophenyl)-3-methoxy-6-oxopyridazin-1(6H)-yl)-3-(4-(cyclopentanefo rmamido)phenyl)propanamido)benzoic acid**

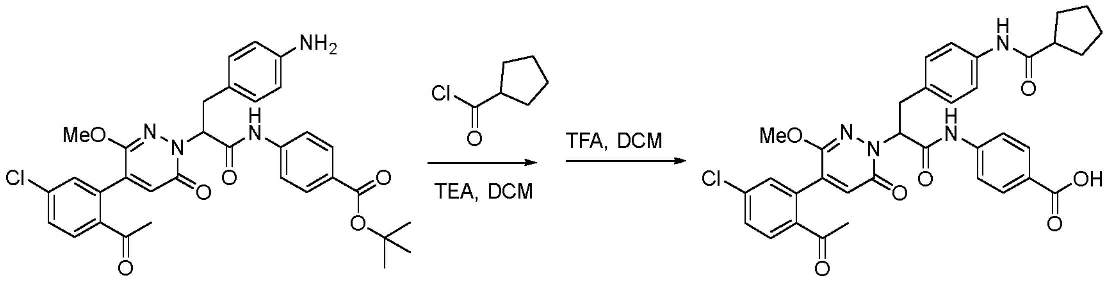


Under nitrogen protection, triethylamine (27 μl, 0.17 mmol) and cyclopentanecarbonyl chloride (15 μl, 0.12 mmol) were added to a solution of tert-butyl 4-(2-(4-(2-acetyl-5-chlorophenyl)-3-methoxy-6-oxopyridazin-1(6H)-yl)-3-(4-aminophenyl)propa namido)benzoate (40 mg, 0.065 mmol) in dichloromethane (1.0 mL), and the reaction was carried out at room temperature for 0.5 hour. The reaction solution was quenched with water (1 mL), diluted with dichloromethane (50 mL), and washed with water (30 mL) and saturated brine (30 mL) successively. It was then dried with anhydrous sodium sulfate and concentrated under reduced pressure. The obtained crude product was directly used in the next reaction. LCMS: RT = 4.44 min, [M+H]+ = 711.12.

The above crude product was dissolved in dichloromethane (1.0 ml), and trifluoroacetate (0.2 ml) was added. The reaction was carried out at room temperature for 45 minutes. The solvent was evaporated under reduced pressure, and the obtained residue was purified by preparative HPLC to give 10 mg of a pale yellow solid, 4-(2-(4-(2-acetyl-5-chlorophenyl)-3-methoxy-6-oxopyridazin-1(6H)-yl)-3-(4-(cyclopentaneform amido)phenyl)propanamido)benzoic acid (yield: 23.0%). LCMS: RT = 3.93 min, [M+H]-= 655.12. $^1$H NMR (500 MHz, DMSO) δ 12.72 (s, 1H), 10.49 (s, 1H), 9.77 (s, 1H), 8.00 (d, J =8.4 Hz, 1H), 7.90 (d, J =8.8 Hz, 2H), 7.73 (d, J =8.8 Hz, 2H), 7.69 (dd, J =8.4, 2.2 Hz, 1H), 7.54 - 7.48 ( m, 3H), 7.21 (d, J =8.5 Hz, 2H), 6.90 (s, 1H), 5.70 (dd, J =10.3, 4.8 Hz, 1H), 3.68 (s, 3H), 3.50 - 3.44 (m , 2H), 2.76 - 2.69 (m, 1H), 2.54 (s, 3H), 1.86 - 1.76 (m, 2H), 1.73 - 1.62 (m, 4H), 1.57 - 1.49 (m, 2H).

## Example 41

**Synthesis of (S)-4-(2-(4-(2-acetyl-5-fluorophenyl)-3-methoxy-6-oxopyridazin-1(6H)-yl)-3-phenylpropana mido)benzoic acid**

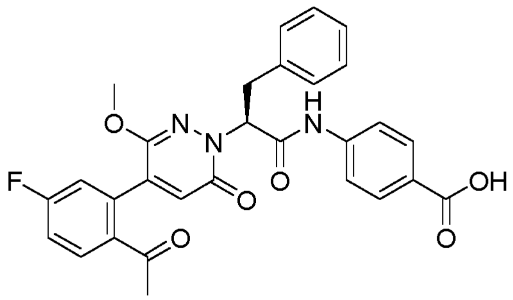

The specific synthetic route is as follows.

**Step A: Synthesis of 1-(4-fluoro-2-(4,4,5,5-tetramethyl-1,3,2-dioxaborolan-2-yl)phenyl)ethan-1-one**

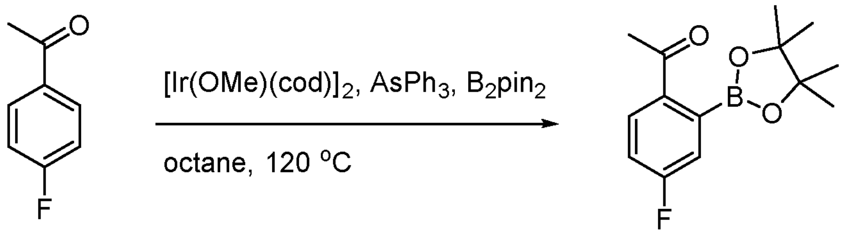


Under nitrogen protection, triphenylarsenic (80 mg, 0.26 mmol) and $[Ir(OMe)(cod)]_2$ (44 mg, 0.066 mmol) were added to a solution of 4-fluoroacetophenone (3.0 g, 21.7 mmol), bis(pinacolato)diboron (1.15 g, 4.34 mmol) in n-octane (21.8 mL), and the mixture was reacted at 120°C for 18 hours. After the reaction solution was cooled to room temperature, it was diluted with ethyl acetate (100 mL), washed with water (80 mL) and saturated brine (80 mL) successively. It was then dried with anhydrous sodium sulfate and concentrated under reduced pressure. The obtained residue was purified by silica gel column chromatography (eluent: petroleum ether/ethyl acetate=50/1-20/1). 615 mg of a pale yellow solid, 1-(4-fluoro-2-(4,4,5,5-tetramethyl-1,3,2-dioxaborolan-2-yl)phenyl)ethan-1-one, was obtained (yield: 54.0%). LCMS: RT = 4.01 min, [M-H]- = 263.04.

**Step B: Synthesis of 5-(2-acetyl-5-fluorophenyl)-6-methoxy-2-(4-methoxybenzyl)pyridazin-3(2H)-one**

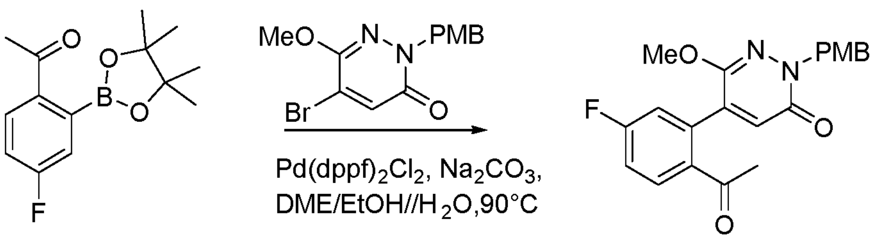


Under nitrogen protection, ethanol (0.38 mL), water (0.38 mL) and $Pd(dppf)_2Cl_2$ (24 mg, 0.034 mmol) were added to a solution of 1-(4-fluoro-2-(4,4,5,5-tetramethyl-1,3,2-dioxaborolan-2-yl)phenyl)ethan-1-one (205 mg, 0.67 mmol), 5-bromo-6-methoxy-2-(4-methoxybenzyl)pyridazin-3(2H)-one (146 mg, 0.45 mmol), sodium carbonate (142 mg, 1.34 mmol) in ethylene glycol dimethyl ether (3.0 mL), and the mixed solution was reacted at 90°C for 1 hour. After the reaction solution was cooled to room temperature, it was extracted with ethyl acetate (30 mL×2), and the organic phases were combined and washed with water (40 mL) and saturated brine (40 mL) successively. It was then dried with anhydrous sodium sulfate and concentrated under reduced pressure. The obtained residue was purified by silica gel column chromatography (eluent: petroleum ether/ethyl acetate=2/1). 120 mg of a pale yellow oily product, 5-(2-acetyl-5-fluorophenyl)-6-methoxy-2-(4-methoxybenzyl)pyridazin-3(2H)-one, was obtained(yield: 70.0%). LCMS: RT = 3.39 min, [M+H]+ = 383.16.

**Step C: Synthesis of 5-(2-acetyl-5-fluorophenyl)-6-methoxypyridazin-3(2H)-one**

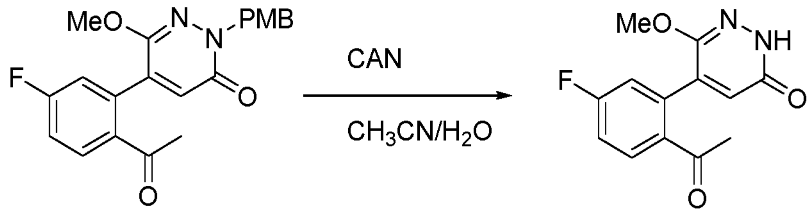


Ceric ammonium nitrate (1.38 g, 2.51 mmol) was added to a solution of 5-(2-acetyl-5-fluorophenyl)-6-methoxy-2-(4-methoxybenzyl)pyridazin-3(2H)-one (120 mg, 0.31 mmol) in acetonitrile/water (2.4 mL/0.8 mL) under ice-water bath. After the addition was completed, the ice-water bath was removed, and the reaction was carried out at room temperature for 0.5 hour. The reaction solution was extracted with ethyl acetate (50 mL×2), and the organic phases were combined and washed with water (50 mL×2) and saturated brine (50 mL) successively. It was then dried with anhydrous sodium sulfate and concentrated under reduced pressure. The obtained crude product was directly used in the next reaction. LCMS: RT = 3.00 min, [M+H]+ = 263.11.

**Step D: Synthesis of tert-butyl (S)-4-(2-(4-(2-acetyl-5-fluorophenyl)-3-methoxy-6-oxopyridazin-1(6H)-yl)3-phenylpropana mido)benzoate**

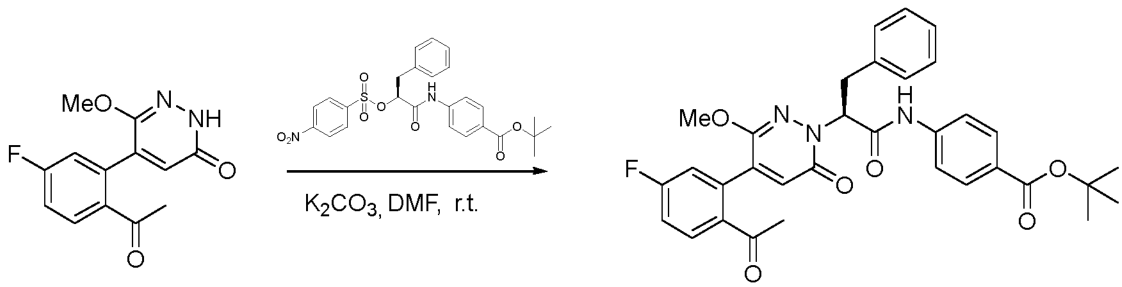


Potassium carbonate (87 mg, 0.63 mmol) and tert-butyl (R)-4-(2-(((4-nitrophenyl)sulfonyl)oxy)-3-phenylpropanamido)benzoate (182 mg, 0.35 mmol) was added to a solution of the above crude product in N,N-dimethylformamide (3.1 mL) at room temperature. The reaction was carried out at room temperature overnight.

The reaction solution was extracted with ethyl acetate (50 mL×2), and the organic phases were combined and washed with water (50 mL×2) and saturated brine (50 mL) successively. It was then dried with anhydrous sodium sulfate and concentrated under reduced pressure. The obtained residue was purified by silica gel column chromatography(eluent: petroleum ether/ethyl acetate = 3/1 - 2/1). 153 mg of a colorless oily product, tert-butyl (S)-4-(2-(4-(2-acetyl-5-fluorophenyl)-3-methoxy-6-oxopyridazin-1(6H)-yl)3-phenylpropanamid o)benzoate, was obtained (yield: 83.0%). LCMS: RT = 4.40 min, [M+H]+ = 586.21.

**Step E: Synthesis of (S)-4-(2-(4-(2-acetyl-5-fluorophenyl)-3-methoxy-6-oxopyridazin-1(6H)-yl)-3-phenylpropana mido)benzoic acid**

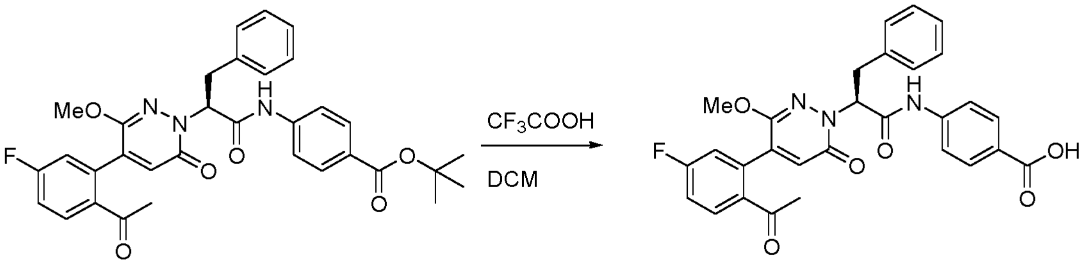


Trifluoroacetate (0.3 mL) was added to a solution of tert-butyl (S)-4-(2-(4-(2-acetyl-5-fluorophenyl)-3-methoxy-6-oxopyridazin-1(6H)-yl)3-phenylpropanamid o)benzoic acid (153 mg, 0.26 mmol) in dichloromethane (2.0 mL) at room temperature. After the addition was completed, the reaction was carried out at room temperature for 0.5 hour, and the solvent was evaporated under reduced pressure. The residue was dissolved in dichloromethane (1.0 ml), and the solution was slowly added dropwise to 30 ml of n-hexane, and a large amount of solid was precipitated. It was filtrated under reduced pressure, and 85 mg of a pale yellow solid, (S)-4-(2-(4-(2-acetyl-5-fluorophenyl)-3-methoxy-6-oxopyridazin-1(6H)-yl)-3-phenylpropanamid o)benzoic acid, was obtained (yield: 62.0%). LCMS: RT = 3.84 min, [M-H]- = 528.07.

**Example 42**

**Synthesis of (S)-4-(2-(4-(3-chloro-2,6-difluorophenyl)-6-oxopyridazin-1(6H)-yl)-3-phenylpropanamido)b enzoic acid**

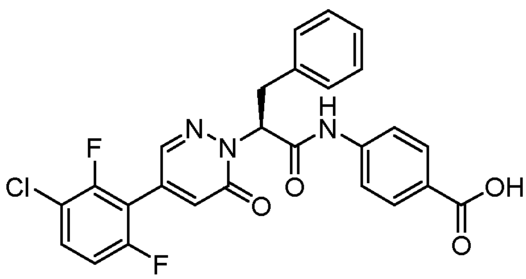

The specific synthetic route is as follows.

**Step A: synthesis of 1-chloro-2,4-difluoro-3-vinylbenzene**

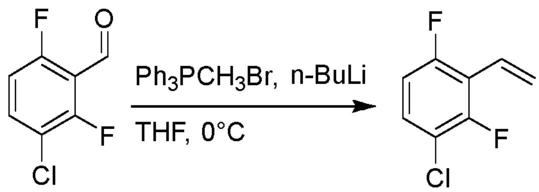


A tetrahydrofuran solution of n-butyllithium (21.3 mL, 33.60 mmol, 1.6 mol/L) was slowly added dropwise to tetrahydrofuran (50.0ml) containing methyltriphenylphosphine bromide (12.18 g, 34.00 mmol) while keeping the temperature at 0°C, and the mixture was stirred at the same temperature for 1 hour. Subsequently, 5-chloro-2,6-difluorobenzaldehyde (5.00 g, 28.32 mmol) in tetrahydrofuran (5.0 mL) was added to the above solution. It was kept stirring at this temperature for 30 minutes, warmed to room temperature and reacted for 2 hours.

The reaction was completed, quenched by adding water, and the mixture was extracted with ethyl acetate (100ml × 3 times). The organic phases were combined, and the organic phases were first washed with saturated brine (50 ml × 2 times), then dried with anhydrous sodium sulfate, and finally concentrated under reduced pressure. The obtained residue was purified by silica gel column chromatography (eluent: pure n-hexane). 1.50 g of colorless transparent

liquid, 1-chloro-2,4-difluoro-3-vinylbenzene, was obtained (yield: 25.2%). LCMS: RT = 4.48 min.

**Step B: synthesis of 2-(3-chloro-2,6-difluorophenyl)acetaldehyde**

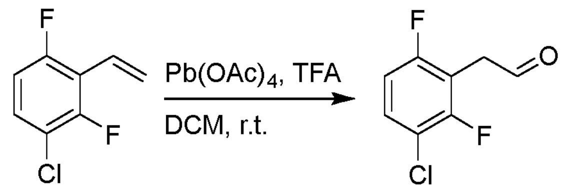


Lead tetraacetate and trifluoroacetic acid were added to a reaction flask at 0°C. Subsequently, a solution of 1-chloro-2,4-difluoro-3-vinylbenzene (1.50 mg, 8.57 mmol) in dichloromethane (15.0 mL) was added dropwise. After 2 minutes, the ice bath was removed, and the reaction was carried out at room temperature for 2 hours.

The reaction was completed, quenched by adding water, and then saturated sodium chloride solution was added until no white solid was produced. After suction filtration, the filter cake was washed with dichloromethane, the filtrate was adjusted to neutral pH with saturated sodium bicarbonate, and the layers were separated. The organic phase was washed with saturated brine (50 mL × 3 times), then dried with anhydrous sodium sulfate, and finally concentrated under reduced pressure. 1.50 g of yellow oily product, 2-(3-chloro-2,6-difluorophenyl)acetaldehyde, was obtained, which was directly used in the next reaction without purification.

**Step C: synthesis of 4-(3-chloro-2,6-difluorophenyl)-5-hydroxyfuran-2(5H)-one**

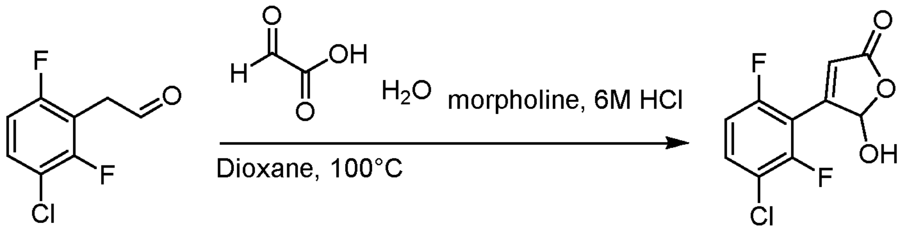


At room temperature, morpholine (719 mg, 8.26 mmol), hydrochloric acid (1.4 mL, 8.6 mmol, 6.0 mol/L), glyoxylic acid hydrate (688 mg, 7.48 mmol) and 2-(3-chloro-2,6-difluorophenyl)acetaldehyde (1.5 g , 0.48 mmol) were successively added to 1,4-dioxane (10.0 ml), and the reaction was refluxed for 2 hours.

The reaction was completed, quenched by adding water, and the mixture was extracted with ethyl acetate (50 ml × 3 times). The organic phases were combined, and the organic phase was first washed with saturated brine (30 ml × 2 times), then dried with anhydrous sodium sulfate, and finally concentrated under reduced pressure. The obtained residue was purified by silica gel column chromatography (eluent: ethyl acetate/n-hexane=1/10). 1.00 g of a yellow solid, 4-(3-chloro-2,6-difluorophenyl)-5-hydroxyfuran-2(5H)-one, was obtained as (yield: 47.3%). LCMS: RT = 3.45 min, $[M-H]^-$= 244.93.

**Step D: synthesis of 5-(3-chloro-2,6-difluorophenyl)pyridazin-3(2H)-one**

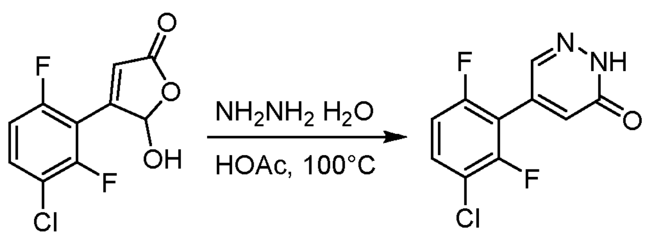

At room temperature, 4-(3-chloro-2,6-difluorophenyl)-5-hydroxyfuran-2(5H)-one (500 mg, 2.03 mmol) was added to glacial acetic acid (3 ml), and then 80% hydrazine hydrate (246 μl, 4.06 mmol) was slowly added. The reaction was carried out at 100°C for 1 hour.

After the reaction was completed, it was cooled to room temperature, and a large amount of solid was precipitated, which was diluted with a small amount of ethyl acetate to disperse the solid. After suction filtration, the filter cake was washed with a small amount of ethyl acetate and dried to obtain 330 mg of white solid, 5-(3-chloro-2,6-difluorophenyl)pyridazin-3(2H)-one (yield: 67.0%). LCMS: RT = 3.28 min, $[M+H]^+$ = 242.98.

**Step E: synthesis of (S)-tert-butyl 4-(2-(4-(3-chloro-2,6-difluorophenyl)-6-oxopyridazin -1(6H)-yl)-3-phenylpropanamido)benzoate**

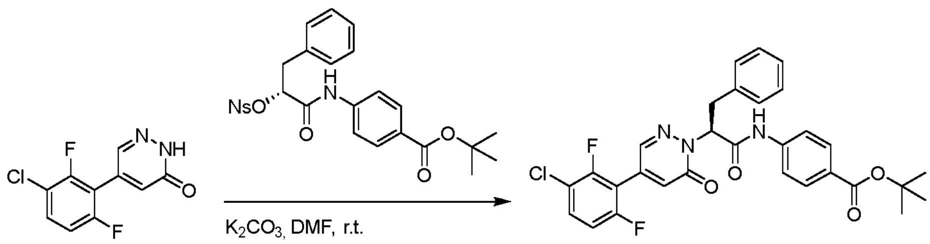


At room temperature, 5-(3-chloro-2,6-difluorophenyl)pyridazin-3(2H)-one (100 mg, 0.42 mmol), tert-butyl (R)-4-(2-(((4-nitrophenyl)sulfonyl)oxy)-3-phenylpropanamido)benzoate (239 mg, 0.45 mmol) and potassium carbonate (285 mg, 2.10 mmol) were added to N,N-dimethylformamide (5.0 ml) and reacted at room temperature for 3 hours.

The reaction was completed, quenched by adding water, and the mixture was extracted with ethyl acetate (20ml × 3 times). The organic phases were combined, and the organic phases were first washed with saturated brine (10ml × 2 times), then dried with anhydrous sodium sulfate, and finally concentrated under reduced pressure. The obtained residue was purified by silica gel column chromatography (eluent: ethyl acetate/n-hexane=1/2). 230 mg of a pale yellow solid, tert-butyl (S)-4-(2-(4-(3-chloro-2,6-difluorophenyl)-6-oxopyridazin-1(6H)-yl)-3-phenylpropanamido)benz oate, was obtained (yield: 96.8%). LCMS: RT = 4.60 min, $[M+H]^+$ = 566.13.

**Step F: synthesis of (S)-4-(2-(4-(3-chloro-2,6-difluorophenyl)-6-oxopyridazin-1(6H)-yl)-3-phenylpropanamido)b enzoic acid**

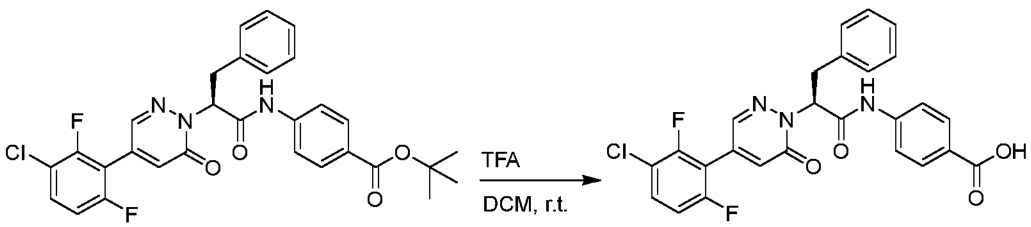


At room temperature, tert-butyl (S)-4-(2-(4-(3-chloro-2,6-difluorophenyl)-6-oxopyridazin-1(6H)-yl)-3-phenylpropanamido)benz oate (230 mg, 0.40 mmol) was added to dichloromethane (4.0 ml), and trifluoroacetic acid (1.0 ml) was added dropwise. the mixture was reacted at room temperature for 3 hours.

After the reaction was completed, dichloromethane was evaporated to dryness and trifluoroacetic acid was pumped dry with an oil pump. The resulting residue was dissolved in dichloromethane (2.0 ml) and added dropwise to n-hexane (30.0 ml) to precipitate a white solid. After suction filtration, the filter cake was washed with n-hexane and dried to obtain 5 mg of light yellow solid, (S)-4-(2-(4-(3-chloro-2,6-difluorophenyl)-6-oxopyridazin-1(6H)-yl)-3-phenylpropanamido)benz oic acid (yield: 75.8%). LCMS: RT = 4.02 min, [M-H]- = 508.06. $^1H$ NMR (500 MHz, DMSO) δ 12.76 (s, 1H), 10.66 (s, 1H), 8.19 (d, J = 1.4 Hz, 1H), 7.92 (d, J = 8.8 Hz, 2H), 7.88-7.81 (m, 1H), 7.72 (d, J = 8.8 Hz, 2H), 7.40 (td, J = 9.0, 1.4 Hz, 1H), 7.33-7.25 (m, 4H), 7.20 (t, J = 7.6 Hz, 2H), 5.90 (dd, J = 9.3, 6.0 Hz, 1H), 3.57-3.46 (m, 2H).

**Example 43**

**Synthesis of (S)-4-(2-(4-(2-acetyl-5-chlorophenyl)-6-oxo-3-phenylpyridazin-1(6H)-yl)-3-phenylpropana mido)benzoic acid**

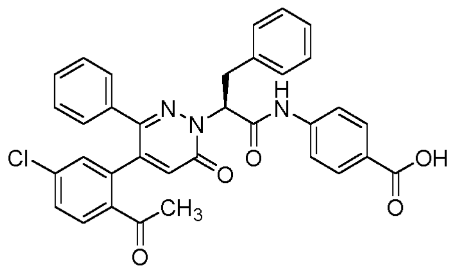

The specific synthetic route is as follows.

**Step A: synthesis of 5-(2-acetyl-5-chlorophenyl)-6-phenylpyridazin-3(2H)-one**

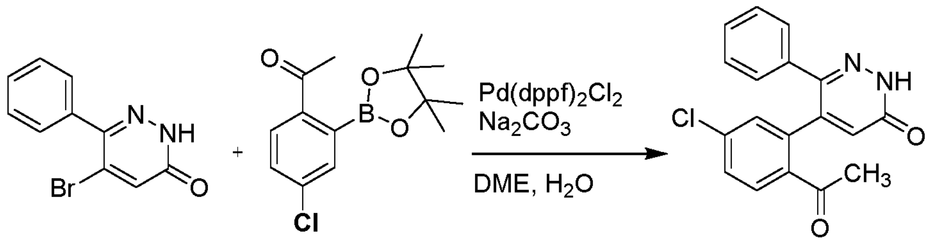


5-bromo-6-phenylpyridazin-3(2H)-one (150 mg, 0.60 mmol) and 1-(4-chloro-2-(4,4,5,5-tetramethyl-1,3,2-dioxaborolan-2-yl)phenyl)ethan-1-one (181 mg, 0.64 mmol) were dissolved in ethylene glycol dimethyl ether (10.0 mL) and water (2.0 mL). Subsequently, sodium carbonate (126 mg, 1.10 mmol) and [1,1'-bis(diphenylphosphino)ferrocene]palladium dichloride (69 mg, 0.090 mmol) were added to the above solution. It was stirred at 120°C for 4 hours.

Water (50 mL) was added to the reaction to dilute the reaction solution. The mixture was extracted with ethyl acetate (20 mL×3 times). The organic phases were combined. The organic phase was first washed with saturated brine (20 ml × 3 times), then dried with anhydrous sodium sulfate, and finally concentrated under reduced pressure. The obtained residue was purified by silica gel column chromatography (eluent: ethyl acetate/petroleum ether=1/2). 30 mg of solid 5-(2-acetyl-5-chlorophenyl)-6-phenylpyridazin-3(2H)-one was obtained (yield: 16.0%). LCMS : RT = 3.55 min, $[M+H]^+$ = 299.20.

**Step B: synthesis of tert-butyl (S)-4-(2-(4-(2-acetyl-5-chlorophenyl)-6-oxo-3-phenylpyridazin-1(6H)-yl)-3-phenylpropana mido)benzoate**

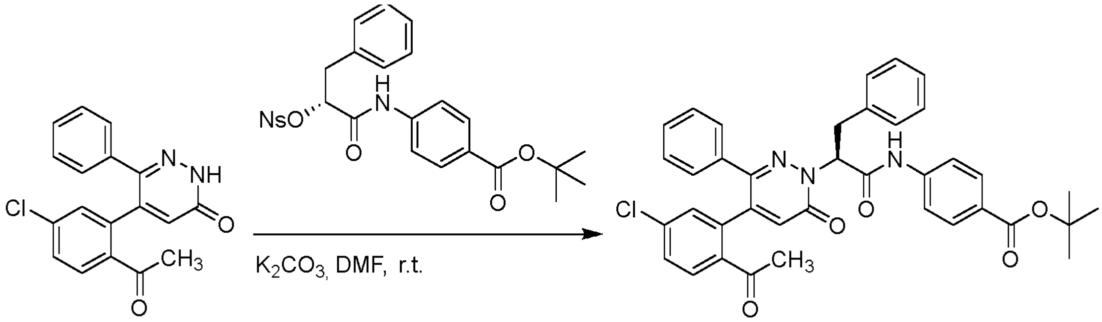


5-(2-acetyl-5-chlorophenyl)-6-phenylpyridazin-3(2H)-one (30 mg, 0.092 mmol) and tert-butyl (R)-4-(2-(((4-nitrophenyl)sulfonyl)oxy)-3-phenylpropanamido)benzoate (58 mg, 0.11 mmol) were dissolved in N,N-dimethylformamide (10.0 mL). Subsequently, potassium carbonate (25 mg, 0.18 mmol) was added to the above solution. It was stirred at room temperature for 4 hours.

Water (20 mL) was added to the reaction to dilute the reaction solution. The mixture was extracted with ethyl acetate (10 mL×3 times). The organic phases were combined. The organic phase was first washed with saturated brine (10 ml × 3 times), then dried with anhydrous sodium sulfate, and finally concentrated under reduced pressure. 18 mg of a crude product, tert-butyl (S)-4-(2-(4-(2-acetyl-5-chlorophenyl)-6-oxo-3-phenylpyridazin-1(6H)-yl)-3-phenylpropanamido )benzoate, was obtained (yield: 30.0%). LCMS: RT= 4.41 min, $[M+H]^+$= 647.13.

**Step C: synthesis of (S)-4-(2-(4-(2-acetyl-5-chlorophenyl)-6-oxo-3-phenylpyridazin-1(6H)-yl)-3-phenylpropana mido)benzoic acid**

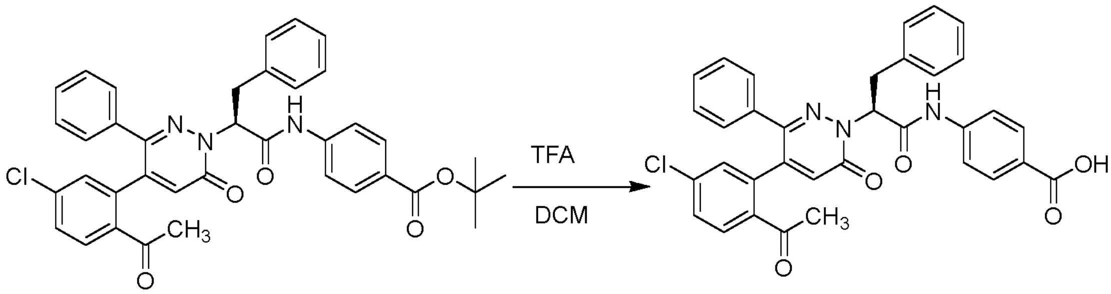


tert-Butyl (S)-4-(2-(4-(2-acetyl-5-chlorophenyl)-6-oxo-3-phenylpyridazin-1(6H)-yl)-3-phenylpropanamido )benzoate (18 mg, 0.027 mmol) was dissolved in dichloromethane (4.0 mL). Subsequently, trifluoroacetic acid (1.0 ml) was added to the above solution, and the mixture was stirred at room temperature for 2 hours.

The reaction solution was concentrated under reduced pressure, and purified by preparative high performance liquid phase chromatography to obtain 13 mg of white solid, (S)-4-(2-(4-(2-acetyl-5-chlorophenyl)-6-oxo-3-phenylpyridazin-1(6H)-yl)-3-phenylpropanamido )benzoic acid (yield: 81.0%). LCMS: RT = 4.20 min, $[M+H]^+$ = 592.14.

**Example 44**

**Synthesis of (S)-4-(2-(4-(2-acetyl-5-chlorophenyl)-3-methoxy-6-oxopyridazin-1(6H)-yl)-N-methyl-3-phen ylpropanamido)benzoic acid**

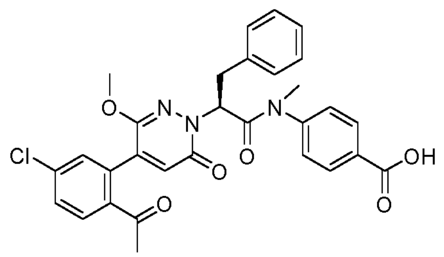

The specific synthetic route is as follows.

**Step A: synthesis of methyl (R)-4-(2-chloro-N-methyl-3-phenylpropanamido)benzoate**

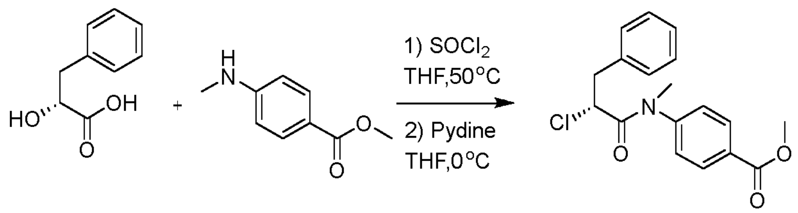


D-phenyllactic acid (0.5 g, 3.0 mmol) was dissolved in dry tetrahydrofuran (40.0 mL) and placed in a dry three-necked flask. Under nitrogen protection, it was stirred under an ice bath for 15 minutes, and thionyl chloride (0.7 mL, 9.0 mmol) was slowly added dropwise to the reaction solution. After 30 minutes, the dropwise addition was completed. It was heated to 70°C and stirred at a constant temperature for 5 hours. The reaction solution was cooled to room temperature, spin-dried, evacuated by an oil pump for 15 minutes, and then dissolved in THF to prepare solution A. Methyl 4-(methylamino)benzoate (500 mg, 3.0 mmol) and diisopropylethylamine (1.5 mL, 9.0 mmol) were dissolved in dry tetrahydrofuran (20.0 mL) and placed in a dry three-necked flask. Under nitrogen protection, it was stirred under an ice bath for 15 minutes, and solution A was slowly added dropwise to the mixture. It was stirred under an ice bath for 1 hour, and monitored by LCMS until the reaction was complete.

Water was added to the reaction solution to quench the reaction. The mixture was extracted with ethyl acetate (40 mL×3 times). The organic phases were combined, and the organic phase was first washed with saturated brine (20 ml × 3 times), then dried with anhydrous sodium sulfate, and finally concentrated under reduced pressure. The obtained residue was purified by silica gel column chromatography (eluent: ethyl acetate/petroleum ether=1/4), 600 mg of a yellow solid, methyl (R)-4-(2-chloro-N-methyl-3-phenylpropanamido)benzoate, was obtained(yield: 67.0%). LCMS: RT = 4.10 min, $[M+H]^+$= 332.11.

**Step B: synthesis of methyl (S)-4-(2-(4-(2-acetyl-5-chlorophenyl)-3-methoxy-6-oxopyridazin-1(6H)-yl)-N-methyl-3-phen ylpropanamido)benzoate**

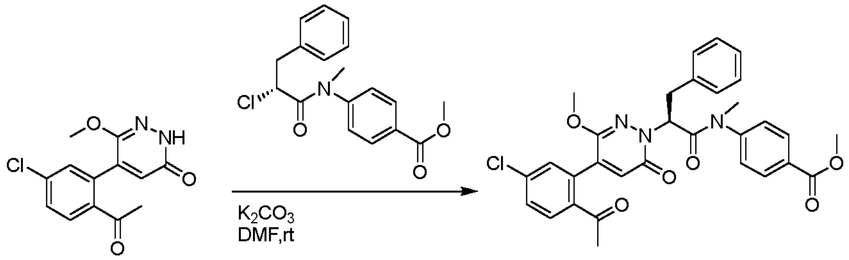


5-(2-acetyl-5-chlorophenyl)-6-ethoxypyridazin-3(2H)-one (60 mg, 0.22 mmol) was dissolved in N,N-dimethylformamide (2.0 ml). Subsequently, potassium carbonate (60 mg, 0.44 mmol) and methyl (R)-4-(2-chloro-N-methyl-3-phenylpropanamido)benzoate (100 mg, 0.32 mmol) were added to the above solution. It was stirred at room temperature

for 15 hours.

Water was added to the reaction solution to quench the reaction. The mixture was extracted with ethyl acetate (20 mL×3 times). The organic phases were combined, and the organic phase was first washed with saturated brine (10 ml × 3 times), then dried with anhydrous sodium sulfate, and finally concentrated under reduced pressure. The obtained residue was purified by silica gel column chromatography (eluent: ethyl acetate/petroleum ether=1/1). 40 mg of yellow solid, methyl (S)-4-(2-(4-(2-acetyl-5-chlorophenyl)-3-methoxy-6-oxopyridazin-1(6H)-yl)-N-methyl-3-phenylp ropanamido)benzoate, was obtained (yield: 32.0%). LCMS: RT = 4.11 min, $[M+H]^+$= 572.08.

**Step C: synthesis of (S)-4-(2-(4-(2-acetyl-5-chlorophenyl)-3-methoxy-6-oxopyridazin-1(6H)-yl)-N-methyl-3-phen ylpropanamido)benzoic acid**

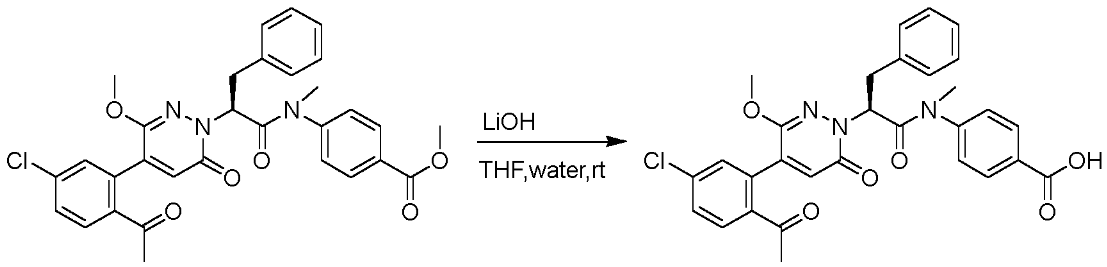


Methyl (S)-4-(2-(4-(2-acetyl-5-chlorophenyl)-3-methoxy-6-oxopyridazin-1(6H)-yl)-N-methyl-3-phenylp ropanamido)benzoate (40 mg, 0.07 mmol) was dissolved in tetrahydrofuran (4.0 mL) and water (1.0 mL). Subsequently, lithium hydroxide (6 mg, 0.14 mmol) was added to the above solution, followed by stirring at room temperature for 6 hours.

Water (10 mL) was added to the reaction solution, and the mixture was extracted with ethyl acetate (20 mL×3 times). The organic phases were combined, and the organic phase was first washed with saturated brine (10 ml × 3 times), then dried with anhydrous sodium sulfate, and finally concentrated under reduced pressure. The obtained residue was purified by preparative high performance liquid chromatography to give 16 mg of yellow solid, (S)-4-(2-(4-(2-acetyl-5-chlorophenyl)-3-methoxy-6-oxopyridazin-1(6H)-yl)-N-methyl-3-phenylp ropanamido)benzoic acid (yield: 41.0%). LC-MS: RT = 3.87 min, $[M+H]^+$= 560.14. $^1H$ NMR (500 MHz, DMSO) δ 12.97 (s, 1H), 7.99 (d, *J* = 8.4 Hz, 1H), 7.88 (d, *J* = 8.4 Hz, 2H), 7.68 (dd, *J* = 8.4, 2.2 Hz, 1H), 7.34 (m, 3H), 7.15 (m, 3H), 6.99 (s, 2H), 6.55 (s, 1H), 5.57 (s, 1H), 3.58 (s, 3H), 3.29 (d, *J* = 14.1, 5.6 Hz, 1H), 3.19 (s, 3H), 3.16 (s, 1H), 2.54 (s, 3H).

**Example 45**

**Synthesis of (S)-4-(2-(4-(5-chloro-2-(2-methoxyacetyl)phenyl)-3-methoxy-6-oxopyridazin-1(6H)-yl)-3-ph enyl-propanamido)benzoic acid**

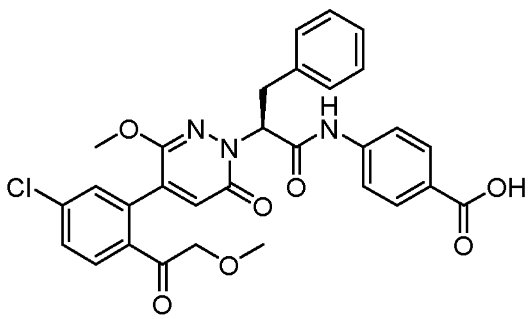

The specific synthetic route is as follows.

**Step A: synthesis of (S)-4-(2-(4-(5-chloro-2-(2-methoxyacetyl)phenyl)-3-methoxy-6-oxopyridazin-1(6H)-yl)-3-phenylpropanamido)benzoic acid**

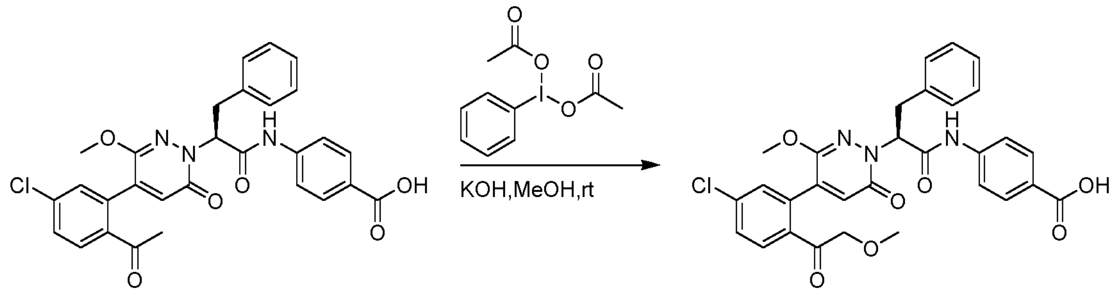


(S)-4-(2-(4-(2-acetyl-5-chlorophenyl)-3-methoxy-6-oxopyridazin-1(6H)-yl)-3-phenylpropan amido)benzoic acid (80 mg, 0.15 mmol) was dissolved in methanol (4.0 mL). Subsequently, potassium hydroxide (33 mg, 0.60 mmol) and diacetyliodobenzene (60 mg, 0.18 mmol) were added to the above solution, and the mixture was stirred at room temperature for 1 hour.

Water (10 mL) was added to the reaction solution, diluted hydrochloric acid (1.0 mL) was added, and the mixture was extracted with ethyl acetate (20 mL×3 times). The organic phases were combined, and the organic phase was washed with saturated brine (10 mL×3 times), dried with anhydrous sodium sulfate, filtered, and concentrated under reduced pressure. The resulting residue was purified by preparative high performance liquid chromatography to obtain 47 mg of yellow solid, (S)-4-(2-(4-(5-chloro-2-(2-methoxyacetyl)phenyl)-3-methoxy-6-oxopyridazin-1(6H)-yl)-3-phenylpropanamido)benzoic acid (yield: 55.0%). LCMS: RT = 3.97 min, $[M+H]^+$= 576.15.

**Example 46**

**Synthesis of 4-(2-(4-(2-acetyl-5-chlorophenyl)-3-methoxy-6-oxopyridazin-1(6H)-yl)-4-methylpentanami do)benzoic acid**

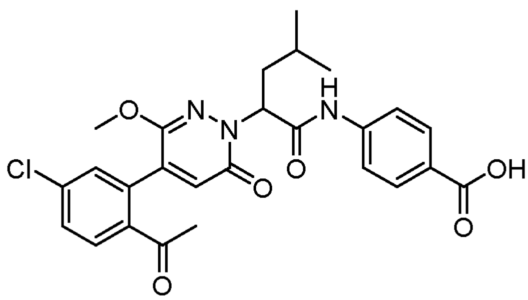

The specific synthetic route is as follows.

**Step A: synthesis of 2-hydroxy-4-methylpentanoic acid**

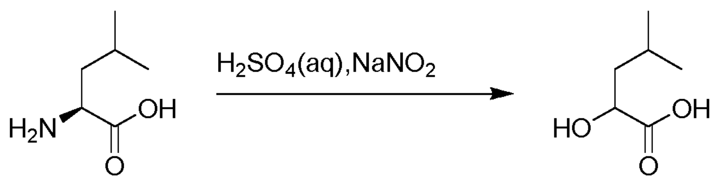

L-leucine (2.0 g, 15.2 mmol) was dissolved in 1 M sulfuric acid (40.0 mL), an aqueous solution of sodium nitrite (8.0 g) was slowly added dropwise under an ice-salt bath. The temperature was naturally warmed to room temperature and it was stirred overnight.

The reaction solution was concentrated under reduced pressure, and the obtained residue was purified by preparative high performance liquid chromatography to obtain 2.0 g of liquid 2-hydroxy-4-methylpentanoic acid (yield: 100.0%). LCMS: RT = 3.69 min.

**Step B: synthesis of tert-butyl 4-(2-hydroxy-4-methylpentanamido)benzoate**

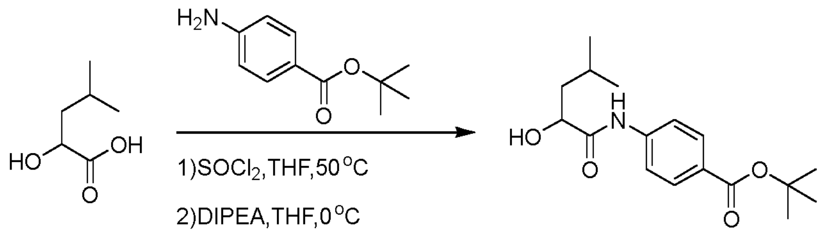


2-hydroxy-4-methylpentanoic acid (2.0 g, 15.0 mmol) was dissolved in dry tetrahydrofuran (40.0 mL) and placed in a dry three-necked flask. Under nitrogen protection, it was stirred under an ice bath for 15 minutes, and thionyl chloride (3.6 g, 30.0 mmol) was slowly added dropwise to the reaction solution. The dropwise addition was completed after 30 minutes. After heating to 50°C and stirring at constant temperature for 3 hours, the reaction solution was cooled to room temperature, spin-dried, evacuated by oil pump for 15 minutes, and then dissolved in THF to prepare solution A. tert-Butyl 4-aminobenzoate (2.5 g, 12.8 mmol) and diisopropylethylamine (5.3 mL, 45.0 mmol) were dissolved in dry tetrahydrofuran (20.0 mL) and placed in a dry three-necked flask. Under nitrogen protection, it was stirred under an ice bath for 15 minutes, and solution A was slowly added dropwise to the mixture. After stirring in an ice bath for 1 hour, the reaction was monitored by LCMS until the reaction was complete.

Water was added to the reaction solution to quench the reaction. The mixture was extracted with ethyl acetate (40 mL×3 times). The organic phases were combined, and the organic phase was first washed with saturated brine (30 ml × 3 times), then dried with anhydrous sodium sulfate, and finally concentrated under reduced pressure. The obtained residue was purified by silica gel column chromatography (eluent: ethyl acetate/petroleum ether=1/4) to obtain 224 mg of a yellow solid, tert-butyl 4-(2-hydroxy-4-methylpentanamido)benzoate (yield: 6.0%). LCMS: RT = 4.16 min, $[M-H]^-$= 306.06.

**Step C: synthesis of tert-butyl 4-(4-methyl-2-(((4-nitrophenyl)sulfonyl)oxy)pentanamido)benzoate**

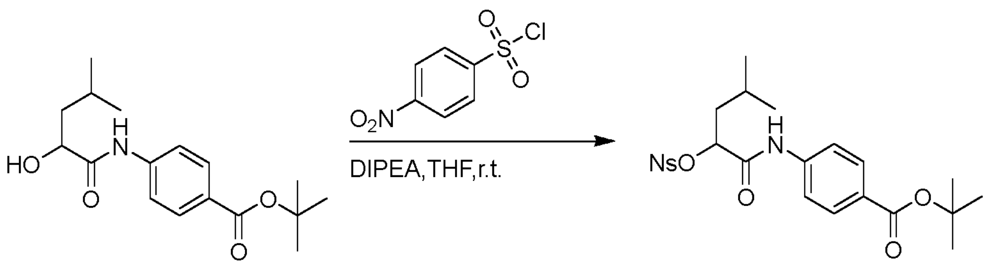


tert-Butyl 4-(2-hydroxy-4-methylpentanamido)benzoate (224 mg, 0.73 mmol) and triethylamine (0.3 mL, 2.1 mmol) were dissolved in dichloromethane (10.0 mL). 4-Nitrobenzenesulfonyl chloride (221 mg, 1.0 mmol) was added to the reaction solution under an ice bath, and the mixture was stirred at room temperature for 2 hours.

Saturated sodium bicarbonate solution (10 mL) was added to the reaction solution to quench the reaction. The mixture was extracted with ethyl acetate (30 mL×3 times). The organic phases were combined. The organic phase was first washed with saturated brine (10 ml × 3 times), then dried with anhydrous sodium sulfate, and finally concentrated under reduced pressure. The obtained residue was dissolved in dichloromethane (4 mL), and it was added dropwise to n-hexane (60 mL) with stirring. A large amount of white solid was precipitated and filtered, and the filter cake was collected

to obtain 300 mg of white solid, tert-butyl 4-(4-methyl-2-(((4-nitrophenyl)sulfonyl)oxy)pentanamido)benzoate (yield: 83.0%). LCMS: RT = 4.44 min.

**Step D: synthesis of tert-butyl 4-(2-(4-(2-acetyl-5-chlorophenyl)-3-methoxy-6-oxopyridazin-1(6H)-yl)-4-methylpentanami do)benzoate**

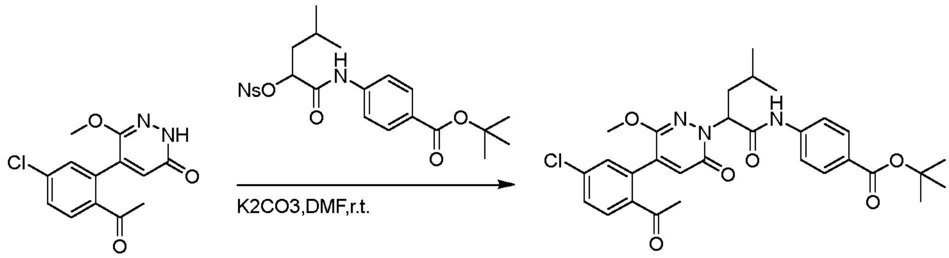


5-(2-acetyl-5-chlorophenyl)-6-methoxypyridazin-3(2H)-one (58 mg, 0.20 mmol) was dissolved in N,N-dimethylformamide (2.0 ml). Subsequently, potassium carbonate (83 mg, 0.60 mmol) and tert-butyl 4-(4-methyl-2-(((4-nitrophenyl)sulfonyl)oxy)pentanoylamino)benzoate (150.0 mg, 0.30 mmol) were added to the above solution. It was stirred at room temperature for 12 hours.

Water was added to the reaction solution to quench the reaction. The mixture was extracted with ethyl acetate (20 mL×3 times). The organic phases were combined, and the organic phase was first washed with saturated brine (10 ml × 3 times), then dried with anhydrous sodium sulfate, and finally concentrated under reduced pressure. The obtained residue was purified by silica gel column chromatography (eluent: ethyl acetate/petroleum ether=1/1). 66 mg of yellow solid, tert-butyl 4-(2-(4-(2-acetyl-5-chlorophenyl)-3-methoxy-6-oxopyridazin-1(6H)-yl)-4-methylpentanamido)b enzoate, was obtained (yield: 24.0%). LCMS: RT = 4.60 min , $[M+H]^+$ = 568.18.

**Step E: synthesis of 4-(2-(4-(2-acetyl-5-chlorophenyl)-3-methoxy-6-oxopyridazin-1(6H)-yl)-4-methylpentanami do)benzoic acid**

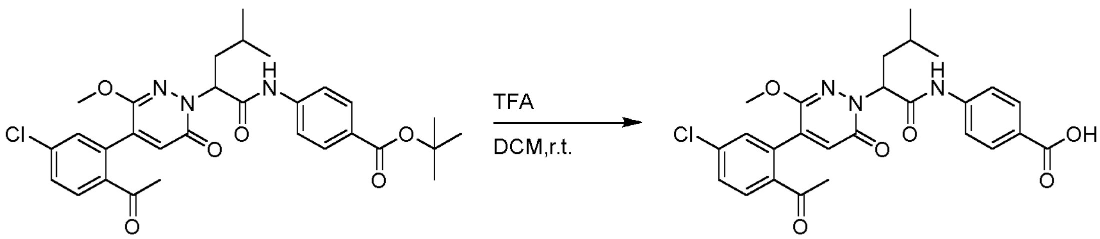


tert-Butyl 4-(2-(4-(5-chloro-2-(2-hydroxyacetyl)phenyl)-3-methoxy-6-oxopyridazin-1(6H)-yl)-4-methylpe ntanamido)benzoate (66 mg, 0.12 mmol) was dissolved in dichloromethane (2.0 mL). Subsequently, trifluoroacetic acid (1.0 ml) was added to the above solution, and the mixture was stirred at room temperature for 1 hour.

The reaction solution was concentrated under reduced pressure under an air bath. The resulted residue was purified by slurrying with dichloromethane and n-hexane to obtain 19 mg of yellow solid, tert-butyl 4-(2-(4-(2-acetyl-5-chlorophenyl)-3-methoxy-6-oxopyridazin-1(6H)-yl)-4-methylpentanamido)b enzoate (yield: 32.0%). LCMS: RT = 3.98 min, $[M-H]^-$= 510.10. $^1$H NMR (400 MHz, DMSO) δ 10.45 (s, 1H), 8.01 (d, *J* = 8.4 Hz, 1H), 7.88 (d, *J*= 8.8 Hz, 2H), 7.74 - 7.66 (m, 3H), 7.56 (d, *J* = 2.2 Hz, 1H), 6.95 (s, 1H), 5.45 (dd, *J*= 10.9, 4.3 Hz, 1H), 3.63 (s, 3H), 2.55 (s, 3H), 2.24 (dd, *J* = 17.7, 6.8 Hz, 1H), 1.78 (dd, *J* = 11.6, 6.7 Hz, 1H), 1.58 (s, 1H), 0.94 (d, *J* = 6.6 Hz, 6H).

**Example 47**

**Synthesis of (S)-4-(2-(4-(2-acetyl-5-(trifluoromethyl)phenyl)-3-methoxy-6-oxopyridazin-1(6H)-yl)-6-phe nylpropanamido)benzoic acid**

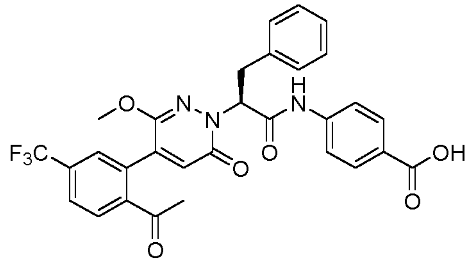

The specific synthetic route is as follows.

**Step A: synthesis of 1-(2-(4,4,5,5-tetramethyl-1,3,2-dioxaborolan-2-yl)-4-(trifluoromethyl)phenyl)ethan-1-one**

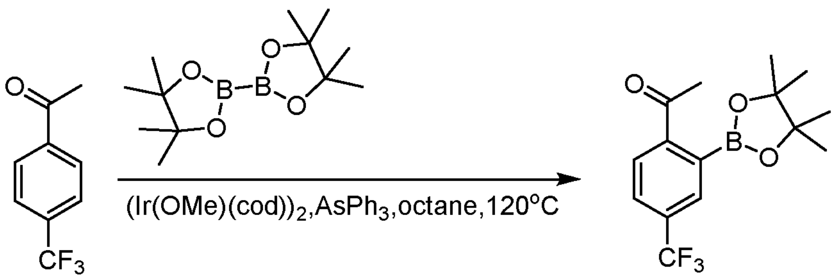


At room temperature, 1-(4-(trifluoromethyl)phenyl)ethan-1-one (8 mL, 39.6 mmol) and 4,4,4',4',5,5,5',5'-octamethyl-2,2'-bis(1,3,2-dioxaborolan) (2.0 g, 7.9 mmol) were dissolved in n-octane (50.0 mL). Subsequently, methoxy(cyclooctadiene)iridium(I) dimer (159 mg, 0.24 mmol) and triphenylarsenic (146 mg, 0.48 mmol) were added to the above solution. It was stirred at 120°C for 18 hours.

Water was added to the reaction solution to quench the reaction solution. The mixture was extracted with ethyl acetate (100 mL×3 times). The organic phases were combined, and the organic phase was first washed with saturated brine (50 ml × 3 times), then dried with anhydrous sodium sulfate, and finally concentrated under reduced pressure. The obtained residue was purified by silica gel column chromatography (eluent: ethyl acetate/petroleum ether=1/4) to obtain 1.2 g of yellow solid, 1-(2-(4,4,5,5-tetramethyl-1,3,2-dioxaborolan-2-yl)-4-(trifluoromethyl)phenyl)ethan-1-one (yield: 49.0%). LCMS: RT = 3.91 min.

**Step B: synthesis of 5-(2-acetyl-5-(trifluoromethyl)phenyl)-6-methoxy-2-(4-methoxybenzyl)pyridazin-3(2H)-one**

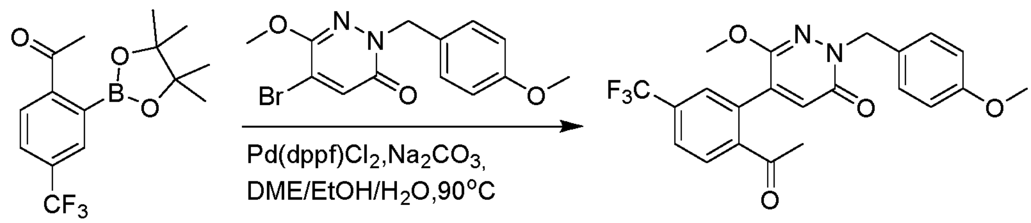


At room temperature, 5-bromo-6-methoxy-2-(4-methoxybenzyl)pyridazin-3(2H)-one (310 mg, 1.0 mmol),

1-(2-(4,4,5,5-tetramethyl-1,3,2-dioxaborolan-2-yl)-4-(trifluoromethyl)phenyl)ethan-1-one (470 mg, 1.5 mmol) and sodium carbonate (210 mg, 2.0 mmol) were added to a three-necked flask. After nitrogen replacement, ethylene glycol dimethyl ether (8 mL), ethanol (1 mL) and water (1 mL) were added. After nitrogen replacement, [1,1'-bis(diphenylphosphino)ferrocene]dichloropalladium dichloromethane complex (59 mg, 0.07 mmol) was added. After nitrogen replacement, the temperature was raised to 90°C and the reaction was continued for 2 hours.

The reaction solution was cooled to room temperature, filtered through a pad of diatomite, the filter cake was washed with ethyl acetate (30 mL×2 times). The filtrate and the washing solution were combined, and concentrated under reduced pressure. The obtained residue was added with water (50 mL). The mixture was extracted with ethyl acetate (50 mL×3 times), and the organic phases were combined. The organic phase was first washed with saturated brine (20 ml × 3 times), then dried with anhydrous sodium sulfate, and finally concentrated under reduced pressure. The obtained residue was purified by silica gel column chromatography (eluent: ethyl acetate/petroleum ether=1/2). 180 mg of a yellow solid, 5-(2-acetyl-5-(trifluoromethyl)phenyl)-6-methoxy-2-(4-methoxybenzyl)pyridazin-3(2H)-one,was obtained (yield: 42.0%). LCMS: RT = 4.04 min, $[M+H]^+$= 433.10.

**Step C: synthesis of 5-(2-acetyl-5-(trifluoromethyl)phenyl)-6-methoxypyridazin-3(2H)-one**

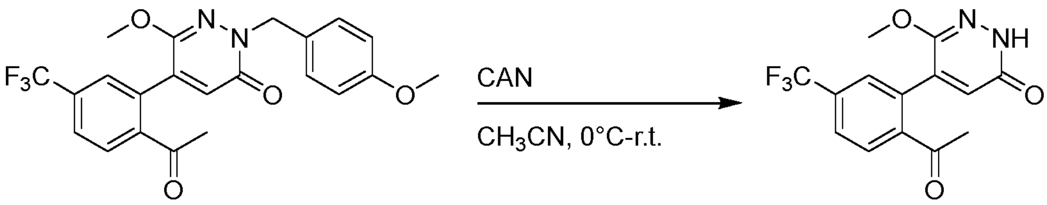


At 0°C, 5-(2-acetyl-5-(trifluoromethyl)phenyl)-6-methoxy-2-(4-methoxybenzyl)pyridazin-3(2H)-one (180 mg, 0.41 mmol) was added to acetonitrile (6 mL) and water (2 mL), followed by slow addition of ceric ammonium nitrate (2.1 g, 4.1 mmol). After the addition was completed, the reaction was carried out at room temperature for 30 minutes.

The reaction was completed, quenched by adding water, and the mixture was extracted with ethyl acetate (30ml × 3 times). The organic phases were combined, and the organic phase was first washed with saturated brine (30ml × 2 times), then dried with anhydrous sodium sulfate, and finally concentrated under reduced pressure. The obtained residue was purified by silica gel column chromatography (eluent: ethyl acetate/n-hexane=1/1). 80 mg of a yellow solid, 5-(2-acetyl-5-(trifluoromethyl)phenyl)-6-methoxypyridazin-3(2H)-one, was obtained (yield: 63.0%). LCMS: RT = 3.33 min, $[M+H]^+$= 313.07.

**Step D: synthesis of tert-butyl (S)-4-(2-(4-(2-acetyl-5-(trifluoromethyl)phenyl)-3-methoxy-6-oxopyridazin-1(6H)-yl)-3-phe nylpropanamido)benzoate**

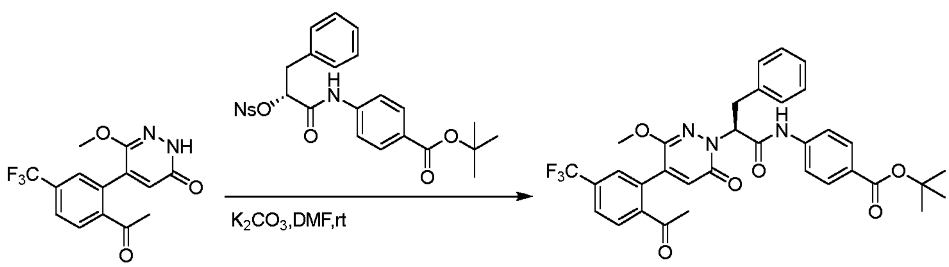


At room temperature, 5-(2-acetyl-5-(trifluoromethyl)phenyl)-6-methoxypyridazin-3(2H)-one (45 mg, 0.14 mmol), tert-butyl (R)-4-(2-(((4-nitrophenyl)sulfonyl)oxy)-3-phenylpropanamido)benzoate (114 mg, 0.22 mmol) and potassium carbonate (38 mg, 0.28 mmol) were added to N,N-dimethylformamide (2.0 mL) and reacted overnight at room temperature.

The reaction was completed, quenched by adding water, and the mixture was extracted with ethyl acetate (10ml × 3 times). The organic phases were combined, and the organic phase was first washed with saturated brine (10ml × 2 times), then dried with anhydrous sodium sulfate, and finally concentrated under reduced pressure. The

obtained residue was purified by silica gel column chromatography (eluent: ethyl acetate/n-hexane=1/1). 58 mg of a light yellow solid, tert-butyl (S)-4-(2-(4-(2-acetyl-5-(trifluoromethyl)phenyl)-3-methoxy-6-oxopyridazin-1(6H)-yl)-3-phenylp ropanamido)benzoate, was obtained (yield: 43.0%). LCMS: RT = 4.52 min, $[M-H]^-$= 634.15.

**Step E: synthesis of (S)-4-(2-(4-(2-acetyl-5-(trifluoromethyl)phenyl)-3-methoxy-6-oxopyridazin-1(6H)-yl)-3-phe nylpropanamido)benzoic acid**

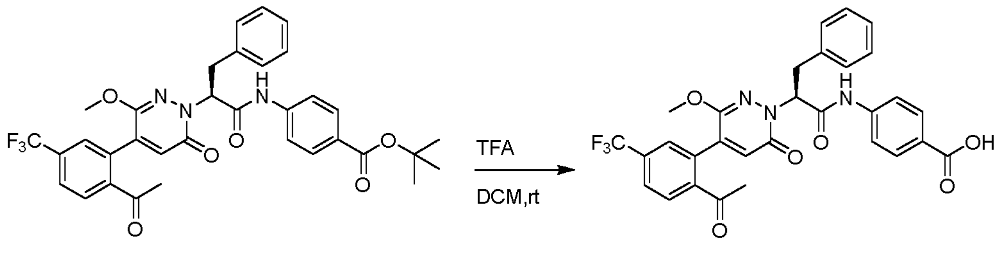


tert-Butyl (S)-4-(2-(4-(2-acetyl-5-(trifluoromethyl)phenyl)-3-methoxy-6-oxopyridazin-1(6H)-yl)-3-phenylp ropanamido)benzoate (80 mg, 0.12 mmol) was dissolved in dichloromethane (2.0 mL). Subsequently, trifluoroacetic acid (0.5 ml) was added to the above solution, and the mixture was stirred at room temperature for 1 hour.

The reaction solution was concentrated under reduced pressure in an air bath. The resulting residue was purified by slurrying with dichloromethane and n-hexane to obtain 33 mg of yellow solid (S)-4-(2-(4-(2-acetyl-5-(trifluoromethyl)phenyl)-3-methoxy-6-oxopyridazin-1(6H)-yl)-3-phenylp ropanamido)benzoic acid (yield: 45.0%). LCMS: RT = 3.99 min, $[M+H]^+$= 580.09. $^1H$ NMR (400 MHz, DMSO) $\delta$ 10.48 (s, 1H), 8.14 (d, $J$ = 8.0 Hz, 1H), 7.98 (d, $J$ = 8.0 Hz, 1H), 7.89 (d, $J$ = 8.8 Hz, 2H), 7.77 (s, 1H), 7.71 (d, $J$ = 8.8 Hz, 2H), 7.34 - 7.23 (m, 5H), 7.18 (t, $J$ = 6.8 Hz, 1H), 6.99 (s, 1H), 5.74 (dd, $J$ = 10.1, 4.9 Hz, 1H), 3.67 (s, 3H), 3.56 - 3.46 (m, 1H), 3.41 (dd, $J$ = 14.1, 5.1 Hz, 1H), 2.57 (s, 3H).

**Example 48 Compound A**

**Synthesis of (S)-4-(2-(4-(5-chloro-2-(4-chloro-1H-1,2,3-triazol-1-yl)phenyl)-6-oxopyrimidin-1(6H)-yl)-3-phenyl-propanamido)benzoic acid**

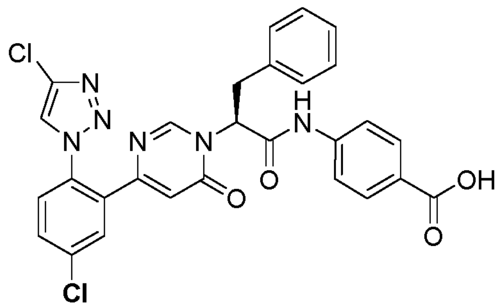

Compound A

The specific synthetic route is as follows.

**Step A: synthesis of 4-chloro-2-(tetramethyl-1,3,2-dioxaborolan-2-yl)aniline**

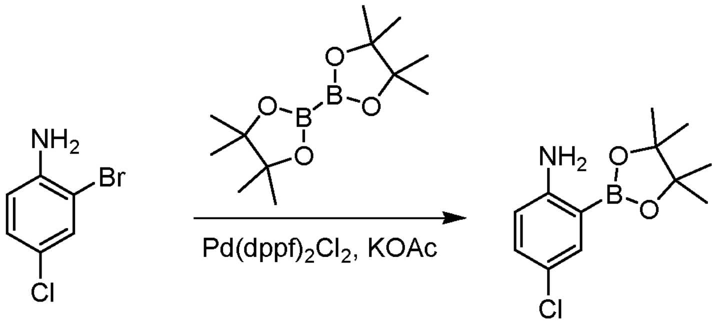


2-bromo-4-chloroaniline (3.0 g, 14.5 mmol), 4,4,5,5-tetramethyl-2-(tetramethyl-1,3,2-dioxaborolan-2-yl)-1,3,2-dioxaborolane (38 g, 150.0 mmol), potassium acetate (2.9 g, 30.0 mmol), and [1,1'-bis(diphenylphosphino)ferrocene]dichloropalladium dichloromethane complex (1.1 g, 1.5 mmol) were dissolved in dimethyl sulfoxide (75 ml). Under nitrogen protection, it was heated at 80°C for 5 hours. The reaction system was cooled to room temperature. Water was added to dissolve the salt, and the reaction solution was filtered. The remaining solids were suspended in dichloromethane and the insoluble solids were filtered out. The filtrate was concentrated and then purified by silica gel column chromatography to obtain 5.2 g of white solid **4-chloro-2-(tetramethyl-1,3,2-dioxaborolan-2-yl)aniline** (yield: 100%). LCMS: RT = 4.40 min, $[M+H]^+$ = 254.10.

**Step B: synthesis of 4-chloro-2-(6-methoxypyrimidin-4-yl)aniline**

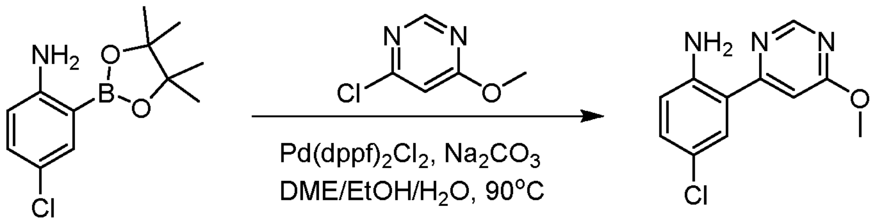


4-chloro-6-methoxypyrimidine (3.9 g, 15.4 mmol), sodium carbonate (3.2 g, 30.8 mmol), ethylene glycol dimethyl ether (16mL), ethanol (2 mL) and water (2 mL) were placed in a three-necked flask. Under nitrogen protection, [1,1'-bis(diphenylphosphino)ferrocene]dichloropalladium dichloromethane complex (1.3 g, 1.5 mmol) was added. 4-chloro-2-(tetramethyl-1,3,2-dioxaborolan-2-yl)aniline (3.31 g, 23.1 mmol) in ethylene glycol dimethyl ether (8 mL) was added, and the reaction solution was heated at 90°C for 2 hours. After the reaction being completed monitored by LCMS, it was cooled to room temperature, filtered through a pad of diatomite, and the filter cake was washed three times with ethyl acetate (30 mL). The filtrate and washing liquid were combined, washed once with water and twice with saturated ammonium chloride. The organic phase was dried with anhydrous sodium sulfate, filtered and spin-dried. The residue was purified by silica gel column chromatography to obtain 1.0 g of a yellow solid, 4-chloro-2-(6-methoxypyrimidin-4-yl)aniline(yield: 28%). LCMS: RT = 3.95 min, $[M+H]^+$ = 236.04.

**Step C: synthesis of 4-{5-chloro-2-[4-(trimethylsilyl)-1H-1,2,3-triazol-1-yl]-phenyl}-6-methoxy-pyrimidine**

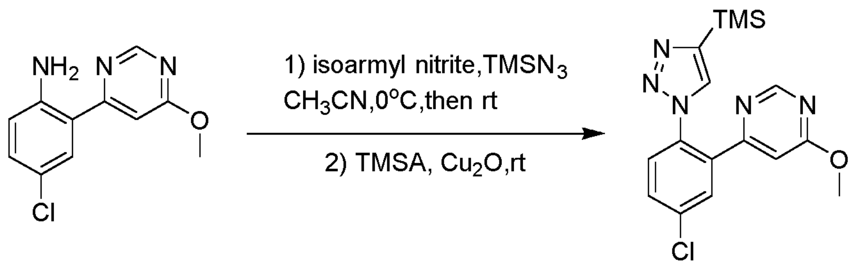

4-chloro-2-(6-methoxypyrimidin-4-yl)aniline (0.9 g, 3.8 mmol) was dissolved in acetonitrile (60 mL). 3-methylbutylnitrite (0.6 mL, 5.8 mmol) was added at 0°C, followed by adding azidotrimethylsilane (0.6 mL, 5.8 mmol) dropwise. It was observed that gas was produced. After 10 minutes, the ice bath was removed and the reaction was warmed to room temperature. After 1 hour, ethynyltrimethylsilane (1.8 mL, 11.4 mmol) and cuprous oxide (0.06 g, 0.36 mmol) were added and the reaction was stirred for 1 additional hour. Ethyl acetate and saturated aqueous ammonium chloride were added to the reaction solution to separate the layers. The organic phase was washed with brine, dried with anhydrous sodium sulfate, filtered and concentrated. Further purification was performed by silica gel column chromatography to obtain 730 mg of a yellow solid, 4-{5-chloro-2-[4-(trimethylsilyl)-1H-1,2,3-triazol-1-yl]-phenyl}-6-methoxypyrimidine (yield: 45%). LCMS: RT = 2.04 min, $[M+H]^+$ = 360.10.

**Step D: synthesis of 4-[5-chloro-2-(4-chloro-1H-1,2,3-triazol-1-yl)phenyl]-6-methoxypyrimidine**

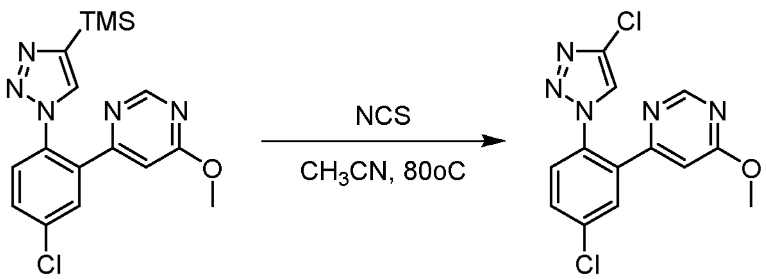


4-{5-Chloro-2-[4-(trimethylsilyl)-1H-1,2,3-triazol-1-yl]phenyl}-6-methoxypyrimidine (700 mg, 1.94 mmol) was dissolved in acetonitrile (20 mL), and N-chlorosuccinimide (0.9 g, 7.2 mmol) and silica gel (2.9 g, 50.44 mmol) were added to the solution. The reaction solution was stirred at 80°C for 1 hour. The reaction solution was then filtered to remove the silica and the collected silica was washed with ethyl acetate. The filtrate was washed with water and brine, and concentrated. The residue was further purified by silica gel column chromatography to obtain 450 mg of a yellow solid, 4-[5-chloro-2-(4-chloro-1H-1,2,3-triazol-1-yl)phenyl]-6-methoxypyrimidine (yield 72%). LCMS: RT = 2.00 min, $[M+H]^+$ = 322.05.

**Step E: synthesis of 6-[5-chloro-2-(4-chloro-1H-1,2,3-triazol-1-yl)phenyl]pyrimidin-4-ol**

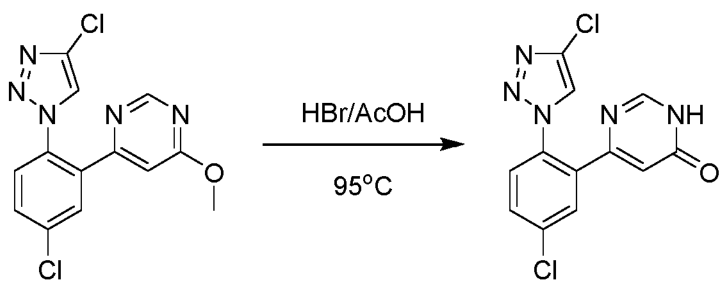


48% aqueous hydrobromic acid (1.5 mL, 13.3 mmol) was added to a solution of 4-[5-chloro-2-(4-chloro-1H-1,2,3-triazol-1-yl)phenyl]-6-methoxypyrimidine (450 mg, 1.4 mmol) in acetic acid (3 mL). The mixture was stirred at 95°C for 1 hour. The reaction solution was concentrated to dryness, and then separated with ethyl acetate and saturated sodium bicarbonate solution. The organic phase was concentrated, and the residue was purified by silica gel column chromatography to obtain 190 mg of yellow solid, 6-[5-chloro-2-(4-chloro-1H-1,2,3-triazol-1-yl)phenyl]pyrimidine-4-ol (yield: 44%). LCMS: RT = 1.74 min, $[M-H]^-$ = 305.97.

**Step F: synthesis of tert-butyl (S)-4-(2-(4-(5-chloro-2-(4-chloro-1H-1,2,3-triazol-1-yl) phenyl)-6-oxopyrimidine-1(6H)-yl)-3-phenylpropanamido)benzoate**

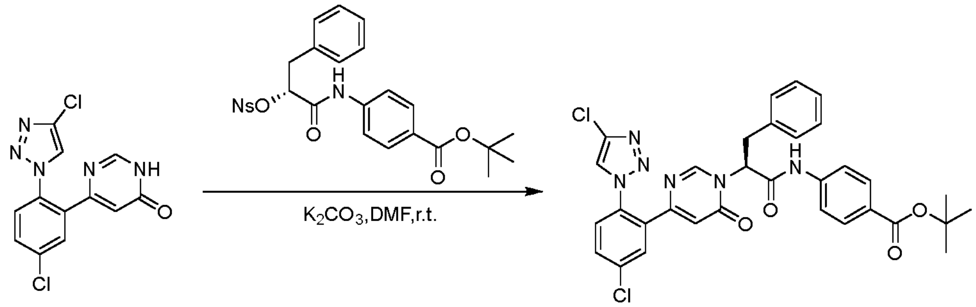


At room temperature, 6-[5-chloro-2-(4-chloro-1H-1,2,3-triazol-1-yl)phenyl]pyrimidin-4-ol (45 mg, 0.15 mmol), tert-butyl (R)-4-(2-(((4-nitrophenyl)sulfonyl)oxy)-3-phenylpropanamido)benzoate (93 mg, 0.18 mmol) and potassium carbonate (40 mg, 0.3 mmol) were added to N,N-dimethylformamide (3.0 mL), and the mixture was reacted at room temperature overnight. Water was added to the reaction solution to quench the reaction. The mixture was extracted with ethyl acetate (40 mL×3 times). The organic phases were combined, and the organic phase was first washed with saturated brine (30 ml × 2 times), then dried with anhydrous sodium sulfate, and finally concentrated under reduced pressure. The obtained residue was purified by silica gel column chromatography to obtain 150 mg of yellow liquid, tert-butyl (S)-4-(2-(4-(5-chloro-2-(4-chloro-1H-1,2,3-triazol-1-yl)phenyl)-6-oxopyrimidine-1(6H)-yl)-3-ph enylpropanamido)benzoate (yield: 59%). LCMS: RT =2.00min, $[M+H]^+$ =631.18.

**Step F: synthesis of (S)-4-(2-(4-(5-chloro-2-(4-chloro-1H-1,2,3-triazol-1-yl)phenyl)-6-oxopyrimidine-1(6H)-yl)-3 -phenylpropanamido)benzoic acid**

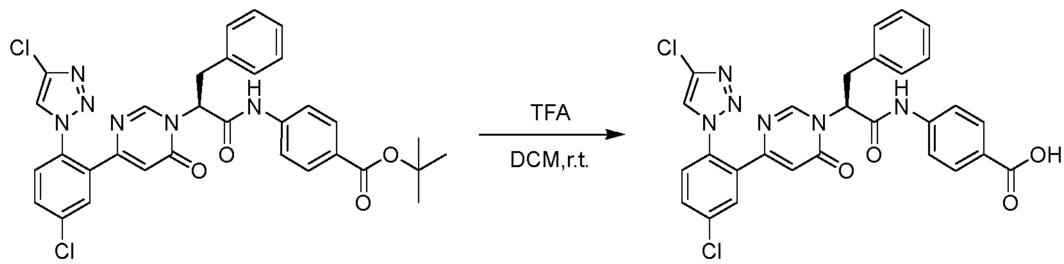


tert-Butyl (S)-4-(2-(4-(5-chloro-2-(4-chloro-1H-1,2,3-triazol-1-yl)phenyl)-6-oxopyrimidine-1(6H)-yl)-3-ph enylpropanamido)benzoate (150 mg, 0.25 mmol) was dissolved in dichloromethane (2.0 mL). Subsequently, trifluoroacetic acid (0.5 ml) was added to the above solution, and the mixture was stirred at room temperature for 1 hour. The reaction solution was concentrated under reduced pressure in an air bath. The resulting residue was purified by preparative chromatography to obtain 70 mg of white solid, (S)-4-(2-(4-(5-chloro-2-(4-chloro-1H-1,2,3-triazole-1-yl)phenyl)-6-oxopyrimidine-1(6H)-yl)-3-p henylpropanamido)benzoic acid (yield: 59%). LCMS: RT = 2.00min, $[M+H]^+$ = 573.16. $^1$H NMR (400 MHz, $CD_3OD$) $\delta$ 10.36 (s, 1H), 8.36 (s, 1H), 8.18 (s, 1H), 7.87 (dd, *J* = 12.0, 5.1 Hz, 2H), 7.72 (d, *J* = 2.3 Hz, 1H), 7.66-7.47 (m, 4H), 7.28-7.07 (m, 5H), 6.22 (d, *J* = 0.8 Hz, 1H), 5.74 (dd, *J* = 10.5, 6.2 Hz, 1H), 3.49 (dd, *J* = 14.1, 6.3 Hz, 1H), 3.34-3.24 (m, 1H).

**Example 49 Compound B**

**Synthesis of (S)-4-(2-(4-(2-acetyl-5-chlorophenyl)-5-methoxy-2-oxopyridinium-1(2H)-yl)-3-phenylpropa namido)benzoic acid**

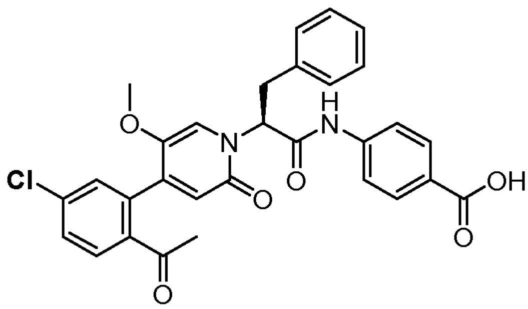

Compound B

The specific synthetic route is as follows.

**Step A: synthesis of (2,5-dimethoxypyridin-4-yl)boronic acid**

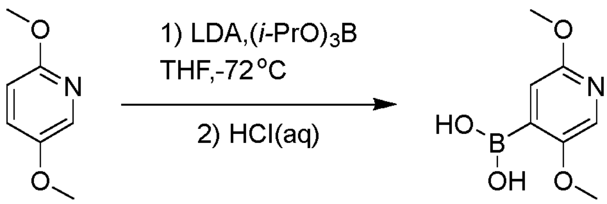


2,5-Dimethoxypyridine (10.0 g, 71.9 mmol) was dissolved in dry tetrahydrofuran (40 mL) and placed in a dry three-necked flask. Under nitrogen protection, it was stirred under a dry ice/ethanol bath for 15 minutes, and lithium diisopropylamide (20 mL, 2.0 M in THF) was slowly added dropwise to the reaction solution. The dropwise addition was completed after 30 minutes. After stirring for 3 h under a dry ice/ethanol bath, triisopropyl borate (33.0 mL, 143.8 mmol) was added to the mixture, which was then naturally warmed to room temperature and stirred at constant temperature for 18 h. After the reaction being completed monitored by LCMS, dilute hydrochloric acid was added to the reaction solution to adjust the pH to 3-4. After stirring for 15 minutes, the solvent was removed by rotary evaporation, and the residue was slurried with acetonitrile to obtain 10.6 g of white solid, (2,5-dimethoxypyridine-4-yl)boronic acid (yield: 80%). LCMS: RT = 1.73min, $[M+H]^+$ = 184.08.

**Step B: synthesis of 1-(4-chloro-2-(2,5-dimethoxypyridin-4-yl)phenyl)ethan-1-one**

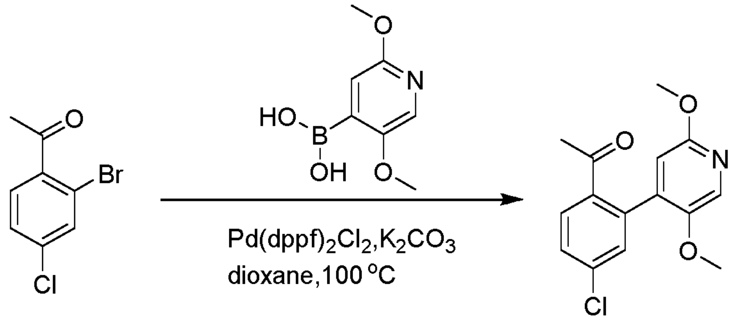


2-Bromo-4-chloroacetophenone (14.8 g, 63.6 mmol) and (2,5-dimethoxypyridin-4-yl)boronic acid (9.7 g, 53.0 mmol) were dissolved in 1,4-dioxane (40 mL), and potassium carbonate (14.6 g, 106 mol) was dissolved in water (10 mL) and placed in a dry three-necked flask. Under nitrogen protection, [1,1'-bis(diphenylphosphino)ferrocene] dichloropalladium dichloromethane complex (3.87 g, 5.3 mmol) was added to the reaction solution. Under nitrogen protection,

it was heated to 100 °C and stirred at constant temperature for 18 hours. After the reaction being completed monitored by LCMS, it was cooled to room temperature and filtered through a pad of diatomite. The filter cake was washed three times with EA (30 mL), and the filtrate and washing liquid were combined, washed once with water and twice with saturated ammonium chloride. The organic phase was dried with anhydrous sodium sulfate, filtered, and rotated to dryness. The residue was purified by silica gel column chromatography to obtain 8.2 g of yellow solid, 1-(4-chloro-2-(2,5-dimethoxypyridin-4-yl)phenyl)ethan-1-one (yield: 53%). LCMS: RT = 4.03 min, $[M+H]^+$ = 292.03.

**Step C: synthesis of 4-(2-acetyl-5-chlorophenyl)-5-methoxypyridin-2(1H)-one**

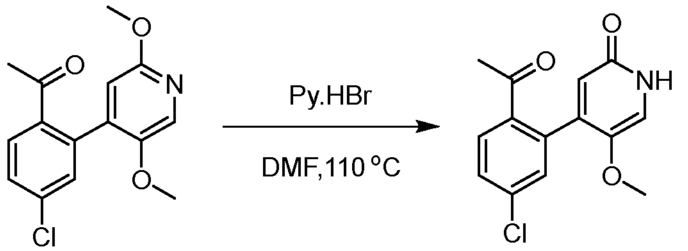


1-(4-chloro-2-(2,5-dimethoxypyridin-4-yl)phenyl)ethan-1-one (8.2 g, 28 mmol) and pyridine hydrobromide (22 g, 140 mmol) were dissolved in N,N-dimethylformamide (20 mL) and placed in a dry flask. Under nitrogen protection, it was heated to 110 °C and stirred at constant temperature for 4 h. After the reaction being completed monitored by LCMS, it was cooled to room temperature. The reaction solution was added dropwise to 100 mL of water, and 5% sodium carbonate was added to adjust the pH to 10-11. The mixture was extracted four times with DCM (40 mL×4). The organic phases were combined, and the organic phase was dried with anhydrous sodium sulfate, filtered, and spin-dried. The residue was dissolved in DCM (10 mL), then added dropwise to n-hexane (120 mL), and a large amount of solid was precipitated and filtered. The filter cake, i.e., the crude product, was collected, which was further purified by silica gel column chromatography to obtain 6.4 g of yellow solid, 4-(2-acetyl-5-chlorophenyl)-5-methoxypyridin-2(1H)-one (yield: 82%). LCMS: RT = 3.81 min, $[M-H]^-$ = 277.04.

**Step D: synthesis of tert-butyl (S)-4-(2-(4-(2-acetyl-5-chlorophenyl)-5-methoxy-2-oxopyridinium-1(2H)-yl)-3-phenylpropa namido)benzoate**

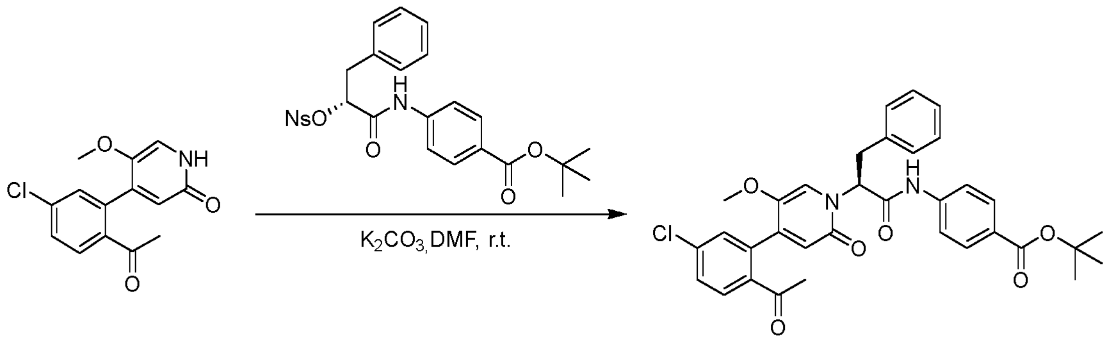


At room temperature, 4-(2-acetyl-5-chlorophenyl)-5-methoxypyridin-2(1H)-one (1.5 g, 5.4 mmol), tert-butyl (R)-4-(2-(((4-nitrophenyl)sulfonyl)oxy)-3-phenylpropanamido)benzoate (4.0 g, 7.6 mmol) and potassium carbonate (1.5 g, 10.8 mmol) were added to N,N-dimethylformamide (20.0 mL) and reacted overnight at room temperature. Water was added to the reaction solution to quench the reaction. The mixture was extracted with ethyl acetate (40 mL×3 times). The organic phases were combined, and the organic phase was first washed with saturated brine (30 ml × 2 times), then dried with anhydrous sodium sulfate, and finally concentrated under reduced pressure. The obtained residue was purified by silica gel column chromatography (eluent: ethyl acetate/n-hexane=1/2). 1.9 g of a yellow solid, tert-butyl (S)-4-(2-(4-(2-acetyl-5-chlorophenyl)-5-methoxy-2-oxopyridinium-1(2H)-yl)-3-phenylpropanam ido)benzoate, was obtained (yield: 59%). LCMS: RT = 4.42 min, [M+H]+ = 601.18.

**Step E: synthesis of (S)-4-(2-(4-(2-acetyl-5-chlorophenyl)-5-methoxy-2-oxopyridinium-1(2H)-yl)-3-phenylpropa namido)benzoic acid**

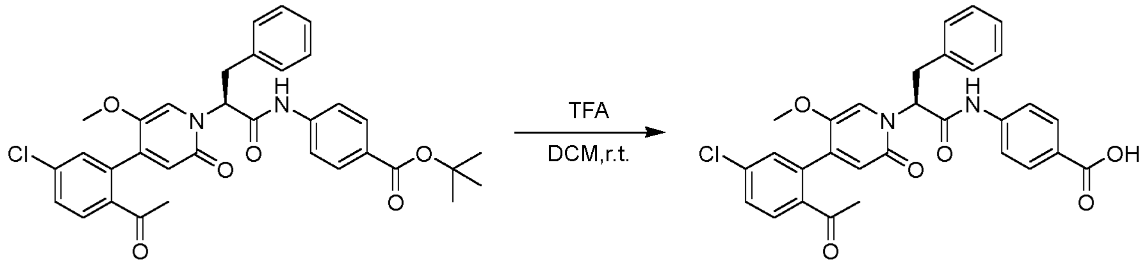


(tert-butyl S)-4-(2-(4-(2-acetyl-5-chlorophenyl)-3-ethoxy-6-oxopyridazin-1(6H)-yl)-3-phenylpropanamido) benzoate (1.9 g, 3.2 mmol) was dissolved in dichloromethane (12.0 mL). Subsequently, trifluoroacetic acid (0.5 ml) was added to the above solution, and the mixture was stirred at room temperature for 1 hour. The reaction solution was concentrated under reduced pressure in an air bath. The obtained residue was slurried with methanol and purified to obtain 1.0 g of a yellow solid, (S)-4-(2-(4-(2-acetyl-5-chlorophenyl)-5-methoxy-2-oxopyridinium-1(2H)-yl)-3-phenylpropanam ido)benzoic acid (yield: 59%). LCMS: RT = 3.88 min, $[M-H]^-$ =543.06. $^1H$ NMR (400 MHz, DMSO) $\delta$ 10.82 (s, 1H), 7.92 (d, $J$ = 8.8 Hz, 2H), 7.82 (d, $J$ = 8.3 Hz, 1H), 7.76 (d, $J$ = 8.8 Hz, 2H), 7.61 (dd, $J$ = 8.4, 2.3 Hz, 2H), 7.42 (s, 1H), 7.38 (s, 1H), 7.33-7.23 (m, 4H), 7.22-7.14 (m, 1H), 6.30 (s, 1H), 6.02 (dd, $J$ = 9.5, 6.6 Hz, 1H), 3.53 (s, 3H), 3.49-3.44 (m, 2H), 2.36 (s, 3H).

**Example 50: Detection of the biological activity of the compounds of the present invention on the inhibition of human coagulation factor XIa by absorptiometry**

1. Experimental materials

Enzyme: Human Factor XIa (ENZYME RESEARCH, Cat. No. HFXIa 1111a)
Substrate: S-2366™: (CHROMOGENIX, Cat. No. 82109039)
Buffer: 145 mM NaCl, 5 mM KCl, 1 mg/mL PEG 8000, 30 mM HEPES, pH 7.4

2. Experimental Procedure

10mM test compound dissolved in 100% DMSO was diluted with 100% DMSO to 1000, 200, 40, 8, 1.6, 0.32, 0.064, 0.0128, 0.00256, 0.00128 μM; 98 μL of FXIa enzyme solution (77.7ng/mL) was added to each well of a 96-well plate, and 98 μL of buffer was added to the blank wells. 2 μL of compounds of different concentrations were added, and the blank and control wells were added with DMSO instead. They were mixed with a shaker, and incubated at 37°C for 20 minutes.

Finally, 100 μL of 800 μM substrate was added to each well, and its absorbance was measured at 405 nm.

3. Data processing

Curve fitting was performed with GraphPad Prism software, and $IC_{50}$ values were calculated, as shown in Table 1.

Table 1: IC50 of the compounds of the present invention inhibiting human FXIa

| EXAMPLE | hFXIa $IC_{50}$ (nM) |
|---|---|
| 2 | 45.6 |
| 3 | 51.21 |
| 4 | 32.59 |

(continued)

| EXAMPLE | hFXIa $IC_{50}$ (nM) |
|---|---|
| 5 | 8.89 |
| 6 | 21.15 |
| 7 | 24.25 |
| 8 | 30.3 |
| 14 | 59.32 |
| 15 | 1.52 |
| 16 | 13.2 |
| 19 | 7.61 |
| 20 | 20.6 |
| 21 | 21.85 |
| 22 | 16.15 |
| 23 | 22.5 |
| 24 | 23 |
| 25 | 18.55 |
| 27 | 79.49 |
| 28 | 218 |
| 29 | 63.79 |
| 32 | 1.65 |
| 33 | 19.94 |
| 34 | 139.55 |
| 35 | 16.54 |
| 36 | 11.59 |
| 39 | 16.36 |
| 40 | 13.86 |
| 42 | 1400 |

Conclusion: The compounds of the present invention have obvious inhibitory activity on human FXIa.

Example 51: **Determination of the anticoagulant effect of the compounds of the present invention on human plasma *in vitro***

1. Experimental materials

Plasma: Human blood was collected in a vacuum blood collection tube containing 3.2% sodium citrate (volume ratio 1:9), centrifuged at 3000 rpm for 10 min at room temperature, and the plasma was collected, divided into EP tubes, and stored at -80 °C.
Reagents: APTT assay kit (Activated partial thromboplastin time assay kit, mindray), calcium chloride solution.
Instrument: coagulation meter (mindray, C2000-A)

2. Experimental method

The frozen human plasma in aliquots was thawed at room temperature and mixed well. 10 mM test compound dissolved in 100% DMSO was diluted with 100% DMSO to 1500, 750, 375, 187.5, 93.75, 46.88, 23.44, 11.72 μM. 98 μL of human plasma was added to a 1.5 mL EP tube, then 2 μL of compounds of different concentrations were added, and 2 μL of 100% DMSO was added for the blank group. They were incubated under a water bath at 37°C for 10 min, and the samples were placed in the corresponding position of the coagulation analyzer to conduct APTT determination of the compounds.

3. Data processing

Curve fitting was performed with GraphPad Prism software, and FC1.5× and FC2× values were calculated respectively, i.e., the concentrations of the compounds corresponding to the APTT of 1.5× and 2× blank control group. The results are shown in Table 2.

Table 2: Anticoagulant effect of the compounds of the present invention on human plasma *in vitro*

| EXAMPLE | aPTT EC1.5×(μM) | aPTT EC2×(μM) |
|---|---|---|
| 1 | 9.31 | >30 |
| 2 | 3.455 | >15 |
| 4 | 2.171 | 11.938 |
| 5 | 1.18 | 4.414 |
| 6 | 0.771 | 2.892 |
| 7 | 2.073 | 12.58 |
| 8 | 2.67 | >15 |
| 15 | 0.531 | 1.749 |
| 16 | 1.005 | 4.768 |
| 19 | 0.641 | 2.817 |
| 20 | 2.418 | 28.32 |
| 21 | 0.777 | 3.848 |
| 22 | 1.346 | 9.382 |
| 23 | 4.236 | >15 |
| 24 | 0.769 | 2.785 |
| 25 | 1.782 | 9.452 |
| 32 | 0.483 | 1.319 |
| 33 | 1.527 | 7.691 |
| 35 | 1.271 | 5.987 |
| 36 | 1.348 | 6.562 |
| 39 | 1.638 | 6.859 |
| 40 | 2.636 | 14.94 |

Conclusion: It can be seen from Table 2 that the compounds of the present invention have obvious anticoagulant effects on human plasma.

Example 52: **Investigation of the selectivity of the compounds of the present invention to coagulation factors**

1. Experimental materials

Enzyme: hFXa: Human Factor Xa: 71nkat. hFIIa: HT5146L. hFVIIa: Human Factor VIIa: hFVIIa 4591L. kallikrein: LOT180223.
Substrate: S-2222™: CHROMOGENIX, NO864682. S-2238™: CHROMOGENIX, NO770996. S-2288™: CHROMOGENIX, NO378902. ADG302.
Buffer:

hFXa buffer: 100 mM NaCl, 5 mM $CaCl_2$, 33% ethylene glycol, 50 mM Tris (pH 7.5).
hFIIa buffer: 0.145 M NaCl, 0.005 M KCl, 1 mg/ml PEG-8000, 0.030 M HEPES (pH 7.4).
hFVIIa buffer: 0.145 M NaCl, 0.005 M KCl, 1 mg/ml PEG-8000, 0.030 M HEPES (pH 7.4).
kallikrein buffer: 50 mM Tris, 50 mM Mimidazole and 150 mM NaCl (pH 8.2).

2. Experimental Procedure

10 mM test compound dissolved in 100% DMSO was diluted with 100% DMSO to 1000, 200, 40, 8, 1.6 μM. 98 μL of enzyme solution was added to each well of a 96-well plate, while 98 μL of buffer was added to the blank wells. 2 μL of compounds of different concentrations were added, while the blank and control wells were added with DMSO instead. They were mixed with a shaker, and incubated at 37°C for 20 min.

The concentrations of hFXa and S-2222™ were FXa (1:28) and 800 μmol/ L, respectively. The concentrations of hFIIa and S-2238™ were hFIIa (0.06 U/ml) and 500 μmol/L, respectively. The concentrations of hFVIIa and S-2288™ were hFVIIa (80 nM) and 1600 μmol/L, respectively. The concentrations of kallikrein and substrate were kallikrein (20 nM) and 1600 μmol/L, respectively.

Finally, 100 μL substrate was added to each well, and its absorbance was measured at 405 nm.

3. Data processing

Curve fitting was performed with GraphPad Prism software, and $IC_{50}$ values were calculated, as shown in Table 3.

Table 3: Investigation of the selectivity of the compounds of the present invention to coagulation factors

| EXAMPLE | hFXa IC50(μM) | hFIIa IC50(μM) | hFVIIa IC50(μM) | hKallikrein IC50(nM) |
|---|---|---|---|---|
| 19 | >100 | >100 | >100 | 523.9±60.2 |
| 32 | >100 | >100 | >100 | 102.9±14.9 |

Conclusion: The compounds of the present invention have good selectivity to other coagulation factors.

Example 53: **Investigation of the pharmacokinetic characteristics of the compounds of the present invention**

1. Experimental materials

SD rats: male, 180-250 g, purchased from Guangdong Medical Laboratory Animal Center. Cynomolgus monkey: male, 4-6 kg, purchased from Guangzhou Chunsheng Biological Research Institute Co., Ltd. Beagle dog: male, 8-12 kg, developed in Kanglong Chemical (Ningbo) New Drug Technology Co., Ltd.

Reagents: DMSO (dimethyl sulfoxide), PEG-400 (polyethylene glycol 400), normal brine, heparin, acetonitrile, formic acid, and propranolol (internal standard) are commercially available.

Instrumentation: Thermo Fisher Scientific LC-MS (U300 UPLC, TSQ QUANTUMN ULTRA triple quadrupole mass spectrometer).

2. Experimental method

The compound was weighed and dissolved in DMSO-PEG-400-physiological brine (5:60:35, v/v/v) system.

After the rats/monkeys were administered intravenously or by gavage, 200 μL of venous blood was collected at 5 min (not collected when administered by gavage), 15 min, 30 min, 1 h, 2 h, 4 h, 6 h, 8 h, and 24 h in heparinized EP tubes, centrifuged at 12000rpm for 2 min, and the plasma was frozen at -80°C for testing. A certain amount of the test substance was precisely weighed and dissolved in DMSO to 1 mg/mL, which was used as a stock solution. An appropriate amount of compound stock solution was accurately pipetted and diluted with acetonitrile to prepare a standard series of solutions. 20 μL of each of the above standard series solutions were accurately pipetted, added with 180 μL of blank plasma, vortexed and mixed to prepare plasma samples equivalent to plasma concentrations of 1, 3, 10, 30, 100, 300, 1000, 3000 and 5000 ng/mL. Two-sample analysis was performed for each concentration to establish a standard curve. 20 μL of plasma was taken out, added with 200 μL of internal standard propranolol (5 ng/mL) in acetonitrile, vortexed and mixed. It was centrifuged at 4000 rpm for 5 min, and the supernatant was collected for LC-MS analysis. LC-MS detection conditions were as follows:

Chromatographic column: Thermo Scientific HYPERSIL GOLD C-18 UPLC column, 100*2.1 mm, 1.9 μm.

Mobile phase: water (0.1% formic acid)-acetonitrile for gradient elution according to the table below.

| Time (min) | Water (with 0.1% formic acid) | Acetonitrile |
|---|---|---|
| 0 | 90% | 10% |
| 0.6 | 90% | 10% |
| 1 | 10% | 90% |
| 2.6 | 10% | 90% |
| 2.61 | 90% | 10% |
| 4 | 90% | 10% |

3. Data processing

After blood drug concentrations being detected by LC-MS, WinNonlin 6.1 software was used to calculate pharmacokinetic parameters by non-compartmental model method. The results are shown in Tables 3 and 4.

Table 4: Rat pharmacokinetic parameters of the compounds of the present invention

| EXAMPLE | Route of administration | Tmax (h) | Cmax (ng/mL) | AUC (h*ng/mL) | $T_{1/2}$ (h) | CL (mL/min/kg) | Vss (L/kg) | F (%) |
|---|---|---|---|---|---|---|---|---|
| 6 | iv | 0.083 | 1370 | 604 | 0.162 | 27.6 | 0.432 | / |
| | ig | 1 | 30.5 | / | / | / | / | 1.7 |
| 15 | iv | 0.083 | 1840 | 599 | 0.466 | 29.1 | 0.471 | / |
| | ig | 0.5 | 3 | / | / | / | / | 0.1 |
| 16 | iv | 0.083 | 2530 | 1090 | 0.676 | 16.6 | 0.444 | / |
| | ig | 0.375 | 160 | 481 | 1.35 | / | / | 8.1 |
| 19 | iv | 0.083 | 2500 | 1020 | 0.181 | 16.4 | 0.269 | / |
| | ig | 1.25 | 768 | 2510 | 2.52 | / | / | 24.6 |
| 21 | iv | 0.0998 | 1600 | 743 | 0.492 | 25.3 | 0.277 | / |
| | ig | 1.5 | 166 | 608 | 2.32 | / | / | 14.9 |
| 24 | iv | 0.083 | 1270 | 457 | 2.8 | 36.5 | 1.14 | / |
| | ig | 0.5 | 43.7 | 93.5 | 1.74 | / | / | 3.9 |
| 25 | iv | 0.083 | 1780 | 889 | 0.8 | 22.6 | 0.915 | / |
| | ig | 0.625 | 125 | 461 | 2.64 | / | / | 8.6 |
| 32 | iv | 0.083 | 2200 | 922 | 1.65 | 23.5 | 0.741 | / |
| | ig | 0.333 | 242 | 788 | 3.77 | / | / | 5.4 |

(continued)

| EXAMPLE | Route of administration | Tmax (h) | Cmax (ng/mL) | AUC (h*ng/ mL) | $T_{1/2}$ (h) | CL (mL/min/kg) | Vss (L/kg) | F (%) |
|---|---|---|---|---|---|---|---|---|
| Compound A | iv | 0.083 | 4600 | 1410 | 0.589 | 11.9 | 0.124 | / |
| | ig | 0.5 | 180 | 576 | 1.26 | / | / | 8.2 |
| Example 143 (CN201680058331) | iv | 0.083 | 4900 | 2780 | 2.4 | 6.0 | 0.341 | / |
| | ig | 2.0 | 18.1 | 105 | 7.88 | / | / | 0.8 |

Table 5: Cynomolgus monkey pharmacokinetic parameters of the compounds of the present invention

| EXAMPLE | Route of administration | Tmax (h) | Cmax (ng/mL) | AUC (h*ng/mL) | $T_{1/2}$ (h) | CL (mL/min/kg) | Vss (L/kg) | F (%) |
|---|---|---|---|---|---|---|---|---|
| 19 | iv | 0.083 | 2690 | 1430 | 2.83 | 12 | 0.65 | / |
| | ig | 2.5 | 198 | 2480 | 7.07 | / | / | 17.3 |
| 21 | iv | 0.083 | 1010 | 455 | 1.27 | 18.3 | 0.699 | / |
| | ig | 1.5 | 27.2 | 357 | 11.4 | / | / | 6.3 |
| Compound B | iv | 0.083 | 8759 | 4220 | 1.2 | 4.1 | 0.2 | / |
| | ig | 2.00 | 108 | 1486 | 8.0 | / | / | 4.1 |

Table 6: Beagle dog pharmacokinetic parameters of the compounds of the present invention

| EXAMPLE | Route of administration | Tmax (h) | Cmax (ng/mL) | AUC (h*ng/mL) | $T_{1/2}$ (h) | CL (mL/min/kg) | Vss (L/kg) | F (%) |
|---|---|---|---|---|---|---|---|---|
| 19 | iv | 0.083 | 2579.7 | 1405.1 | 4.2 | 11.8 | 0.8 | / |
| | ig | 1.25 | 2320 | 9232.2 | 3.6 | / | / | 65.5 |
| 32 | iv | 0.083 | 2187 | 756 | 7.77 | 22.7 | 2.64 | / |
| | ig | 0.25 | 1597 | 3769 | 4.51 | / | / | 49.6 |

Conclusion: The compounds of the present invention have certain orally absorption in rats and monkeys, have good oral absorption in dogs, and the clearance rate *in vivo* is moderately slow. Most of the compounds have a long oral half-life and have good pharmacokinetic characteristics.

Example 54: **Investigation of the caco-2 data of the compounds of the present invention**

Experimental materials:

Medium: DMEM (Corning), FBS (Corning), double antibody (Solarbio), 96-well HTS transwell plate (Corning), Caco-2 cells.

Experimental method: Caco-2 cells were cultured on 96-well HTS transwell plate for 14-18 days, and the TEER value of each well was detected to ensure that the cells in each well formed a complete monolayer. Drug was added and it was incubated for 2h to detect the drug concentrations of A-B and B-A.

Data processing: PappA-B and PappB-A values were calculated: Papp = (VA×[drug]acceptor)/(Area×Time×[drug]initial,donor); Efflux Ratio was calculated: Efflux Ratio=Papp(B-A)/Papp(A-B).

Table 7: Caco-2 data of the compounds of the present invention

| EXAMPLE | Papp (A-B) ($10^{-6}$, cm/s) | Papp (B-A) ($10^{-6}$, cm/s) | Efflux Ratio |
|---|---|---|---|
| 19 | 1.54 | 25.15 | 16.31 |
| 32 | 1.2 | 21.93 | 18.34 |
| Compound B | 1.55 | 13.56 | 8.75 |

Conclusion: The membrane permeability of the compound of the present invention is good.

Example 55: **Investigation of CYP enzyme inhibition by the compounds of the present invention**

Experimental materials:

Liver microsomes (150-donor, Corning, Cat. 452117; Lot. 38292), NADPH.

Experimental method:

A 0.2 mg/mL microsome system was prepared, each test substance and substrate were added, and the final concentration of the test substance was 50 μM. After pre-incubating for 8 min, 10 mM NADPH was added to start the reaction, and the final concentration of NADPH was 1 mM. After a period of incubation, an internal standard such as methanol was added to stop the reaction. The amount of substrate metabolites generated in each reaction well was detected.
Data processing: Taking the metabolite generation in the blank well as 100%, the reduction of metabolite generation in each well of the test substance was calculated, and the inhibition rate was calculated.

Table 8: CYP enzyme inhibition data of the compounds of the present invention

| EXAMPLE | CYP Inhibition IC50 (μM) | | | | | | | |
|---|---|---|---|---|---|---|---|---|
| | CYP1A2 | CYP2B6 | CYP2C8 | CYP2C9 | CYP2C19 | CYP2D6 | CYP3A4 | CYP3A5 |
| 19 | >50 | >50 | ~50 | >50 | >50 | >50 | >50 | >50 |
| 32 | >50 | >50 | ~30 | ~50 | >50 | >50 | >50 | >50 |

Conclusion: The compounds of the present invention have no inhibition on major CYP enzymes, and the risk of DDI is small.

Example 56: **Investigation on hERG of the compound of the present invention**

Experimental materials:

HEK293-hERG stably transfected cell line (invitrogen). DMEM medium (Gibco), HEPES (invitrogen), Blasticidin (invitrogen)

Experimental method:

HEK293-hERG stably transfected cells were used for experiments when they were cultured to a degree of polymerization of 40%-80%. First, a blank solvent was applied to the cells to establish a baseline. Compounds were tested after the hERG current was found to be stable for 5 minutes. In the presence of test compounds, hERG currents were recorded for approximately 5 minutes to reach a steady state, and then 5 sweep frequencies were captured. To ensure good performance of cultured cells and manipulations, the same batch of cells was also tested using the positive control dofetilide.
Data processing:

$$\text{Peak current inhibition} = (1\text{-Peak tail current compound/Peak tail current vehicle})*100$$

Table 9: hERG experimental data of the compound of the present invention

| EXAMPLE | hERG IC50 [μM] | Comment |
|---|---|---|
| 19 | > 10 | 1.17% inhibition at 10 μM |
| 32 | > 10 | 2.80% inhibition at 10 μM |

Conclusion: The compounds of the present invention have higher IC50 for hERG current and better cardiac safety.

The above-mentioned embodiments are preferred embodiments of the present invention. However, the embodiments of the present invention are not limited by the above-mentioned examples, and any other changes, modifications, substitutions, combinations, and simplifications that do not deviate from the spirit and principle of the present invention should be equivalent replacement methods, and are all included within the protection scope of the present invention.

## Claims

1. A compound of formula (I), or a stereoisomer, a tautomer, a pharmaceutically acceptable salt thereof,

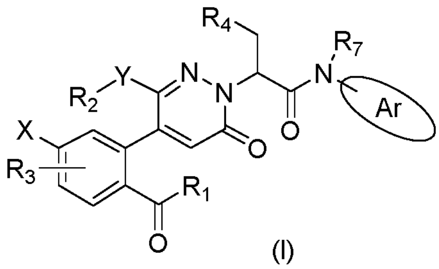

(I)

wherein:

$R_1$ is selected from the group consisting of alkyl, haloalkyl, alkoxy, alkoxyalkyl, and hydroxyalkyl;
X is selected from the group consisting of halogen, alkoxy, and haloalkyl;
$R_3$ is hydrogen atom or halogen;
Y is selected from the group consisting of oxygen atom, nitrogen atom, and a bond;
$R_2$ is selected from the group consisting of hydrogen atom, benzene ring, alkyl, alkoxy, alkoxyalkyl, hydroxyalkyl, haloalkyl, heterocycloalkyl, and cycloalkylmethylene;
$R_4$ is selected from the group consisting of alkyl, benzyl, and aryl or heteroaryl substituted by one $R_6$, wherein $R_6$ is selected from the group consisting of alkyl, halogen, cyano, substituted or unsubstituted amido, substituted or unsubstituted oxopiperazinyl, and substituted or unsubstituted 2-piperidinonyl, wherein substituted amido, substituted oxopiperazinyl, and substituted 2-piperidinonyl is substituted by a substituent selected from the group consisting of alkyl, cycloalkyl, and alkoxyalkyl;
Ar is selected from the group consisting of benzene ring and indole substituted with one or two $R_5$, indazole, quinoxaline, benzimidazole, indolin-2-one, isoquinolin-1(2*H*)-one, and 3,4-dihydroquinolin-2(1*H*)-one, wherein $R_5$ is selected from the group consisting of hydrogen, halogen, alkoxy, hydroxyl, carboxyl, sulfonic acid group, sulfonamido, and amide group; and
$R_7$ is hydrogen or alkyl.

2. The compound, or the stereoisomer, the tautomer, the pharmaceutically acceptable salt thereof according to claim 1, wherein the alkyl is $C_{1-4}$ alkyl, the $C_{1-4}$ alkyl is selected from the group consisting of methyl, ethyl, propyl, isopropyl, n-butyl, isopropyl butyl, sec-butyl, and tert-butyl.

3. The compound, or the stereoisomer, the tautomer, the pharmaceutically acceptable salt thereof according to claim 1, wherein the alkoxy group is $C_{1-4}$ alkoxy, the $C_{1-4}$ alkoxy is selected from the group consisting of methoxy, ethoxy, propoxy, isopropoxy, n-butoxy, isobutoxy, sec-butoxy, and tert-butoxy; the alkoxyalkyl is $C_{1-4}$ alkoxy $C_{1-4}$ alkyl, the $C_{1-4}$ alkoxy $C_{1-4}$ alkyl is selected from the group consisting of methoxymethyl, methoxyethyl, methoxypropyl, methoxybutyl, ethoxymethyl, ethoxyethyl, ethoxypropyl, ethoxybutyl, propoxymethyl, propoxyethyl, propoxypropyl, propoxybutyl, butoxymethyl, butoxyethyl, butoxypropyl, and butoxybutyl.

**4.** The compound, or the stereoisomer, the tautomer, the pharmaceutically acceptable salt thereof according to claim 1, wherein the halogen is selected from the group consisting of fluorine, chlorine, bromine and iodine, the haloalkyl is an alkyl of which one or more hydrogen atoms are substituted by halogen, the hydroxyalkyl is an alkyl of which one or more hydrogen atoms are substituted by hydroxyl, the heterocycloalkyl is an alkyl of which one or more hydrogen atoms are substituted by heterocyclic ring, and the cycloalkylmethylene is a methyl of which one or more hydrogen atoms are substituted by cycloalkyl.

**5.** The compound, or the stereoisomer, the tautomer, the pharmaceutically acceptable salt thereof according to claim 1, wherein the heterocycloalkyl is 4- to 10-membered heterocycloalkyl, the 4- to 10-membered heterocycloalkyl is selected from the group consisting of

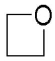, 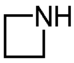, 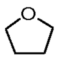, 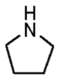, 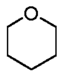, 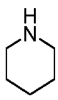, 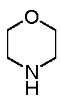, 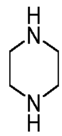, 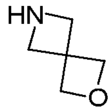, 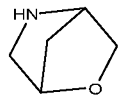

; the aryl is phenyl; the heteroaryl is 5- to 12-membered heteroaryl, wherein the 5- to 12-membered heteroaryl is selected from the group consisting of

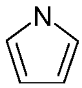, 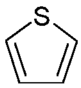, 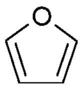, 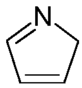, 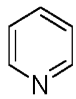, 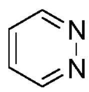,

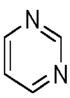, 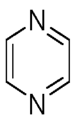, 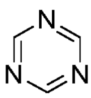, 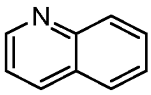, 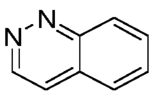, 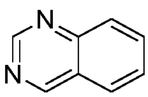, 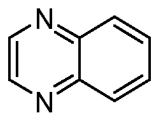, 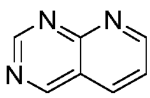,

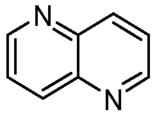 and 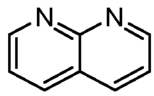.

**6.** The compound, or the stereoisomer, the tautomer, the pharmaceutically acceptable salt thereof according to claim 1, wherein the cycloalkyl is $C_{3-6}$ cycloalkyl, the $C_{3-6}$ cycloalkyl is selected from the group consisting of cyclopropyl, cyclobutyl, cyclopentyl, and cyclohexyl.

**7.** The compound, or the stereoisomer, the tautomer, the pharmaceutically acceptable salt thereof according to claim 1, wherein

$R_1$ is selected from the group consisting of methyl, ethyl, hydroxymethyl, difluoromethyl, fluoromethyl, and methoxymethyl;
X is selected from the group consisting of chlorine, fluorine, and trifluoromethyl;
$R_3$ is hydrogen;
Y is a bond and $R_2$ is hydrogen or

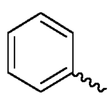,

or Y is oxygen and $R_2$ is selected from the group consisting of hydrogen, methyl, ethyl, phenyl, hydroxyethyl,

cyclopropylmethyl, methoxyethyl, isopropyl, difluoromethyl,

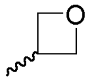

and $CF_3CH_2$-;

$R_4$ is selected from the group consisting of phenyl, 4-fluorophenyl, 4-bromophenyl, 3-methylphenyl, 4-methylphenyl, benzyl, isopropyl,

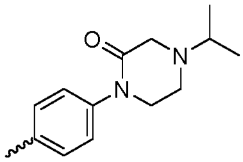
,

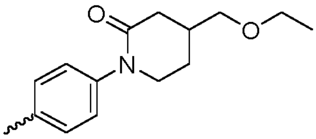
,
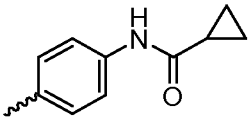
,
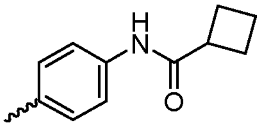
, and
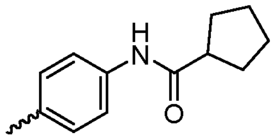
;

Ar is selected from the group consisting of

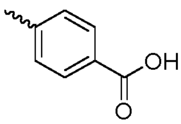
,
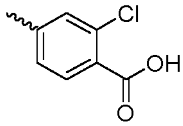
,
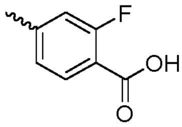
,

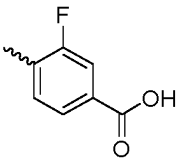
,
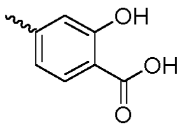
,
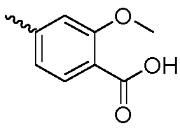
,
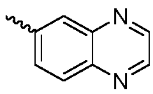
,
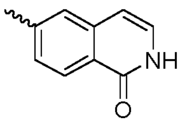
,
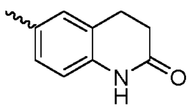
,

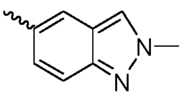
,
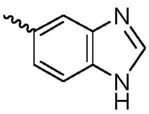
,
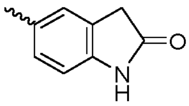
,
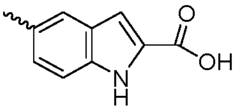
,
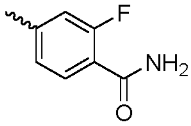
,
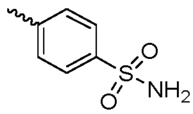

and

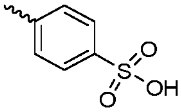
;

$R_7$ is hydrogen or methyl.

**8.** The compound, or the stereoisomer, the tautomer, the pharmaceutically acceptable salt thereof according to claim 1, wherein the compound or the pharmaceutically acceptable salt thereof is selected from following compounds:

| | structural formula | | structural formula |
|---|---|---|---|
| 1 | 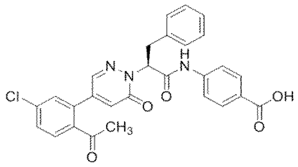 | 24 | 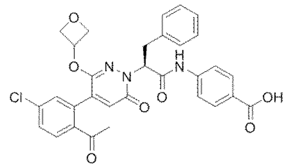 |
| 2 | 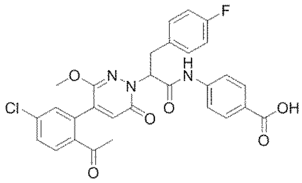 | 25 | 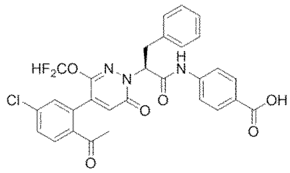 |
| 3 | 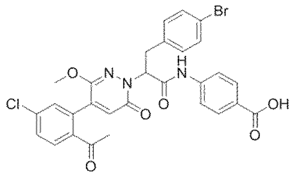 | 26 | 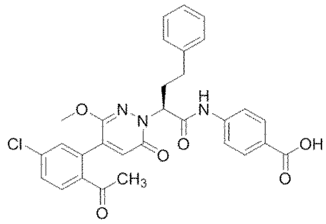 |
| 4 | 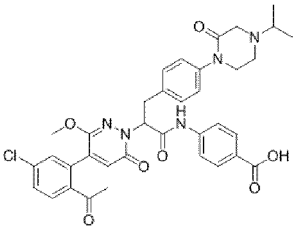 | 27 | 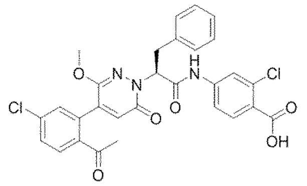 |
| 5 | 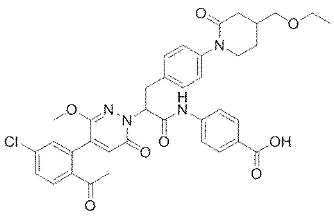 | 28 | 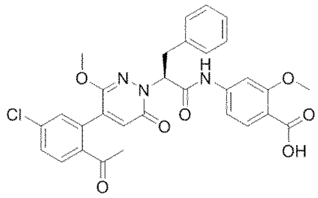 |
| 6 | 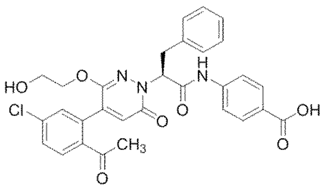 | 29 | 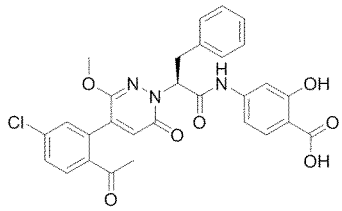 |
| 7 | 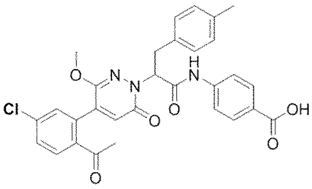 | 30 | 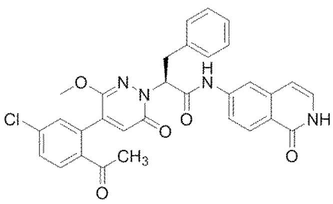 |

(continued)

| | structural formula | | structural formula |
|---|---|---|---|
| 8 | 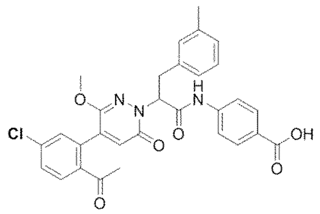 | 31 | 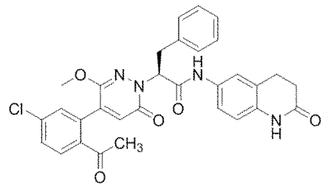 |
| 9 | 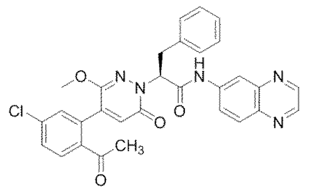 | 32 | 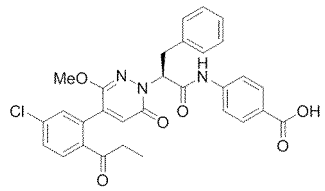 |
| 10 | 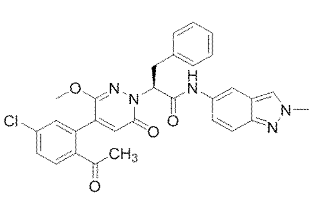 | 33 | 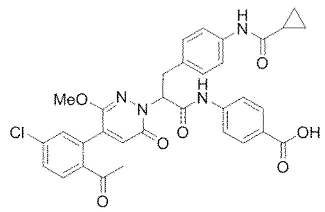 |
| 11 | 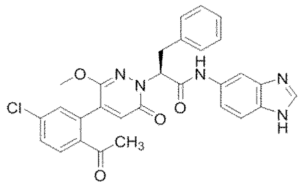 | 34 | 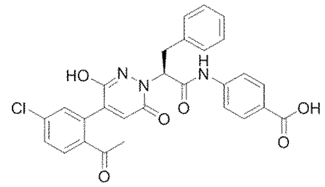 |
| 12 | 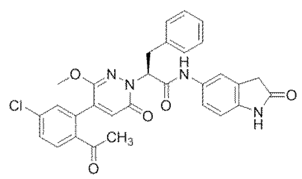 | 35 | 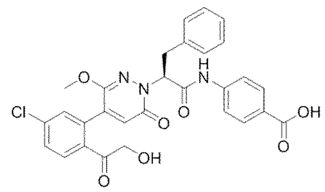 |
| 13 | 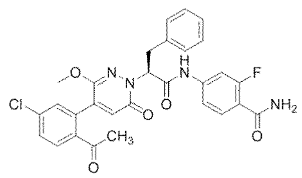 | 36 | 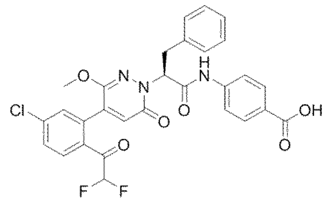 |
| 14 | 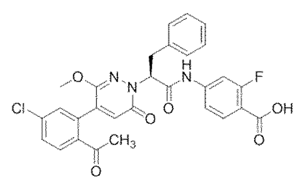 | 37 | 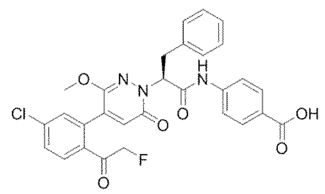 |

(continued)

| | structural formula | | structural formula |
|---|---|---|---|
| 15 | 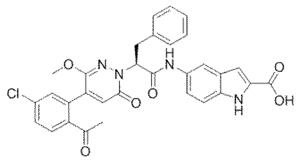 | 38 | 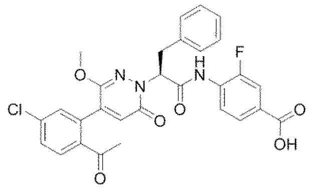 |
| 16 | 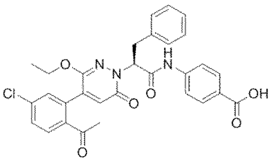 | 39 | 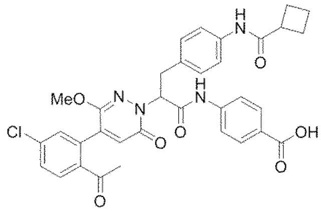 |
| 17 | 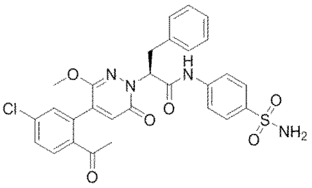 | 40 | 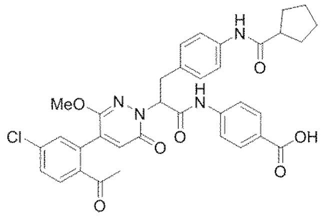 |
| 18 | 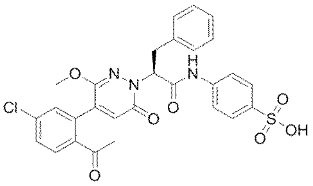 | 41 | 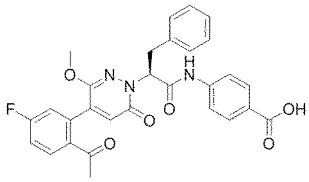 |
| 19 | 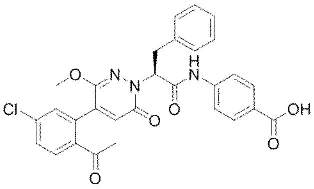 | 43 | 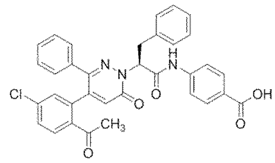 |
| 20 | 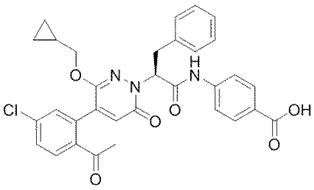 | 44 | 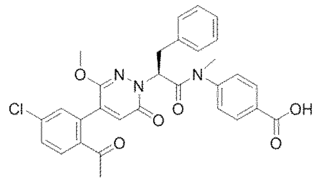 |
| 21 | 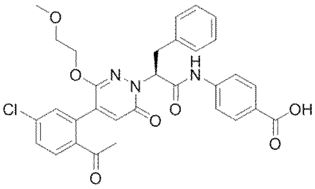 | 45 | 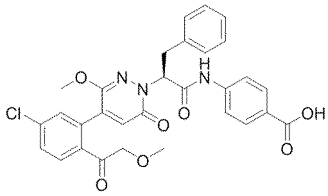 |

(continued)

| | structural formula | | structural formula |
|---|---|---|---|
| 22 | 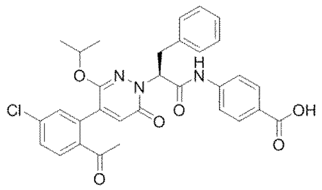 | 46 | 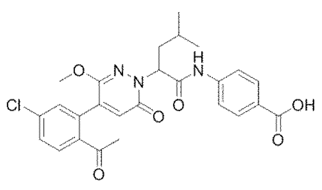 |
| 23 | 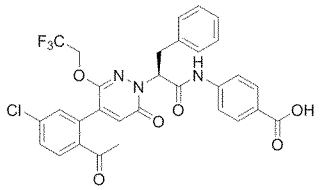 | 47 | 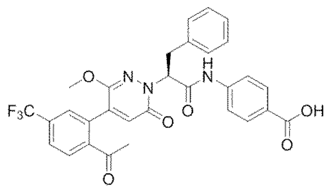 |

**9.** The compound, or the stereoisomer, the tautomer, the pharmaceutically acceptable salt thereof according to claim 1, wherein the pharmaceutically acceptable salt is prepared by the compound and a pharmaceutically acceptable acid or base.

**10.** The compound, or the stereoisomer, the tautomer, the pharmaceutically acceptable salt thereof according to any one of claims 1-9, wherein more than one hydrogen atoms of the compound are substituted with the isotope deuterium.

**11.** A pharmaceutical composition comprising the compound, or the stereoisomer, the tautomer, the pharmaceutically acceptable salt thereof according to any one of claims 1-10, and one or more pharmaceutically acceptable carriers.

**12.** Use of the compound, or the stereoisomer, the tautomer, the pharmaceutically acceptable salt thereof according to any one of claims 1-10 in manufacture of a medicament for treating FXIa-related diseases, preferably, thrombosis-related diseases.

**INTERNATIONAL SEARCH REPORT**

International application No.
**PCT/CN2020/117257**

**A. CLASSIFICATION OF SUBJECT MATTER**

C07D 401/14(2006.01)i; C07D 401/12(2006.01)i; C07D 401/10(2006.01)i; A61K 31/501(2006.01)i; A61K 31/495(2006.01)i; A61K 31/395(2006.01)i; A61P 7/02(2006.01)i; A61P 9/10(2006.01)i

According to International Patent Classification (IPC) or to both national classification and IPC

**B. FIELDS SEARCHED**

Minimum documentation searched (classification system followed by classification symbols)

C07D401/-; A61K31/-; A61P7/-; A61P9/-

Documentation searched other than minimum documentation to the extent that such documents are included in the fields searched

Electronic data base consulted during the international search (name of data base and, where practicable, search terms used)

CNABS; CNTXT; VEN; WOTXT; USTXT; EPTXT; CNKI; 万方; STN: 信立泰, 吴俊军, 陆银锁, 肖瑛, 洪泽新, 吴建立, 邢伟, 因子, XIa, FXIa, 抑制剂, 哒嗪, 酰胺, 甘氨酸, 血栓, 凝血, 心肌梗塞, 脑梗塞, 动脉粥样硬化, factor, inhibitor?, pyridazin+, amid+, glycine, thrombus, thrombo+, coagulant?, myocardial, cerebral, infarction, atherosclero+, structure of formula (I)

**C. DOCUMENTS CONSIDERED TO BE RELEVANT**

| Category* | Citation of document, with indication, where appropriate, of the relevant passages | Relevant to claim No. |
|---|---|---|
| X | WO 2018041122 A1 (JIANGSU HENGRUI MEDICINE CO., LTD. et al.) 08 March 2018 (2018-03-08)<br>claims 1-24 | 1-12 |
| X | CN 108137549 A (SQUIBB BRISTOL MYERS CO.) 08 June 2018 (2018-06-08)<br>claims 1-12 | 1-12 |
| A | CN 107793396 A (JIANGSU HENGRUI MEDICINE CO., LTD. et al.) 13 March 2018 (2018-03-13)<br>entire document | 1-12 |
| A | US 2017291892 A1 (BAYER PHARMA AG) 12 October 2017 (2017-10-12)<br>entire document | 1-12 |
| A | GB 2497806 A (ONO PHARMACEUTICAL CO.) 26 June 2013 (2013-06-26)<br>entire document | 1-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 November 2020** | **15 December 2020** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/117257**

| Patent document cited in search report | Publication date (day/month/year) | Patent family member(s) | Publication date (day/month/year) |
|---|---|---|---|
| WO 2018041122 A1 | 08 March 2018 | US 2019185464 A1 | 20 June 2019 |
| | | EP 3486242 A4 | 04 December 2019 |
| | | JP 2019528299 A | 10 October 2019 |
| | | KR 20190040037 A | 16 April 2019 |
| | | AU 2017317988 A1 | 21 February 2019 |
| | | CN 108495850 A | 04 September 2018 |
| | | US 10633375 B2 | 28 April 2020 |
| | | BR 112019003557 A2 | 21 May 2019 |
| | | EP 3486242 A1 | 22 May 2019 |
| | | US 2020199115 A1 | 25 June 2020 |
| | | CA 3031592 A1 | 08 March 2018 |
| | | IN 201917003243 A | 24 May 2019 |
| | | VN 64991 A | 26 August 2019 |
| | | HK 1255566 A0 | 23 August 2019 |
| CN 108137549 A | 08 June 2018 | ES 2754599 T3 | 20 April 2020 |
| | | WO 2017023992 A1 | 09 February 2017 |
| | | US 10336730 B2 | 02 July 2019 |
| | | EP 3331872 A1 | 13 June 2018 |
| | | US 2018222889 A1 | 09 August 2018 |
| | | KR 20180031037 A | 27 March 2018 |
| | | EP 3331872 B1 | 25 September 2019 |
| | | JP 2018522046 A | 09 August 2018 |
| | | US 2019382378 A1 | 19 December 2019 |
| CN 107793396 A | 13 March 2018 | None | |
| US 2017291892 A1 | 12 October 2017 | US 10167280 B2 | 01 January 2019 |
| | | ES 2722425 T3 | 12 August 2019 |
| | | EP 3197872 A1 | 02 August 2017 |
| | | WO 2016046166 A1 | 31 March 2016 |
| | | EP 3197872 B1 | 30 January 2019 |
| GB 2497806 A | 26 June 2013 | ZA 201404561 A | 30 December 2015 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9630396 A
- WO 9941276 A
- WO 2013093484 A
- WO 2004002405 A
- WO 2013056060 A
- WO 2017005725 A
- WO 2017023992 A
- WO 2018041122 A
- CN 201680058331


### Non-patent literature cited in the description


- *N Engl J Med,* 1991, vol. 325, 153-8
- *Blood,* 2003, vol. 101, 4783-4788
- *Blood,* 2010, vol. 116 (19), 3981-3989
- *Blood,* 2009, vol. 114, 2878-2883
- *Thromb Haemost,* 2011, vol. 105, 269-273
- Remington: The Science and Practice of Pharmacy. Lippincott, Williams & Wilkins, 2005
- *Angewandte Chemie, International Edition,* 2010, vol. 49 (46), 8729-8732